(19) Europäisches Patentamt / European Patent Office / Office européen des brevets

(11) **EP 4 775 571 A1**

(12) **EUROPEAN PATENT APPLICATION**
published in accordance with Art. 153(4) EPC

(43) Date of publication:
**15.07.2026 Bulletin 2026/29**

(21) Application number: **24862105.4**

(22) Date of filing: **06.09.2024**

(51) International Patent Classification (IPC):
***C07D 401/14*** (2006.01) ***C07D 401/04*** (2006.01)
***C07D 413/14*** (2006.01) ***C07D 405/14*** (2006.01)
***A61K 31/4192*** (2006.01) ***A61K 31/4439*** (2006.01)
***A61P 11/06*** (2006.01) ***A61P 35/00*** (2006.01)

(52) Cooperative Patent Classification (CPC):
**A61K 31/4192; A61K 31/4439; A61P 11/06; A61P 35/00; C07D 401/04; C07D 401/14; C07D 405/14; C07D 413/14**

(86) International application number:
**PCT/CN2024/117612**

(87) International publication number:
**WO 2025/051267 (13.03.2025 Gazette 2025/11)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority:
**08.09.2023 CN 202311157448**
**17.11.2023 CN 202311536485**
**13.12.2023 CN 202311711346**
**10.01.2024 CN 202410036503**
**05.02.2024 CN 202410163449**
**12.03.2024 CN 202410279138**
**08.04.2024 CN 202410411109**
**11.06.2024 CN 202410746126**
**26.07.2024 CN 202411008547**

(71) Applicant: **Haisco Pharmaceutical Group Co., Ltd.**
**Shannan, Tibet 856000 (CN)**

(72) Inventors:
- **LI, Yao**
  **Chengdu, Sichuan 611130 (CN)**
- **CHEN, Lei**
  **Chengdu, Sichuan 611130 (CN)**
- **REN, Lei**
  **Chengdu, Sichuan 611130 (CN)**
- **ZHANG, Haoliang**
  **Chengdu, Sichuan 611130 (CN)**
- **ZHANG, Guobiao**
  **Chengdu, Sichuan 611130 (CN)**
- **SONG, Changwei**
  **Chengdu, Sichuan 611130 (CN)**
- **WANG, Jiancheng**
  **Chengdu, Sichuan 611130 (CN)**
- **WANG, Yaoling**
  **Chengdu, Sichuan 611130 (CN)**
- **TANG, Pingming**
  **Chengdu, Sichuan 611130 (CN)**
- **ZHANG, Chen**
  **Chengdu, Sichuan 611130 (CN)**
- **YAN, Pangke**
  **Chengdu, Sichuan 611130 (CN)**

(74) Representative: **AWA Denmark A/S**
**Strandgade 56**
**1401 Copenhagen K (DK)**

(54) **LPAR1 ANTAGONIST AND USE THEREOF**

(57) An LPAR1 antagonist and the use thereof. The present invention relates to a compound as shown in formula (I), or a stereoisomer, a deuterated substance, or a pharmaceutically acceptable salt thereof, and a pharmaceutical composition thereof, and the use thereof in the preparation of a drug for treating/preventing LPAR1-mediated diseases, wherein each group in formula (I) is as defined in the description.

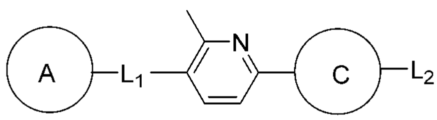

(I)

EP 4 775 571 A1

## Description

Technical Field

The present invention belongs to the field of pharmaceuticals, and particularly relates to a small molecule compound having LPAR1 antagonistic activity, or a stereoisomer, a deuterated substance, or a pharmaceutically acceptable salt thereof, and the use thereof in the preparation of a drug for treating related diseases.

Background Art

Lysophosphatidic acid (LPA) is a small molecule glycerophosphate with a molecular weight ranging from 430 to 480 D. LPA is widely present in the human body. After binding to its receptors, it can activate multiple cellular signalling pathways, participating in the regulation of biological processes such as cell proliferation, differentiation, apoptosis, and neurotransmitter release. It plays an important role in diseases such as cancer, fibrosis, neuronal dysfunction, and bone metabolism disorders. LPA is primarily generated through the hydrolysis of lysophospholipids (mainly lysophosphatidylcholine) by autotaxin. In a bleomycin-induced pulmonary fibrosis model, the level of LPA in bronchoalveolar lavage fluid is significantly increased, leading to increased vascular permeability and the development of pulmonary fibrosis. LPA can also mediate, through fibroblasts, the production of various paracrine mediators that act on epithelial cells, leukocytes, and endothelial cells, regulating tissue remodelling, angiogenesis, inflammation, wound healing, and tumour progression. LPA can even induce the ectodomain shedding of epidermal growth factor (EGF) family ligands from fibroblasts, activate the release of soluble factors, and-partly through EGFR action-stimulate lung epithelial cells, thereby amplifying the local fibroblast response. Recent studies have also revealed that the LPA-LPA1 signalling pathway promotes apoptosis of lung epithelial cells while inhibiting fibroblast apoptosis in idiopathic pulmonary fibrosis (IPF), suggesting its potential role in regulating the progression of fibrosis following lung injury. Research indicates that LPA is closely associated with organ fibrosis, primarily mediated through the lysophosphatidic acid receptor (LPAR) 1. To date, six LPAR subtypes (LPAR1-LPAR6) have been identified, among which LPAR1 has become a major focus of research in recent years. Clinical studies have confirmed that LPAR antagonists exert therapeutic effects against idiopathic pulmonary fibrosis. It was also found that the LPAR1 antagonist BMS-986020 can effectively improve lung function in patients with idiopathic pulmonary fibrosis.

Summary of the Invention

The present invention provides a small molecule compound having LPAR1 antagonistic activity, or a stereoisomer, a deuterated substance, or a pharmaceutically acceptable salt thereof, which has excellent effects such as good activity, superior physicochemical properties, favourable formulability, excellent pharmacokinetic properties, high bioavailability and low toxic and side effects, wherein the compound has a structure of formula (I), (I-1), (I-2), (I-2a), (I-2b), (I-2c), (I-3), (I-4), (I-5) or (I-6),

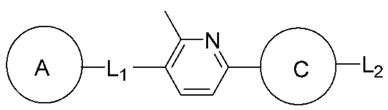

(I)

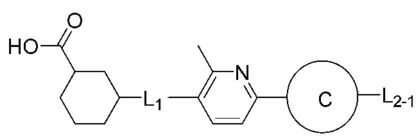

(I-1)

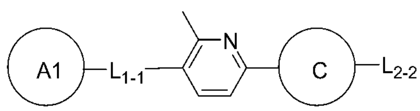

(I-2)

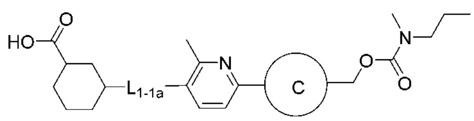

(I-2a)

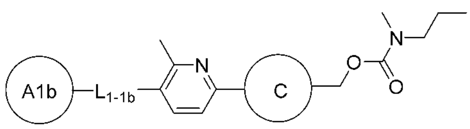

(I-2b)

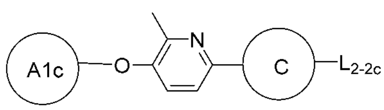

(I-2c)

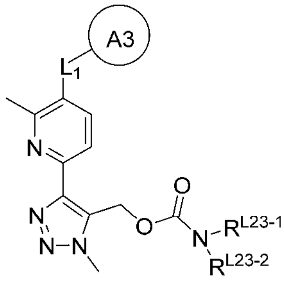
(I-3)

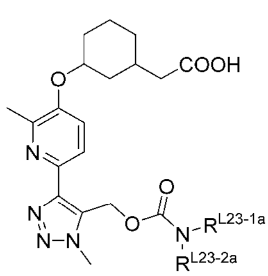
(I-4)

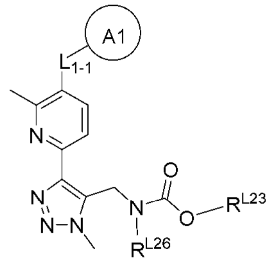
(I-5)

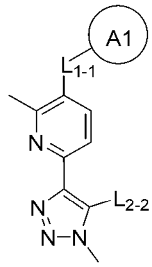
(I-6)

ring C is

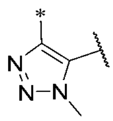,

wherein the "*" end is connected to a pyridine ring on the left side, and the "



" end is connected to $L_2$; in some embodiments, ring C is selected from

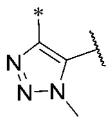 and 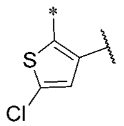,

wherein the "*" end is connected to a pyridine ring on the left side, and the "



" end is connected to $L_2$;

ring A is selected from 3- to 12-membered carbocyclyl and 4- to 12-membered heterocyclyl, or ring A is absent, wherein the heterocyclyl contains 1-3 heteroatoms selected from N, O and S; the carbocyclyl and heterocyclyl are optionally further substituted with 1-4 $R^A$;

when ring A is absent, $L_1$ is selected from $C_{1-6}$ alkyl substituted with one COOH, $C_{2-6}$ alkenyl, and $C_{2-6}$ alkynyl;

when ring A is absent, $L_1$ is selected from $-OC_{1-6}$ alkyl substituted with one COOH, $C_{2-6}$ alkenyl, and $C_{2-6}$ alkynyl;

when rings A1 and A1b are each independently absent, $L_{1-1}$ and $L_{1-1b}$ are each independently selected from $-OC_{1-6}$ alkyl substituted with one COOH, $C_{2-6}$ alkenyl, and $C_{2-6}$ alkynyl; in some embodiments, when ring A1 is absent, $L_{1-1}$ is $-OC_{1-6}$ alkyl substituted with one COOH; in some embodiments, when each ring A, A1 or A1b is independently absent, $L_1$, $L_{1-1}$, and $L_{1-1b}$ are each independently selected from $-OCH_2C(CH_3)_2CH_2COOH$ and $-OCH_2CH_2C(CH_3)_2COOH$;

in some embodiments, ring A is selected from 4- to 12-membered carbocyclyl and 4- to 12-membered heterocyclyl, wherein the heterocyclyl contains 1-3 heteroatoms selected from N, O and S; the carbocyclyl and heterocyclyl are optionally further substituted with 1-4 $R^A$;

in some embodiments, ring A is selected from 4- to 8-membered monocyclic carbocyclyl, 6- to 12-membered bicyclic carbocyclyl, 4- to 8-membered monocyclic heterocyclyl, 6- to 12-membered bicyclic heterocyclyl, and 6- to 10-membered bridged heterocyclyl, wherein the carbocyclyl, heterocyclyl, and bridged heterocyclyl are optionally substituted with 1-4 $R^A$;

in some embodiments, ring A is selected from 4- to 8-membered monocyclic cycloalkyl, 6- to 12-membered fused cycloalkyl, 4- to 8-membered monocyclic heterocycloalkyl, 6- to 12-membered fused heterocycloalkyl, 6- to 10-membered spiroheterocyclyl, and 6- to 10-membered bridged heterocyclyl, wherein the heterocycloalkyl, fused heterocycloalkyl, spiroheterocyclyl, and bridged heterocyclyl contain 1-3 heteroatoms selected from N, O and S; the cycloalkyl, heterocycloalkyl, fused heterocycloalkyl, spiroheterocyclyl, and bridged heterocyclyl are optionally further substituted with 1-4 $R^A$;

in some embodiments, ring A is selected from one of the groups formed by the following structures optionally substituted with 1-4 $R^A$:

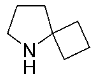, 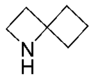, 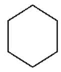,

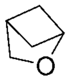, 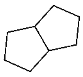, 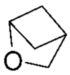, 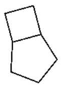, 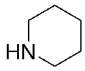, 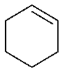, 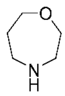, 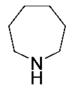, or 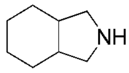, 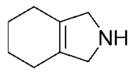,

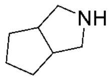, 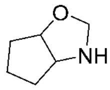, 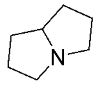, 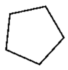, 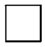, 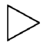, 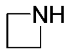, 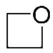, 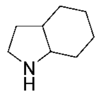, 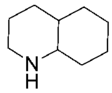,

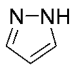, 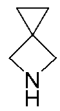, 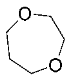, 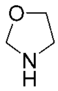, 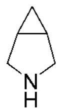, 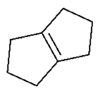, or 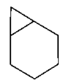,

or ring A is absent;

rings A1, A1b, and A1c are each independently selected from 3- to 12-membered carbocyclyl and 4- to 12-membered heterocyclyl, or are absent, wherein the heterocyclyl contains 1-3 heteroatoms selected from N, O and S; the carbocyclyl and heterocyclyl are optionally further substituted with 1-4 $R^A$;

in some embodiments, rings A1, A1b, and A1c are each independently selected from 3- to 8-membered monocyclic cycloalkyl, 6- to 12-membered fused cycloalkyl, 4- to 8-membered monocyclic heterocycloalkyl, 6- to 12-membered fused heterocycloalkyl, 6- to 10-membered spiroheterocyclyl, and 6- to 10-membered bridged heterocyclyl, or are absent, wherein the heterocycloalkyl, fused heterocycloalkyl, spiroheterocyclyl, and bridged heterocyclyl contain 1-3 heteroatoms selected from N, O and S; the cycloalkyl, heterocycloalkyl, fused heterocycloalkyl, spiroheterocyclyl, and bridged heterocyclyl are optionally further substituted with 1-4 $R^A$;

ring A1 is selected from 4- to 12-membered carbocyclyl and 4- to 12-membered heterocyclyl, wherein the heterocyclyl contains 1-3 heteroatoms selected from N, O and S; the carbocyclyl and heterocyclyl are optionally further substituted with 1-4 $R^A$; and $L_{1-1}$-A1 is not

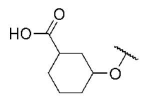;

in some embodiments, ring A1 is selected from 4- to 8-membered monocyclic cycloalkyl, 6- to 12-membered fused cycloalkyl, 4- to 8-membered monocyclic heterocycloalkyl, 6- to 12-membered fused heterocycloalkyl, 6- to 10-membered spiroheterocyclyl, and 6- to 10-membered bridged heterocyclyl, wherein the heterocycloalkyl, fused heterocycloalkyl, spiroheterocyclyl, and bridged heterocyclyl contain 1-3 heteroatoms selected from N, O and S; the cycloalkyl, heterocycloalkyl, fused heterocycloalkyl, spiroheterocyclyl, and bridged heterocyclyl are optionally further substituted with 1-4 $R^A$; and $L_{1-1}$-A1 is not

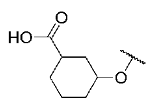;

in some embodiments, ring A1 is selected from one of the groups formed by the following structures optionally substituted with 1-4 $R^A$:

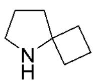, 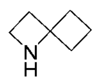, 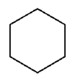,

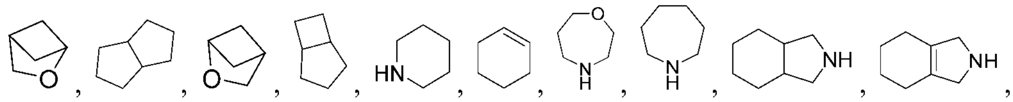

, , , , , , , , , ,

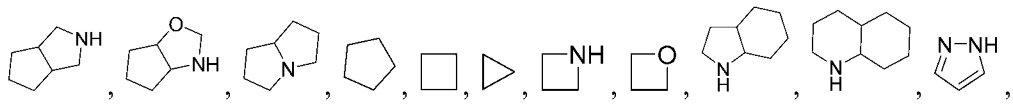

, , , , , , , , , , , , , ,

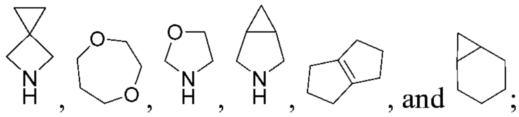

, , , , , and ;

and $L_{1-1}$-A1 is not

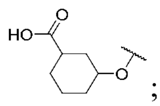

;

in some embodiments, ring A1 is selected from one of the groups formed by the following structures optionally substituted with 1-4 $R^A$:

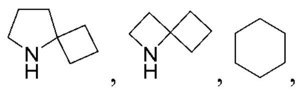

, , ,

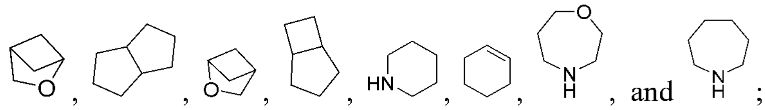

, , , , , , , and ;

and $L_{1-1}$-A1 is not

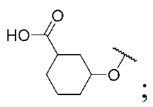

;

ring A1b is selected from 3- to 12-membered carbocyclyl and 4- to 12-membered heterocyclyl, or is absent, wherein the heterocyclyl contains 1-3 heteroatoms selected from N, O and S; the carbocyclyl and heterocyclyl are optionally further substituted with 1-4 $R^A$; and $L_{1-16}$-A1b is not

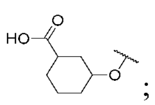

;

in some embodiments, ring A1b is selected from 4- to 12-membered carbocyclyl and 4- to 12-membered heterocyclyl, wherein the heterocyclyl contains 1-3 heteroatoms selected from N, O and S; the carbocyclyl and heterocyclyl are optionally further substituted with 1-4 $R^A$; and $L_{1-16}$-A1b is not

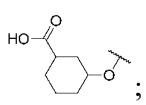

;

in some embodiments, ring A1b is selected from 4- to 8-membered monocyclic cycloalkyl, 6- to 12-membered fused cycloalkyl, 4- to 8-membered monocyclic heterocycloalkyl, 6- to 12-membered fused heterocycloalkyl, 6- to 10-membered spiroheterocyclyl, and 6- to 10-membered bridged heterocyclyl, wherein the heterocycloalkyl, fused

heterocycloalkyl, spiroheterocyclyl, and bridged heterocyclyl contain 1-3 heteroatoms selected from N, O and S; the cycloalkyl, heterocycloalkyl, fused heterocycloalkyl, spiroheterocyclyl, and bridged heterocyclyl are optionally further substituted with 1-4 $R^A$; and $L_{1-16}$-A1b is not

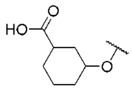;

in some embodiments, ring A1b is selected from one of the groups formed by the following structures optionally substituted with 1-4 $R^A$:

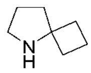, 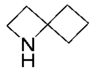, 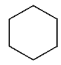,

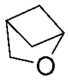, 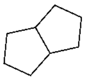, 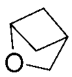, 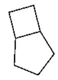, 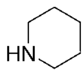, 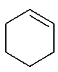, 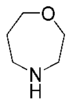, 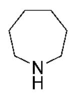 or 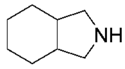, 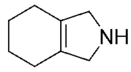,

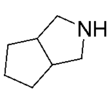, 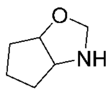, 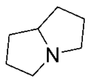, 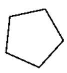, 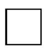, 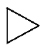, 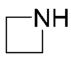, 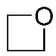, 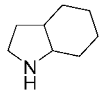, 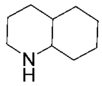, 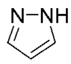,

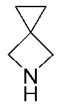, 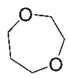, 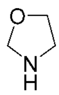, 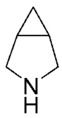, 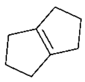, or 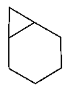,

or ring A1b is absent; and $L_{1-16}$-A1b is not

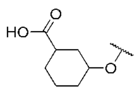;

in some embodiments, ring A1c is selected from 3- to 12-membered carbocyclyl and 4- to 12-membered heterocyclyl, or is absent, wherein the heterocyclyl contains 1-3 heteroatoms selected from N, O and S; the carbocyclyl and heterocyclyl are optionally further substituted with 1-4 $R^A$; and O-Alc is not

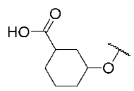;

in some embodiments, ring A1c is selected from 4- to 12-membered carbocyclyl and 4- to 12-membered heterocyclyl, wherein the heterocyclyl contains 1-3 heteroatoms selected from N, O and S; the carbocyclyl and heterocyclyl are optionally further substituted with 1-4 $R^A$; and O-Alc is not

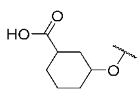;

in some embodiments, ring A1c is selected from 4- to 8-membered monocyclic cycloalkyl, 6- to 12-membered fused cycloalkyl, 4- to 8-membered monocyclic heterocycloalkyl, 6- to 12-membered fused heterocycloalkyl, 6- to 10-membered spiroheterocyclyl, and 6- to 10-membered bridged heterocyclyl, wherein the heterocycloalkyl, fused heterocycloalkyl, spiroheterocyclyl, and bridged heterocyclyl contain 1-3 heteroatoms selected from N, O and S; the cycloalkyl, heterocycloalkyl, fused heterocycloalkyl, spiroheterocyclyl, and bridged heterocyclyl are optionally further substituted with 1-4 $R^A$; and O-Alc is not

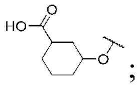;

in some embodiments, ring A1c is selected from one of the groups formed by the following structures optionally substituted with 1-4 $R^A$:

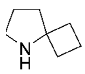, 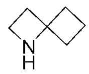, 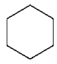,

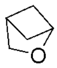, 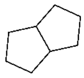, 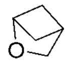, 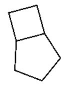, 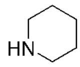, 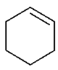, 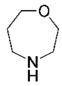, 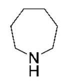, 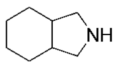, 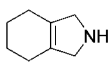, 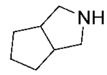,

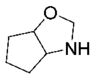, 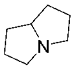, 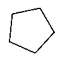, 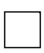, 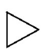, 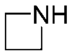, 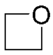, 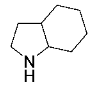, 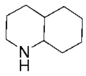, 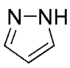, 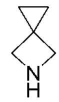, 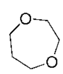,

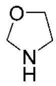, 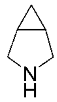, 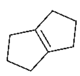, and 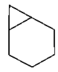,

or ring A1c is absent; and O-Alc is not

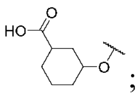;

each $R^A$ is independently selected from H, halogen, =O, CN, OH, $C_{1-6}$ alkyl, $C_{2-6}$ alkenyl, $C_{2-6}$ alkynyl, $-OC_{1-4}$ alkyl, $-(CH_2)_p$-(5- to 10-membered heterocyclyl), -(a 3- to 6-membered carbocyclic ring)-$COOR^{a1}$, $-(CH_2)_p-COOR^{a1}$, $-(CH_2)_p-C(O)NR^{a1}R^{a2}$, $-(CH_2)_p-C(O)NHC(O)R^{a1}$, $-(CH_2)_p-C(O)NHS(O)_2R^{a1}$, $-(CH_2)_p-C(O)NHS(O)R^{a1}$, $-(CH_2)_p-S(O)_2OH$, $-(CH_2)_p-S(O)_2NHC(O)R^{a1}$, or $-(CH_2)_p-P(O)(OH)_2$, or is selected from $=CH_2$, $=CF_2$, $=CH-CH_3$, and $=C-(CH_3)_2$, wherein the heterocyclyl contains 1-3 heteroatoms selected from N, O and S; the alkyl, alkenyl, alkynyl and heterocyclyl are optionally further substituted with 1-4 groups selected from halogen, OH, $NH_2$, CN, and -O-halogenated $C_{1-4}$ alkyl;

in some embodiments, each $R^A$ is independently selected from H, halogen, CN, OH, $C_{1-6}$ alkyl, $C_{2-6}$ alkenyl, $C_{2-6}$ alkynyl, $-OC_{1-4}$ alkyl, $-(CH_2)_p$-(5- to 10-membered heterocyclyl), $-(CH_2)_p-COOR^{a1}$, $-(CH_2)_p-C(O)NR^{a1}R^{a2}$, $-(CH_2)_p-C(O)NHC(O)R^{a1}$, $-(CH_2)_p-C(O)NHS(O)_2R^{a1}$, $-(CH_2)_p-C(O)NHS(O)R^{a1}$, $-(CH_2)_p-S(O)_2OH$, $-(CH_2)_p-S(O)_2NHC(O)R^{a1}$, or $-(CH_2)_p-P(O)(OH)_2$, or is selected from $=CH_2$ and $=CF_2$, wherein the heterocyclyl contains 1-3 heteroatoms selected from N, O and S; the alkyl, alkenyl, alkynyl and heterocyclyl are optionally further substituted with 1-4 groups selected from halogen, OH, $NH_2$, CN, and -O-halogenated $C_{1-4}$ alkyl;

in some embodiments, each $R^A$ is independently selected from H, halogen, =O, CN, OH, $C_{1-4}$ alkyl, $C_{2-4}$ alkenyl, $C_{2-4}$ alkynyl, $-OC_{1-2}$ alkyl, $-(CH_2)_p$-(5- to 8-membered heterocyclyl), -(a 3- to 6-membered carbocyclic ring)-$COOR^{a1}$, and $-(CH_2)_p-COOR^{a1}$, or is selected from $=CH_2$ and $=CF_2$, wherein the heterocyclyl contains 1-3 heteroatoms selected from N, O and S; the alkyl, alkenyl, alkynyl, and heterocyclyl are optionally further substituted with 1-4 groups selected from halogen, OH, $NH_2$, CN, and -O-halogenated $C_{1-2}$ alkyl;

in some embodiments, each $R^A$ is independently selected from H, halogen, CN, OH, $C_{1-4}$ alkyl, $C_{2-4}$ alkenyl, $C_{2-4}$ alkynyl, $-OC_{1-2}$ alkyl, $-(CH_2)_p$-(5- to 8-membered heterocyclyl), and $-(CH_2)_p-COOR^{a1}$, or is selected from $=CH_2$ and $=CF_2$, wherein the heterocyclyl contains 1-3 heteroatoms selected from N, O and S; the alkyl, alkenyl, alkynyl, and heterocyclyl are optionally further substituted with 1-4 groups selected from halogen, OH, $NH_2$, CN, and -O-halogenated $C_{1-2}$ alkyl;

in some embodiments, each $R^A$ is independently selected from H, halogen, CN, OH, -COOH, $-CH_2-COOH$, and $-CH_2CH_2-COOH$, or is $=CH_2$;

in some embodiments, each $R^A$ is independently selected from H, F, Cl, CN, OH, -COOH, $-CH_2-COOH$, and $-CH_2CH_2-COOH$, or is $=CH_2$;

in some embodiments, rings A1, A1b, and A1c are each independently selected from the following structures:

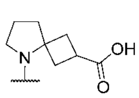, 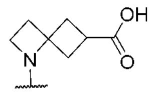, 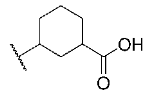,

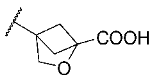, 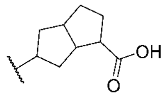, 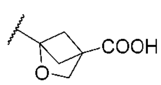, 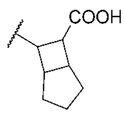, 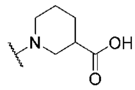, 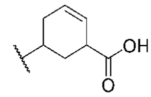,

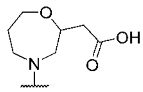, 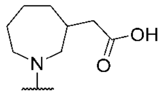, or 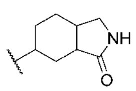, 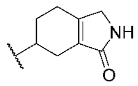, 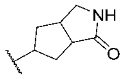, 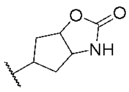,

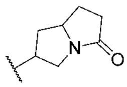, 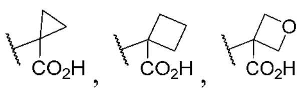, , , 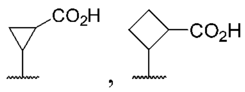, , 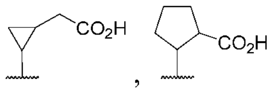, ,

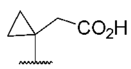, 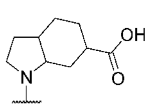, 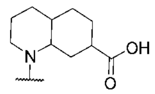, 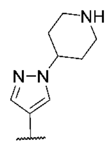, 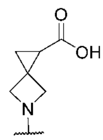, 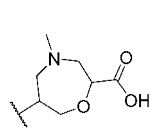,

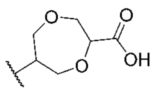, 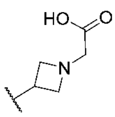, 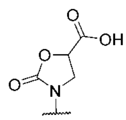, 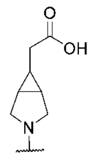, 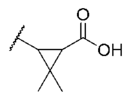, 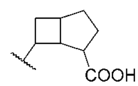, 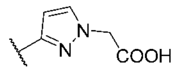,

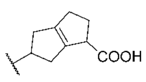, 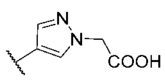, 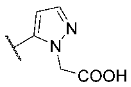, 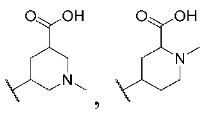, , 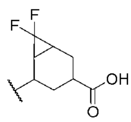, 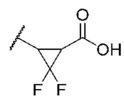,

and

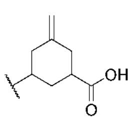;

or are selected from

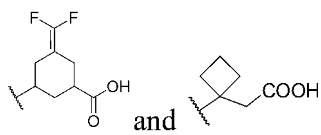 and ;

ring A3 is selected from

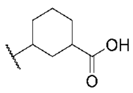, 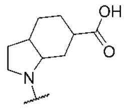, and 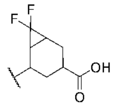;

$L_1$ is selected from a bond, $C_{1-6}$ alkyl, $C_{2-6}$ alkenyl, $C_{2-6}$ alkynyl, -O-, -O-$C_{1-6}$ alkyl-, -S-, -S-$C_{1-6}$ alkyl-, -C(O)$NR^{L1}$-, -$NR^{L1}$C(O)-,

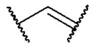,

-$NR^{L1}$-, and -$NR^{L1}$-$C_{1-6}$ alkyl-, wherein the alkyl, alkenyl, and alkynyl are optionally further substituted with 1-4 $R^{L1}$;
in some embodiments, $L_1$ is selected from a bond, $C_{1-6}$ alkyl, $C_{2-6}$ alkenyl, $C_{2-6}$ alkynyl, -O-, -O-$C_{1-6}$ alkyl-, -S-, -S-$C_{1-6}$ alkyl-, -C(O)$NR^{L1}$-, and -$NR^{L1}$C(O)-, wherein the alkyl, alkenyl, and alkynyl are optionally further substituted with 1-4 $R^{L1}$;
in some embodiments, $L_1$ is selected from a bond, $C_{1-4}$ alkyl, $C_{2-4}$ alkenyl, $C_{2-4}$ alkynyl, -O-, -O-$C_{1-4}$ alkyl-, -S-, -S-$C_{1-4}$ alkyl-, -C(O)$NR^{L1}$-, and -$NR^{L1}$C(O)-, wherein the alkyl, alkenyl, and alkynyl are optionally further substituted with 1-4 $R^{L1}$;
in some embodiments, $L_1$ is selected from a bond, $C_{1-2}$ alkyl, -O-$C_{1-2}$ alkyl-, - S-, and -O-, wherein the alkyl is optionally further substituted with 1-4 $R^{L1}$;
in some embodiments, $L_1$ is selected from a bond, -$OCH_2$-, -S-, -$OCH(CH_3)$-, -$OCH(CF_3)$-,

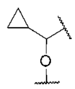,

-O-, -C(=O)-, -$CH_2$-, -NH-, -$N(CH_3)$-, and -$NHCH(CH_3)$-;
in some embodiments, $L_1$ is selected from a bond, -$OCH_2$-, -S-, -$OCH(CH_3)$-, -$OCH(CF_3)$-,

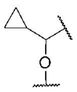,

-O-, -C(=O)-, -NHC(O)-, -$CH_2$-, -NH-, -$N(CH_3)$-, and - $NHCH(CH_3)$-;
in some embodiments, $L_1$ is selected from a bond, -$OCH_2$-, -S-, -$OCH(CH_3)$-, -$OCH(CF_3)$-,

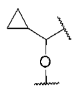,

-O-, -C(=O)-, and -$CH_2$-;
in some embodiments, $L_1$ is selected from -O- and -$N(CH_3)$-;
$L_{1-1}$ is selected from a bond, $C_{1-6}$ alkyl, $C_{2-6}$ alkenyl, $C_{2-6}$ alkynyl, -O-, -O-$C_{1-6}$ alkyl-, -S-, -S-$C_{1-6}$ alkyl-, -C(O)$NR^{L1}$-,

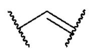,

-$NR^{L1}$-, and -$NR^{L1}$-$C_{1-6}$ alkyl-, wherein the alkyl, alkenyl, and alkynyl are optionally further substituted with 1-4 $R^{L1}$;
in some embodiments, $L_{1-1}$ is selected from a bond, $C_{1-6}$ alkyl, $C_{2-6}$ alkenyl, $C_{2-6}$ alkynyl, -O-, -O-$C_{1-6}$ alkyl-, -S-, -S-$C_{1-6}$ alkyl-, and -C(O)$NR^{L1}$-, wherein the alkyl, alkenyl, and alkynyl are optionally further substituted with 1-4 $R^{L1}$; in some embodiments, $L_{1-1}$ is selected from a bond, $C_{1-4}$ alkyl, $C_{2-4}$ alkenyl, $C_{2-4}$ alkynyl, - O-, -O-$C_{1-4}$ alkyl-, -S-, -S-$C_{1-4}$ alkyl-, and -C(O)$NR^{L1}$-, wherein the alkyl, alkenyl, and alkynyl are optionally further substituted with 1-4 $R^{L1}$; in some embodiments, $L_{1-1}$ is selected from a bond, $C_{1-2}$ alkyl, -O-$C_{1-2}$ alkyl-, -S-, -O-,

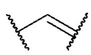,

vinyl, propenyl, ethynyl, and -C(O)NH-, wherein the alkyl is optionally further substituted with 1-4 $R^{L1}$; in some

embodiments, $L_{1-1}$ is selected from a bond, $C_{1-2}$ alkyl, -O-$C_{1-2}$ alkyl-, -S-, and -O-, wherein the alkyl is optionally further substituted with 1-4 $R^{L1}$; in some embodiments, $L_{1-1}$ is selected from a bond, - $OCH_2$-, -$OCH_2CH_2$-, -S-, -$OCH(CH_3)$-, -$OCH(CF_3)$-,

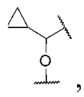
,

-O-, -C(=O)-, -$CH_2$-, - CH=CH-$CH_2$-, ethynyl, and -NHCO-; in some embodiments, $L_{1-1}$ is selected from a bond, -$OCH_2$-, -S-, -$OCH(CH_3)$-, -$OCH(CF_3)$-,

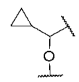
,

-O-, -C(=O)-, and -$CH_2$-;

$L_{1-1a}$ is selected from a bond, $C_{1-4}$ alkyl, $C_{2-4}$ alkenyl, $C_{2-4}$ alkynyl, -O-$C_{1-4}$ alkyl-, -S-, -S-$C_{1-4}$ alkyl-, -C(O)$NR^{L1}$-, -$NR^{L1}$C(O)-,

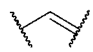
,

-$NR^{L1}$-, and -$NR^{L1}$-$C_{1-6}$ alkyl-, wherein the alkyl, alkenyl, and alkynyl are optionally further substituted with 1-4 $R^{L1}$; in some embodiments, $L_{1-1a}$ is selected from a bond, $C_{1-4}$ alkyl, $C_{2-4}$ alkenyl, $C_{2-4}$ alkynyl, -O-$C_{1-4}$ alkyl-, -S-, -S-$C_{1-4}$ alkyl-, -C(O)$NR^{L1}$-, and -$NR^{L1}$C(O)-, wherein the alkyl, alkenyl, and alkynyl are optionally further substituted with 1-4 $R^{L1}$; in some embodiments, each $L_{1-1a}$ is independently selected from a bond, -$OCH_2$-, -S-, -$OCH(CH_3)$-, -$OCH(CF_3)$-,

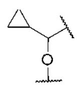
,

-C(=O)-, - $CH_2$-, -CH=CH-$CH_2$-, ethynyl, -NHCO-, -NH-, -$N(CH_3)$-, and -$NHCH(CH_3)$-; in some embodiments, $L_{1-1a}$ is selected from a bond, -$OCH_2$-, -S-, -$OCH(CH_3)$-, - $OCH(CF_3)$-,

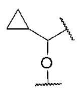
,

-C(=O)-, and -$CH_2$-;

$L_{1-1}$ is selected from a bond, $C_{1-6}$ alkyl, $C_{2-6}$ alkenyl, $C_{2-6}$ alkynyl, -O-, -O-$C_{1-6}$ alkyl-, -S-, -S-$C_{1-6}$ alkyl-, -C(O)$NR^{L1}$-, -$NR^{L1}$C(O)-,

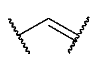
,

-$NR^{L1}$-, and -$NR^{L1}$-$C_{1-6}$ alkyl-, wherein the alkyl, alkenyl, and alkynyl are optionally further substituted with 1-4 $R^{L1}$; in some embodiments, $L_{1-1b}$ is selected from a bond, $C_{1-6}$ alkyl, $C_{2-6}$ alkenyl, $C_{2-6}$ alkynyl, -O-, -O-$C_{1-6}$ alkyl-, -S-, -S-$C_{1-6}$ alkyl-, -C(O)$NR^{L1}$-, and -$NR^{L1}$C(O)-, wherein the alkyl, alkenyl, and alkynyl are optionally further substituted with 1-4 $R^{L1}$; in some embodiments, $L_{1-1b}$ is selected from a bond, $C_{1-4}$ alkyl, $C_{2-4}$ alkenyl, $C_{2-4}$ alkynyl, -O-, -O-$C_{1-4}$ alkyl-, -S-, -S-$C_{1-4}$ alkyl-, -C(O)$NR^{L1}$-, and -$NR^{L1}$C(O)-, wherein the alkyl, alkenyl, and alkynyl are optionally further substituted with 1-4 $R^{L1}$; in some embodiments, $L_{1-1b}$ is selected from a bond, $C_{1-2}$ alkyl, -O-$C_{1-2}$ alkyl-, -S-, -O-,

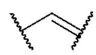
,

vinyl, propenyl, ethynyl, -C(O)NH-, and -NHC(O)-, wherein the alkyl is optionally further substituted with 1-4 $R^{L1}$; in some embodiments, $L_{1-1b}$ is selected from a bond, $C_{1-2}$ alkyl, -O-$C_{1-2}$ alkyl-, -S-, and -O-, wherein the alkyl is optionally further substituted with 1-4 $R^{L1}$; in some embodiments, $L_{1-1b}$ is selected from a bond, -$OCH_2$-, -$OCH_2CH_2$-, -S-,

$-OCH(CH_3)-$, $-OCH(CF_3)-$,

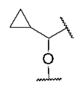,

-O-, - C(=O)-, $-CH_2-$, $-CH=CH-CH_2-$, ethynyl, and -NHCO-; in some embodiments, $L_{1-1b}$ is selected from a bond, $-OCH_2-$, -S-, $-OCH(CH_3)-$, $-OCH(CF_3)-$,

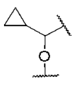,

-O-, - C(=O)-, and $-CH_2-$;

each $R^{L1}$ is independently selected from H, halogen, =O, OH, $C_{1-4}$ alkyl, halogenated $C_{1-4}$ alkyl, 3- to 6-membered cycloalkyl, and -COOH, wherein the alkyl and cycloalkyl are optionally further substituted with 1-4 groups selected from halogen, OH, and $NH_2$; in some embodiments, each $R^{L1}$ is independently selected from H, halogen, =O, OH, $C_{1-4}$ alkyl, halogenated $C_{1-4}$ alkyl, and 3- to 6-membered cycloalkyl, wherein the alkyl and cycloalkyl are optionally further substituted with 1-4 groups selected from halogen, OH, and $NH_2$; in some embodiments, each $R^{L1}$ is independently selected from H, halogen, =O, OH, $C_{1-2}$ alkyl, halogenated $C_{1-2}$ alkyl, and 3- to 6-membered cycloalkyl, wherein the alkyl and cycloalkyl are optionally further substituted with 1-4 groups selected from halogen, OH, and $NH_2$; in some embodiments, each $R^{L1}$ is independently selected from H, halogen, =O, OH, $C_{1-2}$ alkyl, and 3- to 6-membered cycloalkyl; in some embodiments, each $R^{L1}$ is independently selected from H, F, Cl, Br, =O, OH, methyl, ethyl, cyclopropyl, cyclobutyl, cyclopentyl, and cyclohexyl; in some embodiments, each $R^{L1}$ is independently selected from H, halogen, OH, $C_{1-4}$ alkyl, halogenated $C_{1-4}$ alkyl, 3- to 6-membered cycloalkyl, and -COOH, wherein the alkyl and cycloalkyl are optionally further substituted with 1-4 groups selected from halogen, OH, and $NH_2$; in some embodiments, each $R^{L1}$ is independently selected from H, halogen, OH, $C_{1-2}$ alkyl, halogenated $C_{1-2}$ alkyl, 3- to 6-membered cycloalkyl, and -COOH, wherein the alkyl and cycloalkyl are optionally further substituted with 1-4 groups selected from halogen, OH, and $NH_2$; in some embodiments, each $R^{L1}$ is independently selected from H, halogen, OH, $C_{1-2}$ alkyl, and 3- to 6-membered cycloalkyl; $L_2$ is selected from $-(CR^{L21}R^{L22})_p-OC(O)-N(R^{L23})_2$, $-(CR^{L21}R^{L22})pN(R^{L24})S(O)_2N(R^{L25})_2$,$-(CR^{L21}R^{L22})_pN(R^{L26})C(O)N(R^{L27})(R^{L28})$, $-(CR^{L21}R^{L22})_pN(R^{L29})(R^{L30})$,$-C(=O)N(R^{L31})_2$, $-N(R^{L31})-C(=O)OR^{L32}$, $-(CH_2)_pR^{L33}$, $-(CR^{L21}R^{L22})_p-C(O)-N(R^{L21})-C(O)R^{L21}$, $-(CR^{L21}R^{L22})_p-C(O)-N(R^{L29})-S(O)_2R^{L21}$, -(3- to 6-membered cycloalkyl)-$C(=O)N(R^{L25})_2$, -(4- to 7-membered heterocyclyl)-$C(=O)N(R^{L25})_2$, -($C_{2-4}$ alkenyl)-(4- to 7-membered heterocyclyl)-$N(R^{L25})_2$, $-(CR^{L21}R^{L22})_p-SC(O)-N(R^{L25})_2$, - $(CR^{L21}R^{L22})_pN(R^{L29})C(S)N(R^{L27})(R^{L28})$,

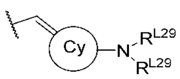,

$-(CR^{L21}R^{L22})_p-OC(O)-R^{L23}$, $-(CR^{L21}R^{L22})_p-O-R^{L23}$, and $-(CR^{L21}R^{L22})_b-N(R^{L26})C(O)O-R^{L23}$, wherein Cy is 4- to 7-membered heterocyclyl; the cycloalkyl and heterocyclyl are optionally further substituted with 1-4 groups selected from halogen, =O, OH, $NH_2$, CN, $C_{1-4}$ alkyl, $C_{1-4}$ alkoxy, halogenated $C_{1-4}$ alkyl, $C_{3-6}$ cycloalkyl, and 5- to 7-membered heterocyclyl;

in some embodiments, $L_2$ is selected from $-(CR^{L21}R^{L22})_p-OC(O)-N(R^{L23})_2$, - $(CR^{L21}R^{L22})_pN(R^{L24})S(O)_2N(R^{L25})_2$, $-(CR^{L21}R^{L22})_pN(R^{L26})C(O)N(R^{L27})(R^{L28})$,$-(CR^{L21}R^{L22})_pN(R^{L29})(R^{L30})$, $-CC=O)N(R^{L31})_2$, $-N(R^{L31})-C(=O)OR^{L32}$, $-(CH_2)pR^{L33}$,$-(CR^{L21}R^{L22})_b-C(O)-N(R^{L29})-C(O)R^{L21}$, $-(CR^{L21}R^{L22})_b-C(O)-N(R^{L29})-S(O)_2R^{L21}$, -(3-to 6-membered cycloalkyl)-$C(=O)N(R^{L25})_2$, -(4- to 7-membered heterocyclyl)-$C(=O)N(R^{L25})_2$, -($C_{2-4}$ alkenyl)-(4- to 7-membered heterocyclyl)-$N(R^{L25})_2$, - $(CR^{L21}R^{L22})_p-SC(O)-N(R^{L25})_2$, $-(CR^{L21}R^{L22})_pN(R^{L29})C(S)N(R^{L27})(R^{L28})$,

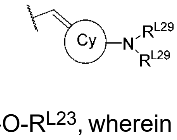,

$-(CR^{L21}R^{L22})p-OC(O)-R^{L23}$, and $-(CR^{L21}R^{L22})_b-O-R^{L23}$, wherein Cy is 4- to 7-membered heterocyclyl; the cycloalkyl and heterocyclyl are optionally further substituted with 1-4 groups selected from halogen, =O, OH, $NH_2$, CN, $C_{1-4}$ alkyl, $C_{1-4}$ alkoxy, halogenated $C_{1-4}$ alkyl, $C_{3-6}$ cycloalkyl, and 5- to 7-membered heterocyclyl;

in some embodiments, $L_2$ is selected from $-(CR^{L21}R^{L22})_p-OC(O)-N(R^{L23})_2$, - $(CR^{L21}R^{L22})_bN(R^{L24})S(O)_2N(R^{L25})_2$, $-(CR^{L21}R^{L22})_pN(R^{L26})C(O)N(R^{L27})(R^{L28})$,$-(CR^{L21}R^{L22})_pN(R^{L29})(R^{L30})$, $-C(=O)N(R^{L31})_2$, $-N(R^{L31})-C(=O)OR^{L32}$,

$-(CH_2)_pR^{L33}$,$-(CR^{L21}R^{L22})_b-C(O)-N(R^{L29})-C(O)R^{L21}$, $-(CR^{L21}R^{L22})_p-C(O)-N(R^{L29})-S(O)_2R^{L21}$,-to 6-membered cycloalkyl)-C(=O)N($R^{L25}$)$_2$, -(4- to 7-membered heterocyclyl)-C(=O)N($R^{L25}$)$_2$, -($C_{2-4}$ alkenyl)-(4- to 7-membered heterocyclyl)-N($R^{L25}$)$_2$, - $(CR^{L21}R^{L22})_p-SC(O)-N(R^{L25})_2$, $-(CR^{L21}R^{L22})_pN^{L29})C(S)N^{L27})(R^{L28})$,

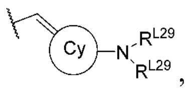

and $-(CR^{L21}R^{L22})_p-OC(O)-R^{L23}$, wherein Cy is 4- to 7-membered heterocyclyl; the cycloalkyl and heterocyclyl are optionally further substituted with 1-4 groups selected from halogen, =O, OH, $NH_2$, CN, $C_{1-4}$ alkyl, $C_{1-4}$ alkoxy, halogenated $C_{1-4}$ alkyl, $C_{3-6}$ cycloalkyl, and 5- to 7-membered heterocyclyl;

in some embodiments, $L_2$ is selected from $-(CR^{L21}R^{L22})_p-OC(O)-N(R^{L23})_2$, - $(CR^{L21}R^{L22})_pN(R^{L24})S(O)_2N(R^{L25})_2$, $-(CR^{L21}R^{L22})_pN(R^{L26})C(O)N(R^{L27})(R^{L28})$,$-(CR^{L21}R^{L22})_pN(R^{L29})(R^{L30})$, -CC=O)N($R^{L31}$)$_2$, -N($R^{L31}$)-C(=O)O$R^{L32}$, -($CH_2$)p$R^{L33}$,$-(CR^{L21}R^{L22})_p-C(O)-N(R^{L29})-C(O)R^{L21}$, $-(CR^{L21}R^{L22})_b-C(O)-N(R^{L29})-S(O)_2R^{L21}$, -(3-to 6-membered cycloalkyl)-C(=O)N($R^{L25}$)$_2$, -(4- to 7-membered heterocyclyl)-C(=O)N($R^{L25}$)$_2$, -($C_{2-4}$ alkenyl)-(4- to 7-membered heterocyclyl)-N($R^{L25}$)$_2$, - $(CR^{L21}R^{L22})_p-SC(O)-N(R^{L25})_2$, $-(CR^{L21}R^{L22})_pN(R^{L29})C(S)N(R^{L27})(R^{L28})$, and

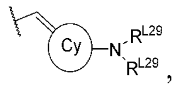

wherein Cy is 4- to 7-membered heterocyclyl; the cycloalkyl and heterocyclyl are optionally further substituted with 1-4 groups selected from halogen, =O, OH, $NH_2$, CN, $C_{1-4}$ alkyl, $C_{1-4}$ alkoxy, halogenated $C_{1-4}$ alkyl, $C_{3-6}$ cycloalkyl, and 5- to 7-membered heterocyclyl; in some embodiments, $L_2$ is selected from $-(CR^{L21}R^{L22})_p-OC(O)-N(R^{L23})_2$, $-(CR^{L21}R^{L22})_bN(R^{L24})S(O)_2N(R^{L25})_2$, $-(CR^{L21}R^{L22})_pN(R^{L26})C(O)N(R^{L27})(R^{L28})$, $-(CR^{L21}R^{L22})_pN(R^{L29})(R^{L30})$,-C(=O)N($R^{L31}$)$_2$, -N($R^{L31}$)-C(=O)O$R^{L32}$, and -($CH_2$)$_p R^{L33}$; in some embodiments, $L_2$ is selected from $-(CR^{L21}R^{L22})_p-OC(O)-N(R^{L23})_2$, - $(CR^{L21}R^{L22})_pN(R^{L24})S(O)_2N(R^{L25})_2$, $-(CR^{L21}R^{L22})_pN(R^{L26})C(O)N(R^{L27})(R^{L28})$,$-(CR^{L21}R^{L22}\ )_pN(R^{L29})(R^{L30})$, -C(=O)N($R^{L31}$)$_2$, and -N($R^{L31}$)-C(=O)O$R^{L32}$; in some embodiments, $L_2$ is selected from $-(CR^{L21}R^{L22})_p-OC(O)-N(R^{L23})_2$, - $(CR^{L21}R^{L22})pN(R^{L24})S(O)_2N(R^{L25})_2$, $-(CR^{L21}R^{L22})_pN(R^{L26})C(O)N(R^{L27})(R^{L28})$,$-(CR^{LZ1}R^{L22})_pN(R^{L29})(R^{L30})$, and -N($R^{L31}$)-C(=O)O$R^{L32}$; in some embodiments, $L_2$ is selected from $-(CR^{L21}R^{L22})_p-OC(O)-N(R^{L23})_2$, - $(CR^{L21}R^{L22})_pN(R^{L24})S(O)_2N(R^{L25})_2$, $-(CR^{L21}R^{L22})_pN(R^{L26})C(O)N(R^{L27})(R^{L28})$, and-$(CR^{L21}R^{L22})_pN(R^{L29})(R^{L30})$;

in some embodiments, $R^{a1}$, $R^{a2}$, $R^{L1}$, $R^{L2}$, $R^{L21}$, $R^{L22}$, $R^{L23}$, $R^{L24}$, $R^{L25}$, $R^{L26}$, $R^{L27}$, $R^{L28}$, $R^{L29}$, $R^{L30}$, and $R^{L31}$ are each independently selected from H, halogen, $C_{1-4}$ alkyl, $C_{2-6}$ alkenyl, $C_{2-6}$ alkynyl, $C_{1-4}$ alkoxy, 5- to 14-membered heterocyclyl, -($CH_2$)$_p$-(4- to 7-membered heterocyclyl), -($CH_2$)$_p$-$C_{3-10}$ cycloalkyl, -($CH_2$)$_p$-O-$C_{3-10}$ cycloalkyl, and -O-$C_{3-10}$ cycloalkyl, wherein the heterocyclyl contains 1-3 heteroatoms selected from N, O and S; the alkyl, alkenyl, alkynyl, cycloalkyl, and heterocyclyl are optionally further substituted with 1-4 groups selected from halogen, =O, =$CH_2$, =$CF_2$, =CH-$CH_3$, =C-($CH_3$)$_2$, OH, $NH_2$, CN, acetyl, -$SCF_3$, - S(O)$_2CH_3$, $C_{1-4}$ alkyl, $C_{1-4}$ alkoxy, halogenated $C_{1-4}$ alkoxy, halogenated $C_{1-4}$ alkyl, $C_{3-10}$ cycloalkyl, 5- to 12-membered heterocyclyl, and -C≡C-$C_{3-10}$ cycloalkyl;

$R^{a1}$, $R^{a2}$, $R^{L1}$, $R^{L2}$, $R^{L21}$, $R^{L22}$, $R^{L23}$, $R^{L24}$, $R^{L25}$, $R^{L26}$, $R^{L27}$, $R^{L28}$, $R^{L29}$, $R^{L30}$, and $R^{L31}$ are each independently selected from H, halogen, $C_{1-4}$ alkyl, $C_{2-6}$ alkenyl, $C_{2-6}$ alkynyl, $C_{1-4}$ alkoxy, 5- to 14-membered heterocyclyl, -($CH_2$)$_p$-(4- to 7-membered heterocyclyl), -($CH_2$)$_p$-$C_{3-10}$ cycloalkyl, and -O-$C_{3-10}$ cycloalkyl, wherein the heterocyclyl contains 1-3 heteroatoms selected from N, O and S; the alkyl, alkenyl, alkynyl, cycloalkyl, and heterocyclyl are optionally further substituted with 1-4 groups selected from halogen, =O, =$CH_2$, =$CF_2$, =CH-$CH_3$, =C-($CH_3$)$_2$, OH, $NH_2$, CN, acetyl, -S(O)$_2CH_3$, $C_{1-4}$ alkyl, $C_{1-4}$ alkoxy, halogenated $C_{1-4}$ alkoxy, halogenated $C_{1-4}$ alkyl, $C_{3-10}$ cycloalkyl, 5- to 12-membered heterocyclyl, and - C≡C-$C_{3-10}$ cycloalkyl;

in some embodiments, $R^{a1}$, $R^{a2}$, $R^{L1}$, $R^{L2}$, $R^{L21}$, $R^{L22}$, $R^{L23}$, $R^{L24}$, $R^{L25}$, $R^{L26}$, $R^{L27}$, $R^{L28}$, $R^{L29}$, $R^{L30}$, and $R^{L31}$ are each independently selected from H, halogen, $C_{1-4}$ alkyl, $C_{2-6}$ alkenyl, $C_{2-6}$ alkynyl, $C_{1-4}$ alkoxy, 5- to 14-membered heterocyclyl, -($CH_2$)$_p$-(4- to 7-membered heterocyclyl), and -($CH_2$)$_p$-$C_{3-10}$ cycloalkyl, wherein the heterocyclyl contains 1-3 heteroatoms selected from N, O and S; the alkyl, alkenyl, alkynyl, cycloalkyl, and heterocyclyl are optionally further substituted with 1-4 groups selected from halogen, =O, =$CH_2$, =$CF_2$, =CH-$CH_3$, =C-($CH_3$)$_2$, OH, $NH_2$, CN, acetyl, -S(O)$_2CH_3$, $C_{1-4}$ alkyl, $C_{1-4}$ alkoxy, halogenated $C_{1-4}$ alkyl, $C_{3-10}$ cycloalkyl, 5- to 12-membered heterocyclyl, and -C≡C-$C_{3-10}$ cycloalkyl;

in some embodiments, $R^{a1}$, $R^{a2}$, $R^{L1}$, $R^{L2}$, $R^{L21}$, $R^{L22}$, $R^{L23}$, $R^{L24}$, $R^{L25}$, $R^{L26}$, $R^{L27}$, and $R^{L28}$ are each independently selected from H, halogen, $C_{1-4}$ alkyl, $C_{2-6}$ alkenyl, $C_{2-6}$ alkynyl, $C_{1-4}$ alkoxy, 5- to 10-membered heterocycloalkyl, -($CH_2$)$_p$-(4-to 7-membered heterocyclyl), and -($CH_2$)$_p$-$C_{3-10}$ cycloalkyl, wherein the heterocyclyl contains 1-3 heteroatoms selected from N, O and S; the alkyl, alkenyl, alkynyl, cycloalkyl, and heterocycloalkyl are optionally further substituted with 1-4 groups selected from halogen, =O, =$CH_2$, =$CF_2$, =CH-$CH_3$, =C-($CH_3$)$_2$, OH, $NH_2$, CN, acetyl,

$-S(O)_2CH_3$, $C_{1-4}$ alkyl, $C_{1-4}$ alkoxy, halogenated $C_{1-4}$ alkyl, $C_{3-10}$ cycloalkyl, 5- to 12-membered heterocyclyl, and $-C{\equiv}C-C_{3-10}$ cycloalkyl;

in some embodiments, $R^{a1}$, $R^{a2}$, $R^{L1}$, $R^{L2}$, $R^{L21}$, $R^{L22}$, $R^{L23}$, $R^{L24}$, $R^{L25}$, $R^{L26}$, $R^{L27}$, $R^{L28}$, $R^{L29}$, $R^{L30}$, and $R^{L31}$ are each independently selected from H, halogen, $C_{1-4}$ alkyl, $C_{2-6}$ alkenyl, $C_{2-6}$ alkynyl, $C_{1-4}$ alkoxy, 5- to 14-membered heterocyclyl, $-(CH_2)_p-$(4- to 7-membered heterocyclyl), and $-(CH_2)_p-C_{3-10}$ cycloalkyl, wherein the heterocyclyl contains 1-3 heteroatoms selected from N, O and S; the alkyl, alkenyl, alkynyl, cycloalkyl, and heterocyclyl are optionally further substituted with 1-4 groups selected from halogen, =O, $=CH_2$, $=CF_2$, $=CH-CH_3$, $=C-(CH_3)_2$, OH, $NH_2$, CN, acetyl, $-S(O)_2CH_3$, $C_{1-4}$ alkyl, $C_{1-4}$ alkoxy, halogenated $C_{1-4}$ alkyl, $C_{3-10}$ cycloalkyl, and 5- to 12-membered heterocyclyl;

in some embodiments, $R^{a1}$, $R^{a2}$, $R^{L1}$, $R^{L2}$, $R^{L21}$, $R^{L22}$, $R^{L23}$, $R^{L24}$, $R^{L25}$, $R^{L26}$, $R^{L27}$, $R^{L28}$, $R^{L29}$, $R^{L30}$, and $R^{L31}$ are each independently selected from H, halogen, $C_{1-4}$ alkyl, $C_{2-6}$ alkenyl, $C_{2-6}$ alkynyl, $C_{3-10}$ cycloalkyl, $C_{1-4}$ alkoxy, 5- to 14-membered heterocyclyl, and $-(CH_2)_p-$(4- to 7-membered heterocyclyl), wherein the heterocyclyl contains 1-3 heteroatoms selected from N, O and S; the alkyl, alkenyl, alkynyl, cycloalkyl, and heterocyclyl are optionally further substituted with 1-4 groups selected from halogen, =O, $=CH_2$, $=CF_2$, OH, $NH_2$, CN, acetyl, $-S(O)_2CH_3$, $C_{1-4}$ alkyl, $C_{1-4}$ alkoxy, halogenated $C_{1-4}$ alkyl, $C_{3-10}$ cycloalkyl, and 5- to 12-membered heterocyclyl;

in some embodiments, $R^{a1}$, $R^{a2}$, $R^{L1}$, $R^{L2}$, $R^{L21}$, $R^{L22}$, $R^{L23}$, $R^{L24}$, $R^{L25}$, $R^{L26}$, $R^{L27}$, $R^{L28}$, $R^{L29}$, $R^{L30}$, and $R^{L31}$ are each independently selected from H, halogen, $C_{1-4}$ alkyl, $C_{2-6}$ alkenyl, $C_{2-6}$ alkynyl, $C_{3-10}$ cycloalkyl, $C_{1-4}$ alkoxy, and 5- to 14-membered heterocyclyl, wherein the heterocyclyl contains 1-3 heteroatoms selected from N, O and S; the alkyl, alkenyl, alkynyl, cycloalkyl, alkoxy, and heterocyclyl are optionally further substituted with 1-4 groups selected from halogen, =O, OH, $NH_2$, CN, acetyl, $C_{1-4}$ alkyl, $C_{1-4}$ alkoxy, halogenated $C_{1-4}$ alkyl, $C_{3-10}$ cycloalkyl, and 5- to 12-membered heterocyclyl;

in some embodiments, $R^{a1}$, $R^{a2}$, $R^{L1}$, $R^{L2}$, $R^{L21}$, $R^{L22}$, $R^{L23}$, $R^{L24}$, $R^{L25}$, $R^{L26}$, $R^{L27}$, and $R^{L28}$ are each independently selected from H, halogen, $C_{1-4}$ alkyl, $C_{2-6}$ alkenyl, $C_{2-6}$ alkynyl, $C_{1-4}$ alkoxy, 5- to 10-membered heterocycloalkyl, $-(CH_2)_p-$(4-to 7-membered heterocyclyl), and $-(CH_2)_p-C_{3-10}$ cycloalkyl, wherein the heterocyclyl contains 1-3 heteroatoms selected from N, O and S; the alkyl, alkenyl, alkynyl, cycloalkyl, and heterocycloalkyl are optionally further substituted with 1-4 groups selected from halogen, =O, $=CH_2$, $=CF_2$, $=CH-CH_3$, $=C-(CH_3)_2$, OH, $NH_2$, CN, acetyl, $-S(O)_2CH_3$, $C_{1-4}$ alkyl, $C_{1-4}$ alkoxy, halogenated $C_{1-4}$ alkyl, $C_{3-10}$ cycloalkyl, and 5- to 12-membered heterocyclyl;

in some embodiments, $R^{a1}$ and $R^{a2}$ are each independently selected from H, halogen, $C_{1-4}$ alkyl, $C_{2-4}$ alkenyl, $C_{2-4}$ alkynyl, $C_{3-6}$ cycloalkyl, and $C_{1-4}$ alkoxy, wherein the alkyl, alkenyl, alkynyl, cycloalkyl, and alkoxy are optionally further substituted with 1-4 groups selected from halogen, =O, OH, $NH_2$, CN, acetyl, $C_{1-4}$ alkyl, $C_{1-4}$ alkoxy, halogenated $C_{1-4}$ alkyl, $C_{3-10}$ cycloalkyl, and 5- to 12-membered heterocyclyl;

in some embodiments, $R^{a1}$ and $R^{a2}$ are each independently selected from H, methyl, ethyl, propyl, isopropyl, cyclopropyl, and cyclobutyl;

in some embodiments, $R^{L21}$ and $R^{L22}$ are each independently selected from H, $C_{1-4}$ alkyl, $C_{2-6}$ alkenyl, $C_{2-6}$ alkynyl, $C_{3-10}$ cycloalkyl, and 5- to 12-membered heterocyclyl, wherein the heterocyclyl contains 1-3 heteroatoms selected from N, O and S; the alkyl, alkenyl, alkynyl, cycloalkyl, and heterocyclyl are optionally further substituted with 1-4 groups selected from halogen, =O, OH, $NH_2$, CN, and $C_{1-4}$ alkyl;

in some embodiments, $R^{L21}$ and $R^{L22}$ are each independently selected from H, $C_{1-2}$ alkyl, $C_{2-4}$ alkenyl, $C_{2-4}$ alkynyl, and $C_{3-6}$ cycloalkyl, wherein the alkyl, alkenyl, alkynyl, cycloalkyl, and heterocyclyl are optionally further substituted with 1-4 groups selected from halogen, =O, OH, $NH_2$, CN, and $C_{1-4}$ alkyl;

in some embodiments, $R^{L21}$ and $R^{L22}$ are each independently selected from H, $C_{1-2}$ alkyl, and $C_{3-6}$ cycloalkyl, wherein the alkyl and cycloalkyl are optionally further substituted with 1-4 groups selected from F, Cl, OH, $NH_2$, and CN;

in some embodiments, $^{L21}$ and $R^{L22}$ are each independently selected from H, methyl, ethyl, and cyclopropyl;

in some embodiments, each $R^{L23}$ is independently selected from $C_{1-4}$ alkyl, - $(CH_2)_p-O-C_{3-6}$ cycloalkyl, $C_{3-6}$ cycloalkyl, $-O-C_{3-6}$ cycloalkyl, $-(CH_2)_p-$(4- to 7-membered heterocycloalkyl), 6- to 12-membered bicyclic heterocycloalkyl,

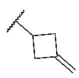,

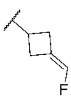, 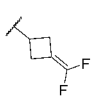, 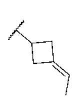, 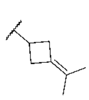, 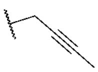, 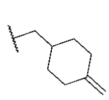, 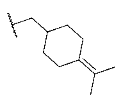, 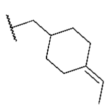, 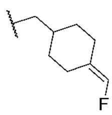,

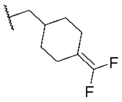, 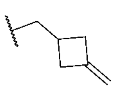, 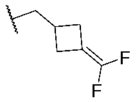, 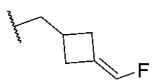, 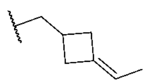, 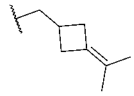,

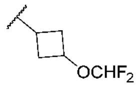, 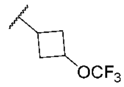,

and 5- to 6-membered heteroaryl, wherein the heterocycloalkyl and heteroaryl contain 1-3 heteroatoms selected from N, O, S, S(O) and $S(O)_2$; the alkyl, heterocycloalkyl and heteroaryl are optionally further substituted with 1-4 groups selected from =O, halogen, OH, $-OCF_3$, $-OCHF_2$, - $SCF_3$, $NH_2$, CN, acetyl, $-S(O)_2CH_3$, and

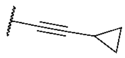;

and when $R^{L21}$ and $R^{L22}$ are both H, and $L_1$ is O, at least one $R^{L23}$ is selected from $-(CH_2)_p$-(4- to 7-membered heterocycloalkyl), 6- to 12-membered bicyclic heterocycloalkyl, 5- to 6-membered heteroaryl,

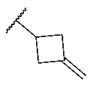, 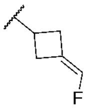, 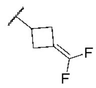, 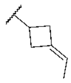,

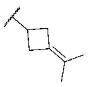, 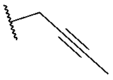, 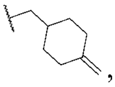, 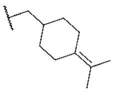, 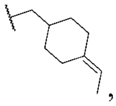, 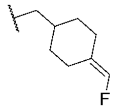, 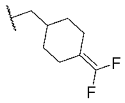, 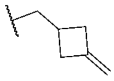,

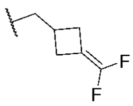, 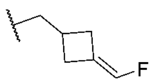, 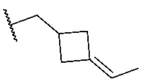, 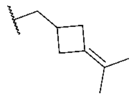, 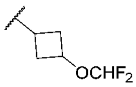, 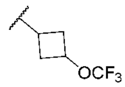,

$C_{1-4}$ alkyl substituted with 1-4 groups selected from $-OCF_3$, $-OCHF_2$, $-SCF_3$, $NH_2$, CN, acetyl, $-S(O)_2CH_3$, and

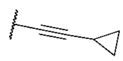,

$-O-C_{3-6}$ cycloalkyl, $-(CH_2)_p-O-C_{3-6}$ cycloalkyl, and $C_{3-6}$ cycloalkyl substituted with 1-4 groups selected from $-OCF_3$, $-OCHF_2$, - $SCF_3$, $NH_2$, CN, acetyl, $-S(O)_2CH_3$, and

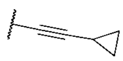,

wherein the heterocycloalkyl and heteroaryl are optionally further substituted with 1-4 groups selected from =O, halogen, OH, $-OCF_3$, $-OCHF_2$, $-SCF_3$, $NH_2$, CN, acetyl, $-S(O)_2CH_3$, and

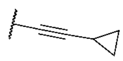;

in some embodiments, each $R^{L23}$ is independently selected from $C_{1-4}$ alkyl, - $(CH_2)_p-O-C_{3-6}$ cycloalkyl, $C_{3-6}$ cycloalkyl, $-O-C_{3-6}$ cycloalkyl, $-(CH_2)_p$-(4- to 7-membered heterocycloalkyl), 6- to 12-membered bicyclic heterocycloalkyl,

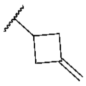,

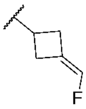, 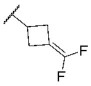, 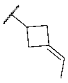, 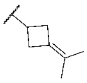, 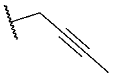, 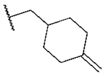, 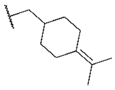, 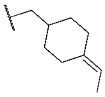, 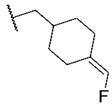,

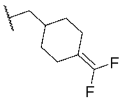, 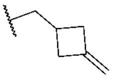, 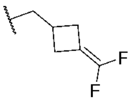, 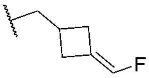, 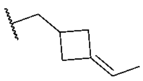, 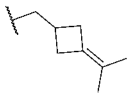,

and 5- to 6-membered heteroaryl, wherein the heterocycloalkyl and heteroaryl contain 1-3 heteroatoms selected from N, O, S, S(O), and $S(O)_2$; the alkyl, heterocycloalkyl and heteroaryl are optionally further substituted with 1-4 groups selected from =O, halogen, OH, $-OCF_3$, $-OCHF_2$, $-SCF_3$, $NH_2$, CN, acetyl, $-S(O)_2CH_3$, and

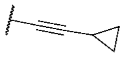;

in some embodiments, each $R^{L23}$ is independently selected from $-(CH_2)_p$-(4-to 7-membered heterocycloalkyl), 6- to 12-membered bicyclic heterocycloalkyl, 5-to 6-membered heteroaryl,

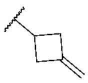, 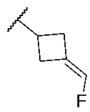, 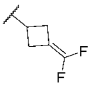, 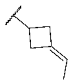, 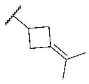, 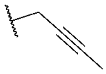,

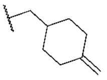, 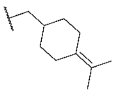, 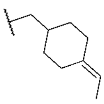, 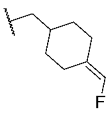, 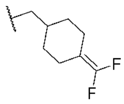, 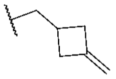, 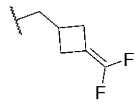,

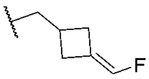, 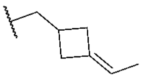, 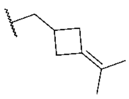,

$C_{1-4}$ alkyl substituted with 1-4 groups selected from $-OCF_3$, $-OCHF_2$, $-SCF_3$, $NH_2$, CN, acetyl, $-S(O)_2CH_3$, and

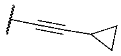,

$-O-C_{3-6}$ cycloalkyl, $-(CH_2)_p-O-C_{3-6}$ cycloalkyl, and $C_{3-6}$ cycloalkyl substituted with 1-4 groups selected from $-OCF_3$, $-OCHF_2$, $-SCF_3$, $NH_2$, CN, acetyl, $-S(O)_2CH_3$, and

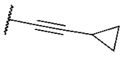,

wherein the heterocycloalkyl and heteroaryl are optionally further substituted with 1-4 groups selected from =O, halogen, OH, $-OCF_3$, $-OCHF_2$, $- SCF_3$, $NH_2$, CN, acetyl, $-S(O)_2CH_3$, and

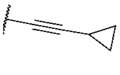;

in some embodiments, each $R^{L23}$ is independently selected from $C_{1-4}$ alkyl, $- O-C_{3-6}$ cycloalkyl, $-(CH_2)_p$-(4- to 7-

membered heterocycloalkyl), 6- to 12-membered bicyclic heterocycloalkyl,

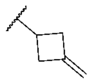, 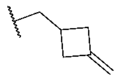, 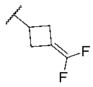, 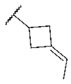, 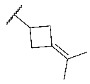,

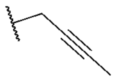, 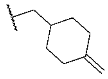, 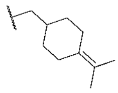, 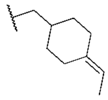, 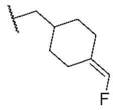, 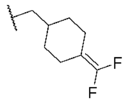,

and 5- to 6-membered heteroaryl, wherein the heterocycloalkyl and heteroaryl contain 1-3 heteroatoms selected from N, O and S; the alkyl, heterocycloalkyl, and heteroaryl are optionally further substituted with 1-4 groups selected from =O, halogen, OH, - $OCF_3$, $NH_2$, CN, acetyl, -$S(O)_2CH_3$, and

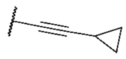;

and when $R^{L21}$ and $R^{L22}$ are both H, at least one $R^{L23}$ is selected from -$(CH_2)_p$-(4- to 7-membered heterocycloalkyl), 6- to 12-membered bicyclic heterocycloalkyl,

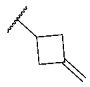, 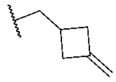,

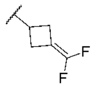, 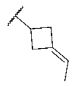, 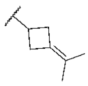,

5- to 6-membered heteroaryl substituted with

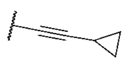, -

$CH_2CH_2OCF_3$, -O-$C_{3-6}$ cycloalkyl,

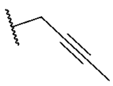, 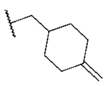, 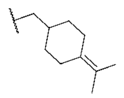, 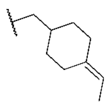,

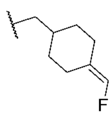, and 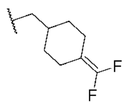;

in some embodiments, each $R^{L23}$ is independently selected from $C_{1-4}$ alkyl, - $(CH_2)_p$-(4- to 7-membered heterocycloalkyl), 6- to 12-membered bicyclic heterocycloalkyl,

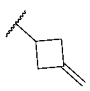, 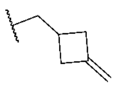, 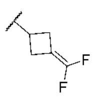, 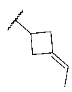, and 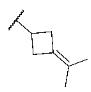,

wherein the heterocycloalkyl contains 1-3 heteroatoms selected from N, O and S; the alkyl and heterocycloalkyl are optionally further substituted with 1-4 groups selected from =O, halogen, OH, $NH_2$, CN, acetyl, and -$S(O)_2CH_3$; and when $R^{L21}$ and $R^{L22}$ are both H, at least one $R^{L23}$ is selected from -$(CH_2)_p$-(4- to 7-membered heterocycloalkyl), 6- to 12-membered bicyclic heterocycloalkyl,

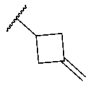, 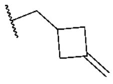,

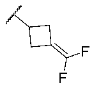, 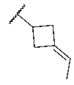, and 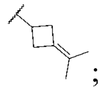;

in some embodiments, each $R^{L23}$ is independently selected from $C_{1-4}$ alkyl, - $(CH_2)_p$-(4- to 7-membered heterocycloalkyl), 6- to 12-membered bicyclic heterocycloalkyl,

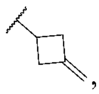, 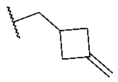, 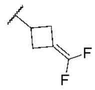, 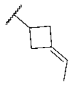, 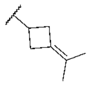,

and 5- to 6-membered heteroaryl, wherein the heterocycloalkyl and heteroaryl contain 1-3 heteroatoms selected from N, O and S; the alkyl, heterocycloalkyl, and heteroaryl are optionally further substituted with 1-4 groups selected from =O, halogen, OH, $NH_2$, CN, acetyl, $-S(O)_2CH_3$, and

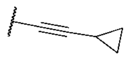;

and when $R^{L21}$ and $R^{L22}$ are both H, at least one $R^{L23}$ is selected from $-(CH_2)_p$-(4- to 7-membered heterocycloalkyl), 6- to 12-membered bicyclic heterocycloalkyl,

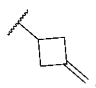, 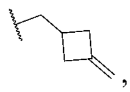, 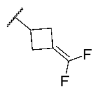, 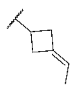, 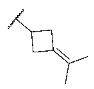,

and 5- to 6-membered heteroaryl substituted with

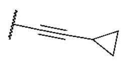;

in some embodiments, each $R^{L23}$ is independently selected from $C_{1-4}$ alkyl, - $(CH_2)_p$-(4- to 7-membered heterocycloalkyl), 6- to 12-membered bicyclic heterocycloalkyl,

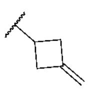, 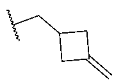, and 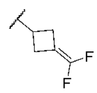,

wherein the heterocycloalkyl contains 1-3 heteroatoms selected from N, O and S; the alkyl and heterocycloalkyl are optionally further substituted with 1-4 groups selected from =O, halogen, OH, $NH_2$, CN, acetyl, and $-S(O)_2CH_3$; and when $R^{L21}$ and $R^{L22}$ are both H, at least one $R^{L23}$ is selected from $-(CH_2)_p$-(4- to 7-membered heterocycloalkyl), 6- to 12-membered bicyclic heterocycloalkyl,

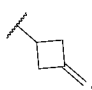, 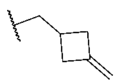, and 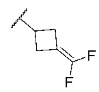;

in some embodiments, each $R^{L23}$ is independently selected from $C_{1-4}$ alkyl and 6- to 12-membered bicyclic heterocycloalkyl, wherein the heterocycloalkyl contains 1-3 heteroatoms selected from N, O and S; the alkyl and heterocycloalkyl are optionally further substituted with 1-4 groups selected from halogen, OH, $NH_2$, CN, and acetyl; and when $R^{L21}$ and $R^{L22}$ are both H, at least one $R^{L23}$ is 6- to 12-membered bicyclic heterocycloalkyl;
in some embodiments, each $R^{L23}$ is independently selected from methyl, ethyl, propyl,

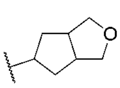, 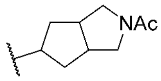, 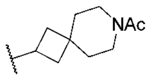, or 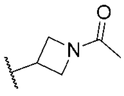, 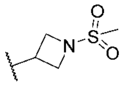, 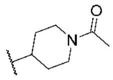,

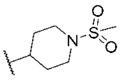, 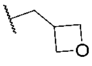, 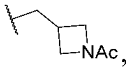, 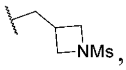, 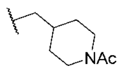, 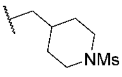, and 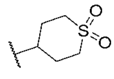;

$R^{L23-1}$ is selected from methyl and ethyl; in some embodiments, $R^{L23-1}$ is methyl;

$R^{L23-2}$ is selected from $-(CH_2)_p$-(4- to 7-membered heterocycloalkyl), $-(CH_2)_p-C_{4-6}$ monocyclic cycloalkyl, $-O-C_{3-6}$ cycloalkyl, and $-CH_2CH_2OCF_3$, wherein the heterocycloalkyl contains 1-2 N atoms; the heterocycloalkyl is optionally further substituted with 1-2 groups selected from $-S(O)_2CH_3$, =O, $=CH_2$, $=CF_2$, $=CH-CH_3$, and $=C-(CH_3)_2$; the cycloalkyl is further substituted with 1-2 groups selected from $=CH_2$, $=CF_2$, $=CH-CH_3$, and $=C-(CH_3)_2$; in some embodiments, $R^{L23-2}$ is selected from $-CH_2CH_2OCF_3$,

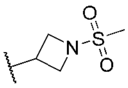, 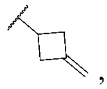, 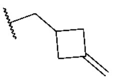, 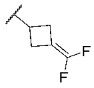, 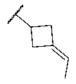, 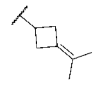,

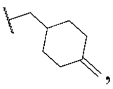, 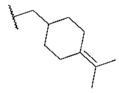, 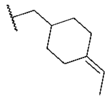, 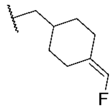, 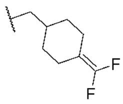, and 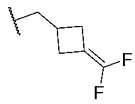;

$R^{L23-1a}$ is selected from H, methyl, and ethyl; in some embodiments, $R^{L23-1a}$ is selected from H and methyl; in some embodiments, $R^{L23-1a}$ is selected from methyl and ethyl;

$R^{L23-2a}$ is selected from $-(CH_2)_p$-(4- to 7-membered heterocycloalkyl), 6- to 12-membered bicyclic heterocycloalkyl, 5- to 6-membered heteroaryl,

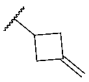,

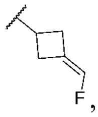, 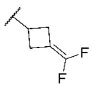, 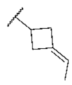, 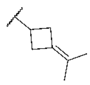, 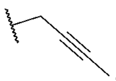, 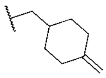, 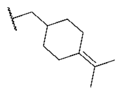, 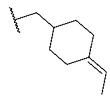, 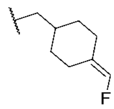,

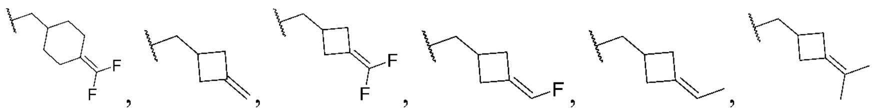,

$C_{1-4}$ alkyl substituted with 1-4 groups selected from $-OCF_3$, $-OCHF_2$, $-SCF_3$, $NH_2$, CN, acetyl, $-S(O)_2CH_3$, and

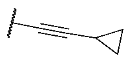,

$-O-C_{3-6}$ cycloalkyl, $-(CH_2)_p-O-C_{3-6}$ cycloalkyl, and $C_{3-6}$ cycloalkyl substituted with 1-4 groups selected from $-OCF_3$, $-OCHF_2$, $-SCF_3$, $NH_2$, CN, acetyl, $-S(O)_2CH_3$, and

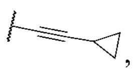,

wherein the heterocycloalkyl and heteroaryl are optionally further substituted with 1-4 groups selected from =O, halogen, OH,$-OCF_3$, $-OCHF_2$, $-SCF_3$, $NH_2$, CN, acetyl, $-S(O)_2CH_3$, and

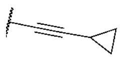;

in some embodiments, $R^{L23\text{-}2a}$ is selected from 4- to 7-membered heterocycloalkyl, $-CH_2$-(4- to 7-membered heterocycloalkyl), 6- to 12-membered bicyclic heterocycloalkyl, 5- to 6-membered heteroaryl,

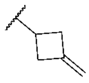, 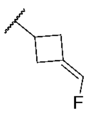, 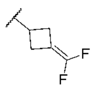, 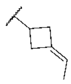, 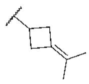, 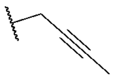,

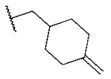, 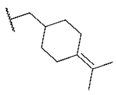, 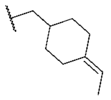, 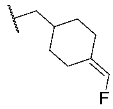, 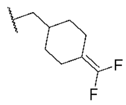, 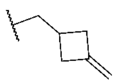, 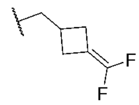,

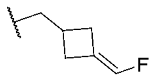, 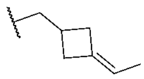, 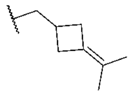,

$C_{2\text{-}4}$ alkyl substituted with 1-4 groups selected from $-OCF_3$, $-OCHF_2$, and $-SCF_3$, $-O-C_{3\text{-}6}$ cycloalkyl, $-(CH_2)_2-O-C_{3\text{-}6}$ cycloalkyl, and $C_{3\text{-}6}$ cycloalkyl substituted with 1 group selected from $-OCF_3$, - $OCHF_2$, $-SCF_3$, $NH_2$, CN, acetyl, $-S(O)_2CH_3$, and

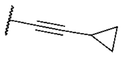,

wherein the heterocycloalkyl and heteroaryl are optionally further substituted with 1, 2, and 3 groups selected from =O, F, Cl, OH, $-OCF_3$, $-OCHF_2$, $-SCF_3$, $NH_2$, CN, acetyl, - $S(O)_2CH_3$, and

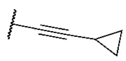;

in some embodiments, each $R^{L24}$ is independently selected from H, $C_{2\text{-}6}$ alkenyl, and $C_{2\text{-}6}$ alkynyl, wherein the alkenyl and alkynyl are optionally further substituted with 1-4 groups selected from halogen, OH, $NH_2$, CN, $C_{3\text{-}10}$ cycloalkyl, and 5- to 12-membered heterocyclyl;

in some embodiments, $R^{L25}$, $R^{L27}$, and $R^{L28}$ are each independently selected from $C_{1\text{-}3}$ alkyl, $C_{2\text{-}6}$ alkenyl, and $C_{2\text{-}6}$ alkynyl, wherein the alkyl, alkenyl, and alkynyl are optionally further substituted with 1-4 groups selected from halogen, OH, $NH_2$, and CN;

in some embodiments, $R^{L25}$, $R^{L27}$, and $R^{L28}$ are each independently selected from $C_{1\text{-}3}$ alkyl, $C_{2\text{-}4}$ alkenyl, and $C_{2\text{-}4}$ alkynyl, wherein the alkyl, alkenyl, and alkynyl are optionally further substituted with 1-4 groups selected from halogen, OH, $NH_2$, and CN;

in some embodiments, $R^{L25}$, $R^{L27}$, and $R^{L28}$ are each independently selected from methyl, ethyl, and propyl;

in some embodiments, $R^{L29}$ is selected from H, $C_{1\text{-}4}$ alkyl, $C_{2\text{-}6}$ alkenyl, and $C_{2\text{-}6}$ alkynyl, wherein the alkyl, alkenyl, and alkynyl are optionally further substituted with 1-4 groups selected from halogen, OH, $NH_2$, and CN; in some embodiments, $R^{L29}$ is selected from H, $C_{1\text{-}4}$ alkyl, $C_{2\text{-}4}$ alkenyl, and $C_{2\text{-}4}$ alkynyl, wherein the alkyl, alkenyl, and alkynyl are optionally further substituted with 1-4 groups selected from halogen, OH, $NH_2$, and CN; in some embodiments, $R^{L29}$ is selected from H, methyl, and ethyl;

in some embodiments, $R^{L30}$ is selected from 6- to 14-membered bicyclic or tricyclic heterocyclyl, wherein the heterocyclyl contains 1-3 heteroatoms selected from N, O and S; the heterocyclyl is optionally further substituted with 1-4 groups selected from halogen, CN, OH, $NH_2$, $C_{1\text{-}4}$ alkyl, and halogenated $C_{1\text{-}4}$ alkyl; and $R^{L30}$ is not

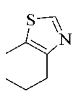, 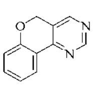, and 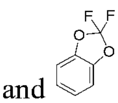;

in some embodiments, $R^{L30}$ is selected from

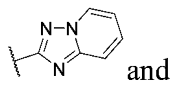 and 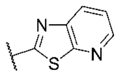 ;

the compound is not

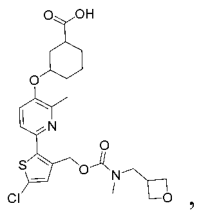 , 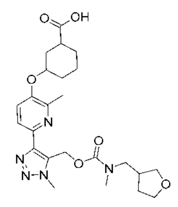 , 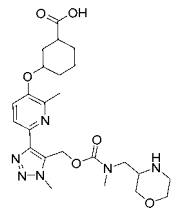

and

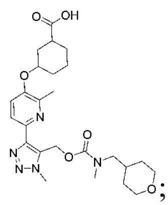 ;

$L_{2-1}$ is selected from $-(CR^{L21}R^{L22})_p-OC(O)-N(R^{L23})_2$, - $(CR^{L21}R^{L22})_pN(R^{L24})S(O)_2N(R^{L25})_2$, $-(CR^{L21}R^{L22})_pN(R^{L26})C(O)N(R^{L27})(R^{L28})$,$-(CR^{L21}R^{L22})_pN(R^{L29})(R^{L30})$, $-(CR^{L21}R^{L22})_p-C(O)-N(R^{L29})-C(O)R^{L21}$, $-(CR^{L21}R^{L22})_p-C(O)-N(R^{L29})-S(O)_2R^{L21}$, -(3- to 6-membered cycloalkyl)-$C(=O)N(R^{L25})_2$, -(4- to 7-membered heterocyclyl)-$C(=O)N(R^{L25})_2$, -($C_{2-4}$ alkenyl)-(4- to 7-membered heterocyclyl)-$N(R^{L25})_2$, $-(CR^{L21}R^{L22})_p-SC(O)-N(R^{L25})_2$, - $(CR^{L21}R^{L22})_pN(R^{L29})C(S)N(R^{L27})(R^{L28})$,

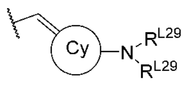 ,

$-(CR^{L21}R^{L22})_p-OC(O)-R^{L23}$, $CR^{L21}R^{L22})_p-O-R^{L23}$, and $-(CR^{L21}R^{L22})_p-N(R^{L26})C(O)O-R^{L23}$, wherein Cy is 4- to 7-membered heterocyclyl; the cycloalkyl and heterocyclyl are optionally further substituted with 1-4 groups selected from halogen, =O, OH, $NH_2$, CN, $C_{1-4}$ alkyl, $C_{1-4}$ alkoxy, halogenated $C_{1-4}$ alkyl, $C_{3-6}$ cycloalkyl, and 5- to 7-membered heterocyclyl;

in some embodiments, $L_{2-1}$ is selected from $-(CR^{L21}R^{L22})_p-OC(O)-N(R^{L23})_2$, - $(CR^{L21}R^{L22})_pN(R^{L24})S(O)_2N(R^{L25})_2$, $(CR^{L21}R^{L22})_pN(R^{L26})C(O)N(R^{L27})(R^{L28})$,$-(CR^{L21}R^{L22})_pN(R^{L29})(R^{L30})$, $-(CR^{L21}R^{L22})_p-C(O)-N(R^{L29})-C(O)R^{L21})$, $-(CR^{L21}R^{L22})_p-C(O)-N(R^{L29})-S(O)_2R^{L21}$, -(3- to 6-membered cycloalkyl)-$C(=O)N(R^{L25})_2$, -(4- to 7-membered heterocyclyl)-$C(=O)N(R^{L25})_2$, -($C_{2-4}$ alkenyl)-(4- to 7-membered heterocyclyl)-$N(R^{L25})2$, $-(CR^{L21}R^{L22})_p-SC(O)-N(R^{L25})_2$, - $(CR^{L21}R^{L22})_pN(R^{L26})C(S)N(R^{L27})(R^{L28})$,

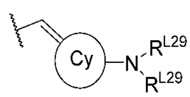 ,

$-(CR^{L21}R^{L22})_p-OC(O)-R^{L23}$, and $-(CR^{L21}R^{L22})_p-O-R^{L23}$, wherein Cy is 4- to 7-membered heterocyclyl; the cycloalkyl and heterocyclyl are optionally further substituted with 1-4 groups selected from halogen, =O, OH, $NH_2$, CN, $C_{1-4}$ alkyl, $C_{1-4}$ alkoxy, halogenated $C_{1-4}$ alkyl, $C_{3-6}$ cycloalkyl, and 5- to 7-membered heterocyclyl;

in some embodiments, $L_{2-1}$ is selected from $-(CR^{L21}R^{L22})_p-OC(O)-N(R^{L23})_2$, - $(CR^{L21}R^{L22})_pN(R^{L24})S(O)_2N(R^{L25})_2$, $-(CR^{L21}R^{L22})_pN(R^{L26})C(O)N(R^{L27})(R^{L28})$,$-(CR^{L21}R^{L22})_pN(R^{L29})(R^{L30})$, $-(CR^{L21}R^{L22})_p-C(O)-N(R^{L29})-C(O)R^{L21}$, $-(CR^{L21}R^{L22})_p-C(O)-N(R^{L29})-S(O)_2R^{L21}$, -(3- to 6-membered cycloalkyl)-$C(=O)N(R^{L25})_2$, -(4- to 7-membered heterocyclyl)-$C(=O)N(R^{L25})_2$, -($C_{2-4}$ alkenyl)-(4- to 7-membered heterocyclyl)-$N(R^{L25})2$, $-(CR^{L21}R^{L22})_p-SC(O)-N(R^{L25})_2$, - $(CR^{L21}R^{L22})_pN(R^{L26})C(S)N(R^{L27})(R^{L28})$,

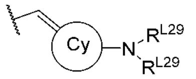,

and $-(CR^{L21}R^{L22})_p$-OC(O)-$R^{L23}$, wherein Cy is 4- to 7-membered heterocyclyl; the cycloalkyl and heterocyclyl are optionally further substituted with 1-4 groups selected from halogen, =O, OH, $NH_2$, CN, $C_{1-4}$ alkyl, $C_{1-4}$ alkoxy, halogenated $C_{1-4}$ alkyl, $C_{3-6}$ cycloalkyl, and 5- to 7-membered heterocyclyl;

in some embodiments, $L_{2-1}$ is selected from $-(CR^{L21}R^{L22})_p$-OC(O)-$N(R^{L23})_2$, - $(CR^{L21}R^{L22})_pN(R^{L24})S(O)_2N(R^{L25})_2$, $-(CR^{L21}R^{L22})_pN(R^{L26})C(O)N(R^{L27})(R^{L28})$,$-(CR^{L21}R^{L22})_pN(R^{L29})(R^{L30})$, $-(CR^{L21}R^{L22})_p$-C(O)-$N(R^{L29})$-C(O)$R^{L21}$, $-(CR^{L21}R^{L22})_p$-C(O)-$N(R^{L29})$-$S(O)_2R^{L21}$, -(3- to 6-membered cycloalkyl)-C(=O)$N(R^{L25})_2$, -(4- to 7-membered heterocyclyl)-C(=O)$N(R^{L25})_2$, -($C_{2-4}$ alkenyl)-(4- to 7-membered heterocyclyl)-$N(R^{L25})_2$, $-(CR^{L21}R^{L22})_p$-SC(O)-$N(R^{L25})_2$, - $(CR^{L21}R^{L22})_pN(R^{L26})C(S)N(R^{L27})(R^{L28})$, and

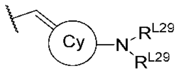,

wherein Cy is 4- to 7-membered heterocyclyl; the cycloalkyl and heterocyclyl are optionally further substituted with 1-4 groups selected from halogen, =O, OH, $NH_2$, CN, $C_{1-4}$ alkyl, $C_{1-4}$ alkoxy, halogenated $C_{1-4}$ alkyl, $C_{3-6}$ cycloalkyl, and 5- to 7-membered heterocyclyl;

in some embodiments, $L_{2-1}$ is selected from $-(CR^{L21}R^{L22})_p$-OC(O)-$N(R^{L23})_2$, - $(CR^{L21}R^{L22})_pN(R^{L24})S(O)_2N(R^{L25})_2$, $-(CR^{L21}R^{L22})_pN(R^{L26})C(O)N(R^{L27})(R^{L28})$, and$-(CR^{L21}R^{L22})_pN(R^{L29})(R^{L30})$;

in some embodiments, $L_{2-1}$ is selected from

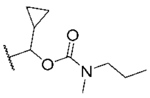, 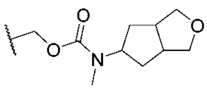,

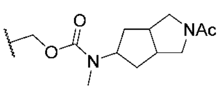, 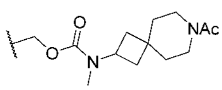, 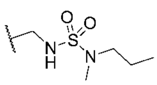, 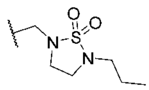, 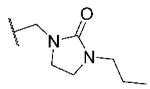,

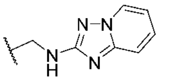, 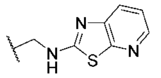, 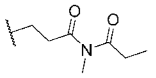, 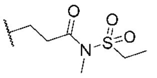, 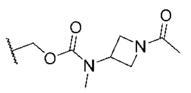,

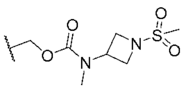, 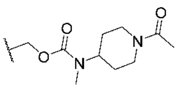, 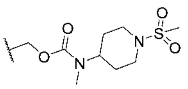, 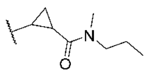, 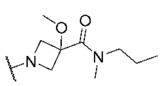,

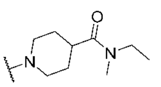, 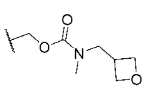, 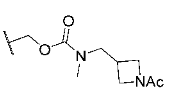, 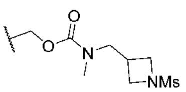, 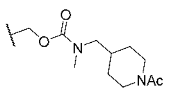,

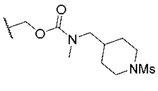, 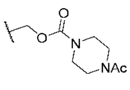, 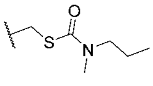, 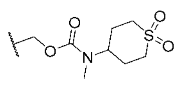, 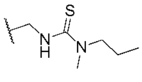, 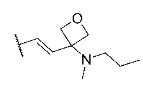,

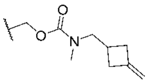, 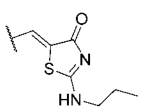, 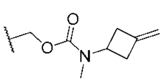, 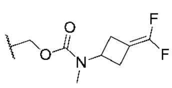, 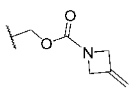, 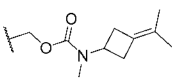,

.

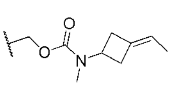, 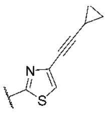, 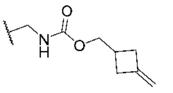, 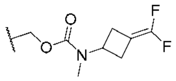, 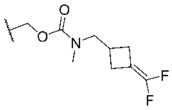,

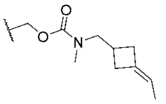, 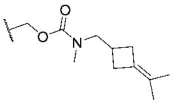, 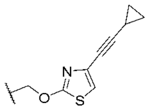, 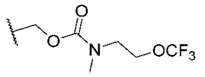, 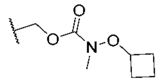,

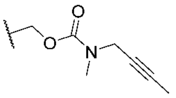, 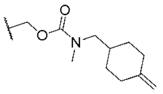, 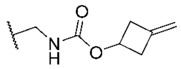, 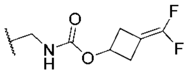, 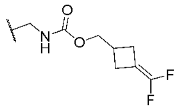,

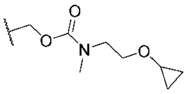, 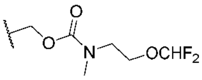, 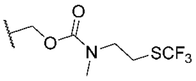, 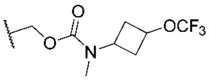,

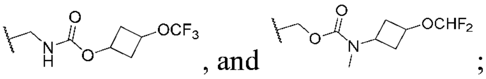, and ;

in some embodiments, $L_{2-1}$ is selected from

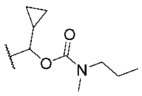, 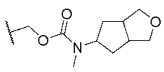,

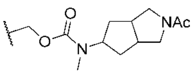, 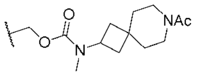, 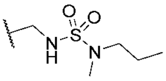, 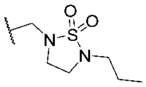, 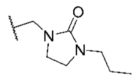,

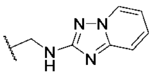, 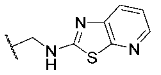, 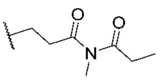, 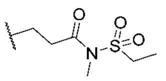, 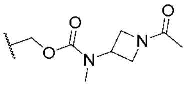,

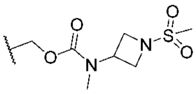, 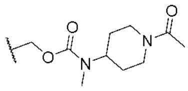, 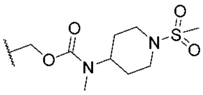, 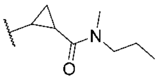, 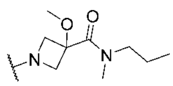,

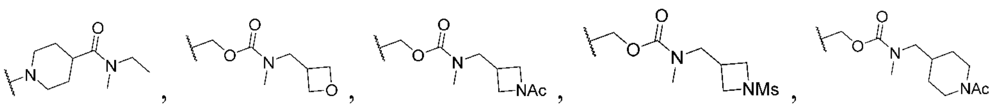, , , , ,

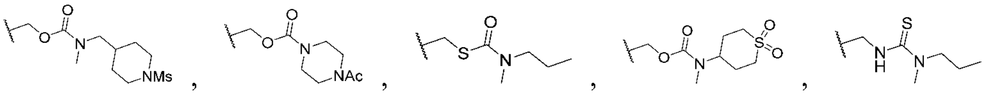, , , , ,

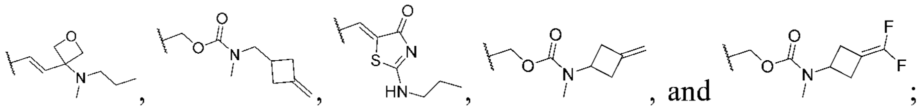, , , , and ;

or $L_{2-1}$ is selected from

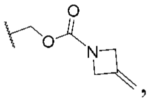, 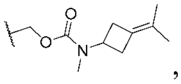, and 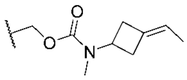;

or $L_{2-1}$ is selected from

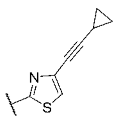, 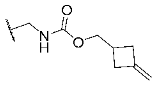, 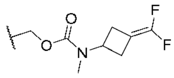, and 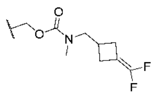;

or $L_{2-1}$ is selected from

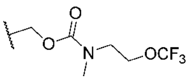, 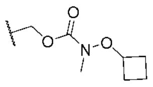, 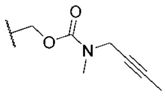, and 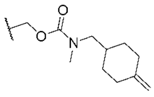;

or $L_{2-1}$ is selected from

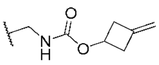, 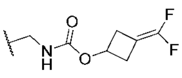, and 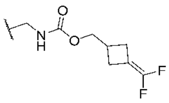;

or $L_{2-1}$ is selected from

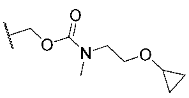, 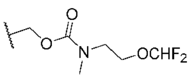, 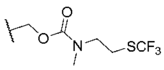, and 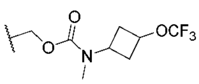;

or $L_{2-1}$ is selected from

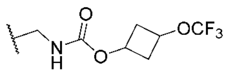 and 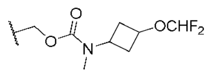;

$L_{2-2}$ and $L_{2-2c}$ are each independently selected from $-(CR^{L21}R^{L22})_p$-OC(O)-N($R^{L23}$)$_2$, $-(CR^{L21}R^{L22})_pN(R^{L24})S(O)_2N(R^{L25})_2$,$-(CR^{L21}R^{L22})_pN(R^{L26})C(O)N(R^{L27})(R^{L28})$, $-(CR^{L21}R^{L22})_pN(R^{L29})(R^{L30})$,$-(CR^{L21}R^{L22})_p$-OC(O)-$R^{L23}$, $-(CR^{L21}R^{L22})_p$-O-$R^{L23}$, and $-(CR^{L21}R^{L22})_p$-N($R^{L26}$)C(O)O-$R^{L23}$;

in some embodiments, $L_{2-2}$ and $L_{2-2c}$ are each independently selected from - $(CR^{L21}R^{L22})_p$-OC(O)-N($R^{L23}$)$_2$, $-(CR^{L21}R^{L22})_pN(R^{L24})S(O)_2N(R^{L25})_2$,$-(CR^{L21}R^{L22})_pN(R^{L26})C(O)N(R^{L27})(R^{L28})$, $-(CR^{L21}R^{L22})_pN(R^{L29})(R^{L30})$,$-(CR^{L21}R^{L22})_p$-OC(O)-$R^{L23}$, and $-(CR^{L21}R^{L22})_p$-O-$R^{L23}$;

in some embodiments, $L_{2-2}$ and $L_{2-2c}$ are each independently selected from - $(CR^{L21}R^{L22})_p$-OC(O)-N($R^{L23}$)$_2$, $-(CR^{L21}R^{L22})_pN(R^{L24})S(O)_2N(R^{L25})_2$,$-(CR^{L21}R^{L22})_pN(R^{L26})C(O)N(R^{L27})(R^{L28})$, $-(CR^{L21}R^{L22})_pN(R^{L29})(R^{L30})$, and-$(CR^{L21}R^{L22})_p$-OC(O)-$R^{L23}$;

in some embodiments, $L_{2-2}$ and $L_{2-2c}$ are each independently selected from - $(CR^{L21}R^{L22})_p$-OC(O)-N($R^{L23}$)$_2$, $-(CR^{L21}R^{L22})_pN(R^{L24})S(O)_2N(R^{L25})_2$,$-(CR^{L21}R^{L22})_pN(R^{L26})C(O)N(R^{L27})(R^{L28})$, and $-(CR^{L21}R^{L22})_pN(R^{L29})(R^{L30})$;

in some embodiments, $L_{2-2c}$ is selected from $-(CR^{L21}R^{L22})_p$-OC(O)-N($R^{L23}$)$_2$,$-(CR^{L21}R^{L22})_pN(R^{L24})S(O)_2N(R^{L25})_2$, $-(CR^{L21}R^{L22})_pN(R^{L26})C(O)N(R^{L27})(R^{L28})$, and-$(CR^{L21}R^{L22})_pN(R^{L29})(R^{L30})$;

in some embodiments, $L_{2-2}$ is selected from $-(CR^{L21}R^{L22})_p$-OC(O)-N($R^{L23}$)$_2$,$-(CR^{L21}R^{L22})_pN(R^{L24})S(O)_2N(R^{L25})_2$, $(CR^{L21}R^{L22})_pN(R^{L26})C(O)N(R^{L27})(R^{L28})$, and-$(CR^{L21}R^{L22})_pN(R^{L29})(R^{L30})$;

in some embodiments, $L_{2-2}$ and $L_{2-2c}$ are each independently selected from

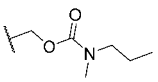, 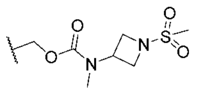, 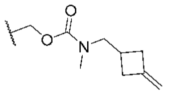, 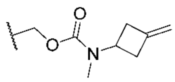, 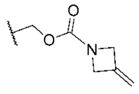,

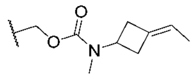, 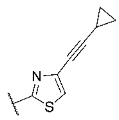, 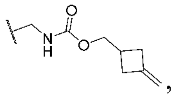, 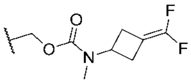, and 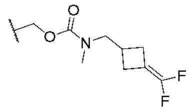;

or $L_{2-2}$ and $L_{2-2c}$ are each independently selected from

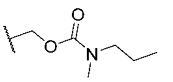,

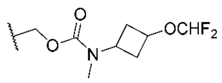, 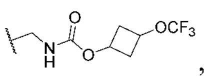, and 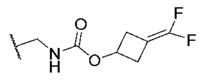;

in some embodiments, $L_{2-2}$ and $L_{2-2c}$ are each independently

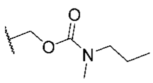;

$R^{L32}$ is selected from $-C_{1-4}$ alkyl-(5- to 10-membered heteroaryl) and $-C_{1-4}$ alkyl-(6- to 10-membered aryl), wherein the heteroaryl and aryl are optionally further substituted with 1-3 substituents selected from halogen, CN, OH, $NO_2$, $NH_2$, halogenated $C_{1-4}$ alkyl, $-OC_{1-4}$ alkyl, $C_{2-6}$ alkenyl or $C_{2-6}$ alkynyl;

in some embodiments, $R^{L32}$ is $-C_{1-4}$ alkyl-(5- to 10-membered heteroaryl), wherein the heteroaryl is optionally further substituted with 1-3 substituents selected from halogen, CN, OH, $NO_2$, $NH_2$, halogenated $C_{1-4}$ alkyl, $-OC_{1-4}$ alkyl, $C_{2-6}$ alkenyl or $C_{2-6}$ alkynyl;

in some embodiments, $R^{L32}$ is $-C_{1-4}$ alkyl-(5- to 10-membered heteroaryl), wherein the heteroaryl is optionally further substituted with 1-3 substituents selected from halogen, CN, OH, $NO_2$, $NH_2$, halogenated $C_{1-4}$ alkyl, $-OC_{1-4}$ alkyl, $C_{2-6}$ alkenyl or $C_{2-6}$ alkynyl;

in some embodiments, $R^{L32}$ is selected from $-C_{1-2}$ alkyl-(5- to 6-membered monocyclic heteroaryl) and $-C_{1-2}$ alkyl-(8- to 10-membered bicyclic heteroaryl), wherein the heteroaryl is optionally further substituted with 1-3 substituents selected from F, Cl, CN, OH, $NO_2$, $NH_2$, and halogenated $C_{1-2}$ alkyl;

in some embodiments, $R^{L32}$ is selected from $-C_{1-2}$ alkyl-(5- to 6-membered monocyclic heteroaryl) and $-C_{1-2}$ alkyl-(8- to 10-membered bicyclic heteroaryl), wherein the heteroaryl is optionally further substituted with 1-3 substituents selected from F, Cl, CN, OH, $NO_2$, $NH_2$, $-CH_2F$, $-CHF_2$, and $-CF_3$;

$R^{L33}$ is selected from $C_{1-4}$ alkyl, halogen, OH, $NH_2$, CN, $C_{2-6}$ alkenyl, $C_{2-6}$ alkynyl, -C(=O)H, -C(=O)OH, $C_{3-10}$ cycloalkyl, $C_{1-4}$ alkoxy, and 5- to 14-membered heterocyclyl, wherein the alkyl, alkoxy, alkenyl, alkynyl, cycloalkyl, and heterocyclyl are optionally further substituted with 1-4 groups selected from halogen, OH, $NH_2$, CN, $N_3$, $C_{1-4}$ alkoxy, halogenated $C_{1-4}$ alkoxy, $C_{3-5}$ cycloalkyl, 4- to 6-membered heterocycloalkyl, 6- to 10-membered aryl, and 5- to 10-membered heteroaryl;

in some embodiments, $R^{L33}$ is $C_{1-4}$ alkyl, wherein the alkyl is optionally further substituted with 1-2 groups selected from halogen, OH, $NH_2$, CN, $N_3$, $C_{1-2}$ alkoxy, fluorinated $C_{1-2}$ alkoxy, $C_{3-5}$ cycloalkyl, 4- to 6-membered heterocycloalkyl, 6- to 10-membered aryl, and 5- to 10-membered heteroaryl;

alternatively, $R^{L24}$ and $R^{L25}$ or $R^{L26}$ and $R^{L27}$, together with the atoms to which they are attached, form a 5- to 7-membered heterocyclic ring containing 1-3 heteroatoms selected from N, O and S, wherein the heterocyclic ring is optionally further substituted with 1-3 groups selected from halogen, =O, OH, $NH_2$, CN, and $C_{1-4}$ alkyl;

alternatively, $R^{L24}$ and $R^{L25}$ or $R^{L26}$ and $R^{L27}$, together with the atoms to which they are attached, form a 5- to 7-membered heterocyclic ring containing 1-3 heteroatoms selected from N, O and S, wherein the heterocyclic ring is optionally further substituted with 1-3 groups selected from halogen, =O, OH, $NH_2$, CN, $C_{1-4}$ alkyl, and acetyl;

alternatively, two $R^{L23}$, together with the atoms to which they are attached, form a 5- to 7-membered heterocyclic ring containing 1-3 heteroatoms selected from N, O and S, wherein the heterocyclic ring is optionally further substituted with 1-3 groups selected from =O, OH, $NH_2$, CN, and acetyl; each p is independently selected from 0, 1, 2, 3 and 4;

in some embodiments, p is selected from 0, 1 or 2;
in some embodiments, p is selected from 0 or 1;
provided that

(1). for formula (I-2), when $L_{2-2}$ is

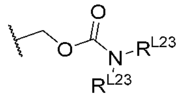,

$L_{1-1}$-A1 is not selected from

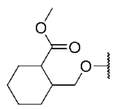, 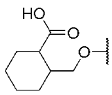, 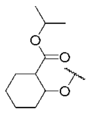, 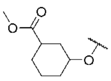, 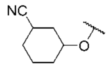, 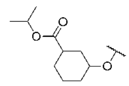, 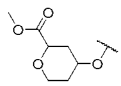, 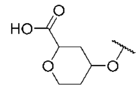,

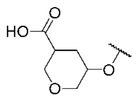, 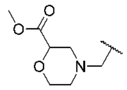, 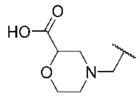, 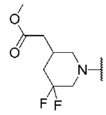, 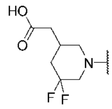, 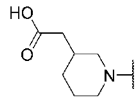, 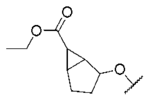,

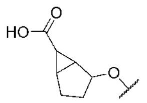, 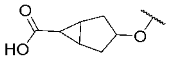, 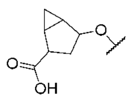, 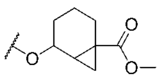, 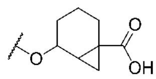, 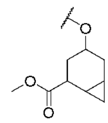,

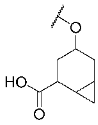, 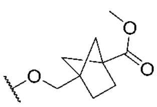, 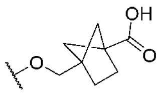, 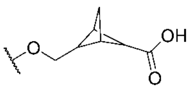, 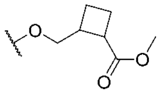, 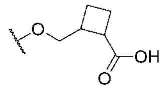,

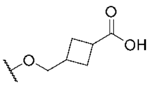, 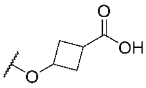, 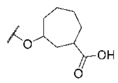, 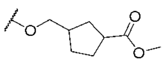, 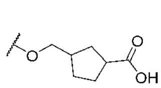, 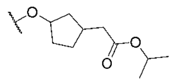,

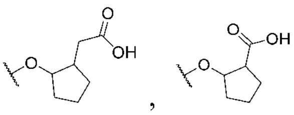, , 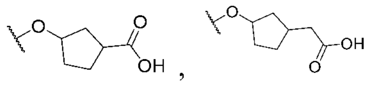, , 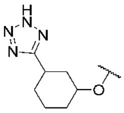, 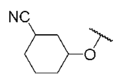,

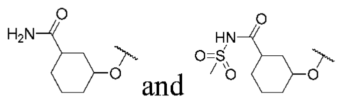 and ;

(2). for formula (I-2), when $L_{2-2}$ is

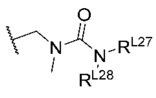,

$L_{1-1}$-A1 is not

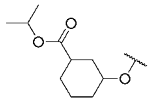;

(3). for formula (I-2b), $L_{1-1b}$-A1b is not selected from

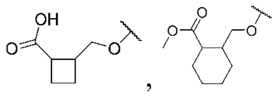,

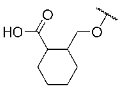, 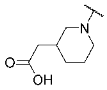, 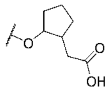, 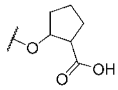, 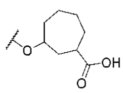, 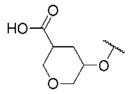, 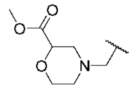,

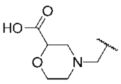, 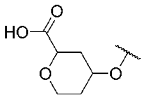, 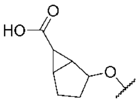, 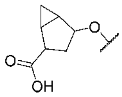, 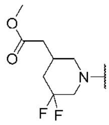, 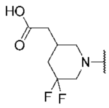, 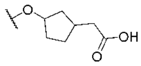,

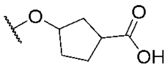, and 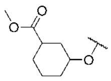;

(4). for formula (I-2c), O-Alc is not selected from

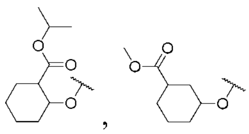,

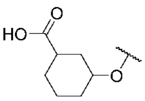, 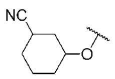, 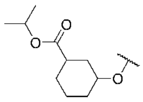, 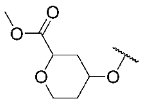, 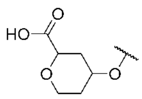, 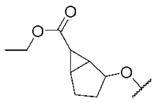,

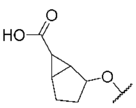, 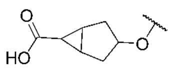, 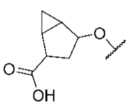, 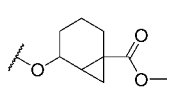, 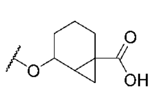, 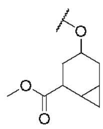,

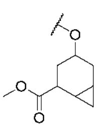, 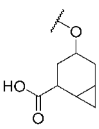, 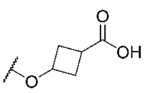, 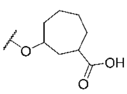, 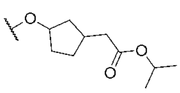, 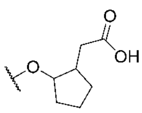, 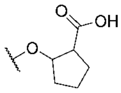,

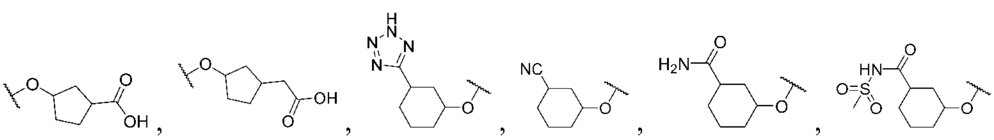,

and

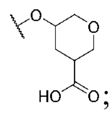;

and the compound is not

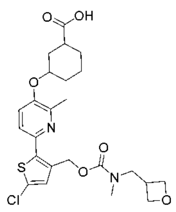, 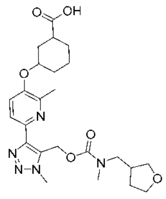, 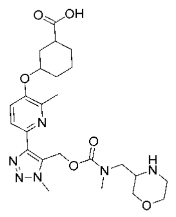

and

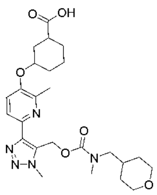.

Specifically, the first technical solution of the present invention relates to the compound as shown in formula (I), or the stereoisomer, the deuterated substance, or the pharmaceutically acceptable salt thereof, wherein

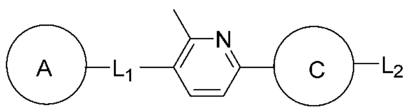

(I)

ring C is

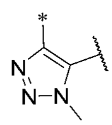,

wherein the "*" end is connected to a pyridine ring on the left side, and the "∿∿" end is connected to $L_2$; in some embodiments, ring C is selected from

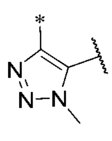 and 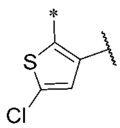,

wherein the "*" end is connected to a pyridine ring on the left side, and the "∿∿" end is connected to $L_2$;

ring A is selected from 3- to 12-membered carbocyclyl and 4- to 12-membered heterocyclyl, or ring A is absent, wherein the heterocyclyl contains 1-3 heteroatoms selected from N, O and S; the carbocyclyl and heterocyclyl are optionally further substituted with 1-4 $R^A$; in some embodiments, ring A is selected from 4- to 12-membered carbocyclyl and 4- to 12-membered heterocyclyl, wherein the heterocyclyl contains 1-3 heteroatoms selected from N, O and S; the carbocyclyl and heterocyclyl are optionally further substituted with 1-4 $R^A$;

each $R^A$ is independently selected from H, halogen, =O, CN, OH, $C_{1-6}$ alkyl, $C_{2-6}$ alkenyl, $C_{2-6}$ alkynyl, $-OC_{1-4}$ alkyl, $-(CH_2)_p$-(5- to 10-membered heterocyclyl), -(a 3- to 6-membered carbocyclic ring)-$COOR^{a1}$, $-(CH_2)_p-COOR^{a1}$, $-(CH_2)_p-C(O)NR^{a1}R^{a2}$, $-(CH_2)_p-C(O)NHC(O)R^{a1}$, $-(CH_2)_p-C(O)NHS(O)_2R^{a1}$, $-(CH_2)_p-C(O)NHS(O)R^{a1}$,

$-(CH_2)_p-S(O)_2OH$, $-(CH_2)_p-S(O)_2NHC(O)R^{a1}$, or $-(CH_2)_p-P(O)(OH)_2$, or $=CH_2$, $=CF_2$, $=CH-CH_3$, and $=C-(CH_3)_2$, wherein the heterocyclyl contains 1-3 heteroatoms selected from N, O and S; the alkyl, alkenyl, alkynyl and heterocyclyl are optionally further substituted with 1-4 groups selected from halogen, OH, $NH_2$, CN, and -O-halogenated $C_{1-4}$ alkyl; in some embodiments, each $R^A$ is independently selected from H, halogen, CN, OH, $C_{1-6}$ alkyl, $C_{2-6}$ alkenyl, $C_{2-6}$ alkynyl, $-OC_{1-4}$ alkyl, $-(CH_2)_p$-(5- to 10-membered heterocyclyl), $-(CH_2)_p-COOR^{a1}$, $-(CH_2)_p-C(O)NR^{a1}R^{a2}$, $-(CH_2)_p-C(O)NHC(O)R^{a1}$, $-(CH_2)_p-C(O)NHS(O)_2R^{a1}$, $-(CH_2)_p-C(O)NHS(O)R^{a1}$, $-(CH_2)_p-S(O)_2OH$, $-(CH_2)_p-S(O)_2NHC(O)R^{a1}$, or $-(CH_2)_p-P(O)(OH)_2$, wherein the heterocyclyl contains 1-3 heteroatoms selected from N, O and S; the alkyl, alkenyl, alkynyl and heterocyclyl are optionally further substituted with 1-4 groups selected from halogen, OH, $NH_2$, CN, and -O-halogenated $C_{1-4}$ alkyl;

$L_1$ is selected from a bond, $C_{1-6}$ alkyl, $C_{2-6}$ alkenyl, $C_{2-6}$ alkynyl, -O-, $-O-C_{1-6}$ alkyl-, -S-, $-S-C_{1-6}$ alkyl-, $-C(O)NR^{L1}-$, $-NR^{L1}C(O)-$,

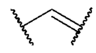,

$-NR^{L1}-$, and $-NR^{L1}-C_{1-6}$ alkyl-, wherein the alkyl, alkenyl, and alkynyl are optionally further substituted with 1-4 $R^{L1}$; in some embodiments, $L_1$ is selected from a bond, $C_{1-6}$ alkyl, $C_{2-6}$ alkenyl, $C_{2-6}$ alkynyl, -O-, $-O-C_{1-6}$ alkyl-, -S-, $-S-C_{1-6}$ alkyl-, $-C(O)NR^{L1}-$, and $-NR^{L1}C(O)-$, wherein the alkyl, alkenyl, and alkynyl are optionally further substituted with 1-4 $R^{L1}$;

each $R^{L1}$ is independently selected from H, halogen, =O, OH, $C_{1-4}$ alkyl, halogenated $C_{1-4}$ alkyl, 3- to 6-membered cycloalkyl, and -COOH, wherein the alkyl and cycloalkyl are optionally further substituted with 1-4 groups selected from halogen, OH, and $NH_2$; in some embodiments, each $R^{L1}$ is independently selected from H, halogen, =O, OH, $C_{1-4}$ alkyl, halogenated $C_{1-4}$ alkyl, and 3- to 6-membered cycloalkyl, wherein the alkyl and cycloalkyl are optionally further substituted with 1-4 groups selected from halogen, OH, and $NH_2$;

when ring A is absent, $L_1$ is selected from $C_{1-6}$ alkyl substituted with one COOH, $C_{2-6}$ alkenyl, and $C_{2-6}$ alkynyl; in some embodiments, when ring A is absent, $L_1$ is selected from $-OC_{1-6}$ alkyl substituted with one COOH, $C_{2-6}$ alkenyl, and $C_{2-6}$ alkynyl; in some embodiments, ring A is absent, and $L_1$ is selected from $-OCH_2C(CH_3)_2CH_2COOH$ and - $OCH_2CH_2C(CH_3)_2COOH$;

$L_2$ is selected from $-(CR^{L21}R^{L22})_p-OC(O)-N(R^{L23})_2$, - $(CR^{L21}R^{L22})_pN(R^{L24})S(O)_2N(R^{L25})_2$, $-(CR^{L21}R^{L22})_pN(R^{L26})C(O)N(R^{L27})(R^{L28})$,$-(CR^{L21}R^{L22})_pN(R^{L29})(R^{L30})$, $-C(=O)N(R^{L31})_2$, $-N(R^{L31})-C(=O)OR^{L32}$, $-(CH_2)_pR^{L33}$,$-(CR^{L21}R^{L22})_p-C(O)-N(R^{L29})-C(O)R^{L21}$, $-(CR^{L21}R^{L22})_p-C(O)-N(R^{L29})-S(O)_2R^{L21}$, -(3-to 6-membered cycloalkyl)-$C(=O)N(R^{L25})_2$, -(4- to 7-membered heterocyclyl)-$C(=O)N(R^{L25})_2$, -($C_{2-4}$ alkenyl)-(4- to 7-membered heterocyclyl)-$N(R^{L25})_2$, - $(CR^{L21}R^{L22})_p-SC(O)-N(R^{L25})_2$, $-(CR^{L21}R^{L22})_pN(R^{L29})C(S)N(R^{L27})(R^{L28})$,

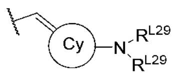,

$-(CR^{L21}R^{L22})_p-OC(O)-R^{L23}$, $-(CR^{L21}R^{L22})_p-O-R^{L23}$, and$-(CR^{L21}R^{L22})_p-N(R^{L26})C(O)O-R^{L23}$, wherein Cy is 4- to 7-membered heterocyclyl; the cycloalkyl and heterocyclyl are optionally further substituted with 1-4 groups selected from halogen, =O, OH, $NH_2$, CN, $C_{1-4}$ alkyl, $C_{1-4}$ alkoxy, halogenated $C_{1-4}$ alkyl, $C_{3-6}$ cycloalkyl, and 5- to 7-membered heterocyclyl; or

$L_2$ is selected from $-(CR^{L21}R^{L22})_p-OC(O)-N(R^{L23})_2$, - $(CR^{L21}R^{L22})_pN(R^{L24})S(O)_2N(R^{L25})_2$, $-(CR\ ^{L21}R^{L22})_pN(R^{L26})C(O)N(R^{L27})(R^{L28})$,$-(CR^{L21}R^{L22})_pN(R^{L29})(R^{L30})$, $-C(=O)N(RL^{31})_2$, $-N(R^{L31})-C(=O)OR^{L32}$, $-(CH_2)_pR^{L33}$,$-(CR^{L21}R^{L22})_p-C(O)-N(R^{L29})-C(O)R^{L21}$, $-(CR^{L21}R^{L22})_p-C(O)-N(R^{L29})-S(O)_2R^{L21}$, -(3-to 6-membered cycloalkyl)-$C(=O)N(R^{L25})_2$, -(4- to 7-membered heterocyclyl)-$C(=O)N(R^{L25})_2$, -($C_{2-4}$ alkenyl)-(4- to 7-membered heterocyclyl)-$N(R^{L25})_2$, - $(CR^{L21}R^{L22})_p-SC(O)-N(R^{L25})_2$, $-(CR^{L21}R^{L22})_pN(R^{L29})C(S)N(R^{L27})(R^{L28})$,

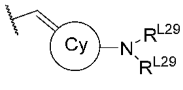,

$-(CR^{L21}R^{L22})_p-OC(O)-R^{L23}$, and $-(CR^{L21}R^{L22})_p-O-R^{L23}$, wherein Cy is 4- to 7-membered heterocyclyl; the cycloalkyl and heterocyclyl are optionally further substituted with 1-4 groups selected from halogen, =O, OH, $NH_2$, CN, $C_{1-4}$ alkyl, $C_{1-4}$ alkoxy, halogenated $C_{1-4}$ alkyl, $C_{3-6}$ cycloalkyl, and 5- to 7-membered heterocyclyl; or

$L_2$ is selected from $-(CR^{L21}R^{L22})_p-OC(O)-N(R^{L23})_2$, - $(CR^{L21}R^{L22})_pN^{L24})S(O)_2(R^{L25})_2$, $-(CR^{L21}R^{L22})_pN(R^{L26})C(O)N(R^{L27})(R^{L28})$,$-(CR^{L21}R^{L22}{}_{)p}N(R^{L21})(R^{L30})$, $-C(=O)N(R^{L31})_2$, $-N(R^{L31})-C(=O)OR^{L32}$, $-(CH_2)_pR^{L33}$,$-(CR^{L21}R^{L22})_p-C(O)-N(R^{L29})-C(O)R^{L21}$, $-(CR^{L21}R^{L22})_p-C(O)-N(R^{L21})-S(O)_2R^{L21}$, -(3-to 6-membered

cycloalkyl)-$C(=O)N(R^{L25})_2$, -(4- to 7-membered heterocyclyl)-$C(=O)N(R^{L25})_2$, -($C_{2-4}$ alkenyl)-(4- to 7-membered heterocyclyl)-$N(R^{L25})_2$, - $(CR^{L21}R^{L22})_p$-SC(O)-$N(R^{L25})_2$, -$(CR^{L21}R^{L22})_pN(R^{L29})C(S)N(R^{L27})(R^{L28})$,

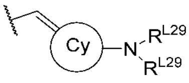 ,

and -$(CR^{L21}R^{L22})_p$-OC(O)-$R^{L23}$, wherein Cy is 4- to 7-membered heterocyclyl; the cycloalkyl and heterocyclyl are optionally further substituted with 1-4 groups selected from halogen, =O, OH, $NH_2$, CN, $C_{1-4}$ alkyl, $C_{1-4}$ alkoxy, halogenated $C_{1-4}$ alkyl, $C_{3-6}$ cycloalkyl, and 5- to 7-membered heterocyclyl; or

$L_2$ is selected from -$(CR^{L21}R^{L22})_p$-OC(O)-$N(R^{L23})_2$, - $(CR^{L21}R^{L22})_pN(R^{L24})S(O)_2N(R^{L25})_2$, -$(CR^{L21}R^{L22})_pN(R^{L26})C(O)N(R^{L27})(R^{L28})$,-$(CR^{L21}R^{L22})_pN(R^{L29})(R^{L30})$, -$C(=O)N(R^{L31})_2$, -$N(R^{L31})$-$C(=O)OR^{L32}$, -$(CH_2)_pR^{L33}$,-$(CR^{L21}R^{L22})_p$-C(O)-$N(R^{L29})$-$C(O)R^{L21}$, -$(CR^{L21}R^{L22})_p$-C(O)-$N(R^{L29})$-$S(O)_2R^{L21}$, -(3-to 6-membered cycloalkyl)-$C(=O)N(R^{L25})_2$, -(4- to 7-membered heterocyclyl)-$C(=O)N(R^{L25})_2$, -($C_{2-4}$ alkenyl)-(4- to 7-membered heterocyclyl)-$N(R^{L25})_2$, - $(CR^{L21}R^{L22})_p$-SC(O)-$N(R^{L25})_2$, -$(CR^{L21}R^{L22})_pN(R^{L29})C(S)N(R^{L27})(R^{L28})$, and

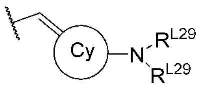 ,

wherein Cy is 4- to 7-membered heterocyclyl; the cycloalkyl and heterocyclyl are optionally further substituted with 1-4 groups selected from halogen, =O, OH, $NH_2$, CN, $C_{1-4}$ alkyl, $C_{1-4}$ alkoxy, halogenated $C_{1-4}$ alkyl, $C_{3-6}$ cycloalkyl, and 5- to 7-membered heterocyclyl;

in some embodiments, $L_2$ is selected from -$(CR^{L21}R^{L22})_p$-OC(O)-$N(R^{L23})_2$,-$(CR^{L21}R^{L22})_pN^{L24})S(O)_2(R^{L25})_2$, -$(CR^{L21}R^{L22})_pN(R^{L26})C(O)N(R^{L27})(R^{L28})$,-$(CR^{L21}R^{L22})_pN(R^{L29})(R^{L30})$, -$C(=O)N(R^{L31})_2$, -$N(R^{L31})$-$C(=O)OR^{L32}$, and-$(CH_2)_pR^{L33}$;

$R^{a1}$, $R^{a2}$, $R^{L1}$, $R^{L2}$, $R^{L21}$, $R^{L22}$, $R^{L23}$, $R^{L24}$, $R^{L25}$, $R^{L26}$, $R^{L27}$, $R^{L28}$, $R^{L29}$, $R^{L30}$, and $R^{L31}$ are each independently selected from H, halogen, $C_{1-4}$ alkyl, $C_{2-6}$ alkenyl, $C_{2-6}$ alkynyl, $C_{1-4}$ alkoxy, 5- to 14-membered heterocyclyl, -$(CH_2)_p$-(4- to 7-membered heterocyclyl), -$(CH_2)_p$-$C_{3-10}$ cycloalkyl, -$(CH_2)_p$-O-$C_{3-10}$ cycloalkyl, and -O-$C_{3-10}$ cycloalkyl, wherein the heterocyclyl contains 1-3 heteroatoms selected from N, O and S; the alkyl, alkenyl, alkynyl, cycloalkyl, and heterocyclyl are optionally further substituted with 1-4 groups selected from halogen, =O, =$CH_2$, =$CF_2$, =CH-$CH_3$, =C-$(CH_3)_2$, OH, $NH_2$, CN, acetyl, -$SCF_3$, -$S(O)_2CH_3$, $C_{1-4}$ alkyl, $C_{1-4}$ alkoxy, halogenated $C_{1-4}$ alkoxy, halogenated $C_{1-4}$ alkyl, $C_{3-10}$ cycloalkyl, 5- to 12-membered heterocyclyl, and -C≡C-$C_{3-10}$ cycloalkyl; or

$R^{a1}$, $R^{a2}$, $R^{L1}$, $R^{L2}$, $R^{L21}$, $R^{L22}$, $R^{L23}$, $R^{L24}$, $R^{L25}$, $R^{L26}$, $R^{L27}$, $R^{L28}$, $R^{L29}$, $R^{L30}$, and $R^{L31}$ are each independently selected from H, halogen, $C_{1-4}$ alkyl, $C_{2-6}$ alkenyl, $C_{2-6}$ alkynyl, $C_{1-4}$ alkoxy, 5- to 14-membered heterocyclyl, -$(CH_2)_p$-(4- to 7-membered heterocyclyl), and -$(CH_2)_p$-$C_{3-10}$ cycloalkyl, wherein the heterocyclyl contains 1-3 heteroatoms selected from N, O and S; the alkyl, alkenyl, alkynyl, cycloalkyl, and heterocyclyl are optionally further substituted with 1-4 groups selected from halogen, =O, =$CH_2$, =$CF_2$, =CH-$CH_3$, =C-$(CH_3)_2$, OH, $NH_2$, CN, acetyl, -$S(O)_2CH_3$, $C_{1-4}$ alkyl, $C_{1-4}$ alkoxy, halogenated $C_{1-4}$ alkyl, $C_{3-10}$ cycloalkyl, 5- to 12-membered heterocyclyl, and -C≡C-$C_{3-10}$ cycloalkyl; or

$R^{a1}$, $R^{a25}$, $R^{L1}$, $R^{L2}$, $R^{L21}$, $R^{L22}$, $R^{L23}$, $R^{L24}$, $R^{L25}$, $R^{L26}$, $R^{L27}$, $R^{L28}$, $R^{L29}$, $R^{L30}$, and $R^{L31}$ are each independently selected from H, halogen, $C_{1-4}$ alkyl, $C_{2-6}$ alkenyl, $C_{2-6}$ alkynyl, $C_{3-10}$ cycloalkyl, $C_{1-4}$ alkoxy, 5- to 14-membered heterocyclyl, -$(CH_2)_p$-(4-to 7-membered heterocyclyl),

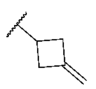 , 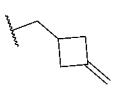 , and 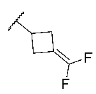 ,

wherein the heterocyclyl contains 1-3 heteroatoms selected from N, O and S; the alkyl, alkenyl, alkynyl, cycloalkyl, and heterocyclyl are optionally further substituted with 1-4 groups selected from halogen, =O, OH, $NH_2$, CN, acetyl, -$S(O)_2CH_3$, $C_{1-4}$ alkyl, $C_{1-4}$ alkoxy, halogenated $C_{1-4}$ alkyl, $C_{3-10}$ cycloalkyl, and 5- to 12-membered heterocyclyl;

in some embodiments, $R^{a1}$, $R^{a2}$, $R^{L1}$, $R^{L2}$, $R^{L21}$, $R^{L22}$, $R^{L23}$, $R^{L24}$, $R^{L25}$, $R^{L26}$, $R^{L27}$, $R^{L28}$, $R^{L29}$, $R^{L30}$, and $R^{L31}$ are each independently selected from H, halogen, $C_{1-4}$ alkyl, $C_{2-6}$ alkenyl, $C_{2-6}$ alkynyl, $C_{3-10}$ cycloalkyl, $C_{1-4}$ alkoxy, and 5- to 14-membered heterocyclyl, wherein the heterocyclyl contains 1-3 heteroatoms selected from N, O and S; the alkyl, alkenyl, alkynyl, cycloalkyl, and heterocyclyl are optionally further substituted with 1-4 groups selected from halogen, =O, OH, $NH_2$, CN, acetyl, $C_{1-4}$ alkyl, $C_{1-4}$ alkoxy, halogenated $C_{1-4}$ alkyl, $C_{3-10}$ cycloalkyl, and 5- to 12-membered heterocyclyl;

in some embodiments, $R^{a1}$, $R^{a2}$, $R^{L1}$, $R^{L2}$, $R^{L21}$, $R^{L22}$, $R^{L23}$, $R^{L24}$, $R^{L25}$, $R^{L26}$, $R^{L27}$, $R^{L28}$, $R^{L29}$, $R^{L30}$, and $R^{L31}$ are each

independently selected from H, halogen, $C_{1-4}$ alkyl, $C_{2-6}$ alkenyl, $C_{2-6}$ alkynyl, $C_{1-4}$ alkoxy, 5- to 14-membered heterocyclyl, $-(CH_2)_p$-(4- to 7-membered heterocyclyl), and $-(CH_2)_p-C_{3-10}$ cycloalkyl, wherein the heterocyclyl contains 1-3 heteroatoms selected from N, O and S; the alkyl, alkenyl, alkynyl, cycloalkyl, and heterocyclyl are optionally further substituted with 1-4 groups selected from halogen, =O, $=CH_2$, $=CF_2$, $=CH-CH_3$, $=C-(CH_3)_2$, OH, $NH_2$, CN, acetyl, $-S(O)_2CH_3$, $C_{1-4}$ alkyl, $C_{1-4}$ alkoxy, halogenated $C_{1-4}$ alkyl, $C_{3-10}$ cycloalkyl, and 5- to 12-membered heterocyclyl;

$R^{L32}$ is selected from $-C_{1-4}$ alkyl-(5- to 10-membered heteroaryl) and $-C_{1-4}$ alkyl-(6- to 10-membered aryl), wherein the heteroaryl and aryl are optionally further substituted with 1-3 substituents selected from halogen, CN, OH, $NO_2$, $NH_2$, halogenated $C_{1-4}$ alkyl, $-OC_{1-4}$ alkyl, $C_{2-6}$ alkenyl or $C_{2-6}$ alkynyl; in some embodiments, $R^{L32}$ is $-C_{1-4}$ alkyl-(5- to 10-membered heteroaryl), wherein the heteroaryl is optionally further substituted with 1-3 substituents selected from halogen, CN, OH, $NO_2$, $NH_2$, halogenated $C_{1-4}$ alkyl, $-OC_{1-4}$ alkyl, $C_{2-6}$ alkenyl or $C_{2-6}$ alkynyl;

$-(CH_2)_{pR}$L33, is selected from $C_{1-4}$ alkyl, halogen, OH, $NH_2$, CN, $C_{2-6}$ alkenyl, $C_{2-6}$ alkynyl, -C(=O)H, -C(=O)OH, $C_{3-10}$ cycloalkyl, $C_{1-4}$ alkoxy, and 5- to 14-membered heterocyclyl, wherein the alkyl, alkoxy, alkenyl, alkynyl, cycloalkyl, and heterocyclyl are optionally further substituted with 1-4 groups selected from halogen, OH, $NH_2$, CN, $N_3$, $C_{1-4}$ alkoxy, halogenated $C_{1-4}$ alkoxy, $C_{3-5}$ cycloalkyl, 4- to 6-membered heterocycloalkyl, 6- to 10-membered aryl, and 5- to 10-membered heteroaryl;

alternatively, $R^{L24}$ and $R^{L25}$ or $R^{L26}$ and $R^{L27}$, together with the atoms to which they are attached, form a 5- to 7-membered heterocyclic ring containing 1-3 heteroatoms selected from N, O and S, wherein the heterocyclic ring is optionally further substituted with 1-3 groups selected from halogen, =O, OH, $NH_2$, CN, and $C_{1-4}$ alkyl;

alternatively, $R^{L24}$ and $R^{L25}$ or $R^{L26}$ and $R^{L27}$ or two $R^{L23}$, together with the atoms to which they are attached, form a 5- to 7-membered heterocyclic ring containing 1-3 heteroatoms selected from N, O and S, wherein the heterocyclic ring is optionally further substituted with 1-3 groups selected from halogen, =O, OH, $NH_2$, CN, $C_{1-4}$ alkyl, and acetyl;

each p is independently selected from 0, 1, 2, 3 and 4.

The second technical solution of the present invention relates to the compound as shown in formula (I), or the stereoisomer, the deuterated substance, or the pharmaceutically acceptable salt thereof, having a structure of formula (I-1), wherein

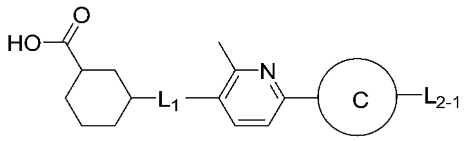

(I-1)

$L_{2-1}$ is selected from $-(CR^{L21}R^{L22})_p-OC(O)-N(R^{L23})_2$, - $(CR^{L21}R^{L22})_pN(R^{L24})S(O)_2N(R^{L25})_2$, $-(CR^{L21}R^{L22})_pN(R^{L26})C(O)N(R^{L27})(R^{L28})$, $-(CR^{L21}R^{L22})_pN(R^{L29})(R^{L30})$, $-(CR^{L21}R^{L22})_p-C(O)-N(R^{L21})-C(O)R^{L21}$, $-(CR^{L21}R^{L22})_p-C(O)-N(R^{L29})-S(O)_2R^{L21}$, -(3- to 6-membered cycloalkyl)-$C(=O)N(R^{L25})_2$, -(4- to 7-membered heterocyclyl)-C(=O)N($R^{L25}$)2, -($C_{2-4}$ alkenyl)-(4- to 7-membered heterocyclyl)-$N(R^{L25})_2$, $-(CR^{L21}R^{L22})_p-SC(O)-N(R^{L25})_2$, - $(CR^{L21}R^{L22})_pN(R^{L26})C(S)N(R^{L27})(R^{L28})$,

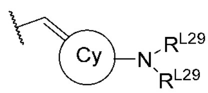 ,

$-(CR^{L21}R^{L22})_p-OC(O)-R^{L23}$, $-(CR^{L21}R^{L22})_p-O-R^{L23}$, and $-(CR^{L21}R^{L22})_p-N(R^{L26})C(O)O-R^{L23}$, wherein Cy is 4- to 7-membered heterocyclyl; the cycloalkyl and heterocyclyl are optionally further substituted with 1-4 groups selected from halogen, =O, OH, $NH_2$, CN, $C_{1-4}$ alkyl, $C_{1-4}$ alkoxy, halogenated $C_{1-4}$ alkyl, $C_{3-6}$ cycloalkyl, and 5- to 7-membered heterocyclyl; or

$L_{2-1}$ is selected from $-(CR^{L21}R^{L22})_p-OC(O)-N(R^{L23})_2$, - $(CR^{L21}R^{L22})_pN^{L24})S(O)_2(R^{L25})_2$, $-(CR^{L21}R^{L22})_pN(R^{L26})C(O)N(R^{L27})(R^{L28})$, $-(CR^{L21}R^{L22})_pN(R^{L29})(R^{L30})$, $-(CR^{L21}R^{L22})_p-C(O)-N(R^{L29})-C(O)R^{L21}$, $-(CR^{L21}R^{L22})$p-C(O)-$N(R^{L29})-S(O)_2R^{L21}$, -(3- to 6-membered cycloalkyl)-$C(=O)N(R^{L25})_2$, -(4- to 7-membered heterocyclyl)-C(=O)N($R^{L25}$)2, -($C_{2-4}$ alkenyl)-(4- to 7-membered heterocyclyl)-$N(R^{L25})_2$, $-(CR^{L21}R^{L22})_p-SC(O)-N(R^{L25})_2$, - $(CR^{L21}R^{L22})_pN(R^{L26})C(S)N(R^{L27})(R^{L28})$,

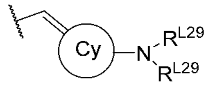,

$-(CR^{L21}R^{L22})_p-OC(O)-R^{L23}$, and $-(CR^{L21}R^{L22})_p-O-R^{L23}$, wherein Cy is 4- to 7-membered heterocyclyl; the cycloalkyl and heterocyclyl are optionally further substituted with 1-4 groups selected from halogen, =O, OH, $NH_2$, CN, $C_{1-4}$ alkyl, $C_{1-4}$ alkoxy, halogenated $C_{1-4}$ alkyl, $C_{3-6}$ cycloalkyl, and 5- to 7-membered heterocyclyl; or

$L_{2-1}$ is selected from $-(CR^{L21}R^{L22})_p-OC(O)-N(R^{L23})_2$, - $(CR^{L21}R^{L22})_pN(R^{L24})S(O)_2N(R^{L25})_2$, $-(CR^{L21}R^{L22})_pN(R^{L26})C(O)N(R^{L27})(R^{L28})$,$-(CR^{L21}R^{L22})_pN(R^{L29})(R^{L30})$, $-(CR^{L21}R^{L22})_p-C(O)-N(R^{L29})-C(O)R^{L21}$, $-(CR^{L21}R^{L22})_p-C(O)-N(R^{L29})-S(O)_2R^{L21}$, -(3- to 6-membered cycloalkyl)-$C(=O)N(R^{L25})_2$, -(4- to 7-membered heterocyclyl)-$C(=O)N(R^{L25})2$, -($C_{2-4}$ alkenyl)-(4- to 7-membered heterocyclyl)-$N(R^{L25})_2$, $-(CR^{L21}R^{L22})_p-SC(O)-N(R^{L25})_2$, - $(CR^{L21}R^{L22})_pN(R^{L26})C(S)N(R^{L27})(R^{L28})$,

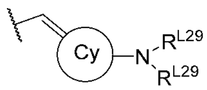,

and $-(CR^{L21}R^{L22})_p-OC(O)-R^{L23}$, wherein Cy is 4- to 7-membered heterocyclyl; the cycloalkyl and heterocyclyl are optionally further substituted with 1-4 groups selected from halogen, =O, OH, $NH_2$, CN, $C_{1-4}$ alkyl, $C_{1-4}$ alkoxy, halogenated $C_{1-4}$ alkyl, $C_{3-6}$ cycloalkyl, and 5- to 7-membered heterocyclyl; or

$L_{2-1}$ is selected from $-(CR^{L21}R^{L22})_p-OC(O)-N(R^{L23})_2$, - $(CR^{L21}R^{L22})_pN(R^{L24})S(O)_2N(R^{L25})_2$, $-(CR^{L21}R^{L22})_pN(R^{L26})C(O)N(R^{L27})(R^{L28})$,$-(CR^{L21}R^{L22})_pN(R^{L29})(R^{L30})$, $-(CR^{L21}R^{L22})_p-C(O)-N(R^{L29})-C(O)R^{L21}$, $-(CR^{L21}R^{L22})_p-C(O)-N(R^{L29})-S(O)_2R^{L21}$, -(3- to 6-membered cycloalkyl)-$C(=O)N(R^{L25})_2$, -(4- to 7-membered heterocyclyl)-$C(=O)N(R^{L25})2$, -($C_{2-4}$ alkenyl)-(4- to 7-membered heterocyclyl)-$N(R^{L25})_2$, $-(CR^{L21}R^{L22})_p-SC(O)-N(R^{L25})_2$, - $(CR^{L21}R^{L22})_pN(R^{L26})C(S)N(R^{L27})(R^{L28})$, and

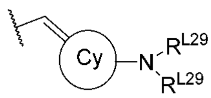,

wherein Cy is 4- to 7-membered heterocyclyl; the cycloalkyl and heterocyclyl are optionally further substituted with 1-4 groups selected from halogen, =O, OH, $NH_2$, CN, $C_{1-4}$ alkyl, $C_{1-4}$ alkoxy, halogenated $C_{1-4}$ alkyl, $C_{3-6}$ cycloalkyl, and 5- to 7-membered heterocyclyl; in some embodiments, $L_{2-1}$ is selected from $-(CR^{L21}R^{L22})_p-OC(O)-N(R^{L23})_2$, $-(CR^{L21}R^{L22})_pN(R^{L24})S(O)_2N(R^{L25})_2$,$-(CR^{L21}R^{L22})_pN(R^{L26})C(O)N(R^{L27})(R^{L28})$, and $-(CR^{L21}R^{L22})_pN(R^{L29})(R^{L30})$;

$R^{L21}$ and $R^{L22}$ are each independently selected from H, $C_{1-4}$ alkyl, $C_{2-6}$ alkenyl, $C_{2-6}$ alkynyl, $C_{3-10}$ cycloalkyl, and 5- to 12-membered heterocyclyl, wherein the heterocyclyl contains 1-3 heteroatoms selected from N, O and S; the alkyl, alkenyl, alkynyl, cycloalkyl, and heterocyclyl are optionally further substituted with 1-4 groups selected from halogen, =O, OH, $NH_2$, CN, and $C_{1-4}$ alkyl;

each $R^{L23}$ is independently selected from $C_{1-4}$ alkyl, $-(CH_2)_p-O-C_{3-6}$ cycloalkyl, $C_{3-6}$ cycloalkyl, $-O-C_{3-6}$ cycloalkyl, $-(CH_2)_p$-(4- to 7-membered heterocycloalkyl), 6- to 12-membered bicyclic heterocycloalkyl,

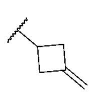, 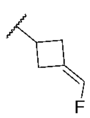, 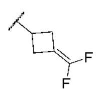, 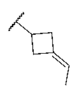,

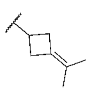, 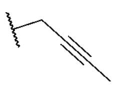, 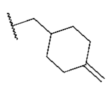, 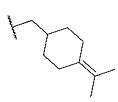, 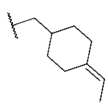, 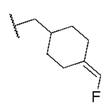, 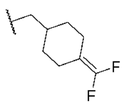, 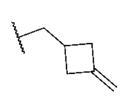,

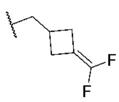, 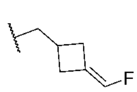, 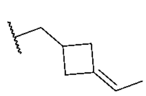, 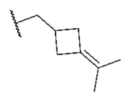,

and 5- to 6-membered heteroaryl, wherein the heterocycloalkyl and heteroaryl contain 1-3 heteroatoms selected from N, O, S, S(O), and $S(O)_2$; the alkyl, heterocycloalkyl and heteroaryl are optionally further substituted with 1-4 groups

selected from =O, halogen, OH, $-OCF_3$, - $OCHF_2$, $-SCF_3$, $NH_2$, CN, acetyl, $-S(O)_2CH_3$, and

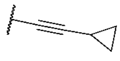;

and when $R^{L21}$ and $R^{L22}$ are both H, and $L_1$ is O, at least one $R^{L23}$ is selected from $-(CH_2)_p$-(4- to 7-membered heterocycloalkyl), 6- to 12-membered bicyclic heterocycloalkyl, 5- to 6-membered heteroaryl,

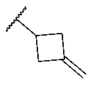, 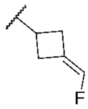, 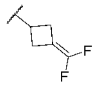, 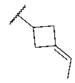,

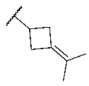, 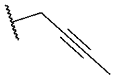, 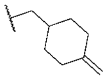, 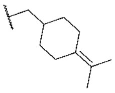, 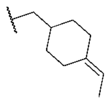, 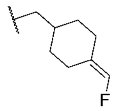, 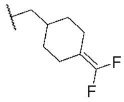, 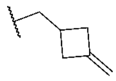,

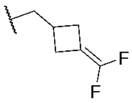, 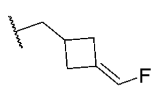, 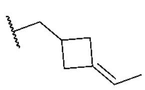, 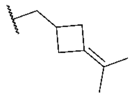,

$C_{1-4}$ alkyl substituted with 1-4 groups selected from $-OCF_3$, $-OCHF_2$, $-SCF_3$, $NH_2$, CN, acetyl, $-S(O)_2CH_3$, and

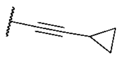,

$-O-C_{3-6}$ cycloalkyl, $-(CH_2)_p-O-C_{3-6}$ cycloalkyl, and $C_{3-6}$ cycloalkyl substituted with 1-4 groups selected from $-OCF_3$, $-OCHF_2$, $-SCF_3$, $NH_2$, CN, acetyl, $-S(O)_2CH_3$, and

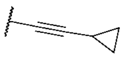,

wherein the heterocycloalkyl and heteroaryl are optionally further substituted with 1-4 groups selected from =O, halogen, OH, - $OCF_3$, $-OCHF_2$, $-SCF_3$, $NH_2$, CN, acetyl, $-S(O)_2CH_3$, and

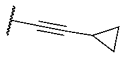;

or

each $R^{L23}$ is independently selected from $C_{1-4}$ alkyl, $-(CH_2)_p$-(4- to 7-membered heterocycloalkyl), 6- to 12-membered bicyclic heterocycloalkyl,

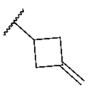,

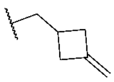, 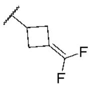, 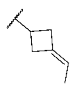, 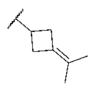,

and 5- to 6-membered heteroaryl, wherein the heterocycloalkyl and heteroaryl contain 1-3 heteroatoms selected from N, O and S; the alkyl, heterocycloalkyl, and heteroaryl are optionally further substituted with 1-4 groups selected from =O, halogen, OH, $NH_2$, CN, acetyl, $-S(O)_2CH_3$, and

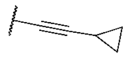;

and when $R^{L21}$ and $R^{L22}$ are both H, at least one $R^{L23}$ is selected from - $(CH_2)_p$-(4- to 7-membered heterocycloalkyl), 6- to 12-membered bicyclic heterocycloalkyl,

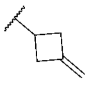, 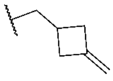, 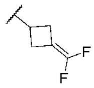, 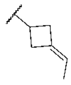, 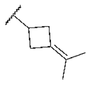,

and 5- to 6-membered heteroaryl substituted with

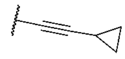;

or

each $R^{L23}$ is independently selected from $C_{1-4}$ alkyl, $-(CH_2)_p$-(4- to 7-membered heterocycloalkyl), 6- to 12-membered bicyclic heterocycloalkyl,

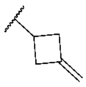,

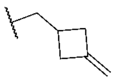, 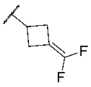, 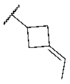, and 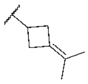,

wherein the heterocycloalkyl contains 1-3 heteroatoms selected from N, O and S; the alkyl and heterocycloalkyl are optionally further substituted with 1-4 groups selected from =O, halogen, OH, $NH_2$, CN, acetyl, and $-S(O)_2CH_3$; and when $R^{L21}$ and $R^{L22}$ are both H, at least one $R^{L23}$ is selected from $-(CH_2)_p$-(4- to 7-membered heterocycloalkyl), 6- to 12-membered bicyclic heterocycloalkyl,

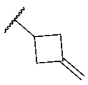, 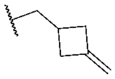, 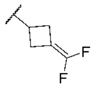, 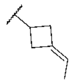, and 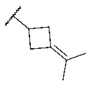;

or

each $R^{L23}$ is independently selected from $C_{1-4}$ alkyl, $-(CH_2)_p$-(4- to 7-membered heterocycloalkyl), 6- to 12-membered bicyclic heterocycloalkyl,

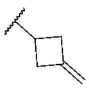,

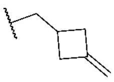, and 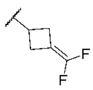,

wherein the heterocycloalkyl contains 1-3 heteroatoms selected from N, O and S; the alkyl and heterocycloalkyl are optionally further substituted with 1-4 groups selected from =O, halogen, OH, $NH_2$, CN, acetyl, and - $S(O)_2CH_3$; and when $R^{L21}$ and $R^{L22}$ are both H, at least one $R^{L23}$ is selected from - $(CH_2)_p$-(4- to 7-membered heterocycloalkyl), 6- to 12-membered bicyclic heterocycloalkyl,

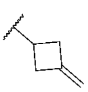, 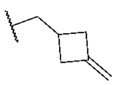, and 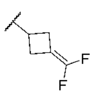;

in some embodiments, each $R^{L23}$ is independently selected from $C_{1-4}$ alkyl and 6- to 12-membered bicyclic heterocycloalkyl, wherein the heterocycloalkyl contains 1-3 heteroatoms selected from N, O and S; the alkyl and heterocycloalkyl are optionally further substituted with 1-4 groups selected from halogen, OH, $NH_2$, CN, and acetyl; and when $R^{L21}$ and $R^{L22}$ are both H, at least one $R^{L23}$ is 6- to 12-membered bicyclic heterocycloalkyl; or each $R^{L23}$ is independently selected from $C_{1-4}$ alkyl, $-(CH_2)_p$-O-$C_{3-6}$ cycloalkyl, $C_{3-6}$ cycloalkyl, -O-$C_{3-6}$ cycloalkyl, $-(CH_2)_p$-(4- to 7-membered heterocycloalkyl), 6- to 12-membered bicyclic heterocycloalkyl,

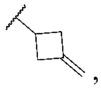, 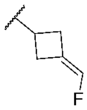, 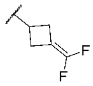, 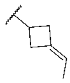,

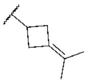, 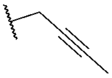, 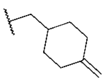, 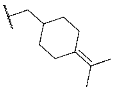, 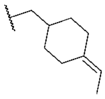, 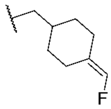, 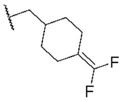, 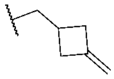,

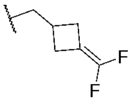, 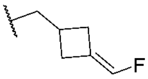, 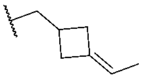, 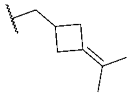, 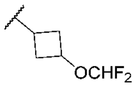, 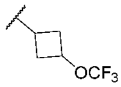,

and 5-to 6-membered heteroaryl, wherein the heterocycloalkyl and heteroaryl contain 1-3 heteroatoms selected from N, O, S, S(O) and $S(O)_2$; the alkyl, heterocycloalkyl and heteroaryl are optionally further substituted with 1-4 groups selected from =O, halogen, OH, $-OCF_3$, $-OCHF_2$, $-SCF_3$, $NH_2$, CN, acetyl, $-S(O)_2CH_3$, and

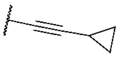;

and when $R^{L21}$ and $R^{L22}$ are both H, and $L_1$ is O, at least one $R^{L23}$ is selected from $-(CH_2)_p$-(4- to 7-membered heterocycloalkyl), 6- to 12-membered bicyclic heterocycloalkyl, 5- to 6-membered heteroaryl,

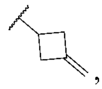, 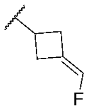, 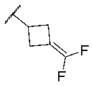, 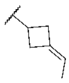,

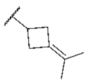, 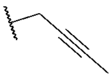, 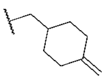, 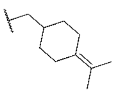, 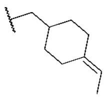, 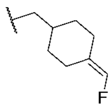, 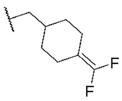, 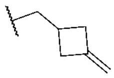,

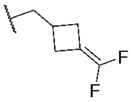, 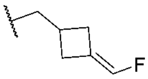, 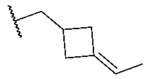, 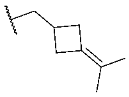, 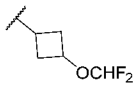, 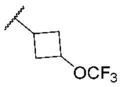, $C_{1-4}$ alkyl substituted with 1-4 groups selected from $-OCF_3$, $-OCHF_2$, $-SCF_3$, $NH_2$, CN, acetyl, $-S(O)_2CH_3$, and

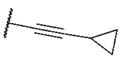,

-O-$C_{3-6}$ cycloalkyl, $-(CH_2)_p$-O-$C_{3-6}$ cycloalkyl, and $C_{3-6}$ cycloalkyl substituted with 1-4 groups selected from $-OCF_3$, $-OCHF_2$, - $SCF_3$, $NH_2$, CN, acetyl, $-S(O)_2CH_3$, and

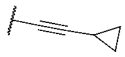,

wherein the heterocycloalkyl and heteroaryl are optionally further substituted with 1-4 groups selected from =O, halogen, OH, $-OCF_3$, $-OCHF_2$, $-SCF_3$, $NH_2$, CN, acetyl, $-S(O)_2CH_3$, and

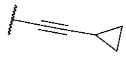;

each $R^{L24}$ is independently selected from H, $C_{2-6}$ alkenyl, and $C_{2-6}$ alkynyl, wherein the alkenyl and alkynyl are optionally further substituted with 1-4 groups selected from halogen, OH, $NH_2$, CN, $C_{3-10}$ cycloalkyl, and 5- to 12-membered heterocyclyl;

$R^{L25}$, $R^{L27}$, and $R^{L28}$ are each independently selected from $C_{1-3}$ alkyl, $C_{2-6}$ alkenyl, and $C_{2-6}$ alkynyl, wherein the alkyl, alkenyl, and alkynyl are optionally further substituted with 1-4 groups selected from halogen, OH, $NH_2$, and CN;

each $R^{L26}$ is independently selected from $C_{2-6}$ alkenyl, $C_{2-6}$ alkynyl, and $C_{3-6}$ cycloalkyl, wherein the alkenyl, alkynyl, and cycloalkyl are optionally further substituted with 1-4 groups selected from halogen, =O, OH, $NH_2$, CN, and $C_{1-4}$ alkyl;

$R^{L29}$ is selected from H, $C_{1-4}$ alkyl, $C_{2-6}$ alkenyl, and $C_{2-6}$ alkynyl, wherein the alkyl, alkenyl, and alkynyl are optionally further substituted with 1-4 groups selected from halogen, OH, $NH_2$, and CN;

$R^{L30}$ is selected from 6- to 14-membered bicyclic or tricyclic heterocyclyl, wherein the heterocyclyl contains 1-3 heteroatoms selected from N, O and S; the heterocyclyl is optionally further substituted with 1-4 groups selected from halogen, CN, OH, $NH_2$, $C_{1-4}$ alkyl, and halogenated $C_{1-4}$ alkyl;

p is selected from 0, 1, 2, 3 and 4;

alternatively, $R^{L24}$ and $R^{L25}$ or $R^{L26}$ and $R^{L27}$, together with the atoms to which they are attached, form a 5- to 7-membered heterocyclic ring containing 1-3 heteroatoms selected from N, O and S, wherein the heterocyclic ring is optionally further substituted with 1-3 groups selected from halogen, =O, OH, $NH_2$, CN, and $C_{1-4}$ alkyl;

alternatively, $R^{L24}$ and $R^{L25}$ or $R^{L26}$ and $R^{L27}$, together with the atoms to which they are attached, form a 5- to 7-membered heterocyclic ring containing 1-3 heteroatoms selected from N, O and S, wherein the heterocyclic ring is optionally further substituted with 1-3 groups selected from halogen, =O, OH, $NH_2$, CN, $C_{1-4}$ alkyl, and acetyl;

alternatively, two $R^{L23}$, together with the atoms to which they are attached, form a 5- to 7-membered heterocyclic ring containing 1-3 heteroatoms selected from N, O and S, wherein the heterocyclic ring is optionally further substituted with 1-3 groups selected from =O, OH, $NH_2$, CN, and acetyl;

provided that (1). $R^{L30}$ is not

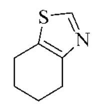, 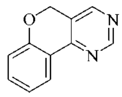 and 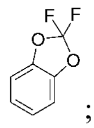;

(2). the compound is not

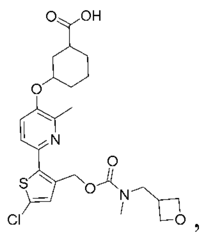, 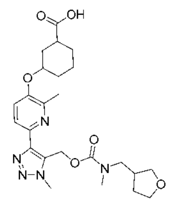, 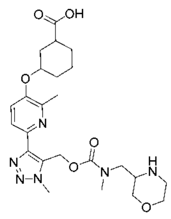

and

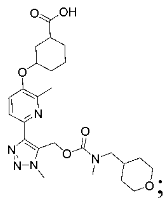;

the definitions of other groups are consistent with those of the first technical solution.

The third technical solution of the present invention relates to the compound as shown in formula (I-1), or the stereoisomer, the deuterated substance, or the pharmaceutically acceptable salt thereof, wherein

$L_{2-1}$ is selected from

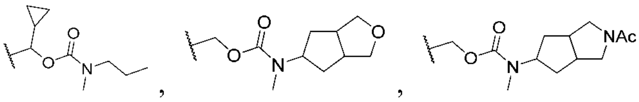,

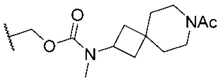, 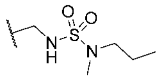, 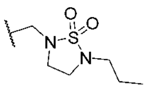, 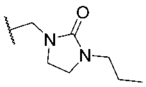, 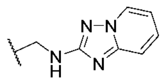,

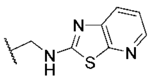, 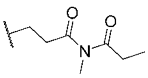, 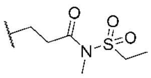, 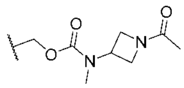, 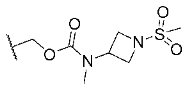,

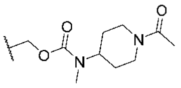, 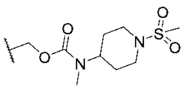, 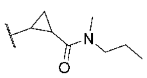, 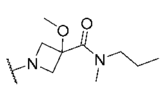, 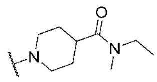,

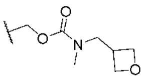, 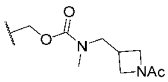, 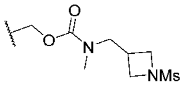, 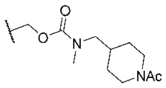, 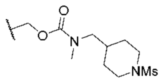,

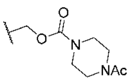, 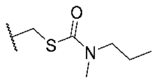, 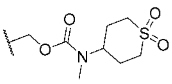, 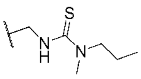, 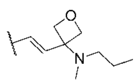, 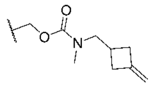,

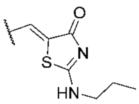, 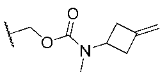, 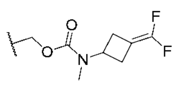, 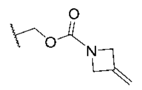, 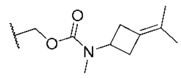,

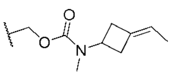, 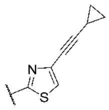, 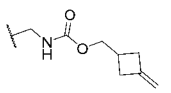, 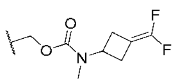, 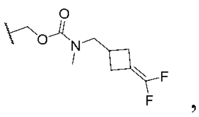,

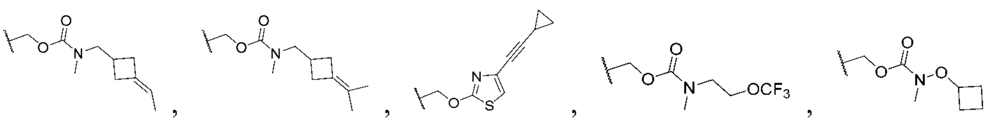,

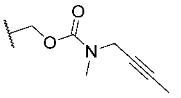, 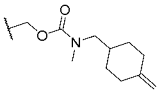, 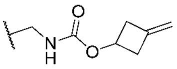, 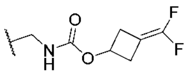, 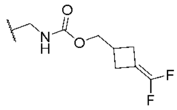,

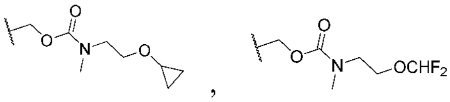, , 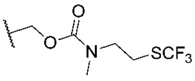, 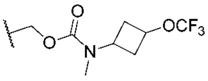,

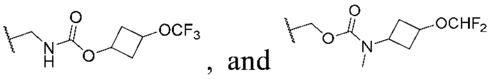, and ;

in some embodiments, $L_{2-1}$ is selected from

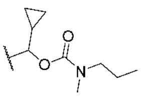, 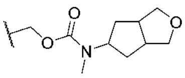, 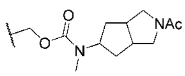, 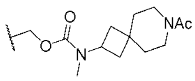, 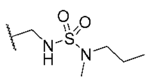,

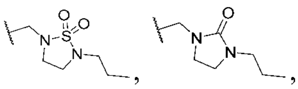, , 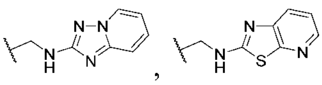, , or 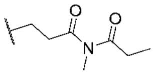, 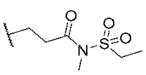,

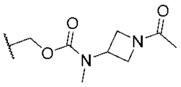, 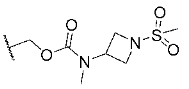, 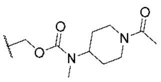, 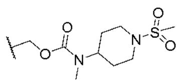, 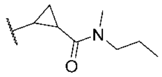,

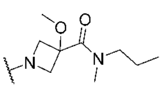, 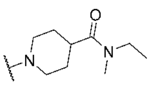, 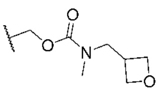, 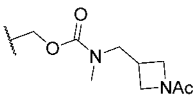, 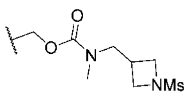,

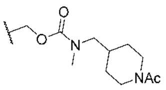, 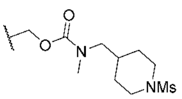, 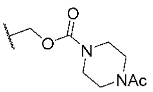, 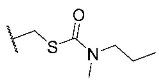, 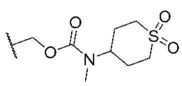,

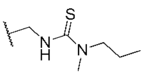, 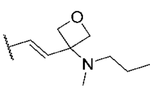, 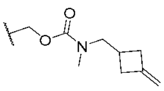, 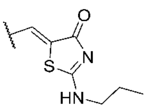, 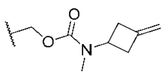,

and

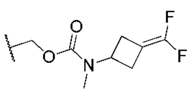;

or $L_{2-1}$ is selected from

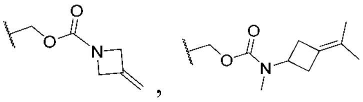, ,

and

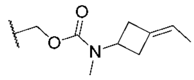;

or $L_{2-1}$ is selected from

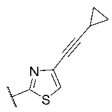, 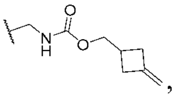, 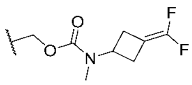,

and

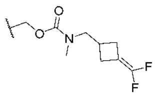;

or $L_{2-1}$ is selected from

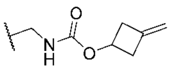, 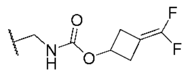,

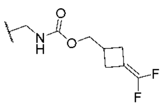, 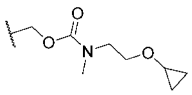, 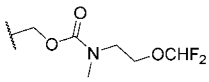, 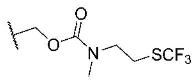,

and

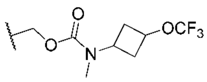;

or $L_{2-1}$ is selected from

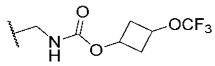 and 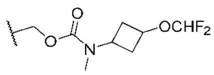;

$L_1$ is selected from a bond, $-OCH_2-$, $-OCH_2CH_2-$, -S-, $-OCH(CH_3)-$, $-OCH(CF_3)-$,

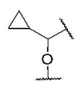,

-O-, -C(=O)-, -NHC(O)-, $-CH_2-$, -NH-, $-N(CH_3)-$, and $-NHCH(CH_3)-$; in some embodiments, $L_1$ is selected from a bond, $-OCH_2-$, -S-, $-OCH(CH_3)-$, $-OCH(CF_3)-$,

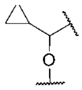,

-O-, -C(=O)-, -NHC(O)-, and $-CH_2-$;
the definitions of other groups are consistent with those of the second technical solution.

The fourth technical solution of the present invention relates to the compound as shown in formula (I), or the stereoisomer, the deuterated substance, or the pharmaceutically acceptable salt thereof, having a structure of formula (I-2), (I-2a), (I-2b), or (I-2c), wherein

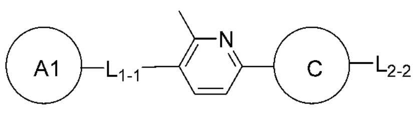

(I-2)

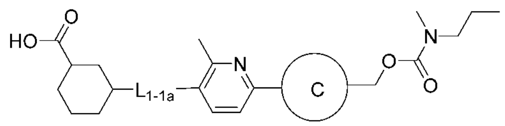

(I-2a)

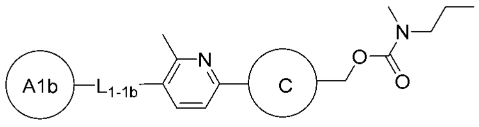

(I-2b)

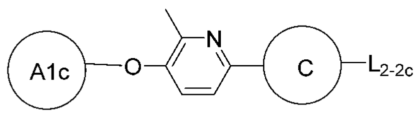

(I-2c)

rings A1, A1b, and A1c are each independently selected from 3- to 12-membered carbocyclyl and 4- to 12-membered heterocyclyl, or are absent, wherein the heterocyclyl contains 1-3 heteroatoms selected from N, O and S; the carbocyclyl and heterocyclyl are optionally further substituted with 1-4 $R^A$; in some embodiments, rings A1, A1b, and A1c are each independently selected from 4- to 12-membered carbocyclyl and 4- to 12-membered heterocyclyl, wherein the heterocyclyl contains 1-3 heteroatoms selected from N, O and S; the carbocyclyl and heterocyclyl are optionally further substituted with 1-4 $R^A$;

each $R^A$ is independently selected from H, =O, halogen, CN, OH, $C_{1-6}$ alkyl, $C_{2-6}$ alkenyl, $C_{2-6}$ alkynyl, -$OC_{1-4}$ alkyl, -$(CH_2)_p$-5- to 10-membered heterocyclyl, - (a 3- to 6-membered carbocyclic ring)-$COOR^{a1}$, -$(CH_2)_p$-$COOR^{a1}$, -$(CH_2)_p$-C(O)$NR^{a1}R^{a2}$, -$(CH_2)_p$-C(O)NHC(O)$R^{a1}$, -$(CH_2)_p$-C(O)NHS$(O)_2R^{a1}$, -$(CH_2)_p$-C(O)NHS(O)$R^{a1}$, -$(CH_2)_p$-S$(O)_2$OH, -$(CH_2)_p$-S$(O)_2$NHC(O)$R^{a1}$, or -$(CH_2)_p$-P(O)$(OH)_2$, or =$CH_2$, =$CF_2$, =CH-$CH_3$, and =C-$(CH_3)_2$, wherein the heterocyclyl contains 1-3 heteroatoms selected from N, O and S; the alkyl, alkenyl, alkynyl and heterocyclyl are optionally further substituted with 1-4 groups selected from halogen, OH, $NH_2$, CN, and -O-halogenated $C_{1-4}$ alkyl; in some embodiments, each $R^A$ is independently selected from H, halogen, CN, OH, $C_{1-6}$ alkyl, $C_{2-6}$ alkenyl, $C_{2-6}$ alkynyl, -$OC_{1-4}$ alkyl, -$(CH_2)_p$-5- to 10-membered heterocyclyl, -$(CH_2)_p$-$COOR^{a1}$, -$(CH_2)_p$-C(O)$NR^{a1}R^{a2}$, -$(CH_2)_p$-C(O)NHC(O)$R^{a1}$, -$(CH_2)_p$-C(O)NHS$(O)_2R^{a1}$, - $(CH_2)$p-C(O)NHS(O)$R^{a1}$, -$(CH_2)_p$-S$(O)_2$OH, -$(CH_2)_p$-S$(O)_2$NHC(O)$R^{a1}$, or -$(CH_2)_p$-P(O)$(OH)_2$, wherein the heterocyclyl contains 1-3 heteroatoms selected from N, O and S; the alkyl, alkenyl, alkynyl and heterocyclyl are optionally further substituted with 1-4 groups selected from halogen, OH, $NH_2$, CN, and -O-halogenated $C_{1-4}$ alkyl; and $L_{1-1}$-A1, $L_{1-1b}$-A1b, and O-Alc are not

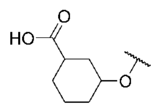;

$L_{1-1}$ and $L_{1-1b}$ are each independently selected from a bond, $C_{1-6}$ alkyl, $C_{2-6}$ alkenyl, $C_{2-6}$ alkynyl, -O-, -O-$C_{1-6}$ alkyl-, -S-, -S-$C_{1-6}$ alkyl-, -C(O)$NR^{L1}$-, - $NR^{L1}$C(O)-,

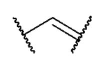,

-$NR^{L1}$-, and -$NR^{L1}$-$C_{1-6}$ alkyl-, wherein the alkyl, alkenyl, and alkynyl are optionally further substituted with 1-4 $R^{L1}$; in some embodiments, $L_{1-1}$ and $L_{1-16}$ are each independently selected from a bond, $C_{1-6}$ alkyl, $C_{2-6}$ alkenyl, $C_{2-6}$ alkynyl, -O-, -O-$C_{1-6}$ alkyl-, -S-, -S-$C_{1-6}$ alkyl-, -C(O)$NR^{L1}$-, and -$NR^{L1}$C(O)-, wherein the alkyl, alkenyl, and alkynyl are optionally further substituted with 1-4 $R^{L1}$;

$L_{1-1a}$ is selected from a bond, $C_{1-4}$ alkyl, $C_{2-4}$ alkenyl, $C_{2-4}$ alkynyl, -O-$C_{1-4}$ alkyl-, -S-, -S-$C_{1-4}$ alkyl-, -C(O)$NR^{L1}$-, -$NR^{L1}$C(O)-

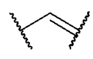,

-$NR^{L1}$-, and -$NR^{L1}$-$C_{1-6}$ alkyl-, wherein the alkyl, alkenyl, and alkynyl are optionally further substituted with 1-4 $R^{L1}$; in some embodiments, $L_{1-1a}$ is selected from a bond, $C_{1-4}$ alkyl, $C_{2-4}$ alkenyl, $C_{2-4}$ alkynyl, -O-$C_{1-4}$ alkyl-, -S-, -S-$C_{1-4}$ alkyl-, -C(O)$NR^{L1}$-, and-$NR^{L1}$C(O)-, wherein the alkyl, alkenyl, and alkynyl are optionally further substituted with 1-4

$R^{L1}$;

each $R^{L1}$ is independently selected from H, halogen, OH, $C_{1-4}$ alkyl, halogenated $C_{1-4}$ alkyl, 3- to 6-membered cycloalkyl, and -COOH, wherein the alkyl and cycloalkyl are optionally further substituted with 1-4 groups selected from halogen, OH, and $NH_2$;

in some embodiments, when rings A1 and A1b are each independently absent, $L_{1-1}$ and $L_{1-16}$ are each independently selected from -$OC_{1-6}$ alkyl substituted with one COOH, $C_{2-6}$ alkenyl, and $C_{2-6}$ alkynyl; in some embodiments, each $R^{L1}$ is independently selected from H, halogen, =O, OH, $C_{1-4}$ alkyl, halogenated $C_{1-4}$ alkyl, and 3- to 6-membered cycloalkyl, wherein the alkyl and cycloalkyl are optionally further substituted with 1-4 groups selected from halogen, OH, and $NH_2$;

$L_{2-2}$ and $L_{2-2c}$ are each independently selected from $-(CR^{L21}R^{L22})_p-OC(O)-N(R^{L23})_2$, $-(CR^{L21}R^{L22})_pN(R^{L24})S(O)_2N(R^{L25})_2$,$-(CR^{L21}R^{L22})_pN(R^{L26})C(O)N(R^{L27})(R^{L28})$, $-(CR^{L21}R^{L22})_pN(R^{L29})(R^{L30})$,$-(CR^{L21}R^{L22})_p-OC(O)-R^{L23}$, $-(CR^{L21}R^{L22})_p-O-R^{L23}$, and $-(CR^{L21}R^{L22})_p-N(R^{L26})C(O)O-R^{L23}$; or

$L_{2-2}$ and $L_{2-2c}$ are each independently selected from $-(CR^{L21}R^{L22})_p-OC(O)-N(R^{L23})_2$, $-(CR^{L21}R^{L22})_pN(R^{L24})S(O)_2N(R^{L25})_2$,$-(CR^{L21}R^{L22})_pN(R^{L26})C(O)N(R^{L27})(R^{L28})$, $-(CR^{L21}R^{L22})_pN(R^{L29})(R^{L30})$, and-$(CR^{L21}R^{L22})_p-O-C(O)-R^{L23}$; or

$L_{2-2}$ and $L_{2-2c}$ are each independently selected from $-(CR^{L21}R^{L22})_p-OC(O)-N(R^{L23})_2$, $-(CR^{L21}R^{L22})_pN(R^{L24})S(O)_2N(R^{L25})_2$,$-(CR^{L21}R^{L22})_pN(R^{L26})C(O)N(R^{L27})(R^{L28})$, and $-(CR^{L21}R^{L22})_pN(R^{L29})(R^{L30})$;

$R^{a1}$, $R^{a2}$, $R^{L1}$, $R^{L2}$, $R^{L21}$, $R^{L22}$, $R^{L23}$, $R^{L24}$, $R^{L25}$, $R^{L26}$, $R^{L27}$, and $R^{L28}$ are each independently selected from H, halogen, $C_{1-4}$ alkyl, $C_{2-6}$ alkenyl, $C_{2-6}$ alkynyl, $C_{1-4}$ alkoxy, 5- to 10-membered heterocycloalkyl, $-(CH_2)_p$-(4- to 7-membered heterocyclyl), $-(CH_2)_p-C_{3-10}$ cycloalkyl, and $-(CH_2)_p-OC_{3-10}$ cycloalkyl, wherein the heterocyclyl contains 1-3 heteroatoms selected from N, O and S; the alkyl, alkenyl, alkynyl, cycloalkyl, and heterocycloalkyl are optionally further substituted with 1-4 groups selected from halogen, =O, $=CH_2$, $=CF_2$, $=CH-CH_3$, $=C-(CH_3)_2$, OH, $NH_2$, CN, acetyl, $-S(O)_2CH_3$, $C_{1-4}$ alkyl, $C_{1-4}$ alkoxy, halogenated $C_{1-4}$ alkyl, halogenated $C_{1-4}$ alkoxy, $-SCF_3$, $C_{3-10}$ cycloalkyl, 5- to 12-membered heterocyclyl, and $-C=C-C_{3-10}$ cycloalkyl; or

$R^{a1}$, $R^{a2}$, $R^{L1}$, $R^{L2}$, $R^{L21}$, $R^{L22}$, $R^{L23}$, $R^{L24}$, $R^{L25}$, $R^{L26}$, $R^{L27}$, and $R^{L28}$ are each independently selected from H, halogen, $C_{1-4}$ alkyl, $C_{2-6}$ alkenyl, $C_{2-6}$ alkynyl, $C_{1-4}$ alkoxy, 5- to 10-membered heterocycloalkyl, $-(CH_2)_p$-(4- to 7-membered heterocyclyl), and $-(CH_2)_p-C_{3-10}$ cycloalkyl, wherein the heterocyclyl contains 1-3 heteroatoms selected from N, O and S; the alkyl, alkenyl, alkynyl, cycloalkyl, and heterocycloalkyl are optionally further substituted with 1-4 groups selected from halogen, =O, $=CH_2$, $=CF_2$, $=CH-CH_3$, $=C-(CH_3)_2$, OH, $NH_2$, CN, acetyl, - $S(O)_2CH_3$, $C_{1-4}$ alkyl, $C_{1-4}$ alkoxy, halogenated $C_{1-4}$ alkyl, $C_{3-10}$ cycloalkyl, 5- to 12-membered heterocyclyl, and $-C\equiv C-C_{3-10}$ cycloalkyl; or

$R^{a1}$, $R^{a2}$, $R^{L1}$, $R^{L2}$, $R^{L21}$, $R^{L22}$, $R^{L23}$, $R^{L24}$, $R^{L25}$, $R^{L26}$, $R^{L27}$, and $R^{L28}$ are each independently selected from H, halogen, $C_{1-4}$ alkyl, $C_{2-6}$ alkenyl, $C_{2-6}$ alkynyl, $C_{1-4}$ alkoxy, 5- to 10-membered heterocycloalkyl, $-(CH_2)_p$-(4- to 7-membered heterocyclyl), and $-(CH_2)_p-C_{3-10}$ cycloalkyl, wherein the heterocyclyl contains 1-3 heteroatoms selected from N, O and S; the alkyl, alkenyl, alkynyl, cycloalkyl, and heterocycloalkyl are optionally further substituted with 1-4 groups selected from halogen, =O, $=CH_2$, $=CF_2$, $=CH-CH_3$, $=C-(CH_3)_2$, OH, $NH_2$, CN, acetyl, - $S(O)_2CH_3$, $C_{1-4}$ alkyl, $C_{1-4}$ alkoxy, halogenated $C_{1-4}$ alkyl, $C_{3-10}$ cycloalkyl, and 5-to 12-membered heterocyclyl;

in some embodiments, $R^{a1}$, $R^{a2}$, $R^{L1}$, $R^{L2}$, $R^{L21}$, $R^{L22}$, $R^{L23}$, $R^{L24}$, $R^{L25}$, $R^{L26}$, $R^{L27}$, and $R^{L28}$ are each independently selected from H, halogen, $C_{1-4}$ alkyl, $C_{2-6}$ alkenyl, $C_{2-6}$ alkynyl, $C_{3-10}$ cycloalkyl, $C_{1-4}$ alkoxy, and 5- to 10-membered heterocycloalkyl, wherein the heterocyclyl contains 1-3 heteroatoms selected from N, O and S; the alkyl, alkenyl, alkynyl, cycloalkyl, and heterocycloalkyl are optionally further substituted with 1-4 groups selected from halogen, =O, OH, $NH_2$, CN, acetyl, $C_{1-4}$ alkyl, $C_{1-4}$ alkoxy, halogenated $C_{1-4}$ alkyl, $C_{3-10}$ cycloalkyl, and 5-to 12-membered heterocyclyl;

$R^{L29}$ is selected from H, $C_{1-4}$ alkyl, $C_{2-6}$ alkenyl, and $C_{2-6}$ alkynyl, wherein the alkyl, alkenyl, and alkynyl are optionally further substituted with 1-4 groups 1selected from halogen, OH, $NH_2$, and CN;

$R^{L30}$ is selected from 6- to 14-membered bicyclic or tricyclic heterocyclyl, wherein the heterocyclyl contains 1-3 heteroatoms selected from N, O and S; the heterocyclyl is optionally further substituted with 1-4 groups selected from halogen, CN, OH, $NH_2$, $C_{1-4}$ alkyl, and halogenated $C_{1-4}$ alkyl;

alternatively, $R^{L24}$ and $R^{L25}$ or $R^{L26}$ and $R^{L27}$, together with the atoms to which they are attached, form a 5- to 7-membered heterocyclic ring containing 1-3 heteroatoms selected from N, O and S, wherein the heterocyclic ring is optionally further substituted with 1-3 groups selected from halogen, =O, OH, $NH_2$, CN, and $C_{1-4}$ alkyl;

each p is independently selected from 0, 1, 2, 3 and 4;

provided that

(1). for formula (I-2), when $L_{2-2}$ is

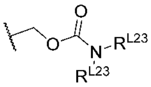,

$L_{1-1}$-A1 is not selected from

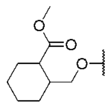, 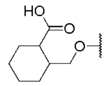, 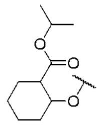, 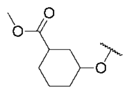, 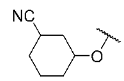, 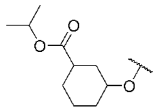, 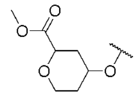,

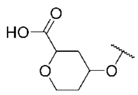, 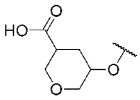, 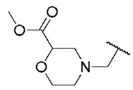, 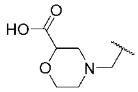, 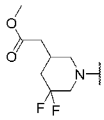, 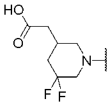, 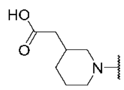,

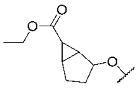, 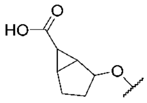, 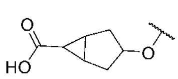, 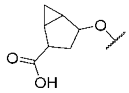, 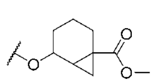, 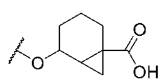,

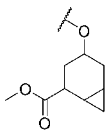, 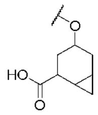, 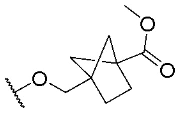, 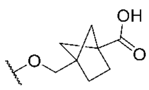, 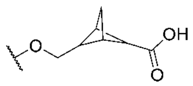, 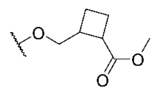,

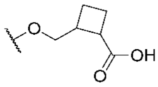, 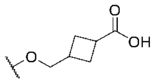, 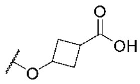, 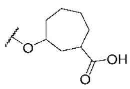, 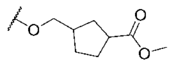, 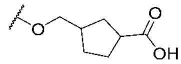,

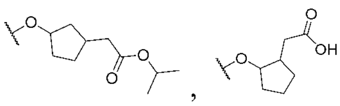, , 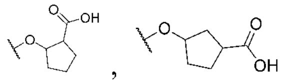, , 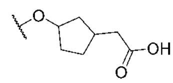, 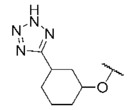,

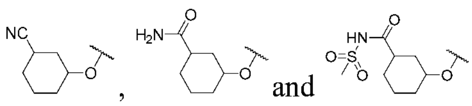, , and ;

(2). for formula (I-2), when $L_{2-2}$ is

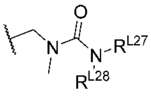,

$L_{1-1}$-A1 is not

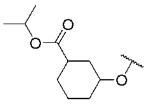;

(3). for formula (I-2b), $L_{1-16}$-A1b is not selected from

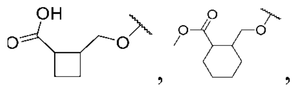, ,

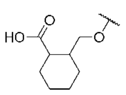, 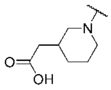, 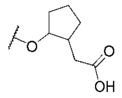, 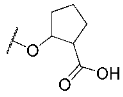, 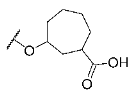, 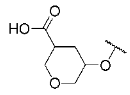, 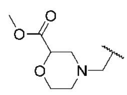,

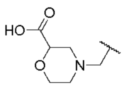, 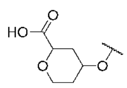, 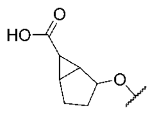, 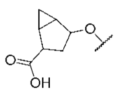, 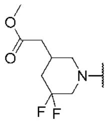, 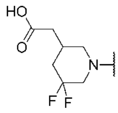, 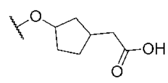,

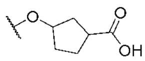, and 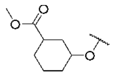;

(4). for formula (I-2c), O-A1c is not selected from

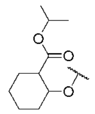, 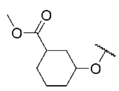,

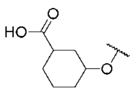, 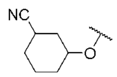, 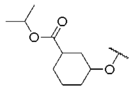, 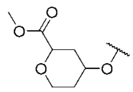, 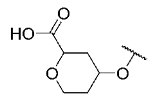, 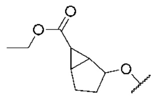,

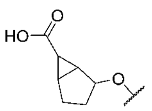, 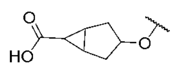, 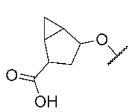, 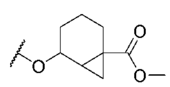, 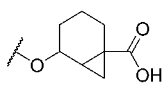, 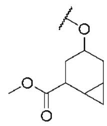,

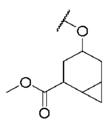, 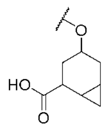, 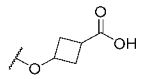, 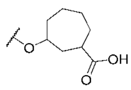, 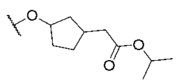, 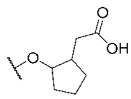, 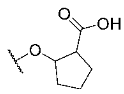,

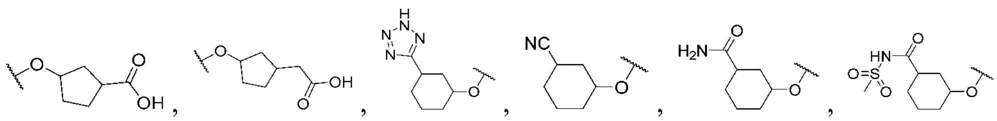 , , , , , ,

and

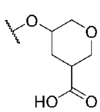;

the definitions of other groups are consistent with those of the first technical solution.

The fifth technical solution of the present invention relates to the compound as shown in formula (I-2), (I-2b), or (I-2c), or the stereoisomer, the deuterated substance, or the pharmaceutically acceptable salt thereof, wherein

rings A1, A1b, and A1c are selected from 3- to 8-membered monocyclic cycloalkyl, 6- to 12-membered fused cycloalkyl, 4- to 8-membered monocyclic heterocycloalkyl, 6- to 12-membered fused heterocycloalkyl, 6- to 10-membered spiroheterocyclyl, and 6- to 10-membered bridged heterocyclyl, or are absent, wherein the heterocycloalkyl, fused heterocycloalkyl, spiroheterocyclyl, and bridged heterocyclyl contain 1-3 heteroatoms selected from N, O and S; the cycloalkyl, heterocycloalkyl, fused heterocycloalkyl, spiroheterocyclyl, and bridged heterocyclyl are optionally further substituted with 1-4 $R^A$; in some embodiments, rings A1, A1b, and A1c are selected from 4- to 8-membered monocyclic cycloalkyl, 6- to 12-membered fused cycloalkyl, 4- to 8-membered monocyclic heterocycloalkyl, 6- to 12-membered fused heterocycloalkyl, 6- to 10-membered spiroheterocyclyl, and 6- to 10-membered bridged heterocyclyl, wherein the heterocycloalkyl, fused heterocycloalkyl, spiroheterocyclyl, and bridged heterocyclyl contain 1-3 heteroatoms selected from N, O and S; the cycloalkyl, heterocycloalkyl, fused heterocycloalkyl, spiroheterocyclyl, and bridged heterocyclyl are optionally further substituted with 1-4 $R^A$;

each $R^A$ is independently selected from H, halogen, =O, CN, OH, $C_{1-4}$ alkyl, $C_{2-4}$ alkenyl, $C_{2-4}$ alkynyl, $-OC_{1-2}$ alkyl, $-(CH_2)_p$-(5- to 8-membered heterocyclyl), - (a 3- to 6-membered carbocyclic ring)-$COOR^{a1}$, $-(CH_2)_p-COOR^{a1}$, or $=CH_2$, $=CF_2$, $=CH-CH_3$ or $=C-(CH_3)_2$, wherein the heterocyclyl contains 1-3 heteroatoms selected from N, O and S; the alkyl, alkenyl, alkynyl, and heterocyclyl are optionally further substituted with 1-4 groups selected from halogen, OH, $NH_2$, CN, and -O-halogenated $C_{1-2}$ alkyl; in some embodiments, each $R^A$ is independently selected from H, halogen, CN, OH, $C_{1-4}$ alkyl, $C_{2-4}$ alkenyl, $C_{2-4}$ alkynyl, $-OC_{1-2}$ alkyl, $-(CH_2)_p$-(5- to 8-membered heterocyclyl), and $-(CH_2)_p-COOR^{a1}$, wherein the heterocyclyl contains 1-3 heteroatoms selected from N, O and S; the alkyl, alkenyl, alkynyl, and heterocyclyl are optionally further substituted with 1-4 groups selected from halogen, OH, $NH_2$, CN, and -O-halogenated $C_{1-2}$ alkyl;

$L_{1-1}$ and $L_{1-16}$ are each independently selected from a bond, $C_{1-4}$ alkyl, $C_{2-4}$ alkenyl, $C_{2-4}$ alkynyl, -O-, $-O-C_{1-4}$ alkyl-, -S-, $-S-C_{1-4}$ alkyl-, $-C(O)NR^{L1}-$, and - $NR^{L1}C(O)-$, wherein the alkyl, alkenyl, and alkynyl are optionally further substituted with 1-4 $R^{L1}$;

each $R^{L1}$ is independently selected from H, halogen, =O, OH, $C_{1-2}$ alkyl, halogenated $C_{1-2}$ alkyl, 3- to 6-membered cycloalkyl, and -COOH, wherein the alkyl and cycloalkyl are optionally further substituted with 1-4 groups selected from halogen, OH, and $NH_2$; in some embodiments, each $R^{L1}$ is independently selected from H, halogen, =O, OH, $C_{1-2}$ alkyl, halogenated $C_{1-2}$ alkyl, and 3- to 6-membered cycloalkyl, wherein the alkyl and cycloalkyl are optionally further substituted with 1-4 groups selected from halogen, OH, and $NH_2$;

the definitions of other groups are consistent with those of the fourth technical solution.

The sixth technical solution of the present invention relates to the compound as shown in formula (I-2), or the stereoisomer, the deuterated substance, or the pharmaceutically acceptable salt thereof, wherein

rings A1, A1b, and A1c are each independently selected from one of the groups formed by the following structures optionally substituted with 1-4 $R^A$:

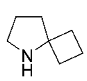, 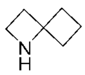, 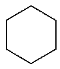, 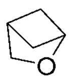, 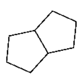, 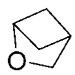, 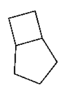, 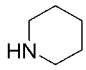, 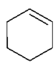, 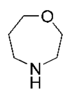, 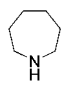,

or

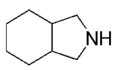, 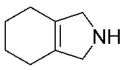, 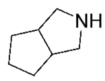, 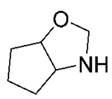, 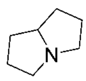, 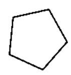, 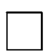, 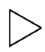, 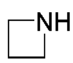, 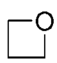,

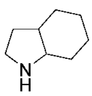, 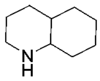, 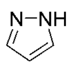, 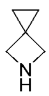, 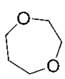, 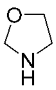, 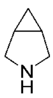, and 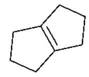;

each $R^A$ is independently selected from H, $C_{1-2}$ alkyl, halogen, CN, OH, - COOH, $-CH_2-COOH$, $-CH_2CH_2-COOH$, $=CH_2$, $=CF_2$, $=CH-CH_3$ or $=C-(CH_3)_2$; in some embodiments, each $R^A$ is independently selected from H, $C_{1-2}$ alkyl, halogen, CN, OH, -COOH, $-CH_2-COOH$, and $-CH_2CH_2-COOH$; in some embodiments, each $R^A$ is independently selected from H, halogen, CN, OH, -COOH, $-CH_2-COOH$, and $-CH_2CH_2-COOH$;

$L_{1-1}$ and $L_{1-16}$ are each independently selected from a bond, $C_{1-2}$ alkyl, $-O-C_{1-2}$ alkyl-, -S-, -O-,

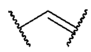,

vinyl, propenyl, ethynyl, -C(O)NH-, and -NHC(O)-, wherein the alkyl is optionally further substituted with 1-4 $R^{L1}$; in some embodiments, $L_{1-1}$ and $L_{1-16}$ are each independently selected from a bond, $C_{1-2}$ alkyl, -O-$C_{1-2}$ alkyl-, -S-, -O-, and -NHC(O)-, wherein the alkyl is optionally further substituted with 1-4 $R^{L1}$;

each $R^{L1}$ is independently selected from H, halogen, =O, OH, $C_{1-2}$ alkyl, and 3- to 6-membered cycloalkyl;

$L_{2-2}$ and $L_{2-2c}$ are each independently selected from

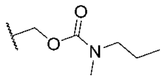,

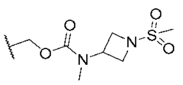, 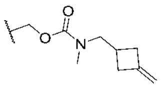, 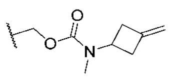, 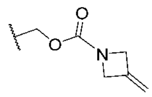, 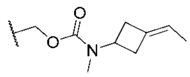,

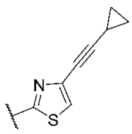, 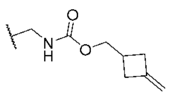, 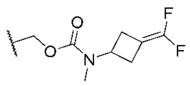, 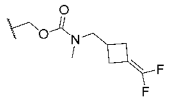, 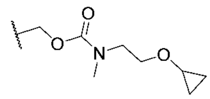,

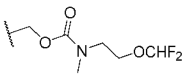, 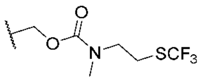, and 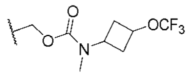;

or

$L_{2-2}$ and $L_{2-2c}$ are each independently

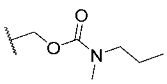;

or

$L_{2-2}$ and $L_{2-2c}$ are each independently selected from

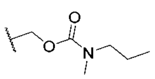,

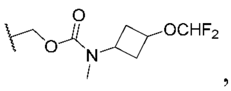, 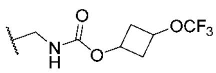, and 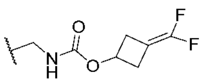;

the definitions of other groups are consistent with those of the fifth technical solution.

The seventh technical solution of the present invention relates to the compound as shown in formula (I-2), (I-2a), (I-2b), or (I-2c), or the stereoisomer, the deuterated substance, or the pharmaceutically acceptable salt thereof, wherein

rings A1, A1b, and A1c are each independently selected from the following structures:

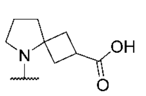, 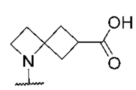, 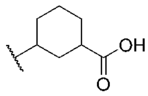, 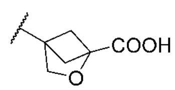, 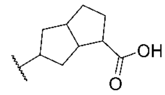,

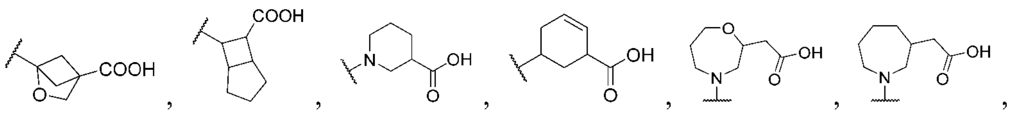 , , , , , , ,

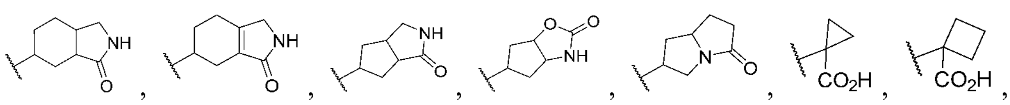 , , , , , , ,

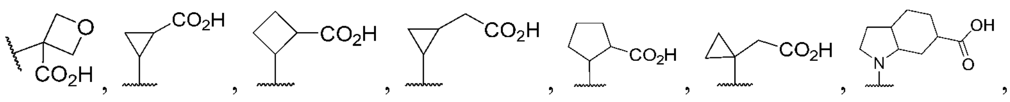 , , , , , , ,

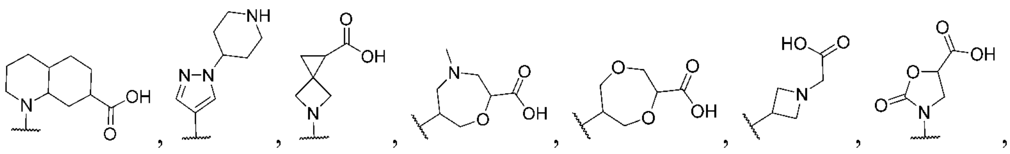 , , , , , , ,

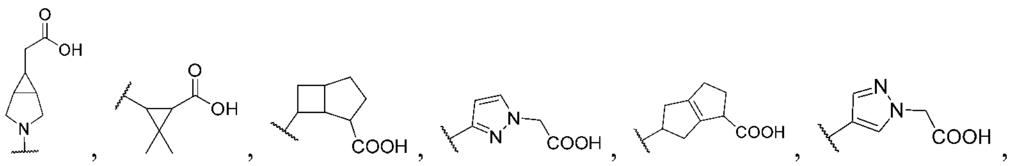 , , , , , ,

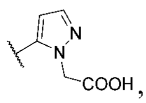 , 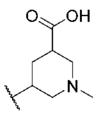 , and 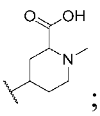 ;

or are selected from

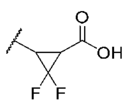 and 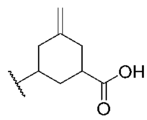 ;

or are selected from

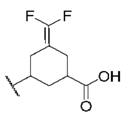 , 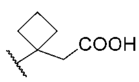 , and 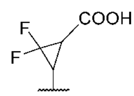 ;

$L_{1-1}$ and $L_{1-16}$ are each independently selected from a bond, $-OCH_2-$, $-OCH_2CH_2-$, -S-, $-OCH(CH_3)-$, $-OCH(CF_3)-$,

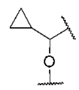 ,

-O-, -C(=O)-, $-CH_2-$, $-CH=CH-CH_2-$, ethynyl, and -NHCO-; in some embodiments, $L_{1-1}$ and $L_{1-16}$ are each independently selected from a bond, $-OCH_2-$, -S-, $-OCH(CH_3)-$, $-OCH(CF_3)-$,

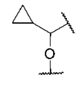 ,

-O-, -C(=O)-, and $-CH_2-$;

each $L_{1-1a}$ is independently selected from a bond, $-OCH_2-$, -S-, $-OCH(CH_3)-$, $-OCH(CF_3)-$,

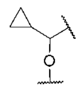,

-C(=O)-, $-CH_2-$, $-CH=CH-CH_2-$, ethynyl, -NHCO-, -NH-, $-N(CH_3)-$, and $-NHCH(CH_3)-$; in some embodiments, each $L_{1-1a}$ is independently selected from a bond, $-OCH_2-$, -S-, $-OCH(CH_3)-$, $-OCH(CF_3)-$,

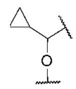,

-C(=O)-, - NHC(O)-, and $-CH_2-$;

the definitions of other groups are consistent with those of the sixth technical solution.

The eighth technical solution of the present invention relates to the compound as shown in formula (I), or the stereoisomer, the deuterated substance, or the pharmaceutically acceptable salt thereof, having a structure of formula (I-6),

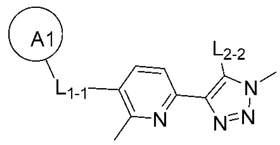

(I-6)

wherein

ring A1 is selected from 3- to 12-membered carbocyclyl and 4- to 12-membered heterocyclyl, or is absent, wherein the heterocyclyl contains 1-3 heteroatoms selected from N, O and S; the carbocyclyl and heterocyclyl are optionally further substituted with 1-4 $R^A$; in some embodiments, ring A1 is selected from 3- to 8-membered monocyclic cycloalkyl, 6- to 12-membered fused cycloalkyl, 4- to 8-membered monocyclic heterocycloalkyl, 6- to 12-membered fused heterocycloalkyl, 6- to 10-membered spiroheterocyclyl, and 6- to 10-membered bridged heterocyclyl, or ring A1 is absent, wherein the heterocycloalkyl, fused heterocycloalkyl, spiroheterocyclyl, and bridged heterocyclyl contain 1-3 heteroatoms selected from N, O and S; the cycloalkyl, heterocycloalkyl, fused heterocycloalkyl, spiroheterocyclyl, and bridged heterocyclyl are optionally further substituted with 1-4 $R^A$; in some embodiments, ring A1 is selected from 4- to 8-membered monocyclic cycloalkyl, 6- to 12-membered fused cycloalkyl, 4- to 8-membered monocyclic heterocycloalkyl, 6- to 12-membered fused heterocycloalkyl, 6- to 10-membered spiroheterocyclyl, and 6- to 10-membered bridged heterocyclyl, wherein the heterocycloalkyl, fused heterocycloalkyl, spiroheterocyclyl, and bridged heterocyclyl contain 1-3 heteroatoms selected from N, O and S; the cycloalkyl, heterocycloalkyl, fused heterocycloalkyl, spiroheterocyclyl, and bridged heterocyclyl are optionally further substituted with 1-4 $R^A$; in some embodiments, ring A1 is selected from the following structures:

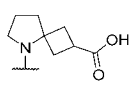, 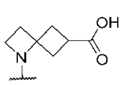, 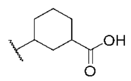, 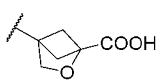, 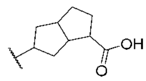, 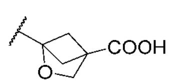,

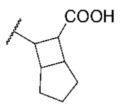, 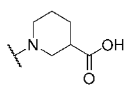, 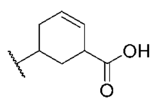, 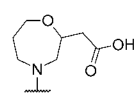, 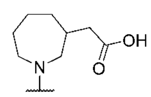, 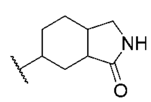,

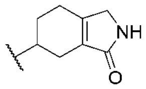, 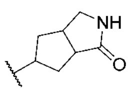, 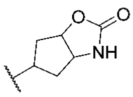, 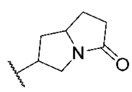, 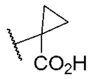, 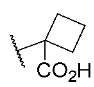, 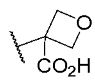,

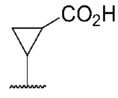, 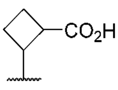, 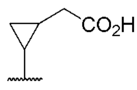, 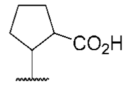, 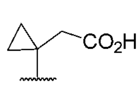, 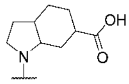, 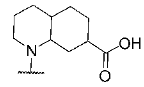,

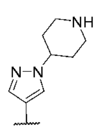, 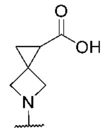, 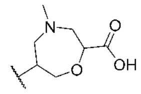, 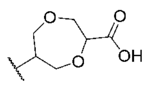, 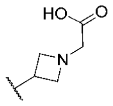, 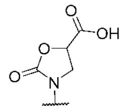, 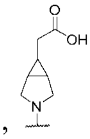, 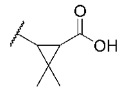,

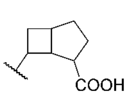, 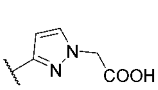, 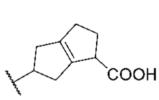, 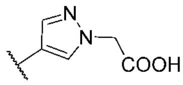, 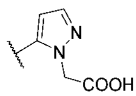, 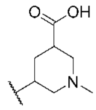,

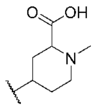, 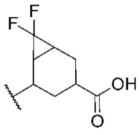, 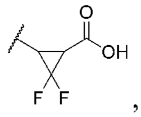, 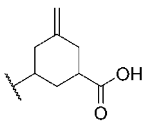, 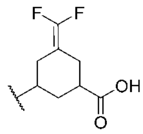, and 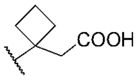;

in some embodiments, when ring A1 is absent, $L_{1-1}$ is $-OC_{1-6}$ alkyl substituted with one COOH;

$L_{1-1}$ is selected from a bond, $C_{1-6}$ alkyl, $C_{2-6}$ alkenyl, $C_{2-6}$ alkynyl, -O-, $-O-C_{1-6}$ alkyl-, -S-, $-S-C_{1-6}$ alkyl-, $-C(O)NR^{L1}-$,

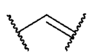,

$-NR^{L1}-$, and $-NR^{L1}-C_{1-6}$ alkyl-, wherein the alkyl, alkenyl, and alkynyl are optionally further substituted with 1-4 $R^{L1}$; in some embodiments, $L_{1-1}$ is selected from a bond, $-OCH_2-$, $-OCH_2CH_2-$, -S-, $-OCH(CH_3)-$, $-OCH(CF_3)-$,

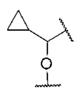,

-O-, -C(=O)-, $-CH_2-$, $-CH=CH-CH_2-$, ethynyl, and -NHCO-;

each $R^A$ is independently selected from H, halogen, CN, OH, $C_{1-6}$ alkyl, $C_{2-6}$ alkenyl, $C_{2-6}$ alkynyl, $-OC_{1-4}$ alkyl, $-(CH_2)_p$-(5- to 10-membered heterocyclyl), - $(CH_2)_p-COOR^{a1}$, $-(CH_2)_p-C(O)NR^{a1}R^{a2}$, $-(CH_2)_p-C(O)NHC(O)R^{a1}$, $-(CH_2)_p-C(O)NHS(O)_2R^{a1}$, $-(CH_2)_p-C(O)NHS(O)R^{a1}$, $-(CH_2)_p-S(O)_2OH$, $-(CH_2)_p-S(O)_2NHC(O)R^{a1}$, $-(CH_2)_p-P(O)(OH)_2$, $=CH_2$, and $=CF_2$, wherein the heterocyclyl contains 1-3 heteroatoms selected from N, O and S; the alkyl, alkenyl, alkynyl and heterocyclyl are optionally further substituted with 1-4 groups selected from halogen, OH, $NH_2$, CN, and -O-halogenated $C_{1-4}$ alkyl; in some embodiments, each $R^A$ is independently selected from H, halogen, CN, OH, -COOH, $-CH_2-COOH$, - $CH_2CH_2-COOH$ or $=CH_2$;

$L_{2-2}$ is selected from $-(CR^{L21}R^{L22})_p-OC(O)-N(R^{L23})_2$, - $(CR^{L21}R^{L22})_bN(R^{L24})S(O)_2N(R^{L25})_2$, $-(CR^{L21}R^{L22})_pN(R^{L26})C(O)N(R^{L27})(R^{L28})$, $-(CR^{L21}R^{L22})_pN(R^{L29})(R^{L30})$, $-C(=O)N(R^{L31})_2$, $-N(R^{L31})-C(=O)OR^{L32}$, $-(CH_2)_pR^{L33}$, $-(CR^{L21}R^{L22})_p-C(O)-N(R^{L29})-C(O)R^{L21}$, $-(CR^{L21}R^{L22})_p-C(O)-N(R^{L29})-S(O)_2R^{L21}$, -(3-to 6-membered cycloalkyl)$-C(=O)N(R^{L25})_2$, -(4- to 7-membered heterocyclyl)$-C(=O)N(R^{L25})_2$, $-(C_{2-4}$ alkenyl)-(4- to 7-membered heterocyclyl)$-N(R^{L25})_2$, - $(CR^{L21}R^{L22})_p-SC(O)-N(R^{L25})_2$, $-(CR^{L21}R^{L22})_pN(R^{L29})C(S)N(R^{L27})(R^{L28})$,

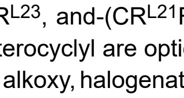,

$-(CR^{L21}R^{L22})_p-OC(O)-R^{L23}$, $-(CR^{L21}R^{L22})_p-O-R^{L23}$, and $-(CR^{L21}R^{L22})_p-N(R^{L26})C(O)O-R^{L23}$, wherein Cy is 4- to 7-membered heterocyclyl; the cycloalkyl and heterocyclyl are optionally further substituted with 1-4 groups selected from halogen, =O, OH, $NH_2$, CN, $C_{1-4}$ alkyl, $C_{1-4}$ alkoxy, halogenated $C_{1-4}$ alkyl, $C_{3-6}$ cycloalkyl, and 5- to 7-membered heterocyclyl; in some embodiments, $L_{2-2}$ is selected from $-(CR^{L21}R^{L22})_p-OC(O)-N(CH_3)R^{L23}$, $-(CR^{L21}R^{L22})_p-O-C(O)-NHR^{L23}$, and $-(CR^{L21}R^{L22})_p-N(R^{L26})C(O)O-R^{L23}$;

$R^{L23}$ is selected from $-(CH_2)_p$-(4- to 7-membered heterocyclyl) and $-(CH_2)_p-C_{3-10}$ cycloalkyl, wherein the heterocyclyl

contains 1-3 heteroatoms selected from N, O and S; the cycloalkyl and heterocyclyl are optionally further substituted with 1-4 groups selected from $=CH_2$, $=CF_2$, $=CH-CH_3$, and $=C-(CH_3)_2$;
$R^{L21}$, $R^{L22}$, $R^{L24}$, $R^{L25}$, $R^{L26}$, $R^{L27}$, $R^{L21}$, and $R^{L29}$ are each independently selected from H and $C_{1-4}$ alkyl.

The ninth technical solution of the present invention relates to the compound as shown in formula (I), or the stereoisomer, the deuterated substance, or the pharmaceutically acceptable salt thereof, having a structure of formula (I-3),

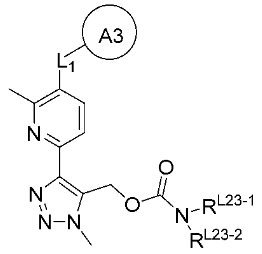

(I-3)

wherein ring A3 is selected from

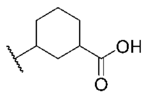 , 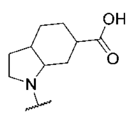 , and 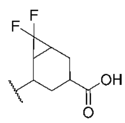 ;

$L_1$ is selected from -O-, $-N(CH_3)-$, and -NHC(O)-;
$R^{L23-1}$ is selected from methyl and ethyl;
$R^{L23-2}$ is selected from $-(CH_2)_p$-(4- to 7-membered heterocycloalkyl), $-(CH_2)_p-C_{4-6}$ monocyclic cycloalkyl, $-(CH_2)_2-O-C_{3-6}$ cycloalkyl, $-O-C_{3-6}$ cycloalkyl, and - $CH_2CH_2OCF_3$, wherein the heterocycloalkyl contains 1-2 N atoms; the heterocycloalkyl is optionally further substituted with 1-2 groups selected from - $S(O)_2CH_3$, =O, $=CH_2$, $=CF_2$, $=CH-CH_3$, and $=C-(CH_3)_2$; the cycloalkyl is further substituted with 1-2 groups selected from $=CH_2$, $=CF_2$, $=CH-CH_3$, and $=C-(CH_3)_2$; in some embodiments, $R^{L23-2}$ is selected from $-CH_2CH_2OCF_3$,

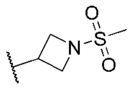 , 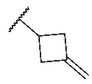 ,

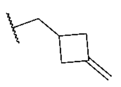 , 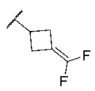 , 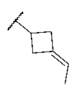 , 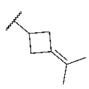 , 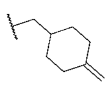 , 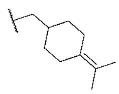 , 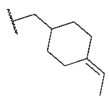 , 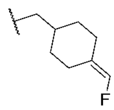 ,

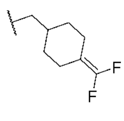 , and 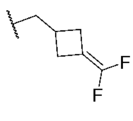 ;

p is selected from 0, 1 and 2.

The tenth technical solution of the present invention relates to the compound as shown in formula (I), or the stereoisomer, the deuterated substance, or the pharmaceutically acceptable salt thereof, having a structure of formula (I-5),

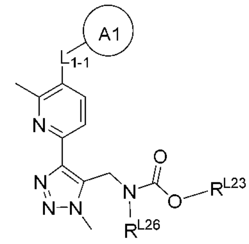

(I-5)

wherein ring A1 is selected from 4- to 8-membered monocyclic cycloalkyl, 6-to 12-membered fused cycloalkyl, 4- to 8-membered monocyclic heterocycloalkyl, 6- to 12-membered fused heterocycloalkyl, 6- to 10-membered spiroheterocyclyl, and 6- to 10-membered bridged heterocyclyl, or is absent; in some embodiments, ring A1 is selected from

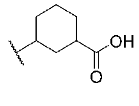, 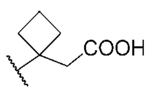, 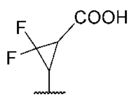, and 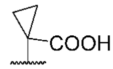,

or is absent;

$L_{1-1}$ is selected from $-OCH_2-$, $-OCH_2CH_2-$, and -O-; or

when ring A1 is absent, $L_{1-1}$ is selected from $-OCH_2CH_2C(CH_3)_2-COOH$ and $-OCH_2C(CH_3)_2CH_2-COOH$;

$R^{L26}$ is selected from H and $C_{1-4}$ alkyl;

$R^{L23}$ is selected from $-(CH_2)_p$-(4- to 7-membered heterocycloalkyl) and $-(CH_2)_p-C_{4-6}$ monocyclic cycloalkyl, wherein the heterocycloalkyl contains 1-2 N atoms; the heterocycloalkyl is optionally further substituted with 1-2 groups selected from $=CH_2$, $=CF_2$, $=CH-CH_3$, $=C-(CH_3)_2$, =CHF, and haloalkoxy; the cycloalkyl is further substituted with 1-2 groups selected from $=CH_2$, $=CH-CH_3$, $=C-(CH_3)_2$, $=CF_2$, =CHF, and haloalkoxy; in some embodiments, $R^{L23}$ is selected from

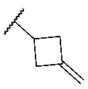, 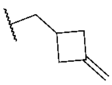, 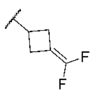, 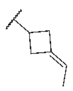, 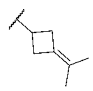, 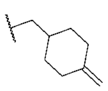, 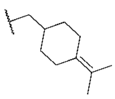, 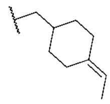,

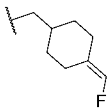, 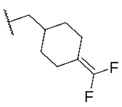, 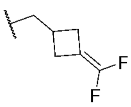, and 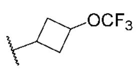;

p is selected from 0, 1 and 2.

The eleventh technical solution of the present invention relates to the compound as shown in formula (I-6), or the stereoisomer, the deuterated substance, or the pharmaceutically acceptable salt thereof,

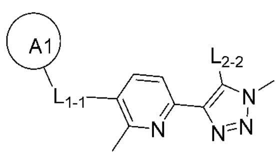

(I-6)

wherein

$L_{2-2}$ is selected from $-(CR^{L21}R^{L22})_p-OC(O)-N(CH_3)R^{L23}$, $-(CR^{L21}R^{L22})_p-OC(O)-NHR^{L23}$, and $-(CR^{L21}R^{L22})_p-N(R^{L26})C(O)O-R^{L23}$;

$R^{L21}$, $R^{L22}$, and $R^{L26}$ are each independently selected from H and $C_{1-4}$ alkyl;

$R^{L23}$ is selected from $-(CH_2)_p$-(4- to 7-membered heterocyclyl) and $-(CH_2)_p-C_{3-10}$ cycloalkyl, wherein the heterocyclyl contains 1-3 heteroatoms selected from N, O and S; the cycloalkyl and heterocyclyl are further substituted with 1-4

groups selected from $=CH_2$, $=CF_2$, $=CH-CH_3$, and $=C-(CH_3)_2$;
ring A1 is selected from 3- to 12-membered carbocyclyl and 4- to 12-membered heterocyclyl, or is absent, wherein the heterocyclyl contains 1-3 heteroatoms selected from N, O and S; the carbocyclyl and heterocyclyl are optionally further substituted with 1-4 $R^A$;
when ring A1 is absent, $L_{1-1}$ is $-OC_{1-6}$ alkyl substituted with one COOH;
$L_{1-1}$ is selected from a bond, $C_{1-6}$ alkyl, $C_{2-6}$ alkenyl, $C_{2-6}$ alkynyl, -O-, $-O-C_{1-6}$ alkyl-, -S-, $-S-C_{1-6}$ alkyl-, $-C(O)NR^{L1}-$,

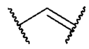,

$-NR^{L1}-$, and $-NR^{L1}-C_{1-6}$ alkyl-, wherein the alkyl, alkenyl, and alkynyl are optionally further substituted with 1-4 $R^{L1}$;
$R^A$, $R^{L1}$, and p are as defined in the first or eighth technical solution above.

More specifically, the present invention relates to the compound as shown in formula (I), or the stereoisomer, the deuterated substance, or the pharmaceutically acceptable salt thereof, wherein the compound is selected from, but not limited to, the structures listed in Table 1 and Table 2 below:

Table 1

| | | | | |
|---|---|---|---|---|
| 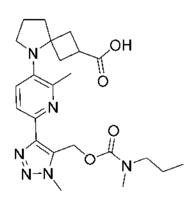 | 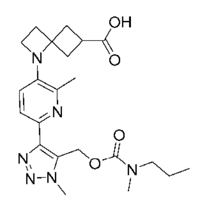 | 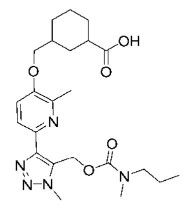 | 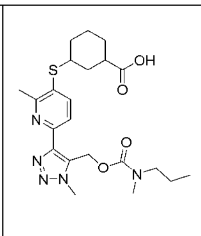 | 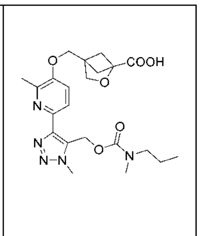 |
| 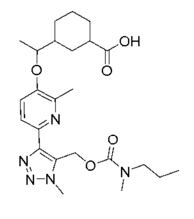 | 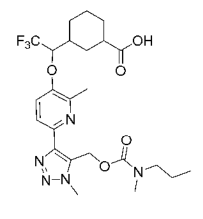 | 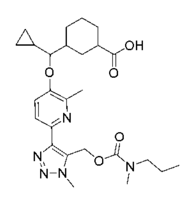 | 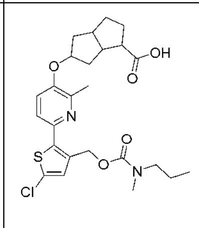 | 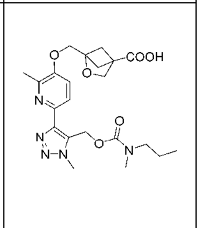 |
| 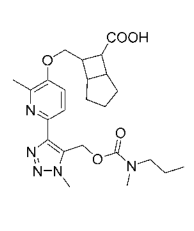 | 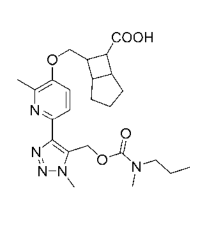 | 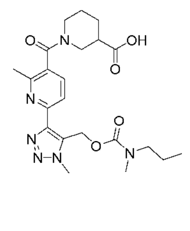 | 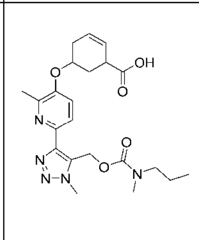 | 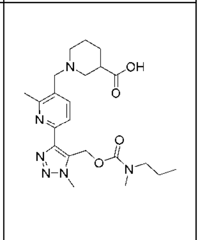 |
| 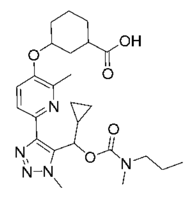 | 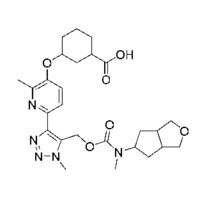 | 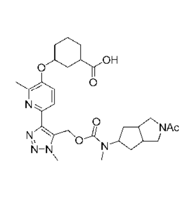 | 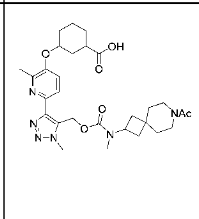 | 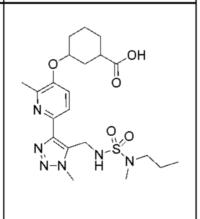 |
| 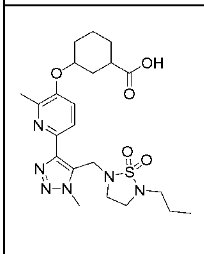 | 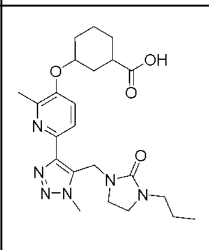 | 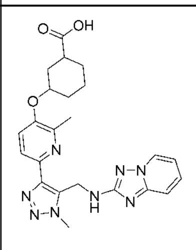 | 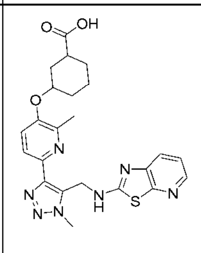 | 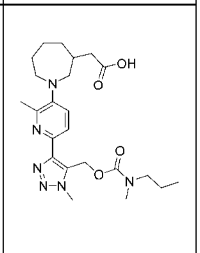 |
| 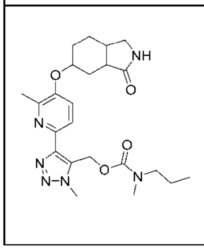 | 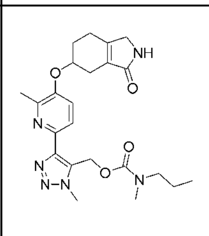 | 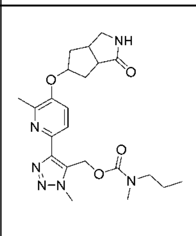 | 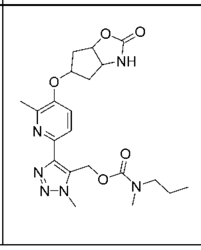 | 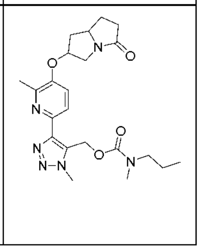 |

| | | | | |
|---|---|---|---|---|
| 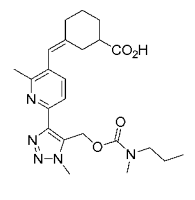 | 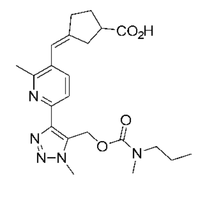 | 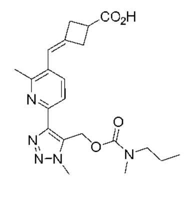 | 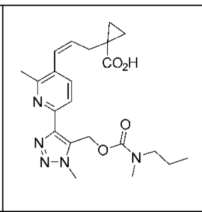 | 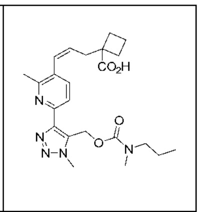 |
| 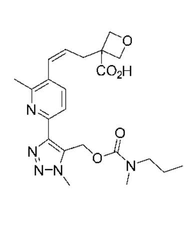 | 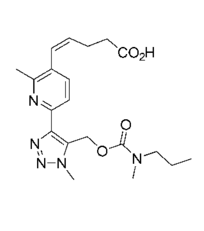 | 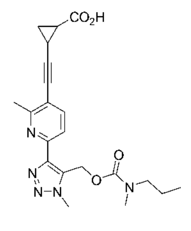 | 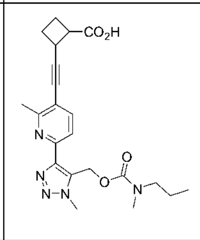 | 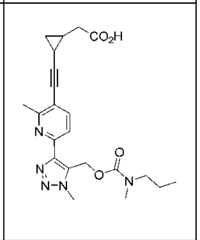 |
| 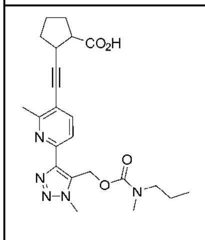 | 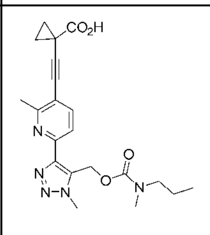 | 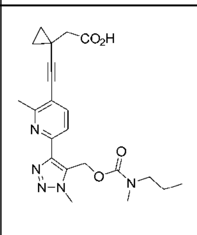 | 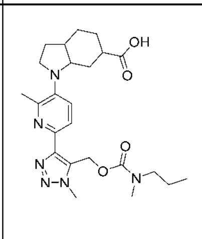 | 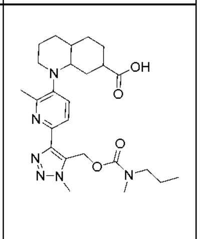 |
| 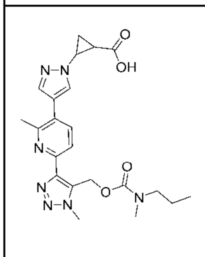 | 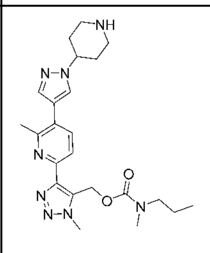 | 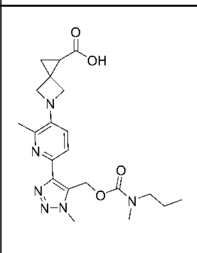 | 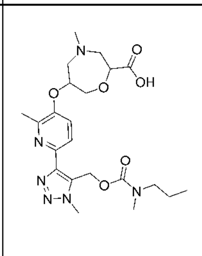 | 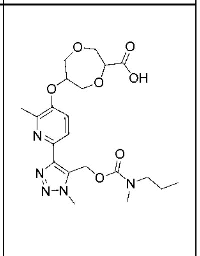 |
| 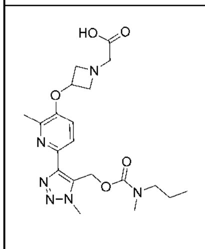 | 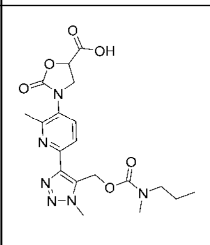 | 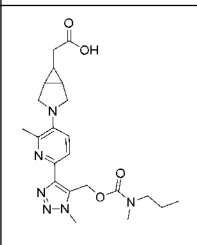 | 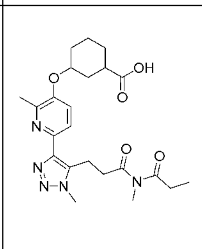 | 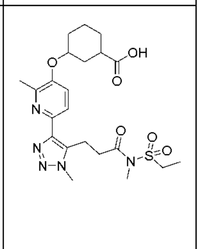 |
| 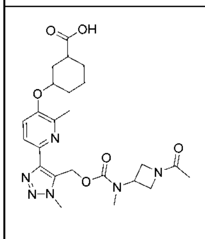 | 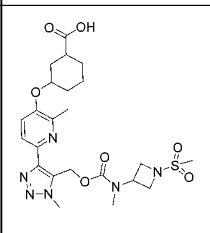 | 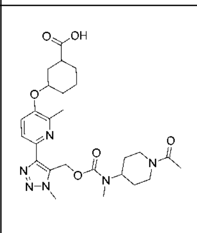 | 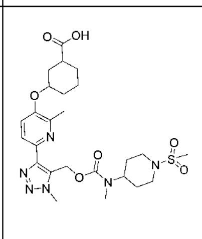 | 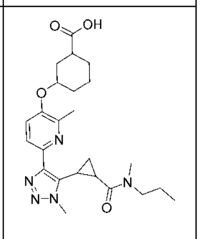 |
| 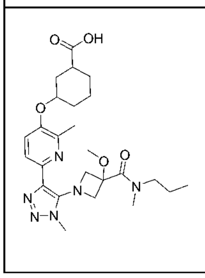 | 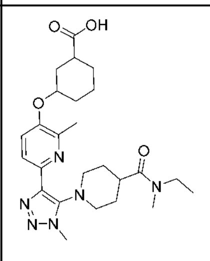 | 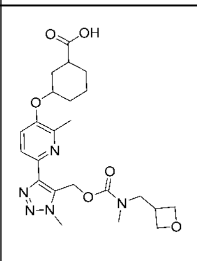 | 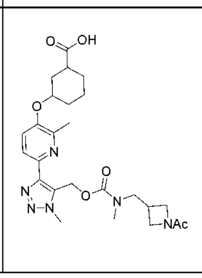 | 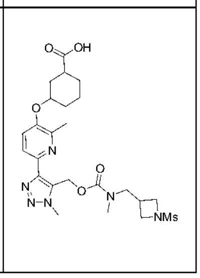 |

| | | | | |
|---|---|---|---|---|
| 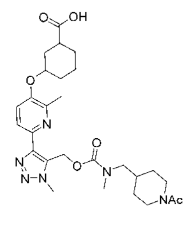 | 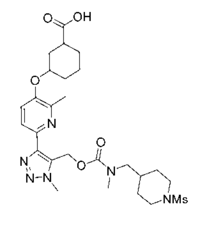 | 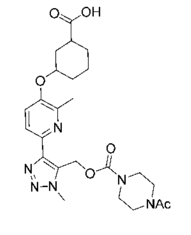 | 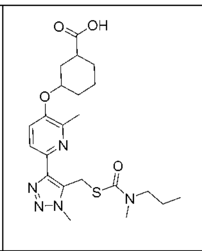 | 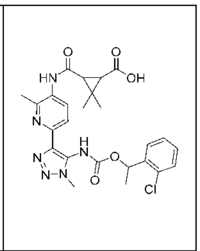 |
| 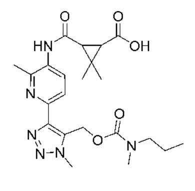 | 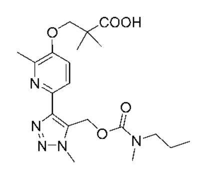 | 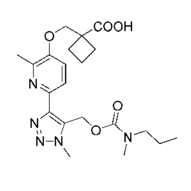 | 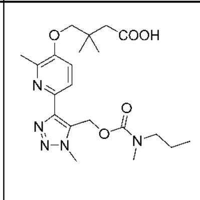 | 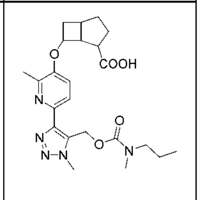 |
| 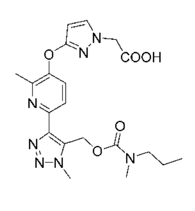 | 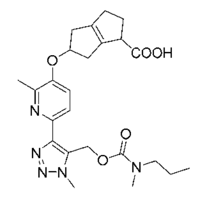 | 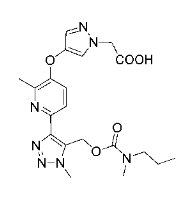 | 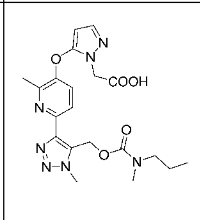 | 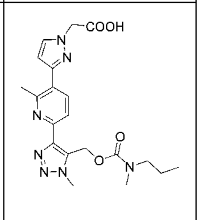 |
| 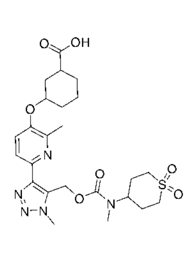 | 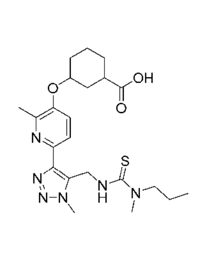 | 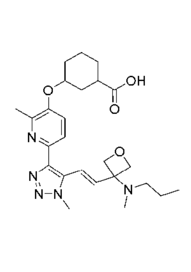 | 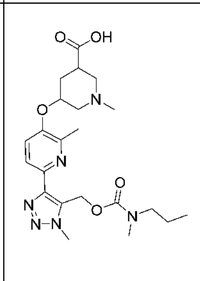 | 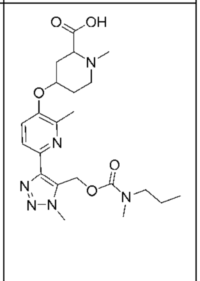 |
| 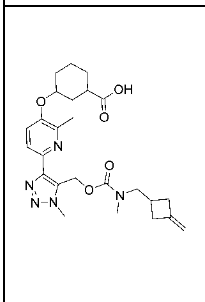 | 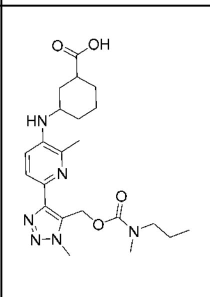 | 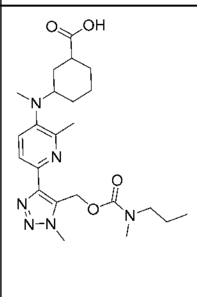 | 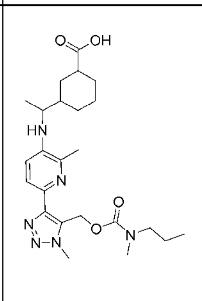 | 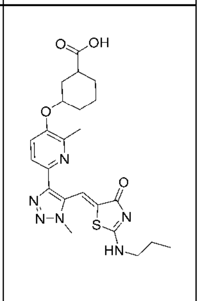 |
| 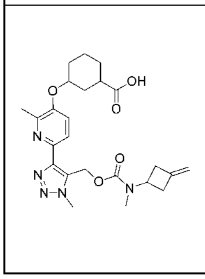 | 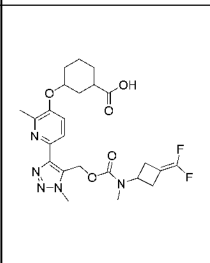 | 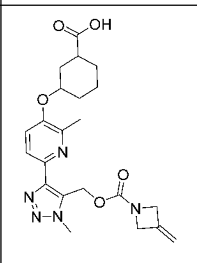 | 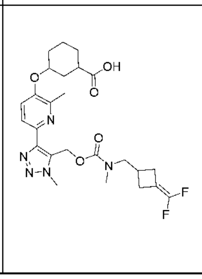 | 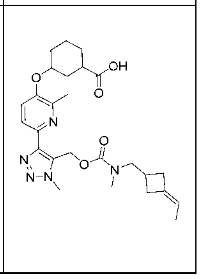 |

| | | | | |
|---|---|---|---|---|
| 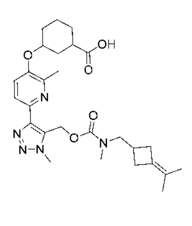 | 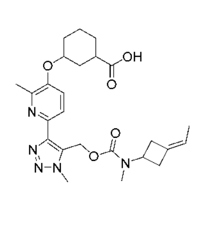 | 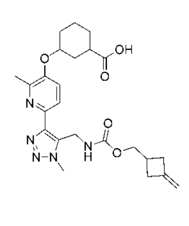 | 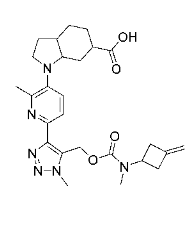 | 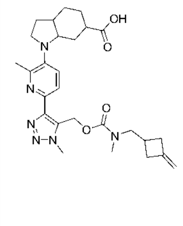 |
| 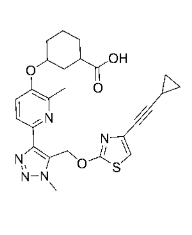 | 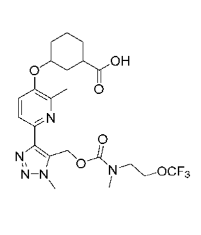 | 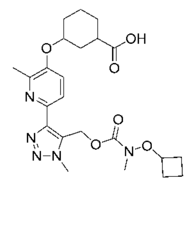 | 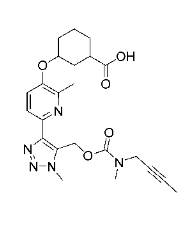 | 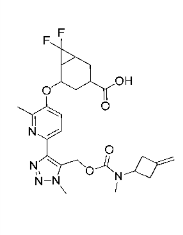 |
| 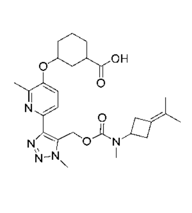 | 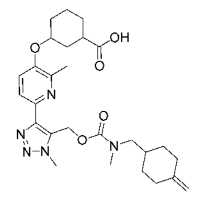 | 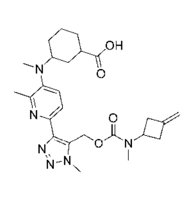 | 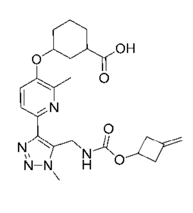 | 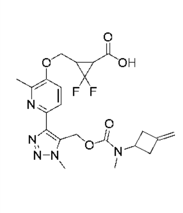 |
| 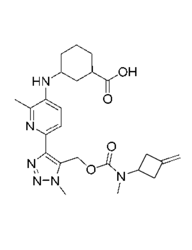 | 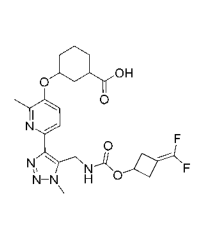 | 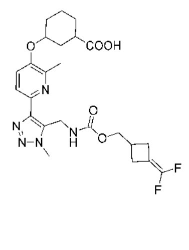 | 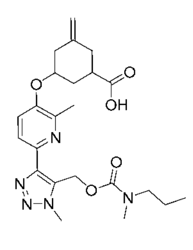 | 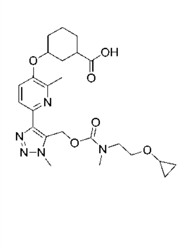 |
| 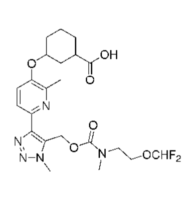 | 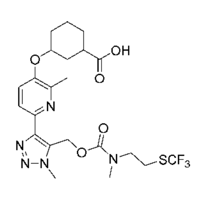 | 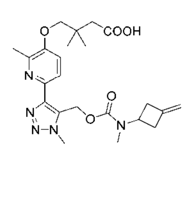 | 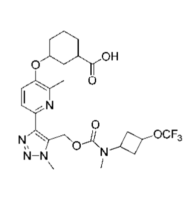 | 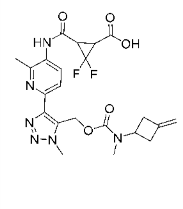 |
| 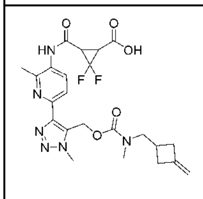 | 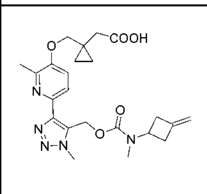 | 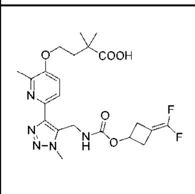 | 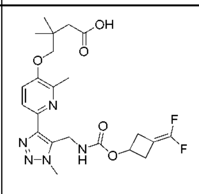 | 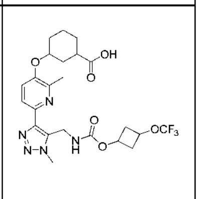 |
| 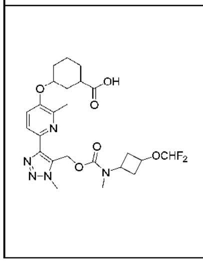 | 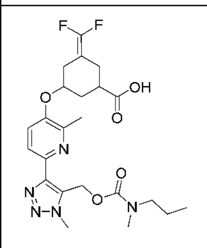 | 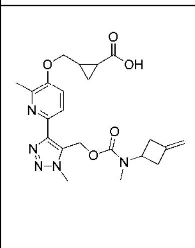 | 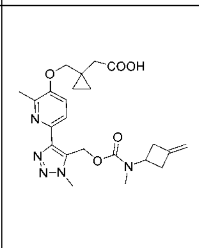 | 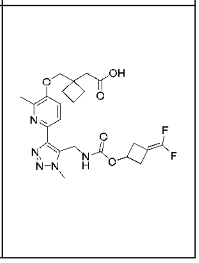 |

| | | | | |
|---|---|---|---|---|
| 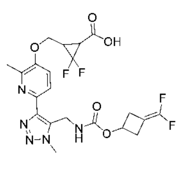 | 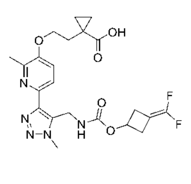 | 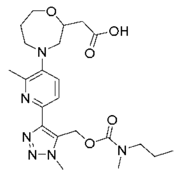 | 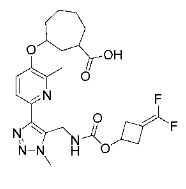 | |

Table 2

| | | | | |
|---|---|---|---|---|
| 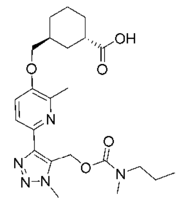 | 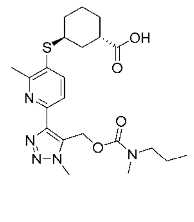 | 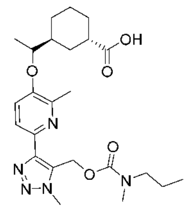 | 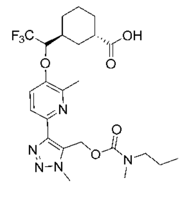 | 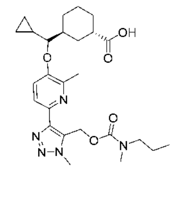 |
| 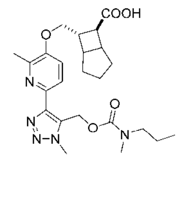 | 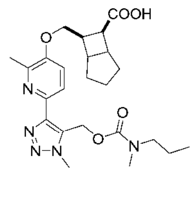 | 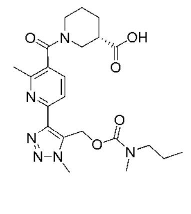 | 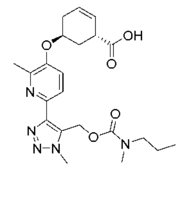 | 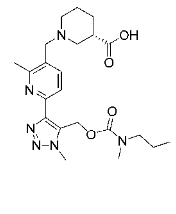 |
| 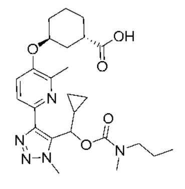 | 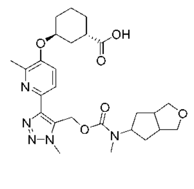 | 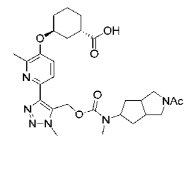 | 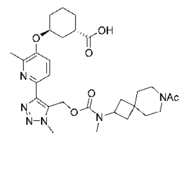 | 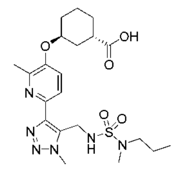 |
| 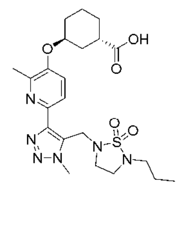 | 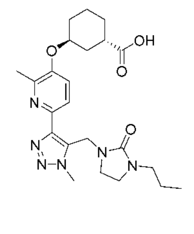 | 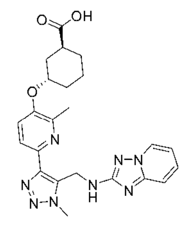 | 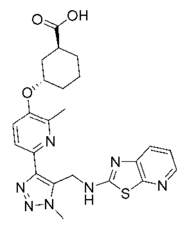 | 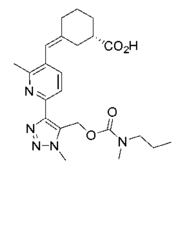 |
| 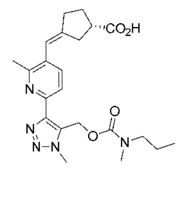 | 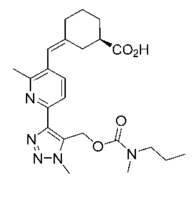 | 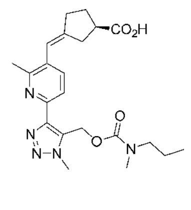 | 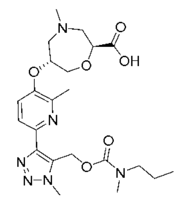 | 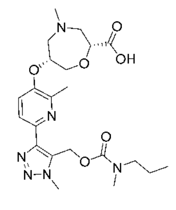 |
| 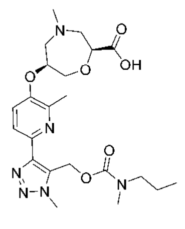 | 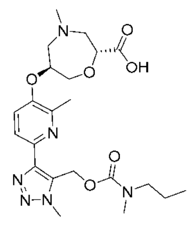 | 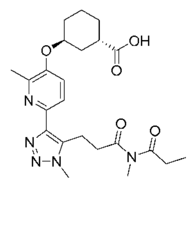 | 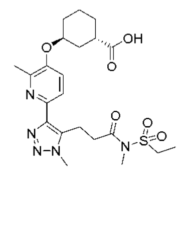 | 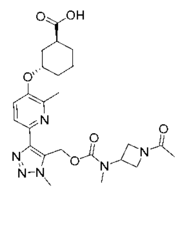 |

| | | | | |
|---|---|---|---|---|
| 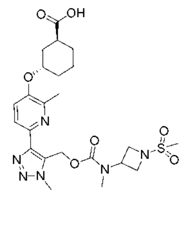 | 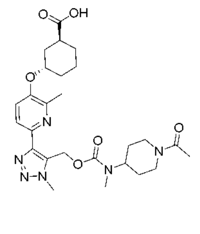 | 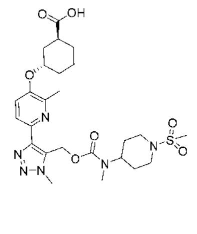 | 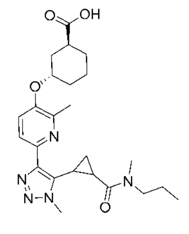 | 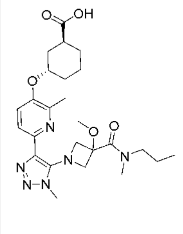 |
| 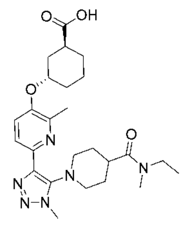 | 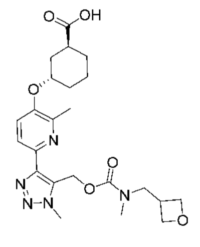 | 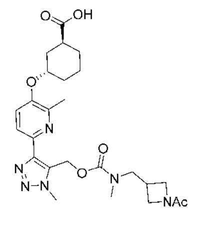 | 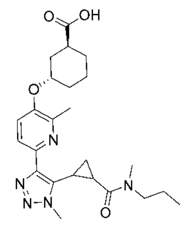 | 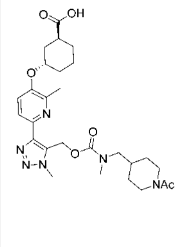 |
| 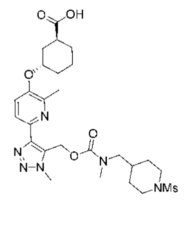 | 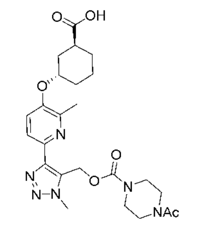 | 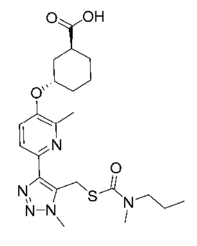 | 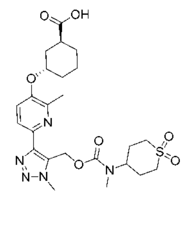 | 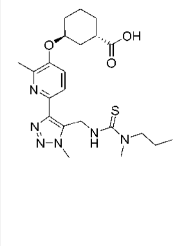 |
| 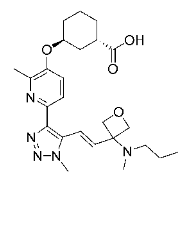 | 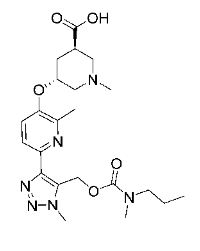 | 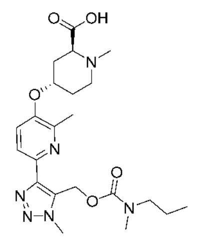 | 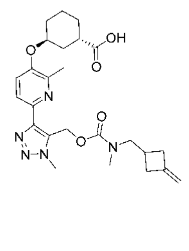 | 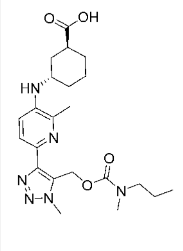 |
| 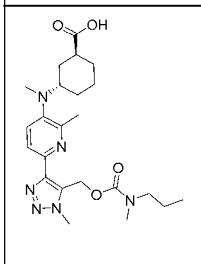 | 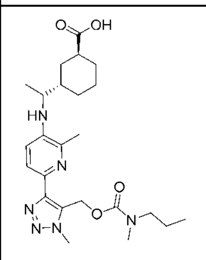 | 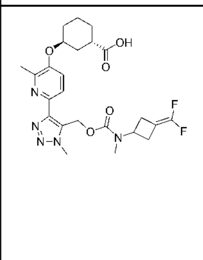 | 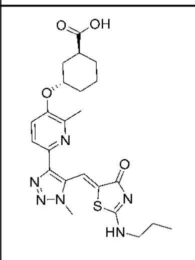 | 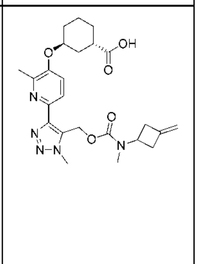 |
| 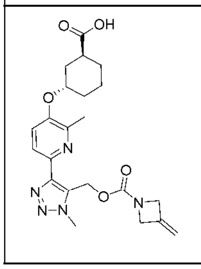 | 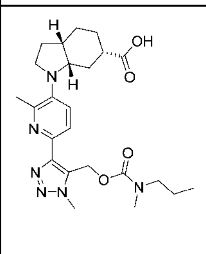 | 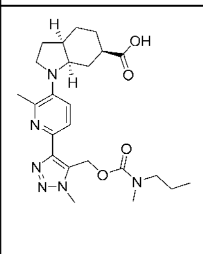 | 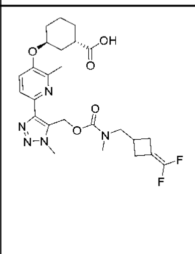 | 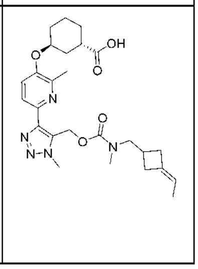 |

| | | | | |
|---|---|---|---|---|
| 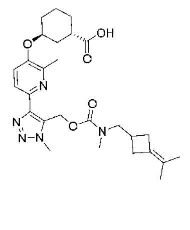 | 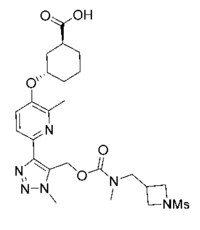 | 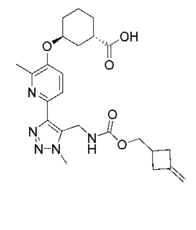 | 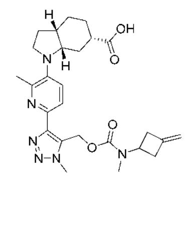 | 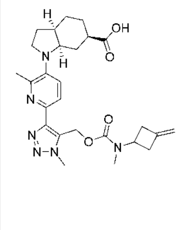 |
| 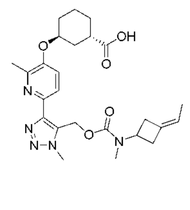 | 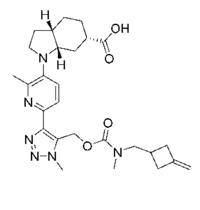 | 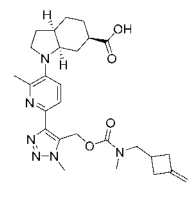 | 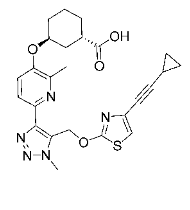 | 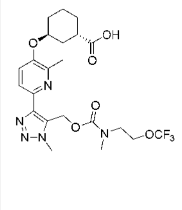 |
| 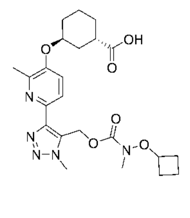 | 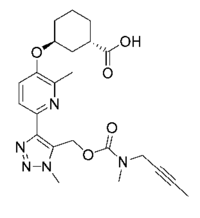 | 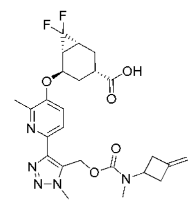 | 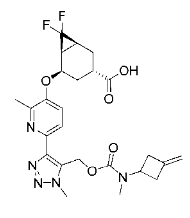 | 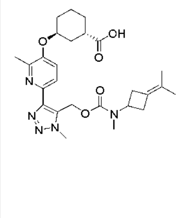 |
| 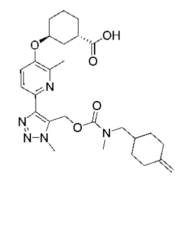 | 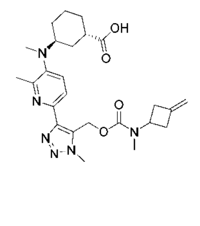 | 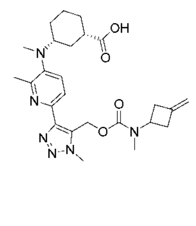 | 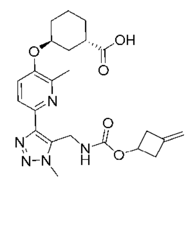 | 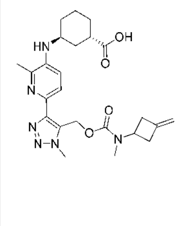 |
| 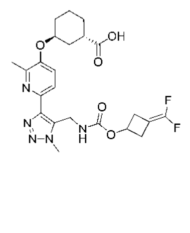 | 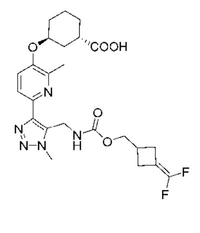 | 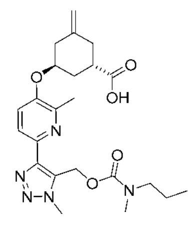 | 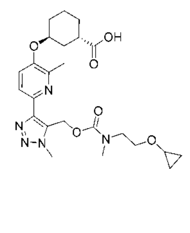 | 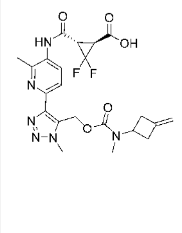 |
| 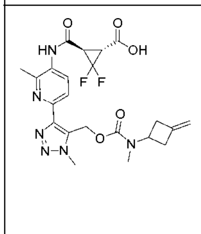 | 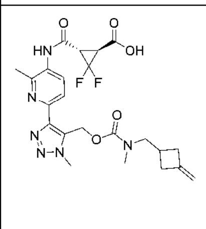 | 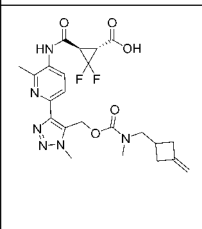 | 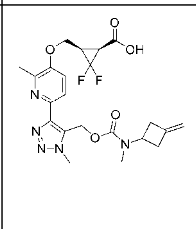 | 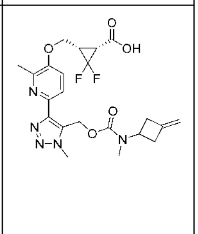 |
| 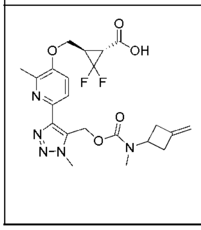 | 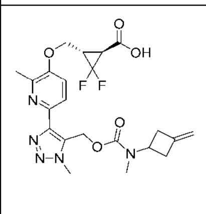 | 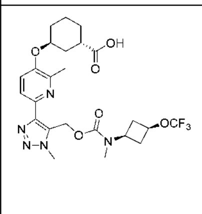 | 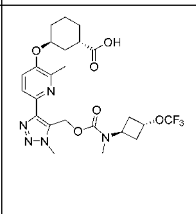 | 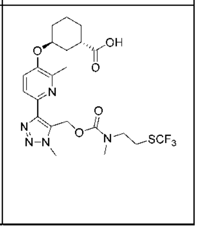 |

| | | | | |
|---|---|---|---|---|
| 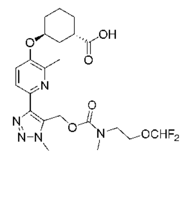 | 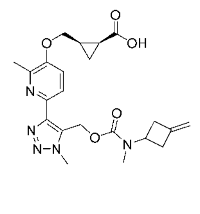 | 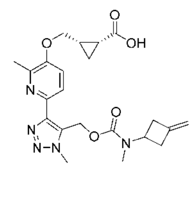 | 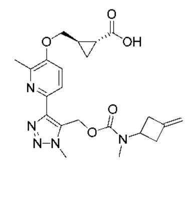 | 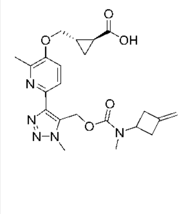 |
| 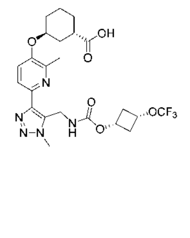 | 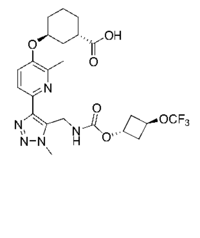 | 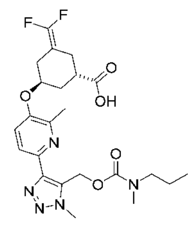 | 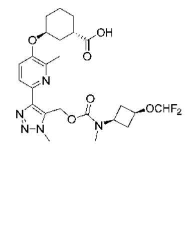 | 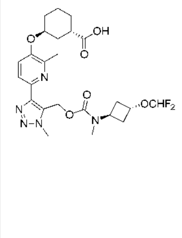 |
| 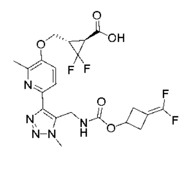 | 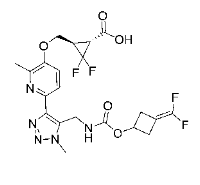 | 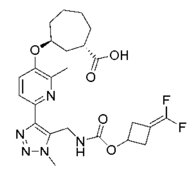 | 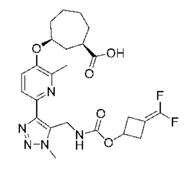 | 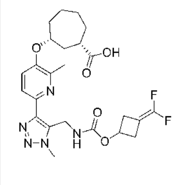 |
| 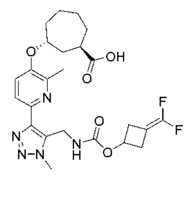 | 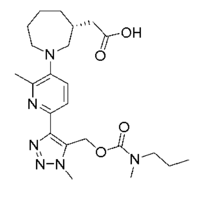 | 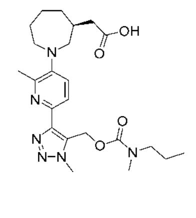 | | |

.

Next, the present invention further provides a pharmaceutical composition comprising the compound, or the stereoisomer, the deuterated substance, or the pharmaceutically acceptable salt thereof according to any of the preceding solutions, and a pharmaceutically acceptable carrier and/or auxiliary material.

Further, the pharmaceutical composition or pharmaceutical preparation of the present invention comprises 1-1500 mg of the compound, or the stereoisomer, the deuterated substance, or the pharmaceutically acceptable salt thereof according to any of the preceding solutions, and a pharmaceutically acceptable carrier and/or auxiliary material.

Further, the present invention further provides the use of the compound, or the stereoisomer, the deuterated substance, or the pharmaceutically acceptable salt thereof according to any of the preceding solutions in the preparation of a drug for treating/preventing an LPAR1-mediated disease. Further, the LPAR1-mediated disease is selected from idiopathic pulmonary fibrosis, progressive pulmonary fibrosis, systemic sclerosis, benign prostatic hyperplasia, multiple sclerosis, nerve injury, and neuralgia, preferably idiopathic pulmonary fibrosis and progressive pulmonary fibrosis.

The present invention further provides a method for treating a disease in a mammal or human, comprising administering to a subject a therapeutically effective amount of the compound, or the stereoisomer, the deuterated substance, or the pharmaceutically acceptable salt thereof according to any of the preceding solutions, or the composition of the present invention, wherein the disease is selected from idiopathic pulmonary fibrosis, progressive pulmonary fibrosis, systemic sclerosis, benign prostatic hyperplasia, multiple sclerosis, nerve injury, and neuralgia. In some embodiments, the mammal described in the present invention does not include human.

The "effective amount" or "therapeutically effective amount" described in the present application refers to administration of a sufficient amount of the compound disclosed in the present application that will alleviate to some extent one or more symptoms of the diseases or conditions being treated. In some embodiments, the outcome is the reduction and/or remission of signs, symptoms or causes of the disease, or any other desired change in the biological system. For example, an "effective amount" in terms of the therapeutic use is an amount of the included compound disclosed in the present application that is required to provide clinically significant reduction of the symptoms of the disease. Examples of the therapeutically effective amount include, but are not limited to 1-1500 mg, 1-1400 mg, 1-1300 mg, 1-1200 mg, 1-1000 mg, 1-900 mg, 1-800 mg, 1-700 mg, 1-600 mg, 1-500 mg, 1-400 mg, 1-300 mg, 1-250 mg, 1-200 mg, 1-150 mg, 1-125 mg, 1-100 mg, 1-80 mg, 1-60 mg, 1-50 mg, 1-40 mg, 1-25 mg, 1-20 mg, 5-1500 mg, 5-1000 mg, 5-900 mg, 5-800 mg, 5-700 mg, 5-600 mg, 5-500 mg, 5-400 mg, 5-300 mg, 5-250 mg, 5-200 mg, 5-150 mg, 5-125 mg, 5-100 mg, 5-90 mg, 5-70 mg, 5-80

mg, 5-60 mg, 5-50 mg, 5-40 mg, 5-30 mg, 5-25 mg, 5-20 mg, 10-1500 mg, 10-1000 mg, 10-900 mg, 10-800 mg, 10-700 mg, 10-600 mg, 10-500 mg, 10-450 mg, 10-400 mg, 10-300 mg, 10-250 mg, 10-200 mg, 10-150 mg, 10-125 mg, 10-100 mg, 10-90 mg, 10-80 mg, 10-70 mg, 10-60 mg, 10-50 mg, 10-40 mg, 10-30 mg, 10-20 mg; 20-1500 mg, 20-1000 mg, 20-900 mg, 20-800 mg, 20-700 mg, 20-600 mg, 20-500 mg, 20-400 mg, 20-350 mg, 20-300 mg, 20-250 mg, 20-200 mg, 20-150 mg, 20-125 mg, 20-100 mg, 20-90 mg, 20-80 mg, 20-70 mg, 20-60 mg, 20-50 mg, 20-40 mg, 20-30 mg; 50-1500 mg, 50-1000 mg, 50-900 mg, 50-800 mg, 50-700 mg, 50-600 mg, 50-500 mg, 50-400 mg, 50-300 mg, 50-250 mg, 50-200 mg, 50-150 mg, 50-125 mg, 50-100 mg; 100-1500 mg, 100-1000 mg, 100-900 mg, 100-800 mg, 100-700 mg, 100-600 mg, 100-500 mg, 100-400 mg, 100-300 mg, 100-250 mg, and 100-200 mg.

The present invention relates to a pharmaceutical composition or pharmaceutical preparation comprising a therapeutically effective amount of the compound, or the stereoisomer, the deuterated substance, or the pharmaceutically acceptable salt thereof according to the present invention, and a carrier and/or auxiliary material. The pharmaceutical composition can be in a unit dosage form (the amount of the active pharmaceutical ingredient per unit dosage form is also referred to as the "dosage strength"). In some embodiments, the pharmaceutical composition comprises the compound, or the stereoisomer, the deuterated substance, or the pharmaceutically acceptable salt thereof according to the present invention in an amount including but not limited to 1-1500 mg, 5-1000 mg, 10-800 mg, 20-600 mg, 25-500 mg, 40-200 mg, 50-100 mg, 1 mg, 1.25 mg, 2.5 mg, 5 mg, 10 mg, 12.5 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, 275 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 850 mg, 900 mg, 950 mg, 1000 mg, 1100 mg, 1200 mg, 1300 mg, 1400 mg, and 1500 mg.

Provided is a method for treating a disease in a mammal, comprising administering to a subject a therapeutically effective amount of the compound, or the stereoisomer, the deuterated substance, or the pharmaceutically acceptable salt thereof according to the present invention, and a pharmaceutically acceptable carrier and/or auxiliary material, wherein the therapeutically effective amount is preferably 1-1500 mg, and the disease is preferably idiopathic pulmonary fibrosis and progressive pulmonary fibrosis.

Provided is a method for treating a disease in a mammal or human, comprising administering to a subject a drug, i.e., the compound, or the stereoisomer, the deuterated substance, or the pharmaceutically acceptable salt thereof according to the present invention, and a pharmaceutically acceptable carrier and/or auxiliary material in a daily dose of 1-1500 mg/day, wherein the daily dose can be a single dose or divided doses; in some embodiments, the daily dose includes, but is not limited to 10-1500 mg/day, 20-1500 mg/day, 25-1500 mg/day, 50-1500 mg/day, 75-1500 mg/day, 100-1500 mg/day, 200-1500 mg/day, 10-1000 mg/day, 20-1000 mg/day, 25-1000 mg/day, 50-1000 mg/day, 75-1000 mg/day, 100-1000 mg/day, 200-1000 mg/day, 25-800 mg/day, 50-800 mg/day, 100-800 mg/day, 200-800 mg/day, 25-400 mg/day, 50-400 mg/day, 100-400 mg/day, and 200-400 mg/day; in some embodiments, the daily dose includes, but is not limited to 1 mg/day, 5 mg/day, 10 mg/day, 20 mg/day, 25 mg/day, 50 mg/day, 75 mg/day, 100 mg/day, 125 mg/day, 150 mg/day, 200 mg/day, 400 mg/day, 600 mg/day, 800 mg/day, 1000 mg/day, 1200 mg/day, 1400 mg/day, and 1500 mg/day.

The present invention relates to a kit, which may comprise a composition in a single-dose or multi-dose form, wherein the kit comprises the compound, or the stereoisomer, the deuterated substance, or the pharmaceutically acceptable salt thereof according to the present invention, and the amount of the compound, or the stereoisomer, the deuterated substance, or the pharmaceutically acceptable salt thereof according to the present invention is the same as that in the above-mentioned pharmaceutical composition.

In the present invention, the amount of the compound, or the stereoisomer, the deuterated substance, or the pharmaceutically acceptable salt thereof according to the present invention is calculated in the form of a free base in each case.

The term "dosage strength" refers to the amount of the active pharmaceutical ingredient contained in each vial, tablet or any other single unit dosage form.

Synthetic route

Those skilled in the art would have been able to prepare the compounds of the present invention by means of combining the document and known organic synthesis techniques, wherein the starting materials used therein are commercially available chemicals and (or) compounds described in chemical documents. "Commercially available chemicals" are obtained from regular commercial sources, and suppliers include: Titan Technology Co., Ltd., Energy Chemical Co., Ltd., Shanghai Demo Co., Ltd., Chengdu Kelong Chemical Co., Ltd., Accela ChemBio Co., Ltd., Pharma-Block Sciences (Nanjing), Inc., WuXi Apptec Co., Ltd., J&K Scientific Co., Ltd., etc.

Specific and similar reactants can be selectively identified by the indexes of known chemicals prepared by the Chemical Abstracts Service of the American Chemical Society, wherein the indexes are available in most public libraries and university libraries and online.

Chemicals that are known but not commercially available in the catalogue are optionally prepared by custom

chemical synthesis plants, wherein many of standard chemical supply plants (for example, those listed above) provide custom synthesis services.

Terms

Unless otherwise specified in the present invention, the terms of the present invention have the following meanings.

The term "halogen" herein refers to F, Cl, Br, I, or isotopes thereof.

The term "halo" or "substituted with halogen" means that a hydrogen atom is substituted with one or more groups selected from F, Cl, Br, I, or isotopes thereof, wherein the upper limit of the number of halogen substituents is equal to the sum of the number of hydrogens that can be substituted in the group to be substituted. Unless otherwise specifically defined, the number of halogen substituents is any integer between 1 and the upper limit, and when the number of halogen substituents is greater than 1, the group to be substituted can be substituted with the same or different halogen.

The term "deuterated" or "deuterated substance" refers to the case where a hydrogen atom on alkyl, cycloalkyl, alkylene, aryl, heteroaryl, mercapto, heterocycloalkyl, alkenyl, alkynyl and other groups is substituted with at least one isotope deuterium, wherein the upper limit of the number of deuterium substituents is equal to the sum of the number of hydrogens that can be substituted in the group to be substituted. Unless otherwise specifically defined, the number of deuterium substituents is any integer between 1 and the upper limit, preferably 1-20 deuterium atoms, more preferably 1-10 deuterium atoms, more preferably 1-6 deuterium atoms, and further preferably 1-3 deuterium atoms.

The term "alkyl" refers to a monovalent linear or branched saturated aliphatic hydrocarbon group. Unless otherwise specially specified, the alkyl refers to an alkyl group comprising 1 to 20 carbon atoms, preferably an alkyl group comprising 1 to 8 carbon atoms, more preferably an alkyl group comprising 1 to 6 carbon atoms, further preferably an alkyl group comprising 1 to 4 carbon atoms, and further preferably an alkyl group comprising 1-2 carbon atoms. Non-limiting examples include methyl, ethyl, n-propyl, isopropyl, n-butyl, sec-butyl, neobutyl, tert-butyl, n-pentyl, isoamyl, neopentyl, n-hexyl, and various branched isomers thereof.

The term "alkylene" refers to a divalent linear or branched saturated alkyl, and examples of alkylene include, but are not limited to, methylene, ethylene, propylene, and butylene.

The term "cycloalkylene" refers to a divalent group of "cycloalkyl", and non-limiting examples include cyclopropylene, cyclobutylene, etc.

The term "carbocyclic ring" or "carbocyclyl" refers to a substituted or unsubstituted, saturated or unsaturated, aromatic or non-aromatic carbocyclic group, including monocyclic carbocyclic rings, bicyclic bridged rings, bicyclic fused rings, bicyclic spiro rings, etc., which have 3 to 12 carbon atoms, preferably 3-10 carbon atoms, and further preferably 3-6 carbon atoms unless otherwise specially specified. The definition thereof comprises cycloalkyl and aryl. In non-limiting examples, monocyclic carbocyclic rings include cyclopropyl, cyclobutyl, cyclopentyl, cyclohexyl, cycloheptyl, phenyl,

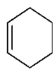,

etc.; bicyclic bridged rings include

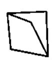, 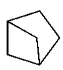, 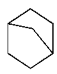,

etc.; bicyclic fused rings include

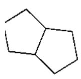, 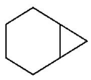, 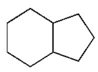,

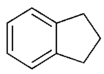, 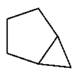,

etc.; and bicyclic spiro rings include

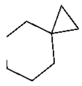, 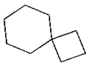, 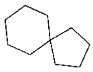,

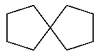, 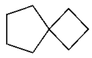, 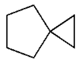,

etc.

The term "cycloalkyl" refers to a monovalent non-aromatic, partially unsaturated or fully saturated, substituted or unsubstituted carbocyclic hydrocarbon group, which typically has 3 to 12 carbon atoms, preferably 3-10 carbon atoms, more preferably 3-6 carbon atoms, and further preferably 3-4 carbon atoms, unless otherwise specially specified. Non-limiting examples include cyclopropyl, cyclobutyl, cyclopentyl, cyclohexyl,

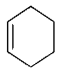,

cycloheptyl, etc.

The term "aryl" refers to an aromatic carbocyclic ring. Non-limiting examples include phenyl, naphthyl, etc.

The term "alkynyl" refers to a monovalent linear or branched unsaturated hydrocarbon group containing one or more carbon-carbon triple bonds. Unless otherwise specifically specified, the alkynyl contains 2-6 carbon atoms, preferably 2-4 carbon atoms, and non-limiting examples include ethynyl, propynyl, propargyl, etc.

The term "alkenyl" refers to a monovalent linear or branched unsaturated hydrocarbon group containing one or more carbon-carbon double bonds. Unless otherwise specifically specified, the alkenyl contains 2-6 carbon atoms, preferably 2-4 carbon atoms, and non-limiting examples include vinyl, propenyl, allyl, 2-butenyl, 1-butenyl, etc.

The term "alkoxy" or "alkyloxy" refers to -O-alkyl. Unless otherwise specifically defined, it is -O-$C_{1-8}$ alkyl, preferably -O-$C_{1-6}$ alkyl, more preferably - O-$C_{1-4}$ alkyl, and further preferably -O-$C_{1-2}$ alkyl. Non-limiting examples include methoxy, ethoxy, n-propoxy, isopropoxy, n-butoxy, secbutoxy, tert-butoxy, n-pentoxy, n-hexyloxy, cyclopropoxy, cyclobutoxy, etc.

The term "haloalkoxy" refers to -O-haloalkyl. Unless otherwise specifically defined, it is -O-halogenated $C_{1-8}$ alkyl, preferably -O-halogenated $C_{1-6}$ alkyl, more preferably -O-halogenated $C_{1-4}$ alkyl, and further preferably -O-halogenated $C_{1-2}$ alkyl. Non-limiting examples include monofluoromethoxy, difluoromethoxy, trifluoromethoxy, difluoroethyloxy, etc.

The term "$C_{1-4}$ alkylacyl" refers to $C_{1-4}$ alkyl-C(O)-. Non-limiting examples include formyl, acetyl, and propionyl.

The term "heterocyclic ring" or "heterocyclyl" refers to a substituted or unsubstituted, saturated or unsaturated, aromatic or non-aromatic ring comprising 1 to 3 heteroatoms selected from N, O, or S unless otherwise specifically defined, including monocyclic heterocyclic rings, bicyclic bridged heterocyclic rings, bicyclic fused heterocyclic rings, bicyclic spiro heterocyclic rings, etc., which are 3- to 12-membered heterocyclic rings, more preferably 4- to 12-membered heterocyclic rings, more preferably 4- to 10-membered heterocyclic rings, and further preferably 4- to 7-membered heterocyclic rings unless otherwise specifically defined. The definition thereof comprises heterocycloalkyl and heteroaryl. The N and S in the heterocyclyl ring may be oxidised into various oxidation states. Heterocyclyl can be connected to a heteroatom or a carbon atom, and non-limiting examples include oxiranyl, aziridinyl, oxetanyl, azetidinyl, 1,3-dioxolanyl, 1,4-dioxolanyl, 1,3-dioxanyl, azepanyl, pyridinyl, furyl, thienyl, pyranyl, N-alkylpyrrolyl, pyrimidinyl, pyrazinyl, pyrazolyl, pyridazinyl, imidazolyl, piperidinyl, piperidinyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, oxazolyl, dihydrooxazolyl, tetrahydrooxazolyl, tetrahydrothiazolyl, tetrahydropyranyl, benzimidazolyl, benzopyridinyl, pyrrolopyridinyl, benzodihydrofuryl, azabicyclo[3.2.1]octyl, azabicyclo[5.2.0]nonanyl, oxatricyclo[5.3.1.1]dodecyl, azaadamantyl, oxaspiro[3.3]heptyl,

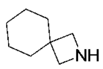, 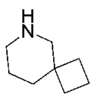, 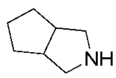, 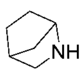, 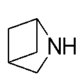, 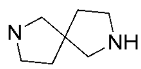,

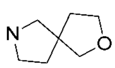, 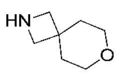, 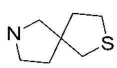, 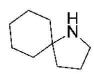, 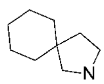,

etc.

The term "heterocyclylene" is a divalent group corresponding to "heterocyclyl", and non-limiting examples include imidazolylene, piperidinylene, aziridinylene, etc.

The term "heteroaromatic ring" or "heteroaryl" refers to an aromatic heterocyclic ring. Non-limiting examples include pyrazolyl, pyrimidinyl, thiazolyl, pyridinyl, furyl, etc.

The term "heterocycloalkyl" refers to a saturated or partially unsaturated, non-aromatic carbocyclic ring comprising 1, 2, 3, 4 and 5 heteroatoms selected from N, S, O, P and Si. The heterocycloalkyl may be monocyclic, bicyclic or polycyclic; the bicyclic or polycyclic heterocycloalkyl may be in the form of a bridged ring, a fused ring, a spiro ring or a combination thereof, and may comprise one or more aromatic rings or heteroaromatic rings, but the ring system is non-aromatic as a whole; and the connection point is on a non-aromatic ring. Typically, the heterocycloalkyl is a 3- to 20-membered ring. When the heterocycloalkyl is monocyclic heterocycloalkyl, it is typically a 3- to 15-membered ring, or a 3- to 10-membered ring, or a 3- to 8-membered ring, or a 3- to 6-membered ring; when the heterocycloalkyl is bicyclic or polycyclic heterocycloalkyl, it is typically a 5- to 12-membered ring, or a 5- to 11-membered ring, or a 6- to 9-membered ring. The heteroatoms N, S and P include their oxidation states C=O, N-O, S=O, S(=O)2, P=O, and P(=O)2. When the heterocycloalkyl is bicyclic or polycyclic, at least one ring contains at least one heteroatom. The heterocycloalkyl can be a bicyclic or polycyclic ring formed by a heteroatom-containing ring and a ring with no heteroatoms, or a bicyclic or polycyclic ring formed by a heteroatom-containing ring and another heteroatom-containing ring. When connected to other groups, the connection point can be either at a heteroatom or a carbon atom. Non-limiting examples of the heterocycloalkyl include azetidinyl, morpholinyl, piperazinyl, piperidyl, tetrahydropyranyl, oxetanyl, pyranyl, azacyclopentenyl, azacyclohexenyl, oxacyclopentenyl, oxacyclohexenyl, etc., and the heterocycloalkyl may be optionally substituted with a substituent.

The term "spiro ring" refers to a polycyclic group sharing one carbon atom (referred to as a spiro atom) between rings, which may contain 0 or at least 1 double or triple bond, and may contain 0 to 5 heteroatoms selected from N, O and S and their oxidation states. Typically, the spiro ring is a 5- to 14-membered ring, or a 5- to 12-membered ring, or a 5- to 10-membered ring. Typically, the spiro ring includes spiro[3.3] (indicating that two 3-membered rings are fused via a common spiro atom), spiro[3.4], spiro[3.5], spiro[3.6], **spiro[4.4],** spiro[4.5], spiro[4.6], spiro[5.5], or spiro[5.6] ring systems. The spiro ring may be spirocyclic, and non-limiting examples thereof include

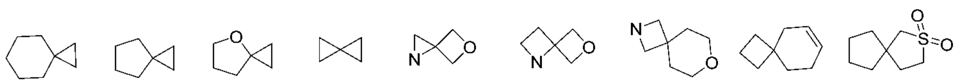 ,

wherein the spiro ring may be optionally substituted with a substituent.

The term "fused ring" refers to a polycyclic group in which the rings share two adjacent ring atoms and one chemical bond, which may contain one or more double or triple bonds, and may contain 0 to 5 heteroatoms selected from N, S and O and their oxidation states. Typically, the fused ring is a 5- to 20-membered ring, or a 5- to 14-membered ring, or a 5- to 12-membered ring, or a 5- to 10-membered ring. Typically, the fused ring includes [3,4]-fused rings (indicating a ring system formed by fusion of a three-membered ring and a four-membered ring; according to IUPC nomenclature rules, either the three-membered or the four-membered ring may serve as the parent ring, and the same applies analogously to the following cases), [3,5]-fused rings, [3,6]-fused rings, [4,4]-fused rings, [4,5]-fused rings, [4,6]-fused rings, [5,5]-fused rings, [5,6]-fused rings, and [6,6]-fused rings. Non-limiting examples of the fused ring include purine, quinoline, isoquinoline, benzopyran, benzofuran, benzothiophene, and

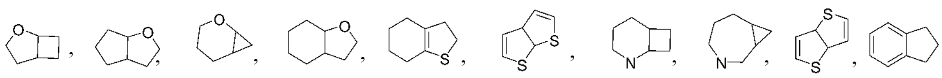 , , , , , , , , , ,

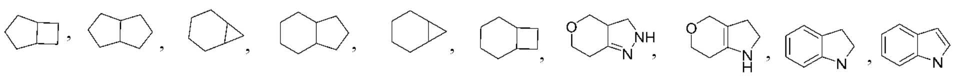 , , , , , , , , , , ;

and the fused ring may be optionally substituted with a substituent.

The term "bridged ring" refers to a ring system in which two rings share two non-adjacent ring atoms, which may contain one or more double or triple bonds. The bridged ring may contain 0 to 5 heteroatoms selected from N, S, and O and their oxidation states. Typically, the bridged ring has 5 to 20, or 5 to 14, or 5 to 12, or 5 to 10 ring atoms. Non-limiting examples of the bridged ring include adamantane,

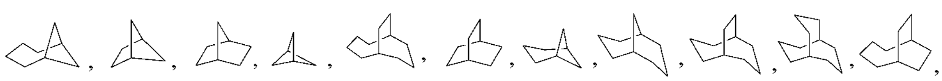 , , , , , , , , , , , ,

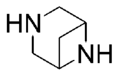, 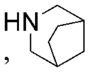, 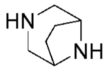, 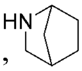, 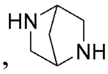, 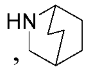, 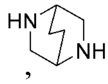, 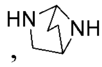, 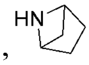.

The term "carbon-fused ring", "fused cycloalkyl", "fused carbocyclyl", or "carbon-fused ring group" refers to a "fused ring" composed solely of carbon atoms. The definition of the "carbon-fused ring", "fused cycloalkyl", "fused carbocyclyl", or "carbon-fused ring group" herein is consistent with that of a fused ring.

The term "fused heterocyclic ring", "fused heterocyclic ring group", "fused heterocycloalkyl", "fused heterocyclyl", or "fused heterocyclic ring group" refers to a "fused ring" containing a heteroatom. The definition of the "fused heterocyclic ring", "fused heterocyclic ring group", "fused heterocycloalkyl", "fused heterocyclyl", or "fused heterocyclic ring group" herein is consistent with that of a fused ring.

The term "spiro heterocyclic ring", "spiroheterocyclyl", "spiro heterocyclic ring group", or "spiroheterocyclyl" refers to a "spiro ring" containing a heteroatom. The definition of the "spiro heterocyclic ring", "spiroheterocyclyl", "spiro heterocyclic ring group" or "spiroheterocyclyl" herein is consistent with that of a spiro ring.

The term "bridged heterocyclic ring", "bridged heterocyclyl", "bridged heterocyclic ring group", or "bridged heterocyclyl" refers to a "bridged ring" containing a heteroatom. The definition of the "bridged heterocyclic ring", "bridged heterocyclyl", "bridged heterocyclic ring group", or "bridged heterocyclyl" herein is consistent with that of a bridged ring. The term "optional" or "optionally" means that the events or circumstances subsequently described may but not necessarily occur, and the description includes the occasions where the events or circumstances occur or do not occur. For example, "alkyl optionally substituted with F" means that the alkyl may but not necessarily be substituted with F, and the description includes the case where the alkyl is substituted with F and the case where the alkyl is not substituted with F.

When the connecting groups listed do not specify their connection direction, their connection direction includes both the left-to-right and right-to-left reading orders. For example, for A-L-B, wherein L is -M-W-, it includes both A-M-W-B and A-W-M-B. A specific example is A-C(O)$NR^{L1}$-B, which includes both A-C(O)$NR^{L1}$-B and A-$NR^{L1}$C(O)-B.

The term "pharmaceutically acceptable salt" refers to a salt of the compound of the present invention, which salt maintains the biological effectiveness and characteristics of a free acid or a free base and is obtained by reacting the free acid with a non-toxic inorganic base or organic base, or reacting the free base with a non-toxic inorganic acid or organic acid.

The term "pharmaceutical composition" represents a mixture of one or more compounds or stereoisomers, solvates, pharmaceutically acceptable salts or co-crystals thereof as described herein and other components including physiologically/pharmaceutically acceptable carriers and/or excipients.

The term "carrier" refers to a system that does not cause significant irritation to the organism and does not eliminate the biological activity and characteristics of the administered compound and can change the way the drug enters the human body and the distribution of the drug in the body, control the release rate of the drug and deliver the drug to targeted organs. Non-limiting examples of the carrier include microcapsule, microsphere, nanoparticle, liposome, etc.

The term "excipient" refers to a substance that is not a therapeutic agent per se, but is used as a diluent, auxiliary material, binder and/or vehicle added to a pharmaceutical composition, thereby improving the disposal or storage properties thereof, or allowing to or promoting the formation of a compound or a pharmaceutical composition into a unit dosage form for administration. As is known to those skilled in the art, pharmaceutically acceptable excipients can provide various functions and can be described as a wetting agent, a buffer, a suspending agent, a lubricant, an emulsifier, a disintegrant, an absorbent, a preservative, a surfactant, a colourant, a flavouring agent, and a sweetening agent. Examples of pharmaceutically acceptable excipients include, but are not limited to: (1) sugars, such as lactose, glucose, and sucrose; (2) starches, such as corn starch and potato starch; (3) cellulose and derivatives thereof, such as sodium carboxymethyl cellulose, ethyl cellulose, cellulose acetate, hydroxypropyl methylcellulose, hydroxypropyl cellulose, microcrystalline cellulose, and croscarmellose (such as croscarmellose sodium); (4) tragacanth powder; (5) malt; (6) gelatine; (7) talc; (8) excipients, such as cocoa butter or suppository wax; (9) oils, such as peanut oil, cottonseed oil, safflower oil, sesame oil, olive oil, corn oil, and soybean oil; (10) diols, such as propylene glycol; (11) polyols, such as glycerol, sorbitol, mannitol, and polyethylene glycol; (12) esters, such as ethyl oleate and ethyl laurate; (13) agar; (14) buffers, such as magnesium hydroxide and aluminium hydroxide; (15) alginic acid; (16) pyrogen-free water; (17) isotonic saline; (18) Ringer's solution; (19) ethanol; (20) pH buffer solutions; (21) polyesters, polycarbonates and/or polyanhydrides; and (22) other non-toxic compatible substances used in a pharmaceutical preparation.

"Isomer" includes "stereoisomer" and "tautomer". The term "stereoisomer" refers to isomers produced as a result of different spatial arrangement of atoms or atomic groups in molecules that are connected to each other in the same sequence. Stereoisomers include cis-trans isomers and optical isomers. The term "tautomer" refers to isomers that can be transformed into each other by a reversible chemical reaction called tautomerisation, which is a transformation between functional groups usually caused by the accompanying migration of a hydrogen atom and a $\pi$ bond (a double bond or a triple bond); such as, the following paired compounds: aldehyde/ketone-enol and imine-enamine.

Detailed Description of Embodiments

The content of the present invention is described in detail by means of the following examples. In examples in which no specific conditions are indicated, experimental methods are carried out under conventional conditions. The listed examples are intended to better illustrate the content of the present invention but should not be construed as limiting the content of the present invention. Non-essential improvements and adjustments made to the embodiments by a person of ordinary skill in the art according to the above Summary of the Invention still fall within the scope of protection of the present invention.

**Test method**

The structure of the compound is determined by nuclear magnetic resonance (NMR) or (and) mass spectrometry (MS). The NMR shift (δ) is given in the unit of $10^{-6}$ (ppm). NMR is measured with (Bruker Avance III 400 and Bruker Avance 300) NMR instrument, and the solvent for determination is deuterated dimethyl sulphoxide (DMSO-$d_6$), deuterated chloroform ($CDCl_3$), and deuterated methanol ($CD_3OD$), and the internal standard is tetramethylsilane (TMS);

MS is determined with Agilent 6120B (ESI) and Agilent 6120B (APCI); and
HPLC is determined with Agilent 1260DAD high pressure liquid chromatograph (Zorbax SB-$C_{18}$ 100 × 4.6 mm, 3.5 μM);
For thin layer chromatography (TLC), silica gel plates from Yantai Huanghai HSGF254 or Qingdao GF254 are used. Silica gel plates with a thickness of 0.15 mm-0.20 mm are employed for TLC, while plates with a thickness of 0.4 mm-0.5 mm are used for TLC separation and purification.

For column chromatography, Yantai Huanghai silica gel of 200-300 mesh is typically used as a carrier.

**Example 1**

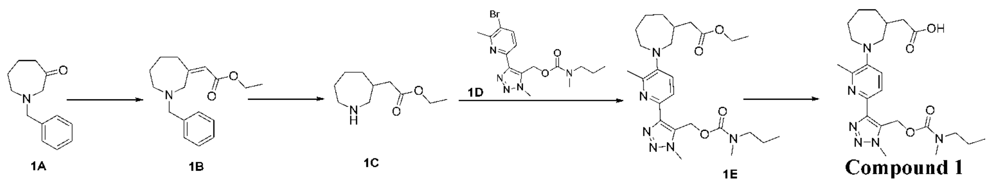


1A 1B 1C 1E Compound 1

Step 1: Ethyl 2-(diethoxyphosphoryl)acetate (2.31 g, 10.32 mmol) was dissolved in tetrahydrofuran (30 mL), and then sodium hydride (0.37 g, 15.33 mmol) was added. The mixture was purged three times with nitrogen, and then reacted at 0°C for half an hour. Subsequently, **1A** (1.5 g, 7.37 mmol) dissolved in a tetrahydrofuran solution was slowly injected into the above reaction system. After the reaction was completed, water was added to quench the reaction, and then the mixture was extracted three times with ethyl acetate. The organic phases were combined and concentrated to dryness, and the residue was purified by normal-phase chromatography (petroleum ether : ethyl acetate = 20 : 1) to afford product 1B (1.6 g, 79%).

LC-MS (ESI): m/z=274.2 $[M+H]^+$.

Step 2: 1B (0.5 g, 1.83 mmol) was dissolved in ethanol (8 mL) and 1M dilute hydrochloric acid solution (2 mL), and then palladium on carbon (0.19 g, 1.83 mmol) was added. The mixture was purged three times with hydrogen and subjected to hydrogenation reaction at room temperature overnight. After the reaction was completed, the reaction solution was filtered through diatomaceous earth, and rinsed repeatedly with dichloromethane and methanol. The filtrate was subjected to rotary evaporation to dryness, and then redissolved in 10 mL of dichloromethane. Excess potassium carbonate was added until the solution reached a weakly basic pH. The mixture was filtered, and the resulting filtrate was concentrated to dryness to afford product **1C** (0.3 g, 88%) in its free form.

LC-MS (ESI): m/z=186.1$[M+H]^+$.

Step 3: 1D (0.5 g, 1.31 mmol, synthesised according to the procedure reported in Journal of Medicinal Chemistry, 2021, vol. 64, # 21, p. 15549-15581), **1C** (0.32 g, 1.73 mmol), caesium carbonate (0.85 g, 2.62 mmol), and Ruphos Pd G3 (0.11 g, 0.13 mmol) were dissolved in 1,4-dioxane (15 mL). The mixture was purged three times with nitrogen, and then reacted at 90°C for 6 hours. After the reaction was completed, the reaction solution was concentrated to dryness, and the residue was purified by normal-phase chromatography (petroleum ether : ethyl acetate = 2 : 1) to afford product **1E** (35 mg, 5.5%).

LC-MS (ESI): m/z=487.1[M+H]+.

Step 4: **1E** (0.035 g, 0.072 mmol) was dissolved in methanol (2 mL), tetrahydrofuran (2 mL) and water (2 mL), and then lithium hydroxide (0.0069 g, 0.29 mmol) was added, and the mixture was reacted at room temperature for 1 hour. After the reaction was completed, 1 M dilute hydrochloric acid was directly added to adjust the pH to weakly acidic. A small amount of water was then added, and the mixture was extracted three times with ethyl acetate. The organic phases were combined and concentrated, and the residue was subsequently purified by preparative HPLC to afford the trifluoroacetate of compound 1 (4 mg, 12%).

LC-MS (ESI): m/z=459.3[M+H]+.

$^1$H NMR (400 MHz, Chloroform-d) δ 7.96 (s, 1H), 7.80 (d, 1H), 5.61 (s, 2H), 4.18 (s, 3H), 3.29 - 3.10 (m, 11H), 2.88 - 2.73 (m, 3H), 2.36 - 2.20 (m, 3H), 1.90 - 1.78 (m, 3H), 1.56 - 1.41 (m, 3H), 0.90 - 0.74 (m, 3H).

Example 2:

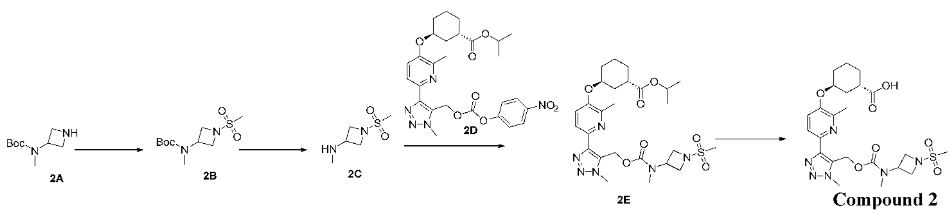

Step 1: Compound 2A (1.00 g, 4.49 mmol, HCl salt) was dissolved in anhydrous tetrahydrofuran (20 mL), triethylamine (682 mg, 6.74 mmol) and methanesulphonyl chloride (617 mg, 5.39 mmol) were successively added with stirring, and the mixture was reacted at room temperature for 4 hours. Upon complete depletion of starting materials as monitored by TLC, the reaction solution was concentrated to about 5 mL, and added dropwise to 15 mL of ice-cold water. The mixture was extracted twice with 20 mL of ethyl acetate, the organic phases were combined and concentrated, and the residue was purified by silica gel column chromatography (petroleum ether : ethyl acetate (v/v) = 1 : 1) to afford compound 2B (680 mg, 57 %).

$^1$H NMR (400 MHz, $CDCl_3$) δ 4.78 (s, 1H), 4.15-4.03 (m, 4H), 2.91-2.87 (m, 6H), 1.46 (s, 9H).

Step 2: Compound 2B (300 mg, 1.13 mmol) was dissolved in a mixed solvent of dichloromethane and trifluoroacetic acid (dichloromethane : trifluoroacetic acid = 3 : 1, 4 mL), and the mixture was reacted at room temperature for 2 hours. Upon complete depletion of starting materials as monitored by TLC, the reaction solution was concentrated to afford crude 2C (220 mg, TFA salt), which was directly used in the next step.

LC-MS (ESI): m/z =165.2 [M+H]+.

Step 3: Compound 2D (200 mg, 0.36 mmol, prepared according to the method reported in patent WO 2017223016A1) and crude 2C (220 mg) were dissolved in 10 mL of tetrahydrofuran, DIPEA (140 mg, 1.08 mmol) was added, and the mixture was reacted at room temperature for 2 hours. Upon complete depletion of starting materials as monitored by LCMS, the reaction solution was concentrated to 2 mL, and added dropwise to 15 mL of ice-cold water. The mixture was extracted twice with 20 mL of ethyl acetate, the organic phases were combined and concentrated, and the residue was purified by silica gel column chromatography (petroleum ether : ethyl acetate (v/v) = 3 : 1) to afford compound **2E** (120 mg, 57 %).

LC-MS (ESI): m/z =579.3 [M+H]+.

Step 4: Compound **2E** (120 mg, 0.21 mmol) was dissolved in a mixed solution of tetrahydrofuran and water (THF : $H_2O$ = 4 : 1, 5 mL), lithium hydroxide monohydrate (28 mg, 0.63 mmol) was added, and the mixture was reacted at room temperature for 16 hours. Upon complete depletion of starting materials as monitored by LCMS, the reaction solution was filtered, the filtrate was concentrated, and the residue was subsequently separated by C18 reversed-phase column chromatography to afford the trifluoroacetate of target compound 2 (21 mg, 17%).

LC-MS (ESI): m/z =537.5 [M+H]+.

$^1$H NMR (400 MHz, DMSO-$d_6$) δ 7.87 - 7.82 (m, 1H), 7.52 - 7.46 (m, 1H), 5.68 (s, 2H), 4.78 (s, 1H), 4.10 (s, 3H), 4.02 - 3.84 (m, 4H), 3.05 - 2.80 (m, 7H), 2.69 - 2.60 (m, 1H), 2.41 (s, 3H), 2.08 - 2.07 (m, 1H), 1.91 - 1.73 (m, 3H), 1.68 - 1.44 (m, 4H).

**Example 3**

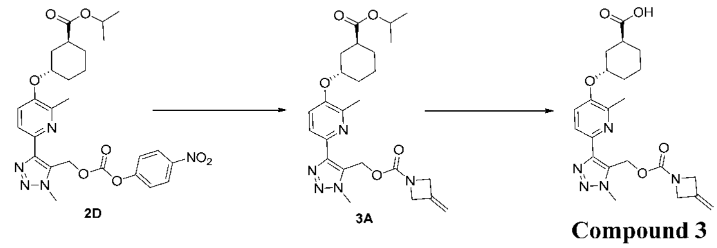

2D 3A Compound 3

Step 1: Compound 2D (204 mg, 0.37 mmol) was dissolved in dichloromethane (5 mL), 3-methylenemidine (38 mg, 0.55 mmol) and excess pyridine were added, and the mixture was reacted at room temperature for 4 h. Upon complete depletion of starting materials, the reaction solution was concentrated, and the crude product was separated by silica gel column chromatography (petroleum ether : ethyl acetate = 1 : 0-3 : 1) to afford target compound **3A** (164 mg, yield: 91.6%).

LC-MS (ESI): m/z= 484.3 $[M+1]^+$.

Step 2: Compound **3A** (163 mg, 0.34 mmol) was dissolved in tetrahydrofuran (10 mL), 1M lithium hydroxide aqueous solution (2 mL) was added, and the mixture was reacted at 50°C for 48 h. Excess citric acid was added to adjust the solution to weakly acidic, the mixture was concentrated, and the resulting residue was separated by silica gel column chromatography (dichloromethane : anhydrous methanol =1 : 0-10 : 1) to afford compound 3 (48 mg, yield: 33.1%).

LC-MS (ESI): m/z= 442.3 $[M+1]^+$.

$^1$H NMR (400 MHz, DMSO-$d_6$) δ 7.83 (d, 1H), 7.46 (d, 1H), 5.74 (s, 2H), 5.66 (s, 2H), 4.81 - 4.73 (m, 1H), 4.48 - 4.38(m, 4H), 4.07 (s, 3H), 2.40 (s, 3H), 2.04 - 1.96 (m, 1H), 1.89 - 1.71 (m, 4H), 1.68 - 1.43 (m, 4H).

**Example 4**

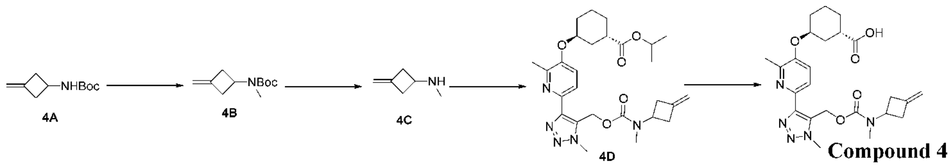

4A 4B 4C 4D Compound 4

Step 1: Compound 4A (500 mg, 2.73 mmol) was added to a reaction flask and dissolved in DMF (10 ml). NaH (160 mg, 4.10 mmol) was added at 0°C, and the mixture was reacted at this temperature for 30 min. Iodomethane (775 mg, 5.46 mmol) was then added, and the resulting mixture was warmed to room temperature and reacted for 3 hours. Upon completion of the reaction as monitored by TLC, the reaction system was diluted with water (50 ml), and then extracted twice with ethyl acetate (50 ml × 2). The organic phases were combined, dried, and concentrated under reduced pressure, and the resulting residue was purified by silica gel column chromatography (petroleum ether : ethyl acetate = 10 : 1) to afford compound **4B** (475 mg, yield: 88.29%).

$^1$H NMR (400 MHz, DMSO-d6) δ 4.84 - 4.80 (m, 2H), 4.50 - 4.24 (m, 1H), 2.90 - 2.80 (m, 2H), 2.78 - 2.70 (m, 2H), 1.39 (s, 9H).

Step 2: Compound **4B** (470 mg, 2.38 mmol) was added to a reaction flask and dissolved in dichloromethane (10 ml), and then trifluoroacetic acid (0.5 ml) was added, and the mixture was reacted at room temperature for 1h. Upon completion of the reaction as monitored by TLC, the reaction system was concentrated under reduced pressure to afford crude compound **4C** (250 mg, TFA salt), which was directly used in the next step.

LC-MS (ESI): m/z=98.1$[M+H]^+$.

Step 3: Compound **2D** (200 mg, 0.36 mmol, synthesised according to the method reported in patent WO 2017223016 A1) and crude compound 4C (250 mg) were added to a reaction flask and dissolved in dichloromethane (10 ml), and then DIPEA (0.62 ml, 3.6 mmol) was added, and the mixture was reacted at room temperature for 3 hours. Upon completion of the reaction as monitored by TLC, the reaction system was concentrated under reduced pressure, and the resulting residue was purified by silica gel column chromatography (petroleum ether : ethyl acetate = 2 : 1) to afford compound **4D** (120 mg, yield: 64.93%).

LC-MS (ESI): m/z=512.3$[M+H]^+$.

Step 4: Compound **4D** (120 mg, 0.23 mmol) was added to a reaction flask and dissolved in THF : MeOH : H2O = 3 : 1 : 1 (15 ml), and then anhydrous lithium hydroxide (22 mg, 0.92 mmol) was added, and the mixture was reacted at 50°C for 16 hours. Upon completion of the reaction as monitored by TLC, the reaction system was concentrated under reduced pressure, and the resulting residue was separated by C18 reversed-phase column chromatography to afford the trifluoroacetate of compound 4 (35 mg, yield: 32.40%).

LC-MS (ESI): m/z=470.4[M+H]$^+$.

$^1$H NMR (400 MHz, DMSO-d6) δ 7.87 - 7.82 (m, 1H), 7.52 - 7.47 (m, 1H), 5.64 (s, 2H), 4.79 (s, 3H), 4.10 (s, 3H), 2.88 - 2.59 (m, 9H), 2.41 (s, 3H), 2.06 - 1.98 (m, 1H), 1.91 - 1.73 (m, 3H), 1.68 - 1.45 (m, 4H).

**Example 5**

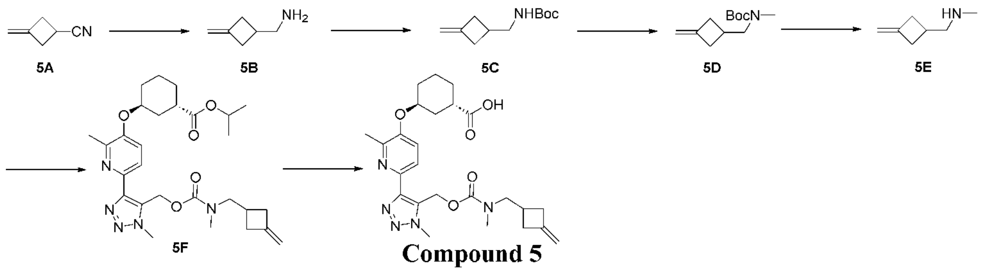
5A 5B 5C 5D 5E 5F

Compound 5

Step 1: Lithium aluminium hydride (815 mg, 21.48 mmol) was dissolved in tetrahydrofuran (10 ml), and then compound 5A (1.0 g, 10.74 mmol) was dissolved in tetrahydrofuran (10 ml) and added to the above system at room temperature, and the resulting mixture was finally reacted at 70°C for 3 hours. Upon completion of the reaction as monitored by TLC, the reaction system was cooled to room temperature, quenched with water (1 ml), stirred at room temperature for 30 min, and filtered. The filtrate was collected and concentrated under reduced pressure to afford compound **5B** (0.9 g, yield: 86.54%).

LC-MS (ESI): m/z=98.2[M+H]$^+$.

Step 2: Compound **5B** (900 mg, 9.26 mmol) was added to a reaction flask and dissolved in tetrahydrofuran (10 ml), and then di-tert-butyl dicarbonate (2.0 g, 9.26 mmol) and triethylamine (2.5 ml, 18.52 mmol) were successively added, and the mixture was reacted at room temperature for 16 hours. Upon completion of the reaction as monitored by TLC, the reaction system was concentrated under reduced pressure, and the resulting residue was purified by silica gel column chromatography (petroleum ether : ethyl acetate = 15 : 1) to afford compound 5C (1.36 g, yield: 95.10%).

$^1$H NMR (400 MHz, DMSO-d6) δ 6.86 (s, 1H), 4.75 - 4.69 (m, 2H), 3.03 - 2.94 (m, 2H), 2.70 - 2.57 (m, 2H), 2.39 - 2.26 (m, 3H), 1.37 (s, 9H).

Starting from compound **5C** and following step 1 to step 4 of Example 4, the trifluoroacetate of compound 5 (40 mg, yield: 34.48%) was obtained.

LC-MS (ESI): m/z=484.4[M+H]$^+$.

$^1$H NMR (400 MHz, DMSO-d6) δ 7.87 - 7.82 (m, 1H), 7.53 - 7.47 (m, 1H), 5.63 (s, 2H), 4.82 - 4.52 (m, 3H), 4.10 (s, 3H), 3.36 - 3.14 (m, 2H), 2.82 - 2.72 (m, 3H), 2.70 - 2.57 (m, 2H), 2.42 (s, 3H), 2.39 - 2.28 (m, 2H), 2.22 - 2.10 (m, 1H), 2.06 - 1.98 (m, 1H), 1.93 - 1.42 (m, 8H).

**Example 6:**

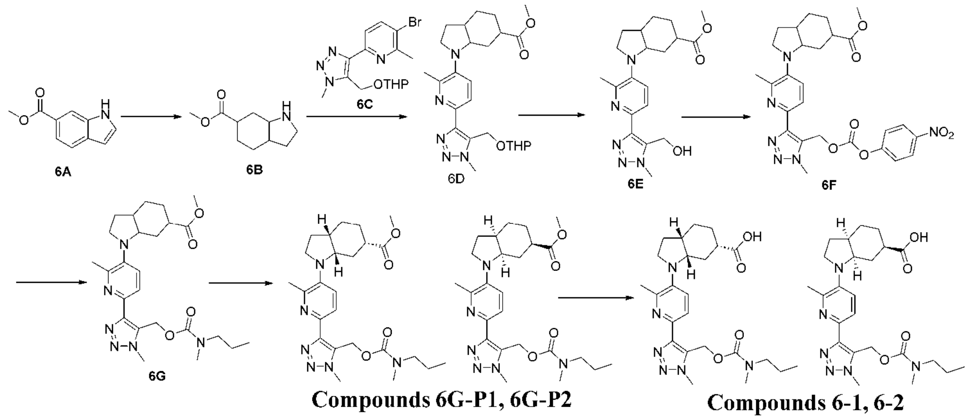
6A 6B 6C 6D 6E 6F 6G

Compounds 6G-P1, 6G-P2 Compounds 6-1, 6-2

Step 1: Compound **6A** (5.00 g, 28.6 mmol) and 70 mL of glacial acetic acid were added to a 200 mL autoclave,

followed by platinum dioxide (1.62 g, 7.15 mmol), and the autoclave was charged with hydrogen to a pressure of 2 MPa. The mixture was heated to 70°C and stirred for 48h. After filtration and concentration, the residue was neutralised to approximately pH 8 with a sodium bicarbonate solution. The crude product was purified by column chromatography (dichloromethane : anhydrous methanol = 70/10) to afford target compound **6B** (2.2 g, 42%).

LC-MS (ESI): m/z=184.1[M+H]+.

Step 2: Intermediate **6C** (1 g, 2.72 mmol) (synthesised with reference to the procedure reported in patent WO 2017223016A1), intermediate **6B** (1 g,5.44 mmol), BrettPhos-G3-Pd (270 mg, 0.25 mmol) and caesium carbonate (1.77 g, 5.44 mmol) were added to 1,4-dioxane (30 mL), and the mixture was purged three times with nitrogen, heated to 95°C and reacted for 12 h. Subsequently, the reaction system was cooled to room temperature, filtered, and concentrated, and the residue was subjected to column chromatography (PE/EA = 2/1) to afford intermediate **6D** (700 mg, 55%).

LC-MS (ESI): m/z = 470.3 [M+H]+.

Step 3: Intermediate **6D** (0.7 g, 1.49 mmol) was dissolved in anhydrous methanol (20 mL), and then pyridine p-toluenesulphonate (750 mg, 2.98 mmol) was added. After the addition was completed, the reaction mixture was stirred at 65°C for 4h, concentrated, and then diluted with water (40 mL), and extracted three times with ethyl acetate (30 mL). The organic phases were combined, dried over anhydrous sodium sulphate, and then concentrated in vacuo to afford a crude product, which was purified by column chromatography (eluents: petroleum ether/ethyl acetate = 20/80) to afford intermediate **6E** (400 mg, 70%).

LC-MS (ESI): m/z = 386.2 [M+H]+.

Step 4: Intermediate **6E** (0.4 g, 1.04 mmol) was dissolved in tetrahydrofuran (30 mL), and then pyridine (410 mg, 5.18 mmol) and 4-nitrophenyl chloroformate (630 mg, 3.13 mmol) were added. After the addition was completed, the reaction mixture was stirred at room temperature for 2h, diluted with water (40 mL), and extracted three times with ethyl acetate (50 mL). The organic phases were combined, dried over anhydrous sodium sulphate, and then concentrated in vacuo to afford a crude product, which was purified by column chromatography (eluents: petroleum ether/ethyl acetate = 40/60) to afford intermediate **6F** (500 mg, yield: 87%).

LC-MS (ESI): m/z = 551.2 [M+H]+.

Step 5: Compound **6F** (500 mg, 0.91 mmol) and N-methylpropylamine (200 mg, 0.73 mmol) were added to a reaction flask and dissolved in tetrahydrofuran (20 ml), and then DIPEA (750 mg, 2.71 mmol) was added, and the mixture was reacted at room temperature for 3 hours. Upon completion of the reaction as monitored by TLC, the reaction system was concentrated under reduced pressure, and the resulting residue was purified by silica gel column chromatography (eluents: petroleum ether/ethyl acetate = 30/70) to afford compound **6G** (400 mg, yield: 91%).

Step 6: Compound **6G** (400 mg) was further subjected to chiral resolution to afford compounds **6G-P1** (150 mg) and **6G-P2** (150 mg).

Analytical method: instrument: SHIMADZU LC-30AD, column: Chiral AD Column; mobile phase: A: $CO_2$, B: 0.05% DEA in MeOH; gradient: 5-10% B in A; flow rate: 3 mL/min; column temperature: 35°C; wavelength: 220 nm.

Preparative method: instrument: Waters 150 Prep-SFC, column: Chiral AD Column; mobile phase: A: $CO_2$, B: 0.1% NH3•H2O in ETOH; gradient: 12% B gradient elution; flow rate: 80 mL/min; column temperature: 25°C; wavelength: 220 nm; cycle time: 10.5 min; sample preparation: sample concentration 10 mg/ml, ethanol solution injection: 1.5 ml per injection. After separation, the fractions were dried using a rotary evaporator at a bath temperature of 35°C, affording compounds **6G-P1** (70 mg, retention time: 1.558 min) and **6G-P2** (80 mg, retention time: 1.799 min).

Step 7: Compound **6G-P1** (150 mg, 0.31 mmol) was added to a reaction flask and dissolved in THF : MeOH : H2O=5 : 1 : 1 (15 ml), and then anhydrous lithium hydroxide (52 mg, 1.24 mmol) was added, and the mixture was reacted at 25°C for 16 hours. Upon completion of the reaction as monitored by TLC, the reaction system was adjusted to approximately pH 7 with 1M HCl, and concentrated under reduced pressure, and the resulting residue was purified and separated by silica gel column chromatography (dichloromethane : anhydrous methanol = 10 : 1) to afford compound **6-1** (50 mg, yield: 34%).

LC-MS (ESI): m/z=471.4[M+H]+.

$^1$H NMR (400 MHz, DMSO-d6) δ 7.74 (d, 1H), 7.30 (d, 1H), 5.64 - 5.67 (m, 2H), 4.08 (s, 3H), 3.82 - 3.87 (m, 1H), 3.60 - 3.64 (m, 1H), 3.15 - 3.17 (m, 1H), 3.04 - 3.07 (m, 1H), 2.95 - 3.00 (m, 1H), 2.74 - 2.80 (m, 3H), 2.44 (s, 3H), 2.37 - 2.44 (m, 1H), 2.16 - 2.19 (m, 1H), 1.88 - 1.93 (m, 1H), 1.72-1.80 (m, 2H), 1.33 - 1.64 (m, 7H), 0.65 - 0.82 (m, 3H).

Compound **6G-P2** (150 mg, 0.31 mmol) was added to a reaction flask and dissolved in THF : MeOH : H2O=5 : 1 : 1 (15 ml), and then anhydrous lithium hydroxide (52 mg, 1.24 mmol) was added, and the mixture was reacted at 25°C for 16 hours. Upon completion of the reaction as monitored by TLC, the reaction system was adjusted to approximately pH 7 with 1M HCl, and concentrated under reduced pressure, and the resulting residue was purified and separated by silica gel column chromatography (dichloromethane : anhydrous methanol = 10 : 1) to afford compound **6-2** (40 mg, yield: 27%).

LC-MS (ESI): m/z=471.4[M+H]+.

$^1$H NMR (400 MHz, DMSO-d6) δ 7.74 (d, 1H), 7.30 (d, 1H), 5.64 - 5.67 (m, 2H), 4.08 (s, 3H), 3.82 - 3.87 (m, 1H), 3.60 - 3.64 (m, 1H), 3.15 - 3.17 (m, 1H), 3.04 - 3.07 (m, 1H), 2.95 - 3.00 (m, 1H), 2.74 - 2.80 (m, 3H), 2.44 (s, 3H), 2.37 - 2.44 (m, 1H), 2.16 - 2.19 (m, 1H), 1.88 - 1.93 (m, 1H), 1.72-1.80 (m, 2H), 1.33 - 1.64 (m, 7H), 0.65 - 0.82 (m, 3H).

## Example 7

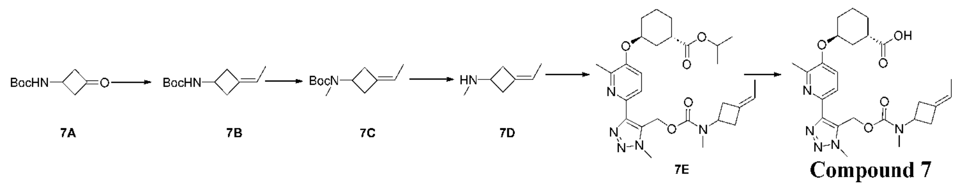

Step 1: Compound ethyltriphenylphosphonium bromide (25 g, 67.48 mmol) was added to a reaction flask and dissolved in tetrahydrofuran (300 ml). The mixture was cooled to -45°C, and then KHMDS (70 ml, 1 mol/L in THF) was added dropwise, and the mixture was reacted at this temperature for 30 min. Subsequently, compound **7A** (10 g, 53.99 mmol) was dissolved in THF (100 ml) and added dropwise to the above system at -45°C. The resulting mixture was then slowly warmed to room temperature and reacted for 16 hours. Upon completion of the reaction as monitored by TLC, the reaction system was quenched with a saturated ammonium chloride solution (200 ml), and then extracted twice with ethyl acetate (150 ml × 2). The organic phases were combined, dried, and concentrated under reduced pressure, and the resulting residue was purified by silica gel column chromatography (petroleum ether : ethyl acetate = 20 : 1) to afford compound **7B** (5.1 g, yield: 47.89%).

$^1$H NMR (400 MHz, DMSO-d6) δ 7.24 - 7.14 (m, 1H), 5.19 - 5.09 (m, 1H), 3.97 - 3.84 (m, 1H), 2.88 - 2.72 (m, 2H), 2.57 - 2.40 (m, 2H), 1.47 - 1.42 (m, 3H), 1.37 (s, 9H).

Starting from compound **7B** and following step 1 to step 4 of Example 4, the trifluoroacetate of compound 7 (53 mg, yield: 40.77%) was obtained.

$^1$H NMR (400 MHz, DMSO-d6) δ 7.89 - 7.82 (m, 1H), 7.56 - 7.50 (m, 1H), 5.67 - 5.58 (m, 3H), 5.20 - 5.11 (m, 1H), 4.82 - 4.76 (m, 1H), 4.10 (s, 3H), 2.76 (s, 3H), 2.72 - 2.55 (m, 4H), 2.43 (s, 3H), 2.06 - 1.74 (m, 4H), 1.71 - 1.35 (m, 8H).

LC-MS (ESI): m/z=484.4[M+H]$^+$.

## Example 8:

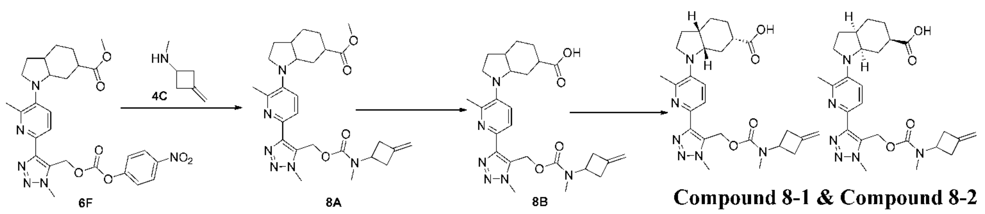


Step 1: Compound **6F** (300 mg, 0.54 mmol) and intermediate 4C (100 mg, 1.03 mmol) were added to a reaction flask and dissolved in tetrahydrofuran (20 ml), and then DIPEA (140 mg, 1.62 mmol) was added, and the mixture was reacted at room temperature for 3 hours. Upon completion of the reaction as monitored by TLC, the reaction system was concentrated under reduced pressure, and the resulting residue was purified by silica gel column chromatography (eluents: petroleum ether/ethyl acetate = 30/70) to afford compound **8A** (300 mg, yield: 91%).

LC-MS (ESI): m/z=509.3[M+H]$^+$.

Step 2: Compound **8A** (300 mg, 0.59 mmol) was added to a reaction flask and dissolved in THF : MeOH : $H_2O$ = 5 : 1 : 1 (10 ml), and then anhydrous lithium hydroxide (120 mg, 2.96 mmol) was added, and the mixture was reacted at 25°C for 16 hours. Upon completion of the reaction as monitored by TLC, the reaction system was adjusted to approximately pH 7 with 1M HCl, and concentrated under reduced pressure, and the resulting residue was purified and separated by silica gel column chromatography (eluents: dichloromethane : anhydrous methanol = 10 : 1) to afford compound **8B** (230 mg, yield: 79%).

LC-MS (ESI): m/z=495.3[M+H]$^+$.

Step 3: Compound **8B** (400 mg) was further subjected to chiral resolution to afford compounds **8-1** (100 mg) and **8-2** (100 mg).

Analytical method: instrument: SHIMADZU LC-30AD, column: Chiral AD Column; mobile phase: A: $CO_2$, B: 0.05% DEA in MeOH; gradient: 5-10% B in A; flow rate: 3 mL/min; column temperature: 35°C; wavelength: 220 nm.

Preparative method: instrument: SFC Prep 150 AP, column: Daicel IG (19 mm × 250 mm); mobile phase: A: $CO_2$,

B: 0.05% $NH_3$•$H_2O$ in ETOH; gradient: 12% B gradient elution; flow rate: 80 mL/min; column temperature: 25°C; wavelength: 220 nm; cycle time: 20 min; sample preparation: sample concentration 10 mg/ml, the sample was dissolved in DMF and filtered through a 0.45 μm filter to prepare a sample solution. After separation, the fractions were dried using a rotary evaporator at a bath temperature of 35°C, affording compounds **8-1** (100 mg, retention time: 10.0 min) and **8-2** (100 mg, retention time: 16.7 min).

**8-1:** (retention time: 10.0 min), $^1$H NMR (400 MHz, DMSO-*d6*) δ 11.77 (s, 1H), 7.75 (d, 1H), 7.31 (d, 1H), 5.71 - 5.63 (m, 2H), 4.79 (s, 2H), 4.43 - 4.39 (m, 1H), 4.08 (s, 3H), 3.87 - 3.83 (m, 1H), 3.66 - 3.60 (m, 1H), 2.99 - 2.92 (m, 1H), 2.87 - 3.58 (m, 7H), 2.43 (s, 3H), 2.41 - 2.32 (m, 1H), 2.24 - 2.21 (m, 1H), 1.92 - 1.87 (m, 1H), 1.80-1.71 (m, 2H), 1.63 - 1.51 (m, 5H).

**8-2:** (retention time: 16.7 min), $^1$H NMR (400 MHz, DMSO-d6) δ 11.77 (s, 1H), 7.75 (d, 1H), 7.31 (d, 1H), 5.71 - 5.63 (m, 2H), 4.79 (s, 2H), 4.43 - 4.39 (m, 1H), 4.08 (s, 3H), 3.87 - 3.83 (m, 1H), 3.66 - 3.60 (m, 1H), 2.99 - 2.92 (m, 1H), 2.87 - 3.58 (m, 7H), 2.43 (s, 3H), 2.41 - 2.32 (m, 1H), 2.24 - 2.21 (m, 1H), 1.92 - 1.87 (m, 1H), 1.80-1.71 (m, 2H), 1.63 - 1.51 (m, 5H).

**Example 9:**

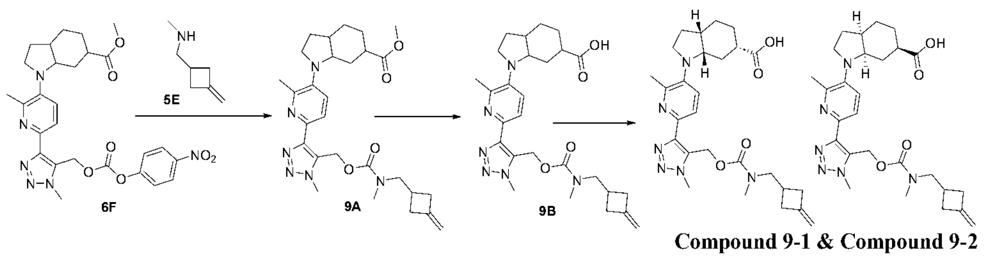


6F 9A 9B Compound 9-1 & Compound 9-2

Starting from compound **6F** and following step 1 to step 3 of Example 8, compounds **9-1** (25 mg) and **9-2** (22 mg) were obtained.

Analytical method: instrument: SHIMADZU LC-30AD, column: Chiral AD Column; mobile phase: A: $CO_2$, B: 0.05% DEA in MeOH; gradient: 5-10% B in A; flow rate: 3 mL/min; column temperature: 35°C; wavelength: 220 nm.

Preparative method: instrument: SFC Prep 150 AP, column: Daicel IG (19 mm × 250 mm); mobile phase: A: $CO_2$, B: 0.05% $NH_3$•$H_2O$ in ETOH; gradient: 12% B gradient elution; flow rate: 80 mL/min; column temperature: 25°C; wavelength: 220 nm; cycle time: 25 min; sample preparation: sample concentration 10 mg/ml, the sample was dissolved in DMF and filtered through a 0.45 μm filter to prepare a sample solution. After separation, the fractions were dried using a rotary evaporator at a bath temperature of 35°C, affording compounds **9-1** (100 mg, retention time: 12.0 min) and **9-2** (100 mg, retention time: 19.6 min).

**9-1:** (retention time: 12.0 min) $^1$H NMR (400 MHz, DMSO-d6) δ 11.68 (s, 1H), 7.75 (d, 1H), 7.31 (d, 1H), 5.70 - 5.63 (m, 2H), 4.74 - 4.59 (m, 2H), 4.08 (s, 3H), 3.87 - 3.83 (m, 1H), 3.66 - 3.60 (m, 1H), 3.33 - 3.29 (m, 1H), 3.25 - 3.19 (m, 1H), 2.99 - 2.93 (m, 1H), 2.80 - 2.65 (m, 4H), 2.53 - 2.50 (m, 1H), 2.44 (s, 3H), 2.44 - 2.32 (m, 3H), 2.26 - 2.14 (m, 2H), 1.94 - 1.87 (m, 1H), 1.80-1.71 (m, 2H), 1.63 - 1.51 (m, 5H).

**9-2:** (retention time: 19.6 min) $^1$H NMR (400 MHz, DMSO-d6) δ 11.68 (s, 1H), 7.75 (d, 1H), 7.31 (d, 1H), 5.70 - 5.63 (m, 2H), 4.74 - 4.59 (m, 2H), 4.08 (s, 3H), 3.87 - 3.83 (m, 1H), 3.66 - 3.60 (m, 1H), 3.33 - 3.29 (m, 1H), 3.25 - 3.19 (m, 1H), 2.99 - 2.93 (m, 1H), 2.80 - 2.65 (m, 4H), 2.53 - 2.50 (m, 1H), 2.44 (s, 3H), 2.44 - 2.32 (m, 3H), 2.26 - 2.14 (m, 2H), 1.94 - 1.87 (m, 1H), 1.80-1.71 (m, 2H), 1.63 - 1.51 (m, 5H).

**Example 10:**

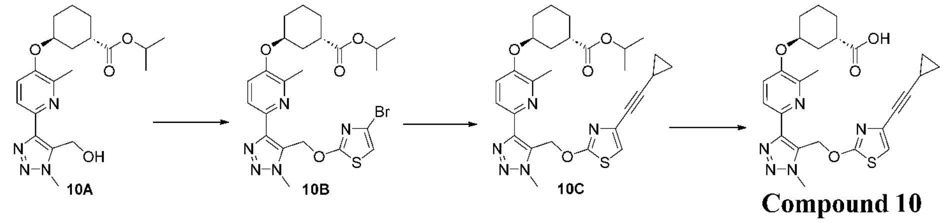

10A 10B 10C Compound 10

Step 1: Compound **10Ab** (200 mg, 0.52 mmol, prepared according to the method reported in patent WO

2017223016 A1) was dissolved in anhydrous tetrahydrofuran (8 mL), and 60% sodium hydride (31 mg, 0.77 mmol) was added in an ice bath. The mixture was stirred for 10 minutes, and then 2,4-dibromothiazole (150 mg, 0.62 mmol) was added, and the resulting mixture was reacted at room temperature for 1 hour. After the reaction was completed, the reaction solution was slowly added dropwise to 0.5 M hydrochloric acid aqueous solution (20 mL) to quench the reaction, and the mixture was extracted with ethyl acetate (15 mL × 3). The organic layers were combined, washed with a saturated sodium chloride aqueous solution (20 mL), dried over anhydrous sodium sulphate, and filtered. The filtrate was concentrated, and the residue was separated and purified by silica gel column chromatography (dichloromethane : methanol (v/v) = 25 : 1) to afford **10B** (145 mg, 51%).

LC-MS (ESI): m/z =550.1 $[M+H]^+$.

Step 2: **10B** (145 mg, 0.27 mmol), cyclopropylacetylene (52 mg, 0.79 mmol), bis(triphenylphosphine)palladium dichloride (19 mg, 0.027 mmol), cuprous iodide (10 mg, 0.05 mmol) and triethylamine (80 mg, 0.79 mmol) were dissolved in anhydrous tetrahydrofuran (8 mL). The mixture was purged with nitrogen and reacted at 40°C under nitrogen overnight. After the reaction was completed, the reaction system was cooled to room temperature. Water (20 mL) was added to the reaction solution, and the mixture was extracted with ethyl acetate (15 mL × 3). The organic layers were combined, washed with saturated sodium chloride aqueous solution (20 mL), dried over anhydrous sodium sulphate, and filtered. The filtrate was concentrated, and the residue was separated and purified by silica gel column chromatography (petroleum ether : ethyl acetate (v/v) = 5 : 1) to afford **10C** (85 mg, 60%).

LC-MS (ESI): m/z =536.3 $[M+H]^+$.

Step 3: **10C** (85 mg, 0.16 mmol) was dissolved in a mixed solution of tetrahydrofuran and water (THF : $H_2O$ (v/v) = 4 : 1, 5 mL), lithium hydroxide monohydrate (20 mg, 0.48 mmol) was added, and the mixture was reacted at room temperature overnight. After the reaction was completed, the reaction system was filtered, the filtrate was concentrated, and the residue was separated and purified by silica gel column chromatography (dichloromethane : methanol (v/v) = 20 : 1) to afford compound **10** (15 mg, 19%).

LC-MS (ESI): m/z =494.1 $[M+H]^+$.

$^1H$ NMR (400 MHz, $CDCl_3$) δ 8.04 - 8.01 (m, 1H), 7.54 - 7.51 (m, 1H), 6.76 (s, 1H), 6.03 - 5.93 (m, 2H), 4.78 (s, 1H), 4.18 (s, 3H), 2.90 - 2.85 (m, 1H), 2.62 (s, 3H), 2.13 - 1.91 (m, 4H), 1.80 - 1.64 (m, 4H), 1.43 - 1.35 (m, 1H), 0.91 - 0.83 (m, 2H), 0.81 - 0.74 (m, 2H).

**Example 11:**

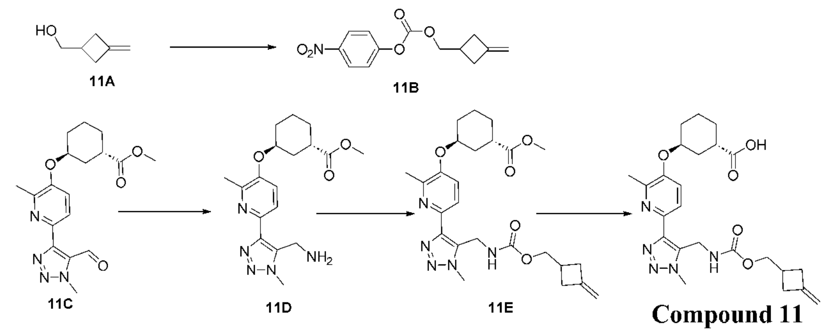

Step 1: Compound **11A** (1.0 g, 10.19 mmol) was added to 20 ml of tetrahydrofuran, followed by pyridine (4030 mg, 50.95 mmol) and 4-nitrophenyl chloroformate (6160 mg, 30.57 mmol), and the mixture was stirred at room temperature for 2 hours. Upon completion of the reaction as monitored by TLC, the reaction system was concentrated under reduced pressure, and the resulting residue was purified by silica gel column chromatography (PE : EA = 5/1) to afford compound **11B** (870 mg, yield: 32%).

LC-MS (ESI): m/z=264.1$[M+H]^+$.

Step 2: Compound **11C** (220 mg, 0.61 mmol, prepared according to the method reported in patent WO 2017223016A1) and ammonium acetate (470 mg, 6.1 mmol) were added to a reaction flask, followed by anhydrous methanol (20 ml), and the mixture was reacted at room temperature for 1 hour. Sodium cyanoborohydride (77 mg, 1.22 mmol) was then added, and the resulting mixture was stirred at room temperature for 16 hours. Upon completion of the reaction as monitored by TLC, the reaction system was concentrated under reduced pressure, and the resulting residue was purified by silica gel column chromatography (DCM : MeOH = 10/1) to afford compound **11D** (45 mg, yield: 20%).

LC-MS (ESI): m/z=360.2$[M+H]^+$.

Step 3: Compound **11D** (45 mg, 0.13 mmol) and compound **11B** (51 mg, 0.20 mmol) were added to a reaction

flask and dissolved in tetrahydrofuran (20 ml), and then DIPEA (50 mg, 0.39 mmol) was added, and the mixture was reacted at room temperature for 3 hours. Upon completion of the reaction as monitored by TLC, the reaction system was concentrated under reduced pressure, and the resulting residue was purified by silica gel column chromatography (eluents: petroleum ether/ethyl acetate = 30/70) to afford compound **11E** (25 mg, yield: 41%).

LC-MS (ESI): m/z=484.2 $[M+H]^+$.

Step 4: Compound **11E** (25 mg, 0.052 mmol) was added to a reaction flask and dissolved in THF : MeOH : $H_2O$ = 5 : 1 : 1 (10 ml), and then lithium hydroxide monohydrate (11 mg, 0.26 mmol) was added, and the mixture was reacted at 25°C for 16 hours. Upon completion of the reaction as monitored by TLC, the reaction system was adjusted to approximately pH 7 with 1M HCl, and concentrated under reduced pressure, and the resulting residue was purified and separated by silica gel column chromatography (eluents: dichloromethane : anhydrous methanol = 10 : 1) to afford compound 11 (15 mg, yield: 62%).

LC-MS (ESI): m/z=470.3$[M+H]^+$.

$^1$H NMR (400 MHz, $CD_3OD$) δ 7.81 (d, 1H), 7.45 (d, 1H), 4.80 - 4.83 (m, 1H), 4.71 - 4.73 (m, 4H), 4.15 (s, 3H), 4.05 - 4.06 (m, 2H), 2.61 - 2.75 (m, 3H), 2.54 - 2.57 (m, 1H), 2.53 (s, 3H), 2.37 - 2.43 (m, 2H), 2.07 - 2.11 (m, 1H),1.87 - 1.93 (m, 3H),1.70 - 1.77 (m, 1H),1.54 - 1.67 (m, 3H).

**Example 12:**

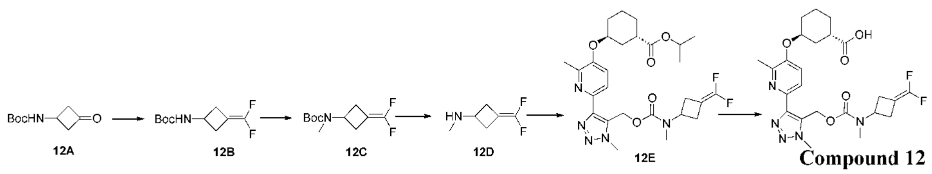

12A 12B 12C 12D 12E Compound 12

Step 1: Potassium tert-butoxide (5.0 g, 44.56 mmol) was added to a three-necked flask and dissolved in DMF (50 ml). The mixture was purged three times with nitrogen and cooled to -45°C, and a solution of **12A** (5.0 g, 27.00 mmol) in DMF (25 ml) and a solution of difluoromethyl (2-pyridyl)sulphone (4.35 g, 22.95 mmol) in DMF (25 ml) were slowly added dropwise. After the dropwise addition was completed, the resulting mixture was slowly warmed to room temperature and reacted for 16 hours. Upon completion of the reaction as monitored by TLC, a saturated ammonium chloride solution (50 ml) and 3M hydrochloric acid aqueous solution (15 ml) were added to the reaction solution, and the mixture was stirred at room temperature for 1 hour. Subsequently, the reaction system was diluted with water (100 ml) and extracted twice with ethyl acetate (100 ml × 2). The organic phases were combined, dried, and concentrated under reduced pressure, and the resulting residue was purified by silica gel column chromatography (PE : EA = 20 : 1) to afford compound **12B** (360 mg, yield: 6.08%).

$^1$H NMR (400 MHz, DMSO-d6) δ 7.40 - 7.30 (m, 1H), 4.13 - 4.00 (m, 1H), 2.95 - 2.84 (m, 2H), 2.65 - 2.54 (m, 2H), 1.38 (s, 9H).

Starting from compound **12B** and following step 1 to step 4 of Example 4, the trifluoroacetate of compound **12** (76 mg, yield: 55.07%) was obtained.

$^1$H NMR (400 MHz, DMSO-d6) δ 7.87 - 7.82 (m, 1H), 7.52 - 7.47 (m, 1H), 5.65 (s, 2H), 4.82 - 4.75 (m, 1H), 4.70 - 4.30 (m, 1H), 4.10 (s, 3H), 2.93 - 2.69 (m, 7H), 2.67 - 2.60 (m, 1H), 2.41 (s, 3H), 2.06 - 1.74 (m, 4H), 1.68 - 1.45 (m, 4H).

LC-MS (ESI): m/z=506.6$[M+H]^+$.

**Example 13**

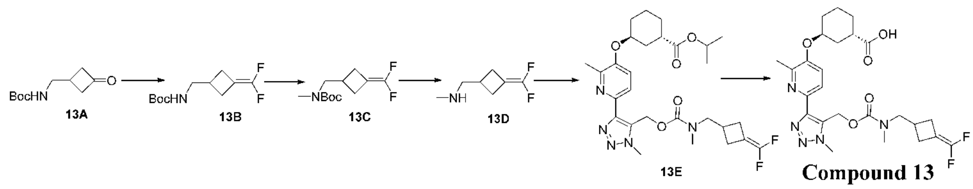

13A 13B 13C 13D 13E Compound 13

Starting from compound **13A** and following step 1 to step 5 of Example 12, the trifluoroacetate of compound **13** (85 mg, yield: 81.73%) was obtained.

$^1$H NMR (400 MHz, DMSO-d6) δ 7.87 - 7.82 (m, 1H), 7.52 - 7.46 (m, 1H), 5.71 - 5.52 (m, 2H), 4.81 - 4.75 (m, 1H), 4.10 (s, 3H), 3.39 - 3.16 (m, 2H), 2.83 - 2.56 (m, 6H), 2.42 (s, 3H), 2.39 - 2.32 (m, 1H), 2.23 - 1.97 (m, 2H), 1.92 - 1.40 (m, 8H).
LC-MS (ESI): m/z=520.6[M+H]$^+$.

**Example 14:**

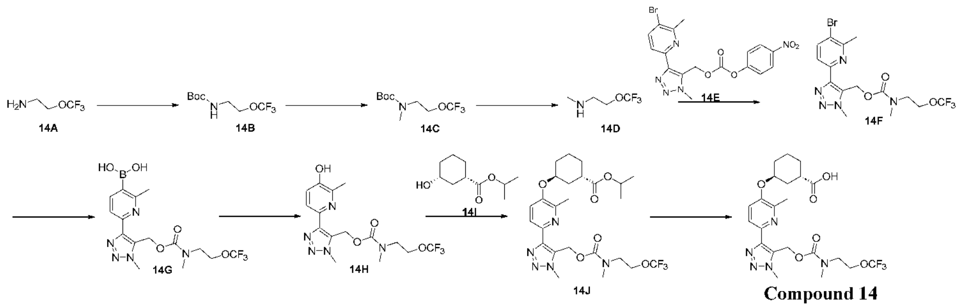

Step 1: At room temperature, **14A** (1.3 g, 10 mmol) was dissolved in dichloromethane (20 ml), triethylamine (3.03 g, 30 mmol) was added, and the mixture was stirred well. Di-tert-butyl dicarbonate (2.18 g, 10 mmol) was then added, and the resulting mixture was reacted for another 16 hours. Upon completion of the reaction as monitored by TLC, the reaction was stopped. EA (30 mL) was added to the reaction solution. The organic phase was washed with water (30 mL × 3) and a saturated sodium chloride aqueous solution (30 mL × 2), dried over anhydrous sodium sulphate, and filtered. The filtrate was concentrated, and the residue was separated and purified by column chromatography (PE : EA = 10 : 1) to afford compound **14B** (640 mg, 27.73%).
LC-MS (ESI): m/z = 230.1 [M+H]$^+$.
Step 2: At room temperature, **14B** (640 mg, 2.79 mmol) was dissolved in dry DMF (5 mL), NaH (134 mg, 3.35 mmol) was added in an ice bath, and the mixture was stirred for 15 minutes. Iodomethane (439 mg, 3.07 mmol) was then added, and the resulting mixture was reacted at room temperature for 1 hour. Upon complete depletion of starting materials as monitored by TLC, the reaction was stopped. Ethyl acetate (15 mL) was added to the reaction solution. The organic phase was washed with water (20 mL × 3) and a saturated sodium chloride aqueous solution (20 mL × 2), dried over anhydrous sodium sulphate, and filtered. The filtrate was concentrated, and the residue was separated and purified by column chromatography (PE : EA = 10 : 1) to afford compound **14C** (320 mg, 47.12%).
LC-MS (ESI): m/z = 244.1 [M+H]$^+$.
Step 3: At room temperature, **14C** (320 mg, 1.32 mmol) was dissolved in dichloromethane (5 mL), a hydrogen chloride dioxane solution (1.32 mL, 5.28 mmol, 4 M) was added, and the mixture was reacted for another 1 hour. Upon complete depletion of starting materials as monitored by TLC, the reaction was stopped. The reaction system was concentrated to afford compound **14D** (180 mg, 95.60%).
LC-MS (ESI): m/z = 144.1 [M+H]$^+$.
Step 4: At room temperature, **14E** (240 mg, 0.54 mmol) (synthesised with reference to the procedure reported in patent WO 2017223016A1) was dissolved in dry dichloromethane (20 mL), triethylamine (209 mg, 1.62 mmol) was added, and the mixture was stirred well. **14D** (170 mg, 1.19 mmol) was added in an ice bath, and the mixture was reacted at room temperature for 16 hours. Upon complete depletion of starting materials as monitored by TLC, the reaction was stopped. Dichloromethane (20 mL) was added to the reaction solution. The organic phase was washed with water (30 mL × 3) and a saturated sodium chloride aqueous solution (30 mL × 2), dried over anhydrous sodium sulphate, and filtered. The filtrate was concentrated, and the residue was separated and purified by column chromatography (PE : EA = 2 : 1) to afford compound **14F** (230 mg, 94.99%).
LC-MS (ESI): m/z = 452.4 [M+H]$^+$.
Step 5: At room temperature, **14F** (230 mg, 0.51 mmol) was dissolved in dry tetrahydrofuran (10 mL), and bis(pinacolato)diboron (259 mg, 1.02 mmol), Pd(dppf)$Cl_2$ (83 mg, 0.10 mmol), and potassium acetate (100 mg, 1.02 mmol) were added. Under nitrogen atmosphere, the mixture was reacted at 80°C for 16 hours. Upon complete depletion of starting materials as monitored by TLC, the reaction was stopped. The reaction system was cooled to room temperature, and ethyl acetate (20 mL) was added to the reaction solution. The organic phase was washed with water (20 mL × 3) and a saturated sodium chloride aqueous solution (20 mL × 2), dried over anhydrous sodium sulphate, and filtered. The filtrate was concentrated to afford compound **14G** (180 mg, 84.84%) without purification.
LC-MS (ESI): m/z = 418.5 [M+H]$^+$.
Step 6: At room temperature, **14G** (180 mg, 0.43 mmol) was dissolved in ethyl acetate (5 mL), hydrogen peroxide

(244 mg, 2.15 mmol, 30%) was added dropwise to the reaction solution in an ice bath, and the mixture was reacted at room temperature for two hours. Upon complete depletion of starting materials as monitored by TLC, the reaction was stopped. Ethyl acetate (10 mL) was added to the reaction solution. The organic phase was washed with water (20 mL × 3) and a saturated sodium chloride aqueous solution (20 mL × 2), dried over anhydrous sodium sulphate, and filtered. The filtrate was concentrated, and the residue was separated and purified by column chromatography (PE : EA = 1 : 1) to afford compound **14H** (90 mg, 53.57%).

LC-MS (ESI): m/z = 390.3 $[M+H]^+$.

Step 7: At room temperature, **14H** (90 mg, 0.23 mmol) was dissolved in dry toluene (5 mL), **141** (86 mg, 0.46 mmol) (synthesised with reference to the procedure reported in patent WO 2017223016A1) and tri-n-butylphosphine (140 mg, 0.69 mmol) were added, and the mixture was stirred well. 1,1'-(Azodicarbonyl)dipiperidine (174 mg, 0.69 mmol) was then added, and the mixture was reacted at 50°C under nitrogen atmosphere for 16 hours. Upon complete depletion of starting materials as monitored by TLC, the reaction was stopped. The reaction system was cooled to room temperature, and ethyl acetate (15 mL) was added to the reaction solution. The organic phase was washed with water (20 mL × 3) and a saturated sodium chloride aqueous solution (20 mL × 2), dried over anhydrous sodium sulphate, and filtered. The filtrate was concentrated, and the residue was separated and purified by column chromatography (PE : EA = 1 : 1) to afford compound **14J** (100 mg, 77.59%).

LC-MS (ESI): m/z = 558.7 $[M+H]^+$.

Step 8: At room temperature, **14J** (100 mg, 0.18 mmol) was dissolved in tetrahydrofuran (3 mL), water (3 mL) was added, and the mixture was stirred well. Lithium hydroxide (22 mg, 0.9 mmol) was then added, and the resulting mixture was reacted at room temperature for 16 hours. Upon complete depletion of starting materials as monitored by TLC, the reaction was stopped. Ethyl acetate (10 mL) was added to the reaction solution, and dilute hydrochloric acid was added to adjust the pH to weakly acidic. The phases were separated, and the organic phase was washed with a saturated sodium chloride aqueous solution (10 mL × 2), dried over anhydrous sodium sulphate, and filtered. The filtrate was concentrated, and the residue was separated and purified by column chromatography (DCM : MeOH = 15 : 1) to afford compound **14** (35 mg, 37.86%).

LC-MS (ESI): m/z = 516.1 $[M+H]^+$.

$^1$H NMR (400 MHz, DMSO-d6) δ 7.85 - 7.83 (d, 1H), 7.48 - 7.46 (d, 1H), 5.69 - 5.64 (d, 2H), 4.77 (s, 1H), 4.18 (s, 1H), 4.09 (s, 3H), 4.00 (s, 1H), 3.54 - 3.44 (d, 2H), 2.86 - 2.81 (d, 3H), 2.68 - 2.59 (m, 1H), 2.40 (s, 3H), 2.03 - 1.99 (m, 1H), 1.88 - 1.77 (m, 3H), 1.66 - 1.45 (m, 4H).

**Example 15**

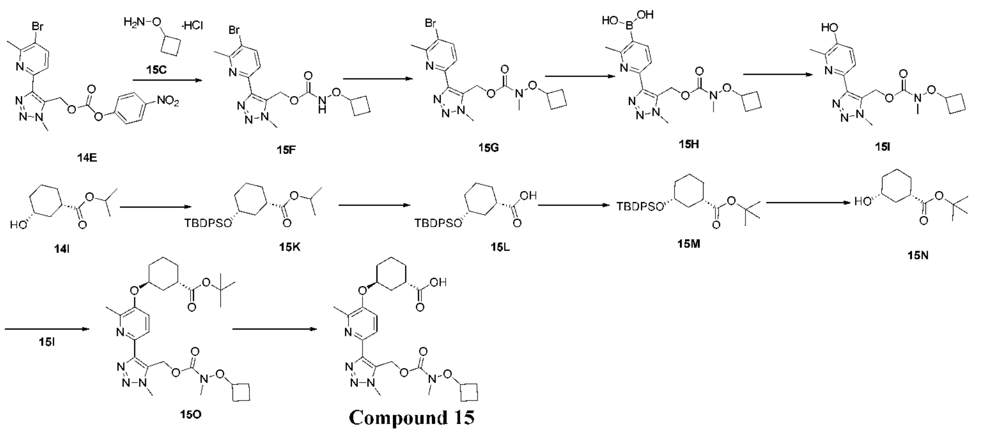

14E 15F 15G 15H 15I
14I 15K 15L 15M 15N
15O Compound 15

Step 1: At room temperature, **14E** (1.5 g, 3.35 mmol) and N,N-diisopropylethylamine (1.73 g, 13.4 mmol) were added to the reaction solution, followed by **15C** (1.1 g, hydrochloride salt, with reference to the procedure reported in ChemMedChem. 2012 Jul;7(7):1230-6.), and the mixture was reacted at room temperature overnight. After the reaction was completed, the reaction solution was concentrated, and the residue was purified by column chromatography (petroleum ether : ethyl acetate (v/v) = 1 : 1) to afford target compound **15F** (0.85 g, 64%).

LC-MS (ESI): m/z= 398.0$[M+H]^+$.

Step 2: **15F** (850 mg, 2.15 mmol) was dissolved in N,N-dimethylformamide (20 mL), sodium hydride (250 mg, 6.45 mmol, 60%) was added at 0°C, and the mixture was reacted for 0.5 hours. Iodomethane (920 mg, 6.45 mmol) was then added, and the resulting mixture was reacted at 40°C overnight. After the reaction was completed, the reaction

solution was quenched with a saturated ammonium chloride solution, extracted with ethyl acetate, washed, and concentrated, and the residue was purified by column chromatography (petroleum ether : ethyl acetate (v/v) = 1 : 1) to afford target compound **15G** (0.5 g, 1.22 mmol).

LC-MS (ESI): m/z= 410.0[M+H]$^+$.

Step 3: **15G** (0.5 g, 1.22 mmol), bis(pinacolato)diboron (0.46 g, 1.83 mmol), potassium acetate (0.30 g, 3.05 mmol), and [1,1'-bis(diphenylphosphino)ferrocene]palladium dichloride dichloromethane complex (89 mg, 0.12 mmol) were dissolved in 1,4-dioxane (10 mL), and the mixture was reacted at 80°C under nitrogen atmosphere for 16 hours. After the reaction was completed, the reaction solution was extracted with ethyl acetate, washed, and concentrated. The resulting residue was directly used in the next step.

LC-MS (ESI): m/z= 376.1 [M+H]$^+$.

Step 4: The product from the previous step was dissolved in ethyl acetate (10 mL), a hydrogen peroxide solution (0.61 mL, 6 mmol, 30%) was added at 0°C, and the mixture was reacted at room temperature for 2 hours. After the reaction was completed, the reaction solution was quenched with a saturated sodium thiosulphate solution, extracted with ethyl acetate, washed, and concentrated, and the residue was purified by column chromatography (petroleum ether : ethyl acetate (v/v) = 1 : 1) to afford target compound **151** (0.3 g, 72%).

LC-MS (ESI): m/z= 348.1[M+H]$^+$.

Step 5: **141** (5 g, 26.85 mmol), tert-butyldiphenylchlorosilane (8.12 g, 29.54 mmol), and imidazole (5.48 g, 80.55 mmol) were dissolved in N,N-dimethylformamide (50 mL), and the mixture was reacted at 60°C for 16 hours. After the reaction was completed, the reaction solution was extracted with ethyl acetate, washed, and concentrated, and the residue was purified by column chromatography (petroleum ether : ethyl acetate (v/v) = 10 : 1) to afford target compound **15K** (10.3 g, 90%).

LC-MS (ESI): m/z=425.3[M+H]$^+$.

Step 6: **15K** (10.3 g, 24.26 mmol) was dissolved in methanol (40 mL), tetrahydrofuran (40 mL), and water (20 mL), and then lithium hydroxide monohydrate (3.05 g, 72.78 mmol) was added, and the mixture was reacted at room temperature for 2 hours. After the reaction was completed, the reaction solution was adjusted to pH=5-6, extracted with ethyl acetate, washed, and concentrated to afford target compound **15L** (9.2 g, 99%).

LC-MS (ESI): m/z= 383.3[M+H]$^+$.

Step 7: **15L** (9.2 g, 24.05 mmol) and N,N-dimethylformamide di-tert-butyl acetal (19.56 g, 96.2 mmol) were dissolved in toluene (150 mL), and the mixture was reacted at 110°C for 24 hours. After the reaction was completed, the reaction solution was extracted with ethyl acetate, washed, and concentrated, and the residue was purified by column chromatography (petroleum ether : ethyl acetate (v/v) = 10 : 1) to afford target compound **15M** (4.1 g, 38%).

LC-MS (ESI): m/z= 383.1[M+H-56]$^+$.

Step 8: **15M** (4.1 g, 9.35 mmol) was dissolved in tetrahydrofuran (40 mL), and then tetrabutylammonium fluoride (18.7 mL, 18.7 mmol, 1M) was added, and the mixture was reacted at room temperature for 16 hours. After the reaction was completed, the reaction solution was extracted with ethyl acetate, washed, and concentrated, and the residue was purified by column chromatography (petroleum ether : ethyl acetate (v/v) = 3 : 1) to afford target compound **15N** (2.2 g, impure).

Step 9: **151** (0.1 g, 0.29 mmol), **15N** (0.17 g, 0.87 mmol), and triphenylphosphine (0.15 g, 0.58 mmol) were dissolved in 1,2-dichloroethane (5 mL), 1,1'-(azodicarbonyl)dipiperidine (0.12 g, 0.58 mmol) was added dropwise at 0°C, and then the mixture was heated to 80°C and reacted for 16 hours. After the reaction was completed, the reaction solution was concentrated, and the residue was purified by column chromatography (petroleum ether : ethyl acetate (v/v) = 1 : 2) to afford target compound **150** (30 mg, 19%).

LC-MS (ESI): m/z= 530.3 [M +1]$^+$.

Step 10: **150** (30 mg, 0.06 mmol) was dissolved in dichloromethane (2 mL), and then trifluoroacetic acid (0.5 mL) was added, and the mixture was reacted at room temperature for 1 hour. After the reaction was completed, the reaction solution was concentrated, and the residue was purified by preparative chromatography to afford the trifluoroacetate of target compound **15** (2.09 mg, 7%).

LC-MS (ESI): m/z= 474.2[M +1]$^+$.

$^1$H NMR (400 MHz, $CDCl_3$) δ 8.01 (d, 1H), 7.63 (d, 1H), 5.70 (dd, 2H), 4.31 (t, 1H), 4.19 (s, 3H), 3.11 (s, 3H), 2.90 (m, 1H), 2.74 (s, 3H), 2.15 - 2.10 (m, 2H), 2.07 - 2.00 (m, 4H), 1.97 - 1.91 (m, 2H), 1.80 - 1.78 (m, 3H), 1.71 - 1.66 (m, 2H), 1.49 - 1.46 (m, 1H), 1.26 (m, 1H).

**Example 16:**

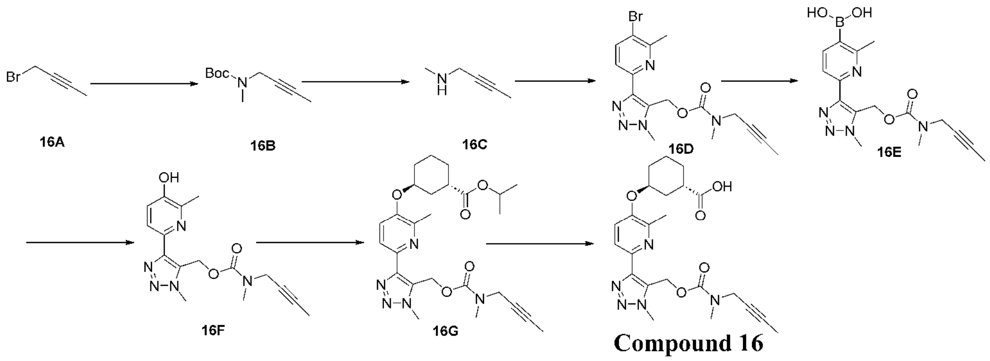

16A 16B 16C 16D 16E 16F 16G Compound 16

Step 1: At room temperature, tert-butyl methylcarbamate (1.3 g, 10 mmol) was dissolved in dry DMF (10 mL), NaH (440 mg, 11 mmol) was added in an ice bath, and the mixture was stirred for 30 minutes. 16A (1.32 g, 10 mmol) was then added, and the resulting mixture was reacted at room temperature for 16 hours. Upon complete depletion of starting materials as monitored by TLC, the reaction was stopped. Ethyl acetate (20 mL) was added to the reaction solution. The organic phase was washed with water (20 mL × 3) and a saturated sodium chloride aqueous solution (20 mL × 2), dried over anhydrous sodium sulphate, and filtered. The filtrate was concentrated, and the residue was separated and purified by column chromatography (PE : EA = 10 : 1) to afford compound **16B** (1.7 g, 94.90%).

LC-MS (ESI): m/z = 184.2 $[M+H]^+$.

Step 2: At room temperature, **16B** (1.7 g, 9.29 mmol) was dissolved in dichloromethane (10 mL), a hydrogen chloride dioxane solution (9.3 mL, 37.15 mmol, 4 M) was added, and the mixture was reacted for another 1 hour. Upon complete depletion of starting materials as monitored by TLC, the reaction was stopped. The reaction system was concentrated to afford compound 16C (760 mg, 98.54%) without purification.

LC-MS (ESI): m/z = 84.2 $[M+H]^+$.

Starting from compound **16C** and following step 4 to step 8 of Example 14, compound **16** (25 mg, 24.83%) was obtained.

LC-MS (ESI): m/z = 456.1 $[M+H]^+$.

$^1$H NMR (400 MHz, $CDCl_3$) δ 7.99 - 7.96 (d, 1H), 7.37 - 7.35 (d, 1H), 5.76 - 5.73 (d, 2H), 4.74 (s, 1H), 4.16 (s, 3H), 4.04 - 3.92 (d, 2H), 2.94 - 2.90 (d, 4H), 2.58 (s, 3H), 2.15-2.11 (d, 1H), 2.01 - 1.89 (m, 3H), 1.80 - 1.78 (d, 3H), 1.74 - 1.64 (m, 4H).

**Example 17**

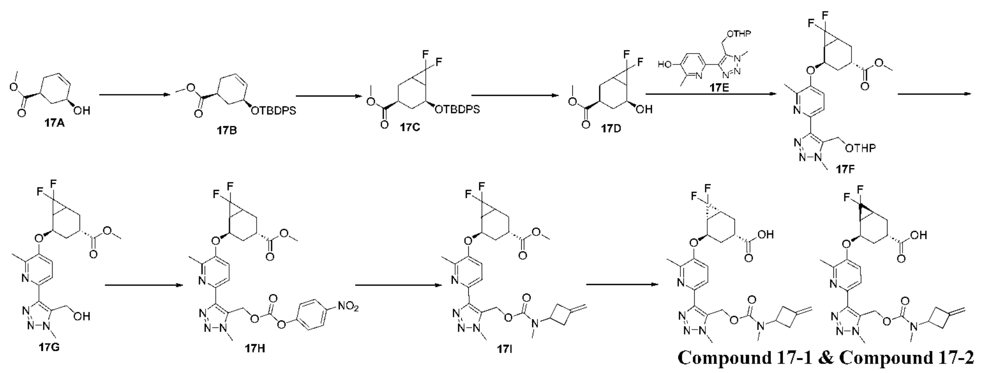

17A 17B 17C 17D 17E 17F 17G 17H 17I Compound 17-1 & Compound 17-2

Step 1: Compound **17A** (synthesised with reference to the procedure reported in patent WO 2019/126093) (4.68 g, 30 mmol) was dissolved in dichloromethane (100 mL), triethylamine (9.18 g, 90 mmol), DMAP (366 mg, 3 mmol) and tert-butyldiphenylchlorosilane (9.1 g, 33 mmol) were added, and the mixture was reacted at room temperature for 3 h. Upon complete depletion of starting materials, the reaction system was concentrated, and the crude product was separated by silica gel column chromatography (petroleum ether : ethyl acetate (v : v) = 20 : 1) to afford target compound **17B** (10.9 g, yield: 92.3%).

Step 2: Compound **17B** (10.9 g, 27.6 mmol) was dissolved in anhydrous toluene (100 mL), trimethylsilyl 2-(fluorosulphonyl)difluoroacetate (34.5 g, 138 mmol) was added, and the mixture was reacted at 110°C overnight. The reaction system was successively washed with saturated sodium thiosulphate and a saturated sodium chloride

aqueous solution. The organic phase was dried and concentrated, and the resulting residue was separated by silica gel column chromatography (petroleum ether : ethyl acetate (v : v) = 20 : 1) to afford target compound **17C** (3.1 g, yield: 25.3%).

Step 3: Compound **17C** (3.1 g, 7.0 mmol) was dissolved in tetrahydrofuran (20 ml), excess tetrabutylammonium fluoride was added, and the mixture was stirred at room temperature overnight. After the reaction was completed, ethyl acetate (100 mL) was added, and the mixture was washed with water (100 mL × 4). The organic phase was collected, dried over anhydrous sodium sulphate, and concentrated, and the resulting crude product was separated by silica gel column chromatography (petroleum ether : ethyl acetate (v : v) = 3 : 1) to afford target title compound **17D** (1.1 g, 78.5%).

Step 4: At room temperature, **17D** (1.1 g, 5.3 mmol) was dissolved in dry toluene (100 mL), **17E** (1.8 g, 6 mmol) (synthesised with reference to the procedure reported in patent WO 2019/126093) and tri-n-butylphosphine (3.2 g, 15.9 mmol) were added, and the mixture was stirred well. 1,1'-(Azodicarbonyl)dipiperidine (4.0 g, 15.9 mmol) was then added, and the mixture was reacted at 50°C under nitrogen atmosphere for 16 hours. Upon complete depletion of starting materials as monitored by TLC, the reaction was stopped. The reaction system was cooled to room temperature, and ethyl acetate (100 mL) was added to the reaction solution. The organic phase was washed with water (100 mL × 3) and a saturated sodium chloride aqueous solution (100 mL × 2), dried over anhydrous sodium sulphate, and filtered. The filtrate was concentrated, and the residue was separated and purified by column chromatography (PE : EA = 1 : 1) to afford target compound **17F** (1.1 g, 43.3%).

LC-MS (ESI): m/z = 493.2 $[M+H]^+$.

Starting from compound **17F** and following steps 3, 4, 5 and 7 of Example 6, the trifluoroacetate of compound **17-1** (preparative HPLC retention time: 18.85 min, 9.3 mg) and the trifluoroacetate of compound **17-2** (HPLC retention time: 19.23 min, 16.5 mg) were obtained. Preparative HPLC method: instrument: waters 2767 preparative liquid chromatography system, column: Sunfire Prep C18 (19 mm × 250 mm) 5 um; mobile phase: A: acetonitrile, B: water (containing 0.1% TFA, v/v); gradient: mobile phase A was increased from 10% to 60% over 20 minutes, gradient elution; flow rate: 15 mL/min.

Compound **17-1** (preparative HPLC retention time: 18.85 min): LC-MS (ESI): m/z =518.8$[M+H]^+$.

Compound **17-2** (preparative HPLC retention time: 19.23 min): $^1$H NMR (400 MHz, DMSO-$d_6$) δ 7.85 (d, 1H), 7.48 (d, 1H), 5.70 (s, 2H), 4.78 (s, 1H), 4.21-4.01 (m, 6H), 3.21 - 3.16 (m, 1H), 2.97 - 2.55 (m, 5H), 2.41 (s, 3H), 2.07 - 1.97 (m, 1H), 1.91 - 1.72 (m, 3H), 1.69 - 1.40 (m, 4H).

LC-MS (ESI): m/z = 518.8 $[M+H]^+$.

## Example 18

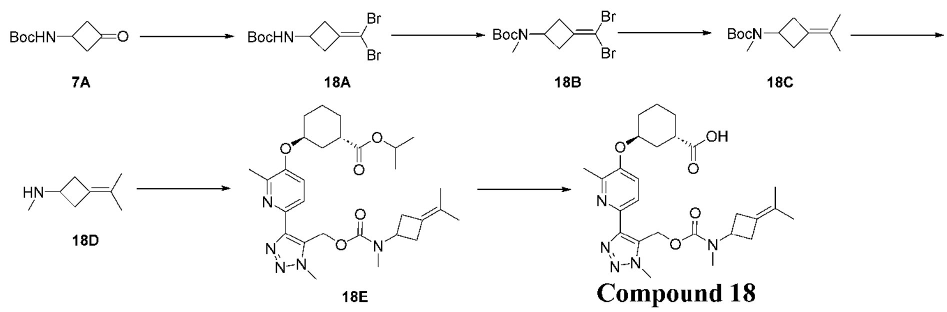

7A 18A 18B 18C

18D 18E Compound 18

Step 1: Compound **7A** (5.0 g, 27.0 mmol) and carbon tetrabromide (17.9 g, 54.0 mmol) were added to a reaction flask and mixed with toluene (200 ml), and then triphenylphosphine (28.3 g, 108.0 mmol) was added, and the mixture was reacted at 80°C for 3 hours. Upon completion of the reaction as monitored by TLC, the reaction system was cooled to room temperature and concentrated under reduced pressure to remove toluene, and the residue was dissolved in ethyl acetate (200 ml), and then washed twice with water (100 ml × 2). The organic phase was collected, dried, and concentrated under reduced pressure, and the resulting residue was purified by silica gel column chromatography (PE : EA = 5 : 1) to afford compound **18A** (5.4 g, yield: 59.01%).

$^1$H NMR (400 MHz, DMSO-d6) δ 7.34 - 7.26 (m, 1H), 4.01 - 3.90 (m, 1H), 2.88 - 2.78 (m, 2H), 2.56 - 2.51 (m, 2H), 1.38 (s, 9H).

Step 2: Compound **18A** (2.0 g, 5.90 mmol) was added to a reaction flask and dissolved in tetrahydrofuran (50 ml), and the mixture was cooled to 0°C. NaH (0.35 g, 8.85 mmol) was then added, and the resulting mixture was reacted at this temperature for 30 min. Subsequently, iodomethane (1.7 g, 11.80 mmol) was added, and the resulting mixture was warmed to room temperature and reacted for 2 hours. Upon completion of the reaction as monitored by TLC, the reaction system was quenched with a saturated ammonium chloride solution (100 ml), and then extracted twice with ethyl acetate

(100 ml × 2). The organic phases were combined, dried, and concentrated under reduced pressure, and the resulting residue was purified by silica gel column chromatography (PE : EA = 30 : 1) to afford compound **18B** (2.02 g, yield: 97.11%).

$^1$H NMR (400 MHz, DMSO-d6) δ 4.39 (s, 1H), 2.86 - 2.71 (m, 7H), 1.40 (s, 9H).

Step 3: Cuprous iodide (10.8 g, 57.20 mmol) was added to a reaction flask and mixed with tetrahydrofuran (60 ml), and a methyl lithium solution (43 ml, 1.6 mol/L in ether) was added dropwise at 0°C. After the dropwise addition was completed, a solution of compound **18B** (2.02 g, 5.72 mmol) in tetrahydrofuran (60 ml) was added. After the dropwise addition was completed, the mixture was warmed to room temperature and reacted for 16 hours. Upon completion of the reaction as monitored by TLC, the reaction system was quenched with a saturated ammonium chloride solution (100 ml), and then extracted twice with ethyl acetate (50 ml × 2). The organic phases were combined, dried, and concentrated under reduced pressure, and the resulting residue was purified by silica gel column chromatography (PE : EA = 20 : 1) to afford compound **18C** (800 mg, yield: 62.02%).

$^1$H NMR (400 MHz, DMSO-d6) δ 4.36 (s, 1H), 2.77 - 2.64 (m, 7H), 1.50 (s, 6H), 1.39 (s, 9H).

Step 4: Compound **18C** (300 mg, 1.33 mmol) was added to a reaction flask and dissolved in dichloromethane (6 ml), and then trifluoroacetic acid (1 ml) was added, and the mixture was reacted at room temperature for 2 hours. Upon completion of the reaction as monitored by TLC, the reaction system was concentrated under reduced pressure to afford compound **18D** (170 mg, TFA salt), which was directly used in the next step.

LC-MS (ESI): m/z=126.2 $[M+H]^+$.

Step 5: Crude compound **18D** (170 mg) was added to a reaction flask and dissolved in dichloromethane (10 ml), and then DIPEA (0.6 ml, 3.60 mmol) and compound **2D** (100 mg, 0.18 mmol) were successively added, and the mixture was reacted at room temperature for 2 hours. Upon completion of the reaction as monitored by TLC, the reaction system was concentrated under reduced pressure, and the resulting residue was purified by silica gel column chromatography (petroleum ether : ethyl acetate = 1 : 1) to afford compound **18E** (90 mg, yield: 92.78%).

LC-MS (ESI): m/z=540.6 $[M+H]^+$.

Step 6: Compound **18E** (90 mg, 0.17 mmol) was added to a reaction flask and dissolved in THF : MeOH : $H_2O$=1 : 1 : 1 (9 ml), and then anhydrous lithium hydroxide (17 mg, 0.68 mmol) was added, and the mixture was reacted at room temperature for 16 hours. Upon completion of the reaction as monitored by TLC, the reaction system was concentrated under reduced pressure, and the resulting residue was separated by C18 reversed-phase column chromatography to afford the trifluoroacetate of compound **18** (43 mg, yield: 50.83%).

$^1$H NMR (400 MHz, DMSO-*d6*) δ 7.89 - 7.81 (m, 1H), 7.52 - 7.47 (m, 1H), 5.64 (s, 2H), 4.81 - 4.75 (m, 1H), 4.09 (s, 3H), 2.75 (s, 3H), 2.70 - 2.56 (m, 5 H), 2.44 - 2.31 (m, 4H), 2.06 - 1.95 (m, 1H), 1.91 - 1.73 (m, 3H), 1.68 - 1.50 (m, 4H), 1.45 (s, 6H).

LC-MS (ESI): m/z=498.3 $[M+H]^+$.

**Example 19**

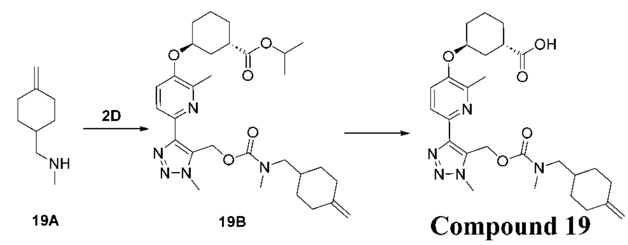


19A 19B **Compound 19**

Step 1: Compound **2D** (200 mg, 0.36 mmol) and compound **19A** (100 mg, 0.72 mmol, synthesised according to the method reported in patent EP585130A2) were added to a reaction flask and dissolved in dichloromethane (10 ml), and then DIPEA (0.20 ml, 1.2 mmol) was added, and the mixture was reacted at room temperature for 3 hours. Upon completion of the reaction as monitored by TLC, the reaction system was concentrated under reduced pressure, and the resulting residue was purified by silica gel column chromatography (petroleum ether : ethyl acetate = 1 : 1) to afford compound **19B** (110 mg, yield: 54.99%).

LC-MS (ESI): m/z=554.6$[M+H]^+$.

Step 2: Compound **19B** (110 mg, 0.20 mmol) was dissolved in a mixed solvent of tetrahydrofuran/water (8 mL, 3 : 1, v : v), and then lithium hydroxide (35 mg, 0.80 mmol) was added, and the reaction solution was reacted at 30°C for 16 hours. Upon completion of the reaction as monitored by TLC, the reaction system was concentrated under reduced pressure, and the resulting residue was purified by silica gel column chromatography (ethyl acetate) and then further separated by C18 reversed-phase column chromatography to afford target compound **19** (32 mg, yield: 31.48%).

LC-MS (ESI): m/z=512.4[M+H]+.

$^1$H NMR (400 MHz, $CDCl_3$) δ 7.97 - 7.94 (m, 1H), 7.22 - 7.19 (m, 1H), 5.76 - 5.74 (m, 2H), 5.35 - 5.21 (m, 1H), 4.70 (s, 1H), 4.62 - 4.47 (m, 1H), 4.13 (s, 3H), 3.23 - 3.16 (m, 1H), 3.06 - 2.98 (m, 1H), 2.91 - 2.82 (m, 4H), 2.49 (s, 3H), 2.17 - 2.13 (m, 1H), 1.98 - 1.92 (m, 4H), 1.80 - 1.74 (m, 3H), 1.70 - 1.64 (m, 5H), 1.58 - 1.56 (m, 2H), 1.25 - 1.07 (m, 2H).

**Example 20:**

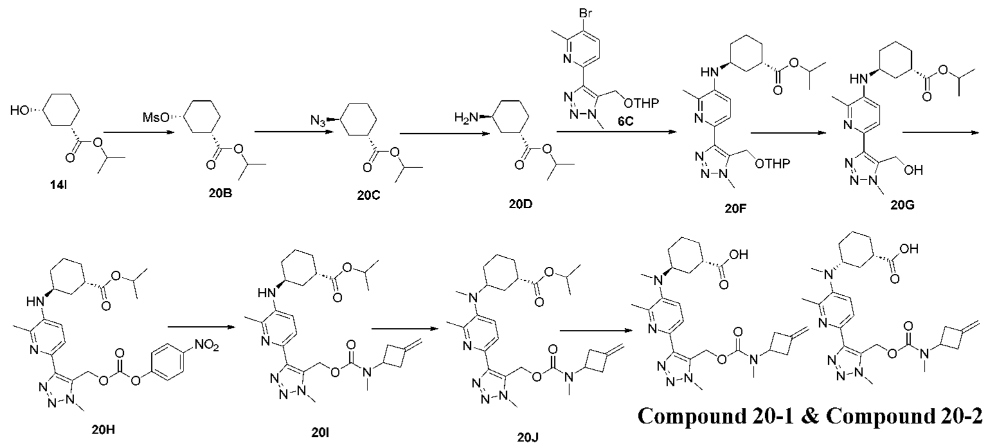

Compound 20-1 & Compound 20-2

Step 1: Compound **141** (1500 mg, 8.05 mmol, prepared according to the method reported in patent WO 2017223016 A1) was added to 40 ml of dichloromethane, and then triethylamine (1630 mg, 16.1 mmol) and methylsulphonyl chloride (1200 mg, 10.47 mmol) were added, and the mixture was stirred at room temperature for 2 hours. Upon completion of the reaction as monitored by TLC, the reaction system was diluted with a saturated sodium bicarbonate solution and extracted with dichloromethane. The organic phase was washed with a saturated sodium chloride aqueous solution, dried over anhydrous sodium sulphate, filtered, and concentrated under reduced pressure to afford compound **20B** (1500 mg, yield: 70%).

Step 2: Compound **20B** (1.5 g, 5.67 mmol) and sodium azide (1.11 g, 17.01 mmol) were added to a reaction flask, followed by DMF (30 ml), and the mixture was heated to 110°C and reacted for 2 hours. The reaction system was cooled, diluted with water, extracted with ethyl acetate, washed with a saturated sodium chloride aqueous solution, dried over anhydrous sodium sulphate, filtered, and concentrated under reduced pressure to afford compound **20C** (1.1 g, yield: 92%).

Step 3: Compound **20C** (1.1 g, 5.21 mmol) and triphenylphosphine (2.73 g, 10.42 mmol) were added to a mixed solvent of tetrahydrofuran (20 ml) and water (20 ml), and the mixture was purged with nitrogen and reacted at room temperature under nitrogen for 10 hours. Upon completion of the reaction as monitored by TLC, the reaction system was concentrated under reduced pressure, and the resulting residue was purified by silica gel column chromatography (eluents: DCM/MeOH = 100/15) to afford compound **20D** (710 mg, yield: 74%).

LC-MS (ESI): m/z=186.1 [M+H]+.

Step 4: Compound **20D** (400 mg, 2.16 mmol) and **6C** (190 mg, 2.16 mmol, prepared according to the method reported in patent WO 2017223016A1) were added to 1,4-dioxane (20 ml), followed by RuphosPdG3 (90 mg, 0.11 mmol) and caesium carbonate (1.41 g, 4.32 mmol). The mixture was purged with nitrogen, heated to 110°C and reacted under nitrogen for 10 hours. Upon completion of the reaction as monitored by TLC, the reaction system was cooled and concentrated under reduced pressure, and the resulting residue was purified by silica gel column chromatography (eluents: PE/EA = 1/1) to afford compound **20F** (500 mg, yield: 49%).

LC-MS (ESI): m/z=472.3 [M+H]+.

Step 5: Compound **20F** (0.5 g, 1.06 mmol) was dissolved in anhydrous methanol (20 mL), and then pyridine p-toluenesulphonate (530 mg, 2.12 mmol) was added. After the addition was completed, the reaction mixture was stirred at 65°C for 4h, concentrated, and then diluted with water (20 mL), and extracted three times with ethyl acetate (30 mL). The organic phases were combined, dried over anhydrous sodium sulphate, and then concentrated in vacuo to afford a crude product, which was purified by column chromatography (eluents: petroleum ether/ethyl acetate = 20/80) to afford compound **20G** (400 mg, 97%).

LC-MS (ESI): m/z = 388.2 [M+H]+.

Step 6: Compound **20G** (0.4 g, 1.03 mmol) was dissolved in tetrahydrofuran (30 mL), and then pyridine (410 mg, 5.15 mmol) and 4-nitrophenyl chloroformate (620 mg, 3.09 mmol) were added. After the addition was completed, the

reaction mixture was stirred at room temperature for 2 hours, diluted with water (40 mL), and extracted three times with ethyl acetate (50 mL). The organic phases were combined, dried over anhydrous sodium sulphate, and then concentrated in vacuo to afford a crude product, which was purified by column chromatography (eluents: petroleum ether/ethyl acetate = 40/60) to afford compound **20H** (140 mg, yield: 25%).

LC-MS (ESI): m/z = 553.2 $[M+H]^+$.

Step 7: Compound **20H** (140 mg, 0.25 mmol) and N-methyl-3-methylenecyclobutan-1-amine hydrochloride (43 mg, 0.33 mmol) were added to a reaction flask and dissolved in tetrahydrofuran (10 ml), and then DIPEA (97 mg, 0.75 mmol) was added, and the mixture was reacted at room temperature for 3 hours. Upon completion of the reaction as monitored by TLC, the reaction system was concentrated under reduced pressure, and the resulting residue was purified by silica gel column chromatography (eluents: petroleum ether/ethyl acetate = 30/70) to afford compound **20I** (120 mg, yield: 93%).

LC-MS (ESI): m/z = 511.3 $[M+H]^+$.

Step 8: Compound **20I** (120 mg, 0.24 mmol) and iodomethane (51 mg, 0.36 mmol) were dissolved in dry tetrahydrofuran (30 mL), and LiHMDS (80 mg, 0.48 mmol) was slowly added in an ice bath. After the addition was completed, the mixture was stirred and reacted for another 2h. Upon complete depletion of starting materials as monitored by TLC, a saturated ammonium chloride solution was added to quench the reaction, and the mixture was extracted three times with ethyl acetate (50 mL). The organic phases were combined, dried over anhydrous sodium sulphate, and concentrated in vacuo to afford a crude product, which was purified by column chromatography (eluents: petroleum ether/ethyl acetate = 40/60) to afford compound **20J** (80 mg, yield: 73%).

LC-MS (ESI): m/z = 525.3 $[M+H]^+$.

Step 9: Compound **20J** (80 mg, 0.15 mmol) was added to a reaction flask and dissolved in THF : MeOH : H2O=5 : 1 : 1 (7 ml), and then lithium hydroxide monohydrate (31 mg, 0.75 mmol) was added, and the mixture was reacted at 25°C for 16 hours. Upon completion of the reaction as monitored by TLC, the reaction system was adjusted to approximately pH 7 with 1M HCl, and concentrated under reduced pressure, and the resulting residue was purified and separated by HPLC to afford compound **20-1** and compound **20-2** (separation method: instrument: SHIMADZU LC-20AP; reversed-phase column: C18; mobile phase: A is 10 mmol $NH_4HCO_3$ in water; B is acetonitrile; gradient: B from 15% to 45% in 16 min; flow rate: 25 mL/min; column temperature: room temperature; wavelength: 210&254 nm).

Compound **20-1:** retention time: 2.039 min.

LC-MS (ESI): m/z=483.3 $[M+H]^+$.

$^1H$ NMR (400 MHz, $CD_3OD$) δ 7.78 (d, 1H), 7.53 (d, 1H), 5.70 (s, 2H), 4.76 - 4.80 (m, 2H), 4.17 (s, 3H), 2.93 - 2.99 (m, 1H), 2.77 - 2.92 (m, 6H), 2.65 - 2.78 (m, 5H), 2.52 (s, 3H), 2.18 - 2.23 (m, 1H), 1.99 - 2.04 (m, 1H), 1.84 - 1.90 (m, 2H), 1.76 - 1.82 (m, 1H), 1.59 - 1.68 (m, 1H), 1.457 - 1.52 (m, 1H), 1.28 - 1.34 (m, 2H).

Compound **20-2:** retention time: 2.141 min.

LC-MS (ESI): m/z=483.3 $[M+H]^+$.

$^1H$ NMR (400 MHz, $CD_3Cl$) δ 7.94 (d, 1H), 7.45 (d, 1H), 5.73 - 5.83 (m, 2H), 4.75 - 4.89 (m, 2H), 4.13 (s, 3H), 3.23 - 3.32 (m, 1H), 2.66 - 3.00 (m, 8H), 2.62 (s, 3H), 2.55 (s, 3H), 1.97 - 2.06 (m, 1H), 1.80 - 1.92 (m, 2H), 1.53 - 1.80 (m, 5H), 1.37 - 1.47 (m, 1H).

**Example 21:**

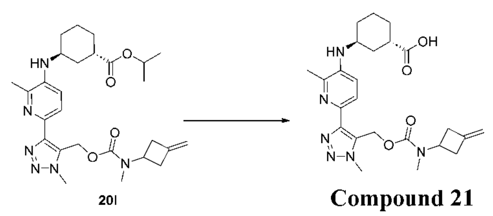

20I

Compound 21

Step 1: Compound **20I** (120 mg, 0.24 mmol) was added to a reaction flask and dissolved in THF : MeOH : $H_2O$ = 5 : 1 : 1 (7 ml), and then lithium hydroxide monohydrate (50 mg, 1.20 mmol) was added, and the mixture was reacted at 25°C for 16 hours. Upon completion of the reaction as monitored by TLC, the reaction system was adjusted to approximately pH 7 with 1M HCl, and concentrated under reduced pressure, and the resulting residue was purified and separated by TLC (DCM : MeOH = 10 : 1) to afford compound 21.

LC-MS (ESI): m/z=469.2 $[M+H]^+$.

$^1H$ NMR (400 MHz, $CDCl_3$) δ 7.95 (d, 1H), 7.14 (d, 1H), 5.73 (s, 2H), 4.78 - 4.87 (m, 2H), 4.12 (s, 3H), 3.70 - 3.79(m, 1H), 2.70 - 2.92 (m, 8H), 2.48 (s, 3H), 2.23 - 2.33 (m, 1H), 1.87 - 2.01 (m, 2H), 1.58 - 1.78 (m, 5H), 1.43 - 1.56 (m,

2H).

**Example 22:**

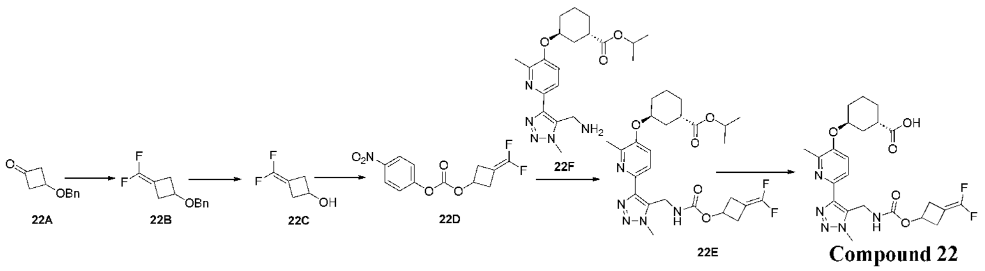

Compound 22

Step 1: Potassium tert-butoxide (9.53 g, 85.13 mmol) was added to a three-necked flask and dissolved in DMF (100 ml). The mixture was purged three times with nitrogen and cooled to -45°C, and a solution of **22A** (10 g, 56.75 mmol) in DMF (25 ml) and a solution of 2-(difluoromethanesulphonyl)pyridine (9.86 g, 51.08 mmol) in DMF (25 ml) were slowly added dropwise. After the dropwise addition was completed, the resulting mixture was reacted at this temperature for one hour. Subsequently, a saturated ammonium chloride solution (30 mL) and a hydrochloric acid aqueous solution (1N, 50 mL) were added, and the mixture was slowly warmed to room temperature and reacted for 16 hours. Upon completion of the reaction as monitored by TLC, the reaction system was diluted with water (100 ml) and extracted twice with methyl tert-butyl ether (100 ml × 2). The organic phases were combined, dried, and concentrated under reduced pressure at 30°C, and the resulting residue was purified by silica gel column chromatography (PE : EA = 10 : 1) to afford compound **22B** (4 g, yield: 33.53%).

$^1$H NMR (400 MHz, $CDCl_3$-*d*) δ 7.29 - 7.20 (m, 5H), 4.36 (s, 2H), 4.11 - 4.03 (m, 1H), 2.86 - 2.79 (m, 2H), 2.64 - 2.55 (m, 2H).

Step 2: Compound **22B** (4 g, 19.05 mmol) was added to a reaction flask and dissolved in dichloromethane (100 ml), and the mixture was purged three times with nitrogen and cooled to -78°C. Boron tribromide (9.52 g, 38.10 mmol) was then added dropwise, and the resulting mixture was reacted at this temperature for one hour. Upon completion of the reaction as monitored by TLC, the reaction system was adjusted to pH greater than 7 with a saturated sodium bicarbonate solution (200 ml) and extracted twice with dichloromethane (50 ml × 2). The organic phases were combined, dried, and concentrated at 20°C, and the resulting residue was purified by silica gel column chromatography (PE : EA = 5 : 1) to afford compound **22C** (1.63 g, yield: 71.33%).

Step 3: Compound **22C** (1.63 g, 13.58 mmol) was added to a reaction flask and dissolved in tetrahydrofuran (20 ml), and then pyridine (5.37 g, 67.90 mmol) and 4-nitrophenyl chloroformate (8.2 g, 40.74 mmol) were added, and the mixture was reacted at room temperature for one hour. Upon completion of the reaction as monitored by TLC, the reaction system was concentrated under reduced pressure, and the resulting residue was purified by silica gel column chromatography (PE : EA = 5 : 1) to afford compound **22D** (2.4 g, yield: 62.02%).

$^1$H NMR (400 MHz, $CDCl_3$-*d*) δ 8.32 - 8.26 (m, 2H), 7.43 - 7.36 (m, 2H), 5.23 - 5.10 (m, 1H), 3.25 - 3.14 (m, 2H), 3.01 - 2.88 (m, 2H).

Step 4: Compound **22F** (100 mg, 0.26 mmol, with reference to WO 2019126093) was dissolved in tetrahydrofuran (5 mL), compound **22D** (150 mg, 0.52 mmol) and *N,N*-diisopropylethylamine (100 mg, 0.78 mmol) were added, and the mixture was reacted at room temperature for 1 h. Upon complete depletion of starting materials, the reaction system was concentrated, and the crude product was separated by silica gel column chromatography (dichloromethane : methanol = 10 : 1) to afford target compound **22E** (108 mg, yield: 78.83%).

LC-MS (ESI): m/z= 534.3 $[M+1]^+$.

Step 5: Compound **22E** (108 mg, 0.20 mmol) was added to a reaction flask and dissolved in THF : MeOH : $H_2O$ = 3 : 1 : 1 (15 ml), and then anhydrous lithium hydroxide (27 mg, 0.61 mmol) was added, and the mixture was reacted at room temperature for 16 hours. Upon completion of the reaction as monitored by LC-MS, the reaction system was adjusted to pH = 4-5 with a hydrochloric acid aqueous solution (1 moL/L) and concentrated under reduced pressure, and the resulting residue was purified and separated by reversed-phase column chromatography to afford compound **22** (45.1 mg, yield: 45.31%).

LC-MS (ESI): m/z=492.2$[M+H]^+$.

$^1$H NMR (400 MHz, DMSO-d6) δ 7.82 (d, 1H), 7.71 (m, 1H), 7.49 (d, 1H), 4.95 - 4.92 (m, 1H), 4.77 - 4.76 (m, 3H), 4.04 (s, 3H), 3.07 - 2.99(m, 2H), 2.73 - 2.54 (m, 3H), 2.44 (s, 3H), 2.02 - 1.92 (m, 1H), 1.88 - 1.74 (m, 3H), 1.70 - 1.43 (m, 4H).

**Example 23:**

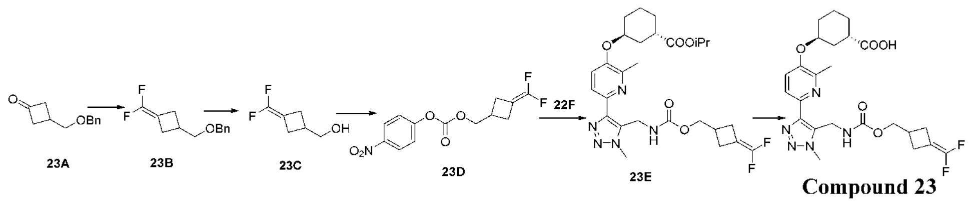

Compound 23

Starting from compound **23A** and following step 1 to step 5 of Example 22, compound **23** (5.4 mg, 6%) was obtained.

LC-MS (ESI): m/z=506.3[M+H]$^+$.

$^1$H NMR (400 MHz, DMSO-d6) δ 7.83 (d, 1H), 7.69-7.63 (m, 1H), 7.49 (d, 1H), 4.80-4.74 (m, 3H), 4.07 - 3.97 (m, 5H), 2.77 - 2.57 (m, 4H), 2.46 - 2.30 (m, 5H), 2.03 - 1.95 (m, 1H), 1.88 - 1.75 (m, 3H), 1.67 - 1.47 (m, 4H).

**Example 24:**

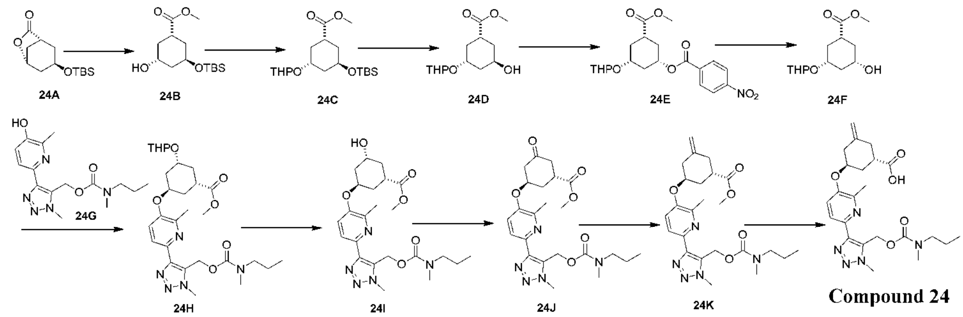

Compound 24

Step 1: Compound **24A** (3.00 g, 11.70 mmol, synthesised according to the method reported in patent WO 2013090929A1) was added to methanol (30 mL), and then sodium methoxide (2.11 g, 11.70 mmol, 30% methanol solution) was added, and the mixture was stirred at room temperature for 2 hours after the addition. Upon completion of the reaction as monitored by TLC, the reaction solution was added to a saturated ammonium chloride solution (50 mL) and extracted three times with dichloromethane (50 mL). The organic phase was washed with a saturated sodium chloride aqueous solution, dried over anhydrous sodium sulphate, filtered, and concentrated under reduced pressure to afford compound **24B** (2.90 g, yield: 86%).

Step 2: Compound **24B** (2.90 g, 10.05 mmol) and 3,4 dihydropyran (1.01 g, 12.06 mmol) were added to dichloromethane (30 mL), p-toluenesulphonic acid (96.0 mg, 0.50 mmol) was added, and the mixture was reacted at room temperature for 2 hours after the addition. Upon completion of the reaction as monitored by TLC, the reaction system was concentrated under reduced pressure, and the resulting residue was purified by silica gel column chromatography (eluents: petroleum ether/ethyl acetate = 5/1) to afford compound **24C** (3.10 g, yield: 83%).

Step 3: Compound **24C** (3.10 g, 8.32 mmol) was added to a tetrahydrofuran (30 mL) solution, followed by tetrabutylammonium fluoride (16.64 mL, 16.64 mmol, 1.0 mol/L tetrahydrofuran solution), and the mixture was reacted at room temperature for 16 hours after the addition. Upon completion of the reaction as monitored by TLC, the reaction system was concentrated under reduced pressure, and the resulting residue was purified by silica gel column chromatography (eluents: petroleum ether/ethyl acetate = 1/1) to afford compound **24D** (1.80 g, yield: 84%).

Step 4: Compound **24D** (1.8 g, 6.97 mmol) was added to dry tetrahydrofuran (40 mL), and then p-nitrobenzoic acid (2.33 g, 13.94 mmol) and 1,1'-(azodicarbonyl)dipiperidine (2.82 g, 13.94 mmol) were successively added. After the addition, the mixture was purged three times with nitrogen and cooled to 0°C-5°C, and finally triphenylphosphine (3.66 g, 13.94 mmol) was added. After the addition, the resulting mixture was warmed to room temperature and reacted at room temperature for 6 hours. Upon completion of the reaction as monitored by TLC, the reaction system was concentrated under reduced pressure, and the resulting residue was purified by silica gel column chromatography (eluents: PET/EA = 4/1) to afford compound **24E** (2.2 g, yield: 77%).

Step 5: Compound **24E** (2.2 g, 5.40 mmol) and sodium carbonate (1.72 g, 16.20 mmol) were added to methanol

(40 mL), and the mixture was reacted at room temperature for 5 hours. Upon completion of the reaction as monitored by TLC, the reaction system was filtered, the filtrate was concentrated under reduced pressure, and the resulting residue was purified by silica gel column chromatography (eluents: petroleum ether/ethyl acetate = 1/1) to afford compound **24F** (1.20 g, yield: 86%).

Step 6: Compound **24G** (1.00 g, 3.13 mmol, synthesised with reference to the method reported in patent WO 2017223016 A1) was added to dry toluene (30 mL), and then compound **24F** (1.13 g, 4.38 mmol) and 1,1'-(azodicarbonyl) dipiperidine (2.37 g, 9.39 mmol) were successively added. After the addition, the mixture was purged three times with nitrogen and cooled to 0°C-5°C, and finally tributylphosphine (1.90 g, 9.39 mmol) was added. After the addition, the resulting mixture was warmed to 60°C and reacted for 16 hours. After the reaction was completed, the reaction system was diluted with water (40 mL) and extracted three times with ethyl acetate (50 mL). The organic phases were combined, dried over anhydrous sodium sulphate, and concentrated in vacuo to afford a crude product, which was purified by column chromatography (eluents: petroleum ether/ethyl acetate = 1/2) to afford compound **24H** (0.95 g, yield: 54%).

LC-MS (ESI): m/z=560.3 $[M+H]^+$.

Step 7: Compound **24H** (0.95 g, 1.70 mmol) was added to methanol (10 mL), followed by trifluoroacetic acid (2 mL), and the mixture was reacted at room temperature for 3 hours after the addition. Upon completion of the reaction as monitored by TLC, the reaction system was concentrated under reduced pressure, and the resulting residue was purified by silica gel column chromatography (eluents: petroleum ether/ethyl acetate = 1/6) to afford compound **24I** (0.61 g, yield: 76%).

LC-MS (ESI): m/z=476.2 $[M+H]^+$.

Step 8: Compound **24I** (0.61 g, 5.40 mmol) and 2-iodoxybenzoic acid (1.08 g, 3.84mmol) were added to ethyl acetate (20 mL), and the mixture was warmed to 75°C and reacted for 6 hours. Upon completion of the reaction as monitored by TLC, the reaction system was filtered, the filtrate was concentrated under reduced pressure, and the resulting residue was purified by silica gel column chromatography (eluents: petroleum ether/ethyl acetate = 1/5) to afford compound **24J** (0.50 g, yield: 82%).

LC-MS (ESI): m/z=474.2 $[M+H]^+$.

Step 9: Compound **24J** (120.0 mg, 0.25 mmol) was added to 5 mL of dry toluene, and the mixture was purged three times with nitrogen and cooled to -5°C-0°C, and TEBBE reagent (1.00 mL, 0.50 mmol, 0.5 mol/L toluene solution) was slowly added dropwise. After the addition, the resulting mixture was reacted at a temperature controlled to -5°C-0°C for 2 hours. Upon completion of the reaction as monitored by TLC, the reaction system was slowly added to 15 mL of saturated sodium bicarbonate solution and extracted three times with ethyl acetate (20 mL). The organic phases were combined, dried over anhydrous sodium sulphate, and concentrated in vacuo to afford a crude product, which was purified by column chromatography (eluents: petroleum ether/ethyl acetate = 1/4) to afford compound **24K** (26.0 mg, yield: 22%).

LC-MS (ESI): m/z=472.1 $[M+H]^+$.

Step 10: **24K** (26.0 mg, 0.055 mmol) was dissolved in methanol (2 mL), tetrahydrofuran (2 mL) and water (2 mL), and then lithium hydroxide (12 mg, 0.28 mmol) was added, and the mixture was reacted at room temperature for 3 hours. After the reaction was completed, 1 M dilute hydrochloric acid was directly added to adjust the pH to weakly acidic. A small amount of water was then added, and the mixture was extracted three times with ethyl acetate (20 mL). The organic phases were combined and concentrated, and the residue was subsequently purified by preparative HPLC to afford compound **24** (8.0 mg, 32%).

LC-MS (ESI): m/z=458.3 $[M+H]^+$.

$^1H$ NMR (400 MHz, $CDCl_3$) δ 8.05 (d, 1H), 7.67 (d, 1H), 5.73 - 5.49 (m, 2H), 4.93 (s, 1H), 4.89 - 4.86 (m, 1H), 4.81 (s, 1H), 4.18 (s, 3H), 3.22 - 3.18 (m, 2H), 2.99 - 2.93 (m, 1H), 2.89 (s, 3H), 2.67 (s, 3H), 2.63 - 2.45 (m, 4H), 2.23 - 2.06 (m, 2H), 1.55 - 1.50 (m, 2H), 0.89 - 0.83 (m, 3H).

## Example 25

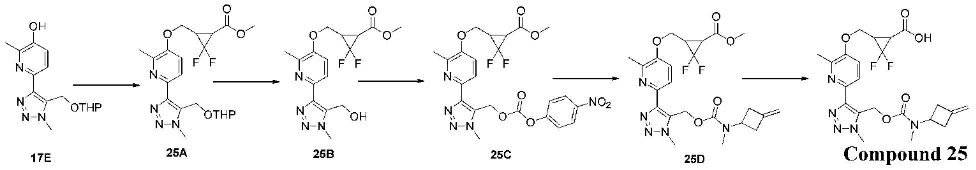

17E 25A 25B 25C 25D Compound 25

Step 1: Compound **17E** (610 mg, 2.00 mmol), methyl 2,2-difluoro-3-(hydroxymethyl)cyclopropane-1-carboxylate (665 mg, 4.00 mmol, document: Helvetica Chimica Acta, 1992, vol. 75, # 3, p. 766-772) and tributylphosphine (1.21 g, 6.00 mmol) were added to a reaction flask and dissolved in toluene (50 ml), and then 1,1'-(azodicarbonyl)dipiperidine (1.51

g, 6.00 mmol) was added, and the mixture was reacted at 50°C for 16 hours. Upon completion of the reaction as monitored by TLC, the reaction system was cooled to room temperature and concentrated under reduced pressure, and the resulting residue was purified by silica gel column chromatography (PE : EA = 2 : 1) to afford compound **25A** (800 mg, yield: 88.40%).

LC-MS (ESI): m/z=453.3[M+H]$^+$.

Step 2: Compound **25A** (800 mg, 1.77 mmol) was added to a reaction flask and dissolved in methanol (50 ml), and then pyridinium 4-methylbenzenesulphonate (490 mg, 1.94 mmol) was added, and the mixture was reacted at 60°C for 4 hours. Upon completion of the reaction as monitored by TLC, the reaction system was cooled to room temperature and concentrated under reduced pressure, and the resulting residue was purified by silica gel column chromatography (PE : EA = 1 : 4) to afford compound **25B** (310 mg, yield: 47.55%).

LC-MS (ESI): m/z=369.2[M+H]$^+$.

Step 3: Compound **25B** (310 mg, 0.84 mmol) was added to a reaction flask and dissolved in dichloromethane (10 ml), and then pyridine (330 mg, 4.2 mmol) and 4-nitrophenyl chloroformate (423 mg, 2.1 mmol) were added, and the mixture was reacted at room temperature for 16 hours. Upon completion of the reaction as monitored by TLC, the reaction system was concentrated under reduced pressure, and the resulting residue was purified by silica gel column chromatography (PE : EA = 1 : 1) to afford compound **25C** (130 mg, yield: 29.02%).

LC-MS (ESI): m/z=534.2[M+H]$^+$.

Step 4: Compound **25C** (130 mg, 0.24 mmol) was added to a reaction flask and dissolved in dichloromethane (10 ml), and then DIPEA (0.25 ml, 1.44 mmol) and compound **4C** (70 mg, 0.72 mmol) were added, and the mixture was reacted at room temperature for 2 hours. Upon completion of the reaction as monitored by TLC, the reaction system was concentrated under reduced pressure, and the resulting residue was purified by silica gel column chromatography (PE : EA = 1 : 2) to afford compound **25D** (115 mg, yield: 96.00%).

LC-MS (ESI): m/z=492.1[M+H]$^+$.

Step 5: Compound **25D** (115 mg, 0.23 mmol) was added to a reaction flask and dissolved in THF : MeOH : $H_2O$=1 : 1 : 1 (9 ml), and then lithium hydroxide (22 mg, 0.92 mmol) was added, and the mixture was reacted at room temperature for 4 hours. Upon completion of the reaction as monitored by TLC, the reaction system was concentrated under reduced pressure, and the resulting residue was separated by C18 reversed-phase column chromatography to afford compound **25** (64 mg, yield: 57.29%).

$^1$H NMR (400 MHz, DMSO-d6) δ 7.88 - 7.83 (m, 1H), 7.51 - 7.46 (m, 1H), 5.64 (s, 2H), 4.78 (s, 2H), 4.33 - 4.26 (m, 1H), 4.21 - 4.13 (m, 1H), 4.09 (s, 3H), 2.87 - 2.73 (m, 5H), 2.71 - 2.53 (m, 5H), 2.39 (s, 3H).

LC-MS (ESI): m/z=478.3[M+H]$^+$.

**Example 26**

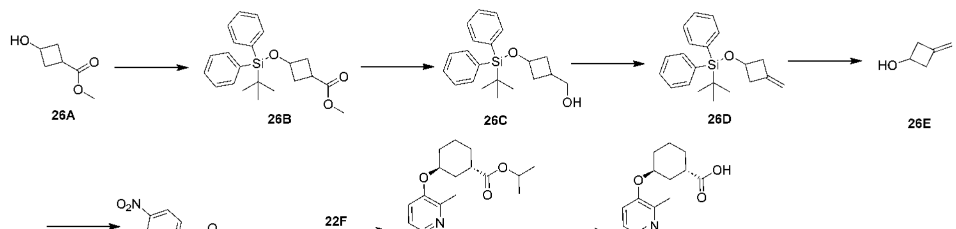


26A 26B 26C 26D 26E

26F 26G Compound 26

Step 1: Compound **26A** (15.0 g, 115.26 mmol) was added to a reaction flask and dissolved in dichloromethane (200 ml), and triethylamine (34.99 g, 345.78 mmol) was added. The mixture was purged three times with nitrogen and cooled to 0°C, and tert-butyldiphenylchlorosilane (38.02 g, 138.31 mmol) was slowly added. After the addition was completed, the resulting mixture was reacted at this temperature for 4 hours. Upon completion of the reaction as monitored by TLC, the reaction system was diluted with water (500 ml) and extracted twice with methyl tert-butyl ether (300 ml × 2). The organic phases were combined, dried, and concentrated under reduced pressure at 40°C, and the resulting residue was purified by silica gel column chromatography (PE : EA = 10 : 1) to afford compound **26B** (40 g, yield: 94.17%).

LC-MS (ESI): m/z=369.3[M+H]$^+$.

Step 2: Compound **26B** (10 g, 27.13 mmol) was added to a reaction flask and dissolved in tetrahydrofuran (50 ml), and the mixture was purged three times with nitrogen and cooled to 0°C. Lithium borohydride (1.18 g, 54.26 mmol) was added, and the resulting mixture was stirred at room temperature for 16 hours. Upon completion of the reaction as monitored by TLC, a saturated ammonium chloride solution (100 ml) was added, and the mixture was extracted twice with

ethyl acetate (50 ml × 2). The organic phases were combined, dried, and concentrated at 45°C, and the resulting residue was purified by silica gel column chromatography (PE : EA = 2 : 1) to afford compound **26C** (4.0 g, yield: 43.29%).

LC-MS (ESI): m/z=341.2[M+H]+.

Step 3: Compound **26C** (2.0g, 5.87 mmol) was added to a reaction flask and dissolved in tetrahydrofuran (20 ml), 2-nitrophenyl selenocyanate (4.00 g, 17.61 mmol) and tri-n-butylphosphine (3.56 g, 17.61 mmol) were added, and the mixture was reacted at room temperature for one hour. The reaction system was concentrated under reduced pressure, dissolved in dichloromethane (20 ml), and cooled to 0°C. m-Chloroperoxybenzoic acid (3.04 g, 17.61 mmol) was added, and the mixture was stirred for 1 hour. The reaction system was concentrated under reduced pressure and dissolved in toluene (20 ml), diisopropylamine (1.78 g, 17.61 mmol) was added, and the resulting mixture was stirred at 90°C for 16 hours. Upon completion of the reaction as monitored by TLC, water (50 ml) was added, and the mixture was extracted with ethyl acetate (50 ml) and concentrated under reduced pressure, and the resulting residue was purified by silica gel column chromatography (PE : EA = 10 : 1) to afford compound **26D** (1.2 g, yield: 63.35%).

$^1$H NMR (400 MHz, $CDCl_3$) δ 7.64 - 7.66 (m, 4H), 7.34 - 7.44 (m, 6H), 4.74 - 4.76 (m, 2H), 4.27 - 4.34 (m, 1H), 2.72 - 2.82 (m, 4H), 1.04 (s, 9H).

Step 4: Compound **26D** (2.0 g, 6.20 mmol) was dissolved in tetrahydrofuran (20 mL), tetrabutylammonium fluoride (12.4 mL, 12.4 mmol) was added, and the mixture was reacted at room temperature for 16 h. Upon complete depletion of starting materials, the reaction system was concentrated to afford crude target compound **26E** (2 g, crude), which was directly used in the next step.

Step 5: Compound **26E** (500 mg, 5.94 mmol) was added to a reaction flask and dissolved in dichloromethane (15 ml), triethylamine (1.80 g, 17.82 mmol) and 4-nitrophenyl chloroformate (1.44 g, 7.13 mmol) were added, and the mixture was reacted at room temperature for 16 hours. Upon completion of the reaction as monitored by TLC, water (20 ml) was added, and the mixture was extracted with dichloromethane (20 ml) and concentrated under reduced pressure, and the resulting residue was purified by silica gel column chromatography (PE : EA = 5 : 1) to afford compound **26F** (0.2 g, yield: 13.15%).

LC-MS (ESI): m/z=250.1[M+H]+.

Step 6: Compound **26F** (200 mg, 0.80 mmol) was added to a reaction flask and dissolved in dichloromethane (10 ml), triethylamine (0.24 g, 2.40 mmol) and compound **22F** (0.34 g, 0.88 mmol) were added, and the mixture was reacted at room temperature for 16 hours. Upon completion of the reaction as monitored by TLC, water (20 ml) was added, and the mixture was extracted with dichloromethane (20 ml) and concentrated under reduced pressure, and the resulting residue was purified by silica gel column chromatography (PE : EA = 1 : 1) to afford compound **26G** (0.18 g, yield: 45.08%).

LC-MS (ESI): m/z=498.2[M+H]+.

Step 7: Compound **26G** (180 mg, 0.27 mmol) was added to a reaction flask and dissolved in methanol (10 ml), lithium hydroxide monohydrate (45 mg, 1.08 mmol) was added, and the mixture was reacted at room temperature for 16 hours. The reaction system was adjusted to pH=4 with 2N hydrochloric acid and concentrated to afford a crude product, which was purified by column chromatography (eluents: dichloromethane : methanol = 10 : 1) to afford a crude target compound, which was subjected to preparative HPLC to afford compound 26 (40 mg, 24.28%).

LC-MS (ESI): m/z = 456.2[M+H]+.

$^1$H NMR (400 MHz, DMSO-d6) δ 7.85 (d, 1H), 7.68 (s, 1H), 7.55 (d, 1H), 4.73 - 4.85 (m, 6H), 4.06 (s, 3H), 2.90 - 2.97 (m, 2H), 2.61 - 2.68 (m, 3H), 2.47(s, 3H), 2.00 - 2.07 (m, 1H), 1.76 - 1.88 (m, 3H), 1.49 - 1.65(m, 4H).

**Example 27**

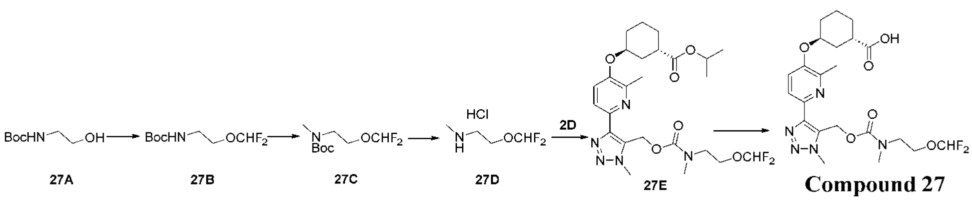


27A 27B 27C 27D 27E Compound 27

Step 1: Into a 250 mL single-necked flask, **27A** (2 g, 12.41 mmol), potassium hydrogen fluoride (3.88 g, 49.64 mmol) and (bromodifluoromethyl)trimethylsilane (5.04 mg, 24.82 mmol) were successively added to a mixed solvent of dichloromethane (20 mL) and water (60 mL). The mixture was purged three times with nitrogen and stirred vigorously at room temperature for 4 hours. After the reaction was completed, the reaction system was extracted three times with dichloromethane. The organic phases were combined, washed with a saturated sodium chloride aqueous solution, dried over anhydrous sodium sulphate, and concentrated under reduced pressure to afford a crude product, which was purified by Biotage Isolera One (40 g silica gel column, eluents: 0-25% EA/PE) to afford compound **27B** (543 mg, yield: 20.72%).

$^1H$ NMR (400 MHz, $CDCl_3$) δ 6.41 - 6.01 (m, 1H), 4.83 (s, 1H), 3.94 - 3.88 (m, 2H), 3.43 - 3.35 (m, 2H), 1.45 (s, 9H).

Step 2: **27B** (535 mg, 2.53 mmol) was dissolved in DMF (10 mL), and the mixture was purged with nitrogen and cooled to 0°C in an ice-water bath with stirring. Sodium hydride (121 mg, 3.04 mmol, 60% Wt) was added, and the mixture was stirred for 5 minutes. Iodomethane (538 mg, 3.79 mmol) was then added, and the resulting mixture was stirred for another 1 hour after the ice-water bath was removed. After the reaction was completed, ice water (30 mL) was added to the reaction flask to quench the reaction, and the mixture was extracted with ethyl acetate (20 mL × 3). The organic phases were combined, washed once with a saturated sodium chloride aqueous solution, dried over anhydrous sodium sulphate, and concentrated to afford crude compound **27C** (800 mg), which was directly used in the next step without further purification.

Step 3: **27C** (800 mg, crude) was added to a single-necked flask and dissolved in dichloromethane (3 mL), a hydrogen chloride dioxane solution (10 mL, 4 mol/L) was added, and the mixture was stirred at room temperature for 1 hour. After the reaction was completed, the reaction system was directly concentrated under reduced pressure to dryness to afford crude compound **27D** (288 mg), which was directly used in the next step without further purification.

Step 4: Crude **27D** (288 mg) was added to a single-necked flask, dichloromethane (10 mL), DIPEA (567 mg, 4.4 mmol) and compound **2D** (120 mg, 0.22 mmol) were added, and the mixture was stirred at room temperature for 16 hours. After the reaction was completed, the reaction system was concentrated under reduced pressure to afford a crude product, which was purified by Biotage Isolera One (12 g silica gel column, eluents: 0-3% MeOH/DCM) to afford compound **27E** (87 mg, yield: 73.30%).

LC-MS (ESI): m/z = 540.2 $[M+H]^+$.

Step 5: **27E** (87 mg, 0.16 mmol) was added to a single-necked flask and dissolved in tetrahydrofuran (10 mL), and water (5 mL) was added. Lithium hydroxide (33 mg, 0.80 mmol) was added with stirring, and the mixture was stirred at room temperature for 16 hours. After the reaction was completed, water (20 mL) and ethyl acetate (20 mL) were added, and the mixture was stirred for 10 minutes, and then the aqueous phase was separated. The organic phase was extracted twice with water (20 mL), and the three aqueous phases were combined and adjusted to a weakly acidic pH with hydrochloric acid (1 N). The mixture was extracted with ethyl acetate (20 mL × 3). The organic phases were combined, washed once with a saturated sodium chloride aqueous solution, dried over anhydrous sodium sulphate, and concentrated under reduced pressure to afford a crude product, which was purified by Biotage Isolera One (12 g silica gel column, eluents: 0-6% MeOH/DCM) to afford compound **27** (41 mg, yield: 51.11 %).

LC-MS (ESI): m/z = 498.3 $[M+H]^+$.

$^1H$ NMR (400 MHz, DMSO-d6) δ 7.90 - 7.80 (m, 1H), 7.56 - 7.46 (m, 1H), 6.88 - 6.30 (m, 1H), 5.73 - 5.61 (m, 2H), 4.78 (s, 2H), 4.09 (s, 3H), 3.95 - 3.76 (m, 2H), 3.46 (s, 2H), 2.90 - 2.78 (m, 3H), 2.63 (s, 1H), 2.42 (s, 3H), 2.09 - 1.98 (m, 1H), 1.87 - 1.76 (m, 2H), 1.70 - 1.46 (m, 4H).

**Example 28**

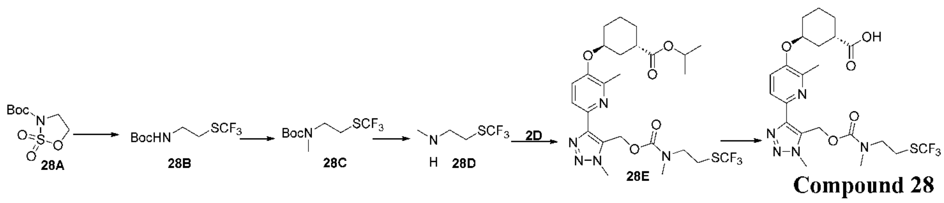


Compound 28

Step 1: **28A** (500 mg, 2.24 mmol) was dissolved in acetonitrile (8 mL), and then tetramethylammonium trifluoromethanethiolate (588 mg, 3.36 mmol) was added, and the mixture was reacted at room temperature overnight. After the reaction was completed, the reaction solution was concentrated by rotary evaporation, dichloromethane (8 mL) and 20% sulphuric acid aqueous solution (5 mL) were added to the residue, and the resulting mixture was stirred at room temperature for 2 h. A saturated sodium bicarbonate solution was slowly added to the reaction solution to quench the reaction, and the mixture was extracted with diethyl ether (15 mL × 3). The organic layers were combined, washed with a saturated sodium chloride aqueous solution (20 mL), dried over anhydrous sodium sulphate, and filtered. The filtrate was concentrated, and the residue was separated and purified by silica gel column chromatography (petroleum ether : ethyl acetate (v/v) = 25 : 1) to afford **28B** (251 mg, 46%).

LC-MS (ESI): m/z =190.0 $[M+H-56]^+$.

Starting from compound **28B** and following step 2 to step 5 of Example 27, compound **28** (21 mg, 18%) was obtained.

LC-MS (ESI): m/z =532.0 $[M+H]^+$.

$^1H$ NMR (400 MHz, $CDCl_3$) δ 8.03 - 8.00 (m, 1H), 7.62 - 7.54 (m, 1H), 5.77 - 5.59 (m, 2H), 4.81 - 4.78 (m, 1H), 4.18 (s, 3H), 3.57 - 3.45 (m, 2H), 3.06 - 3.02 (m, 1H), 2.96 - 2.85 (m, 5H), 2.70 - 2.67 (m, 3H), 2.14 - 1.88 (m, 4H), 1.83 - 1.61 (m, 4H).

**Example 29:**

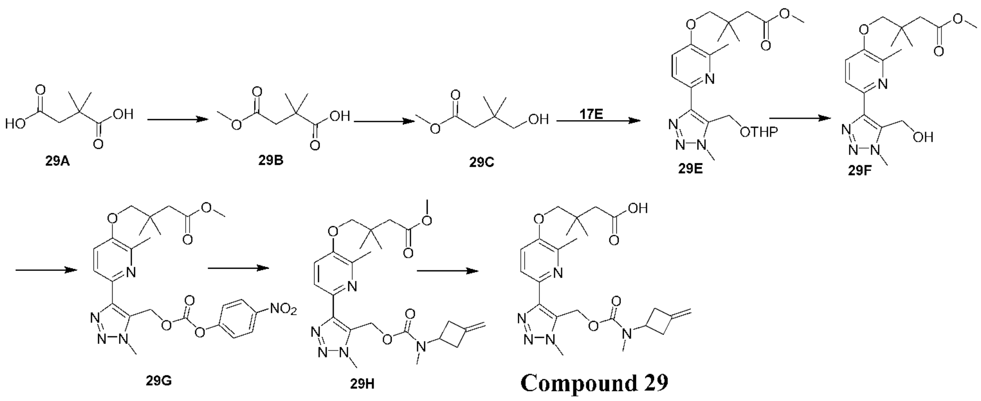


29A 29B 29C 29E 29F

29G 29H Compound 29

Step 1: Compound **29A** (15.00 g, 102.64 mmol) was dissolved in methanol (150 mL), and the mixture was cooled to 0°C-5°C. Concentrated sulphuric acid (2 mL) was slowly added dropwise. After the addition, the resulting mixture was warmed to room temperature and stirred for 17 hours. After the reaction was completed, the reaction system was concentrated under increased pressure, and then a saturated sodium bicarbonate aqueous solution (150 mL) was added portionwise, and the resulting mixture was extracted twice with petroleum ether (100 mL) to remove impurities. The aqueous phase was cooled to 0°C and adjusted to pH=3-4 with 6N hydrochloric acid aqueous solution. The product was then extracted twice with ethyl acetate (100 mL). The combined organic phase was dried over anhydrous sodium sulphate and filtered, and the filtrate was concentrated under reduced pressure to afford compound **29B** (9.20 g, yield: 56%).

Step 2: Compound **29B** (5.00 g, 31.22 mmol) was dissolved in dry tetrahydrofuran (50 mL), and the mixture was purged three times with nitrogen and cooled to 0°C-5°C. Borane-tetrahydrofuran solution (62.44 mL, 62.44 mmol, 1.0mol/L tetrahydrofuran solution) was slowly added dropwise. After the dropwise addition was completed, the resulting mixture was slowly warmed to room temperature and reacted for 3 hours. Upon completion of the reaction as monitored by TLC, the reaction system was cooled to 0°C, and methanol (100 mL) was slowly added dropwise to quench the reaction. After complete quenching, the reaction system was concentrated under reduced pressure, and the resulting residue was purified by normal-phase column chromatography to afford compound **29C** (2.60 g, yield: 57%).

Step 3: Compound **29C** (0.96 g, 6.58 mmol) was added to a dichloromethane solution (10 mL), followed by triethylamine (1.33 g, 13.16 mmol). After the addition, the mixture was cooled to 0°C-5°C. Methanesulphonic anhydride (1.20 g, 6.91 mmol) was added portionwise. After the addition, the resulting mixture was reacted at a temperature controlled to 0°C-5°C for 2 hours. Upon completion of the reaction as monitored by TLC, the reaction system was concentrated under reduced pressure, and the resulting residue was dissolved in N,N-dimethylformamide (10 mL), and then compound **17E** (1.00 g, 3.29 mmol) and caesium carbonate (3.22 g, 9.87 mmol) were successively added. After the addition, the resulting mixture was heated to 100°C and reacted for 15 hours. After the reaction was completed, the resulting reaction system was cooled to room temperature, and water (50 mL) was added. The product was then extracted twice with ethyl acetate (50 mL). The combined organic phase was washed with a saturated sodium chloride aqueous solution, dried over anhydrous sodium sulphate, and filtered. The filtrate was concentrated, and the resulting residue was purified by normal-phase column chromatography to afford compound **29E** (0.23 g, yield: 16%).

LC-MS (ESI): m/z=433.2 $[M+H]^+$.

Step 4: Compound **29E** (0.23 g, 0.53 mmol) was added to methanol (10 mL), followed by trifluoroacetic acid (2 mL), and the mixture was reacted at room temperature for 3 hours after the addition. Upon completion of the reaction as monitored by TLC, the reaction system was concentrated under reduced pressure, and the resulting residue was purified by normal-phase column chromatography to afford compound **29F** (0.15 g, yield: 81%).

LC-MS (ESI): m/z=349.1 $[M+H]^+$.

Step 5: Compound **29F** (0.15 g, 0.43 mmol) was added to dichloromethane (5 mL), and then pyridine (0.14 g, 1.72 mmol) and 4-nitrophenyl chloroformate (0.13 g, 0.65 mmol) were successively added, and the mixture was reacted at room temperature for 3 hours after the addition. Upon completion of the reaction as monitored by TLC, the reaction system was concentrated under reduced pressure, and the resulting residue was purified by normal-phase column chromatography to

afford compound **29G** (0.17 g, yield: 77%).

Step 6: Compound **29G** (0.17 g, 0.33 mmol) was added to tetrahydrofuran (5 mL), and then triethylamine (0.17 g, 1.65 mmol) and compound **4C** (100 mg, TFA salt) were successively added, and the mixture was reacted at room temperature for 3 hours after the addition. Upon completion of the reaction as monitored by TLC, the reaction system was concentrated under reduced pressure, and the resulting residue was purified by normal-phase column chromatography to afford compound **29H** (0.13 g, yield: 83%).

LC-MS (ESI): m/z=472.2 $[M+H]^+$.

Step 7: Compound **29H** (0.13 g, 0.27 mmol) was dissolved in methanol (3 mL), tetrahydrofuran (3 mL) and water (3 mL), and then lithium hydroxide (112 mg, 2.71 mmol) was added, and the mixture was reacted at room temperature for 15 hours. After the reaction was completed, 1 M dilute hydrochloric acid was directly added to adjust the pH to weakly acidic. A small amount of water was then added, and the mixture was extracted three times with ethyl acetate (20 mL). The organic phases were combined and concentrated, and the residue was subsequently purified by preparative HPLC to afford compound **29** (11.0 mg, 9%).

LC-MS (ESI): m/z=458.3 $[M+H]^+$.

$^1$H NMR (400 MHz, DMSO-$d_6$) δ 11.99 (s, 1H), 7.84 (d, 1H), 7.41 (d, 1H), 5.65 (s, 2H), 4.79 (s, 2H), 4.44 - 4.27 (m, 1H), 4.09 (s, 3H), 3.86 (s, 2H), 2.77 - 2.67 (m, 7H), 2.41 (s, 3H), 2.34 (s, 2H), 1.12 (s, 6H).

**Example 30**

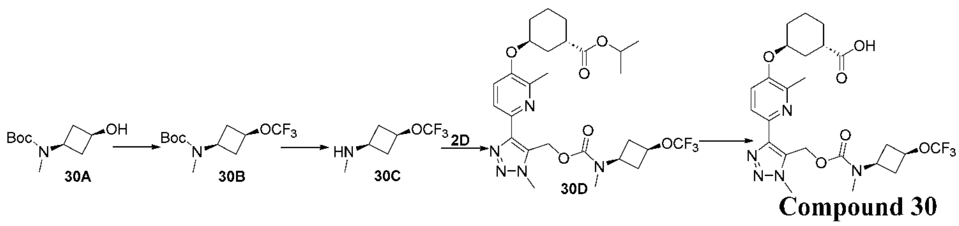

30A 30B 30C 30D
Compound 30

Step 1: **30A** (400 mg, 1.99 mmol), silver trifluoromethanesulphonate (2.04 g, 7.96 mmol), SELECTFLUOR (1.06 g, 2.98 mmol), potassium fluoride (461 mg, 7.96 mmol) and 2-fluoropyridine (772 mg, 7.96 mmol) were dissolved in ethyl acetate (30 mL), and the mixture was stirred well. Trifluoromethyl trimethylsilane (706 mg, 4.98 mmol) was added dropwise, and the mixture was reacted at room temperature for 36 h. After the reaction was completed, 60 mL of water was added, and the mixture was extracted with ethyl acetate (25 mL × 3). The organic layers were combined, washed with a saturated sodium chloride aqueous solution (40 mL), dried over anhydrous sodium sulphate, and filtered. The filtrate was concentrated, and the residue was separated and purified by silica gel column chromatography to afford **30B** (276 mg, 52%).

LC-MS (ESI): m/z = 214.0 $[M+H-56]^+$.

Starting from compound **30B** and following step 3 to step 5 of Example 27, compound **30** (48 mg, 40%) was obtained.

LC-MS (ESI): m/z =542.3 $[M+H]^+$.

$^1$H NMR (400 MHz, $CDCl_3$) δ 8.04 - 8.00 (m, 1H), 7.59 - 7.56 (m, 1H), 5.70 - 5.55 (m, 2H), 4.80 - 4.75 (m, 1H), 4.39 - 4.31 (m, 1H), 4.17 (s, 3H), 2.87 (s, 3H), 2.67 - 2.59 (m, 4H), 2.37 - 2.28 (m, 2H), 2.09 - 2.02 (m, 2H), 1.98 - 1.87 (m, 2H), 1.82 - 1.50 (m, 5H), 1.45 - 1.35 (m, 1H), 0.95 - 0.90 (m, 1H).

**Example 31**

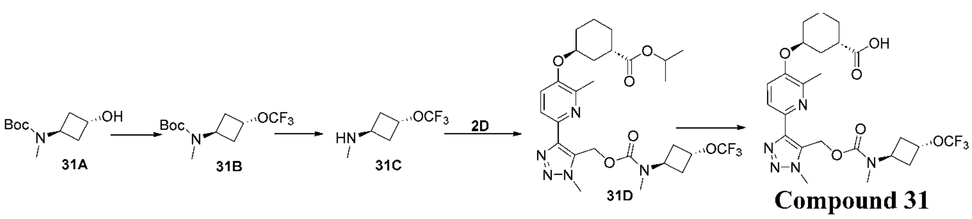

31A 31B 31C 31D
Compound 31

Starting from **31A** (400 mg, 1.99 mmol) and following the procedure described for Example **30,** compound **31** (44

mg, 51%) was synthesised.

LC-MS (ESI): m/z =542.6 [M+H]+.

$^1$H NMR (400 MHz, $CDCl_3$) δ 8.04 - 8.01 (m, 1H), 7.60 - 7.57 (m, 1H), 5.69 - 5.57 (m, 2H), 4.80 - 4.68 (m, 2H), 4.18 (s, 3H), 2.91 - 2.83 (m, 4H), 2.66 (s, 3H), 2.57 - 2.44 (m, 2H), 2.09 - 2.01 (m, 2H), 1.98 - 1.88 (m, 2H), 1.80 - 1.50 (m, 5H), 1.44 - 1.35 (m, 1H), 0.95 - 0.90 (m, 1H).

**Example 32**

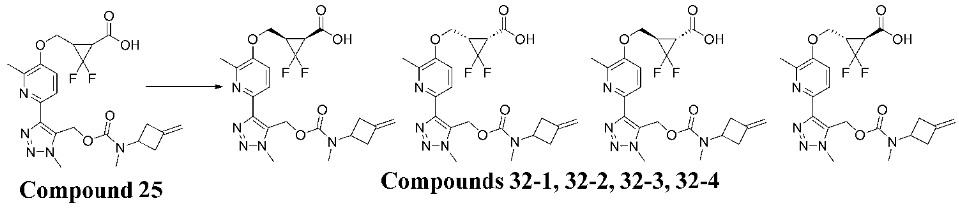

Compound 25

Compounds 32-1, 32-2, 32-3, 32-4

Step 1: Compound **25** (1.34 g) was subjected to chiral resolution to afford compounds **32-1** (6 mg, retention time: 1.07 min), **32-2** (10 mg, retention time: 1.22 min), **32-3** (537 mg, retention time: 1.46 min) and **32-4** (502 mg, retention time: 1.78 min).

Analytical method: instrument: SHIMADZU LC-30AD, column: Chiral AD Column; mobile phase: A: $CO_2$, B: 0.05% DEA in MeOH; gradient: 5-10% B in A; flow rate: 3 mL/min; column temperature: 35°C; wavelength: 220 nm.

Preparative method: instrument: SFC Prep 150 AP, column: Daicel IG (19 mm × 250 mm); mobile phase: A: $CO_2$, B: 0.05% $NH_3$•$H_2O$ in ETOH; gradient: 12% B gradient elution; flow rate: 120 mL/min; column temperature: 25°C; wavelength: 220 nm; cycle time: 25 min; sample preparation: sample concentration 10 mg/ml, the sample was dissolved in DMF and filtered through a 0.45 μm filter to prepare a sample solution. After separation, the fractions were dried using a rotary evaporator at a bath temperature of 35°C, affording compounds **32-1** (6 mg, retention time: 1.07 min), **32-2** (10 mg, retention time: 1.22 min), **32-3** (537 mg, retention time: 1.46 min) and **32-4** (502 mg, retention time: 1.78 min).

32-1: (retention time: 1.07 min), $^1$H NMR (400 MHz, DMSO-d6) δ 7.87 - 7.83 (m, 1H), 7.48 - 7.44 (m, 1H), 5.64 (s, 2H), 4.79 (s, 2H), 4.60 - 4.53 (m, 1H), 4.50 - 4.43 (m, 1H), 4.09 (s, 3H), 2.86 - 2.57 (m, 10H), 2.37 (s, 3H).

LC-MS (ESI): m/z=478.3[M+H]+.

32-2: (retention time: 1.22 min), $^1$H NMR (400 MHz, DMSO-d6) δ 7.87 - 7.84 (m, 1H), 7.50 - 7.47 (m, 1H), 5.65 (s, 2H), 4.79 (s, 2H), 4.58 - 4.52 (m, 1H), 4.47 - 4.40 (m, 1H), 4.09 (s, 3H), 2.88 - 2.63 (m, 10H), 2.37 (s, 3H).

LC-MS (ESI): m/z=478.2[M+H]+.

32-3: (retention time: 1.46 min), $^1$H NMR (400 MHz, DMSO-d6) δ 7.87 - 7.83 (m, 1H), 7.50 - 7.46 (m, 1H), 5.64 (s, 2H), 4.78 (s, 2H), 4.32 - 4.25 (m, 1H), 4.18 - 4.11 (m, 1H), 4.09 (s, 3H), 2.88 - 2.51 (m, 10H), 2.39 (s, 3H).

LC-MS (ESI): m/z=478.2[M+H]+.

32-4: (retention time: 1.78 min), $^1$H NMR (400 MHz, DMSO-*d6*) δ 7.87 - 7.83 (m, 1H), 7.50 - 7.45 (m, 1H), 5.64 (s, 2H), 4.78 (s, 2H), 4.31 - 4.24 (m, 1H), 4.16 - 4.06 (m, 4H), 2.90 - 2.41 (m, 10H), 2.39 (s, 3H).

LC-MS (ESI): m/z=478.2[M+H]+.

**Example 33**

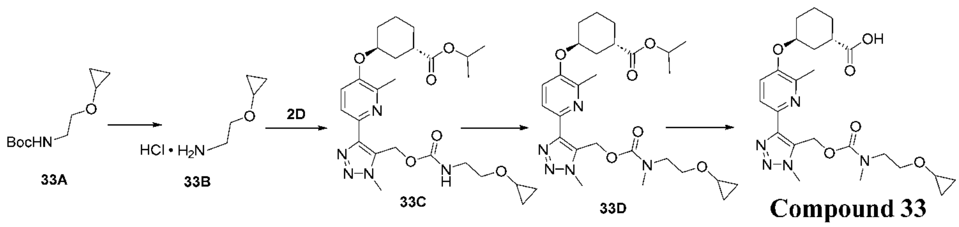


33A 33B 33C 33D Compound 33

Step 1: Compound **33A** (600 mg, 2.98 mmol, synthesised according to the method reported in patent CN 116789556A) was added to a reaction flask and dissolved in dichloromethane (10 ml), and then 4N hydrogen chloride dioxane solution (10 ml) was added, and the mixture was reacted at room temperature for 18 hours. Upon completion of the reaction as monitored by TLC, the reaction system was concentrated under reduced pressure to afford compound **33B**

(330 mg, yield: 80.44%), which was directly used in the next step without further purification.

LC-MS (ESI): m/z = 102.2[M+H]$^+$.

Step 2: Compound **2D** (250 mg, 0.45 mmol) and compound **33B** (185 mg, 1.35 mmol) were added to a reaction flask and dissolved in dichloromethane (10 ml), and then DIPEA (645 mg, 5.0 mmol) was added, and the mixture was reacted at room temperature for 3 hours. Upon completion of the reaction as monitored by TLC, the reaction system was concentrated under reduced pressure, and the resulting residue was purified by silica gel column chromatography to afford compound **33C** (180 mg, yield: 77.30%).

LC-MS (ESI): m/z = 516.3[M+H]$^+$.

Step 3: Compound **33C** (180 mg, 0.35 mmol) was added to a reaction flask and dissolved in DMF (10 ml), and then the mixture was cooled to 0°C. NaH (17 mg, 0.42 mmol) was added, and the resulting mixture was stirred at this temperature for 20 min. Subsequently, iodomethane (75 mg, 0.52 mmol) was added, and the resulting mixture was warmed to room temperature and reacted for 1 hour. Upon completion of the reaction as monitored by TLC, a saturated ammonium chloride solution (20 ml) was added, and then the mixture was extracted with ethyl acetate (20 ml × 2). The organic phases were combined, dried, and concentrated under reduced pressure to afford compound **33D** (180 mg, yield: 95.35%), which was directly used in the next step without further purification.

LC-MS (ESI): m/z = 530.3[M+H]$^+$.

Step 4: Compound **33D** (180 mg, 0.34 mmol) was added to a reaction flask and dissolved in THF : MeOH : $H_2O$ = 1 : 1 : 1 (9 ml), and then anhydrous lithium hydroxide (41 mg, 1.70 mmol) was added, and the mixture was stirred and reacted at room temperature for 18 hours. Upon completion of the reaction as monitored by TLC, dilute hydrochloric acid was added to adjust the pH to weakly acidic, and the mixture was extracted with ethyl acetate (20 mL × 2). The organic phases were combined, dried over anhydrous sodium sulphate, and filtered. The filtrate was concentrated under reduced pressure, and the resulting residue was separated by preparative HPLC to afford compound **33** (0.13 mg, yield: 78.46%).

LC-MS (ESI): m/z=484.4[M+H]$^+$.

$^1$H NMR (400 MHz, $CDCl_3$) δ 8.09 - 8.08 (m, 1H), 7.82 - 7.81 (m, 1H), 5.67 (s, 1H), 5.54 - 5.50 (m, 1H), 4.86 (s, 1H), 4.20 (s, 3H), 3.59 - 3.24 (m, 4 H), 3.24 - 3.23 (m, 1H), 2.95 - 2.88 (m, 4H), 2.79 (s, 3H), 2.06 - 2.00 (m, 2H), 1.92 - 1.67 (m, 6H), 0.49 - 0.43 (m, 4H).

**Example 34**

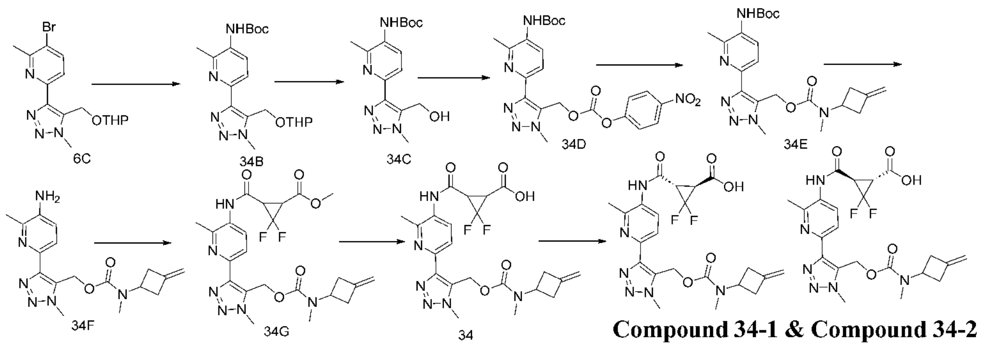

6C 34B 34C 34D 34E 34F 34G 34 Compound 34-1 & Compound 34-2

Step 1: Compound **6C** (5 g, 13.62 mmol), tert-butyl carbamate (3.19 g, 27.24 mmol), caesium carbonate (8.88 g, 27.24 mmol), methanesulphonato(2-dicyclohexylphosphino-2',6'-diisopropoxy-1,1'-biphenyl)(2-amino-1,1'-biphenyl-2-yl)palladium(II) (RuPhos PD G3) (1.14 g, 1.36 mmol) were dissolved in 1,4-dioxane (50 mL), and then the mixture was purged three times with nitrogen and reacted at 100°C for 16 hours. The reaction system was cooled to room temperature and concentrated, and the residue was purified by column chromatography (tetrahydrofuran : petroleum ether = 1%-50%) to afford compound **34B** (5.1 g, yield: 92.84%).

LC-MS (ESI): m/z = 404.2[M+H]$^+$.

Step 2: Compound **34B** (3 g, 7.44 mmol) was dissolved in methanol (30 mL), and then p-toluenesulphonic acid (0.13 g, 0.74 mmol) was added, and the mixture was reacted at 40°C for 2 hours. The reaction system was concentrated, and the residue was purified by column chromatography (tetrahydrofuran : petroleum ether = 1%-50%) to afford compound **34C** (2.1 g, yield: 88.44%).

LC-MS (ESI): m/z = 320.2[M+H]$^+$.

Step 3: Compound **34C** (2 g, 6.26 mmol) and pyridine (0.99 g, 12.52 mmol) were dissolved in dichloromethane (15 mL), and then 4-nitrophenyl chloroformate (1.51 g, 7.51 mmol) was added portionwise, and the mixture was reacted at 25°C for 2 hours. The reaction system was concentrated, and the residue was purified by column chromatography

(tetrahydrofuran : petroleum ether = 1%-50%) to afford compound **34D** (2.5 g, yield: 82.40%).

LC-MS (ESI): m/z = 485.1[M+H]$^+$.

Step 4: Compound **34D** (0.2 g, 0.41 mmol) and triethylamine (0.17 g, 1.64 mmol) were dissolved in dichloromethane (10 mL), and then compound **4C** (0.052 g, 0.53 mmol) was added, and the mixture was reacted at 25°C for 2 hours. The reaction system was concentrated, and the residue was purified by column chromatography (tetrahydrofuran : petroleum ether = 1%-50%) to afford compound **34E** (0.15 g, yield: 82.11 %).

LC-MS (ESI): m/z =443.3[M+H]$^+$.

Step 5: Compound **34E** (0.15 g, 0.34 mmol) was dissolved in dichloromethane (3 mL), and then trifluoroacetic acid (0.3 mL) was added, and the mixture was reacted at room temperature for 2 hours. The reaction system was directly concentrated to afford compound **34F,** which was used in the next step.

LC-MS (ESI): m/z = 343.1[M+H]$^+$.

Step 6: Compound **34F** (0.1 g, 0.29 mmol), trans-2,2-difluoro-3-(methoxycarbonyl)cyclopropanecarboxylic acid (0.10 g, 0.58 mmol) (document: Helvetica Chimica Acta, 1992, vol. 75, # 3, p. 766-772), and N-methylimidazole (0.095 g, 1.16 mmol) were dissolved in acetonitrile (2 mL), and then tetramethylchloroformamidinium hexafluorophosphate (0.24 g, 0.87 mmol) was added, and the mixture was reacted at room temperature for 16 hours. The reaction system was concentrated, and the residue was purified by column chromatography (tetrahydrofuran : petroleum ether = 1%-50%) to afford compound **34G** (0.1 g, yield: 67.87%).

LC-MS (ESI): m/z = 505.1[M+H]$^+$.

Step 7: Compound **34G** (0.1 g, 0.20 mmol) was dissolved in tetrahydrofuran (3 mL) and water (1 mL), and then lithium hydroxide monohydrate (0.034 g, 0.80 mmol) was added, and the mixture was reacted at room temperature for 2 hours. The reaction system was adjusted to pH = 4 with formic acid and concentrated, and the residue was purified by reversed-phase column chromatography (acetonitrile : water = 3%-60%) to afford compound **34** (0.06 g, yield: 61.72%).

LC-MS (ESI): m/z = 491.3[M+H]$^+$.

Step 8: Chiral analysis of compound **34** (400 mg) revealed the presence of two isomers: P1 (retention time: 2.699 min, assigned as compound 34-1), and P2 (retention time: 3.511 min, assigned as compound 34-2). Analytical method: instrument: Waters UPC2 analytical SFC (SFC-H); column: ChiralPak IK, 150 × 4.6 mm I.D., 3 μm; mobile phase: A for CO2 and B for ethanol (0.05% DEA); gradient: 40% B gradient elution; flow rate: 2.5 mL/min; column pressure: 100 bar; column temperature: 35°C; wavelength: 220 nm; cycle time: 4 min.

Preparative method: instrument: WATERS 150 preparative SFC (SFC-26); column: ChiralPak IH, 250 × 30 mm I.D., 10 μm; mobile phase: A for CO2 and B for ethanol (0.1% NH3H2O); gradient: 40% B gradient elution; flow rate: 120 mL/min; column temperature: 38°C; wavelength: 220 nm; cycle time: 4 min; sample preparation: compound was dissolved in about 20 ml of methanol/DCM; injection: 2.2 mL per injection, affording compound **34-1** (142 mg) and compound **34-2** (194 mg).

Compound 34-1: $^1$H NMR (400 MHz, DMSO-*d6*) δ 10.05 (s, 1H), 8.01 - 7.98(m, 1H), 7.88 (d, 1H), 5.67 (s, 2H), 4.79 (s, 2H), 4.11 (s, 3H), 3.45 - 3.41 (m, 1H), 3.38 - 3.32 (d, 2H), 2.89 - 2.73 (m, 6H), 2.46 (s, 3H), 2.35 - 3.30 (m, 1H).

LC-MS (ESI): m/z = 491.3[M+H]+.

Compound 34-2: $^1$H NMR (400 MHz, DMSO-*d6*) δ 10.05 (s, 1H), 8.02 - 7.98(m, 1H), 7.88 (d, 1H), 5.67 (s, 2H), 4.79 (s, 2H), 4.11 (s, 3H), 3.45 - 3.41 (m, 1H), 3.38 - 3.32 (d, 2H), 2.89 - 2.73 (m, 6H), 2.46 (s, 3H), 2.35 - 3.30 (m, 1H).

LC-MS (ESI): m/z = 491.3[M+H]$^+$.

**Example 35**

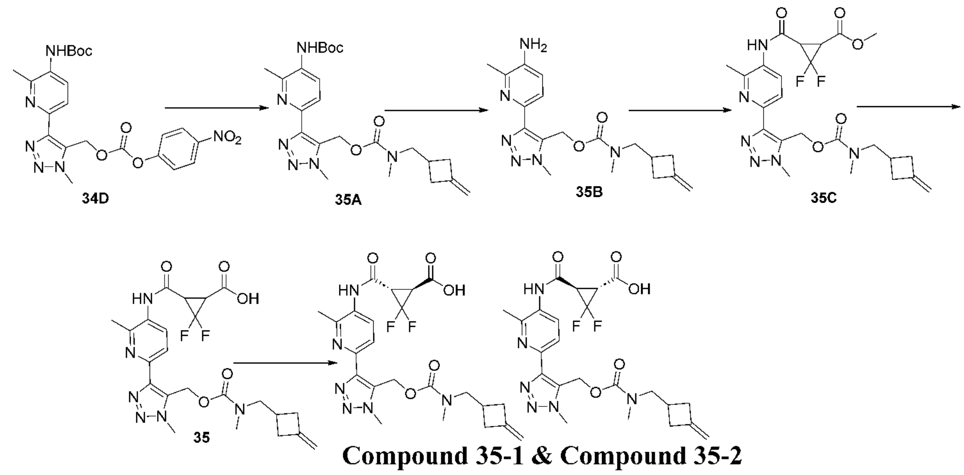

**Compound 35-1 & Compound 35-2**

Starting from compound **34D** and following step 4 to step 8 of Example 34, compound **35-1** (retention time: 2.609 min) and compound **35-2** (retention time: 3.449 min) were obtained. Analytical method: instrument: Waters UPC2 analytical SFC (SFC-H); column: ChiralPak IK, 150 × 4.6 mm I.D., 3 μm; mobile phase: A for CO2 and B for ethanol (0.05% DEA); gradient: 40% B gradient elution; flow rate: 2.5 mL/min; column pressure: 100 bar; column temperature: 35°C; wavelength: 220 nm; cycle time: 4 min.

Preparative method: instrument: WATERS 150 preparative SFC (SFC-26); column: ChiralPak IH, 250 × 30 mm I.D., 10 μm; mobile phase: A for CO2 and B for ethanol (0.1% NH3H2O); gradient: 40% B gradient elution; flow rate: 120 mL/min; column temperature: 38°C; wavelength: 220 nm; cycle time: 4 min; sample preparation: compound was dissolved in about 20 ml of methanol/DCM; injection: 2.2 mL per injection, affording compound **35-1** (17 mg) and compound **35-2** (13 mg).

Compound 35-1: $^1H$ NMR (400 MHz, DMSO-*d6*) δ 10.10 (s, 1H), 8.01 - 7.87 (m, 1H), 7.89 (d, 1H), 5.65 (s, 2H), 4.66 (d, 2H), 4.11 (s, 3H), 3.49 (s, 1H), 3.24 - 3.13 (m, 2H), 2.99 - 3.90 (m, 1H), 2.80 - 2.66 (m, 4H), 2.47 (s, 3H), 2.38 (s, 1H), 2.19 (s, 1H), 1.72 - 1.41 (m, 1H), 1.24 (s, 1H).

LC-MS (ESI): m/z = 505.3$[M+H]^+$.

Compound 35-2: $^1H$ NMR (400 MHz, DMSO-*d6*) δ 10.10 (s, 1H), 8.01-7.87 (m, 1H), 7.89 (d, 1H), 5.65 (s, 2H), 4.66 (d, 2H), 4.11 (s, 3H), 3.49 (s, 1H), 3.24 - 3.13 (m, 2H), 2.99 - 3.90 (m, 1H), 2.80 - 2.66 (m, 4H), 2.47 (s, 3H), 2.38 (s, 1H), 2.19 (s, 1H), 1.72 - 1.41 (m, 1H), 1.24 (s, 1H).

LC-MS (ESI): m/z = 505.3$[M+H]^+$.

## Example 36

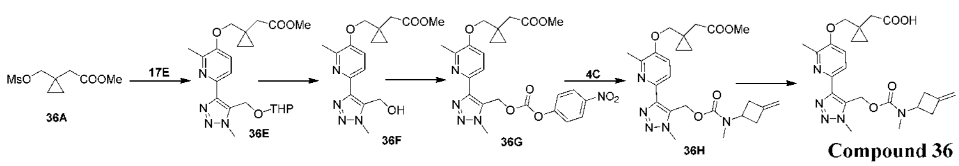


**Compound 36**

Step 1: Compound **36A** (0.1 g, 0.45 mmol, synthesised according to the method reported in patent WO 2008/141462) was added to DMF (5 mL), followed by compound **17E** (0.14 g, 0.45 mmol) and caesium carbonate (0.44 g, 1.35 mmol), and the mixture was reacted at 50°C for 2 hours after the addition. Upon completion of the reaction as monitored by TLC, the reaction system was quenched with water and extracted three times with ethyl acetate (20 mL). The organic phases were combined and concentrated, and the resulting residue was purified by normal-phase column chromatography to afford compound **36E** (120 mg, yield: 62%).

LC-MS (ESI): m/z=431.2 $[M+H]^+$.

**Compound 36** (20 mg) was obtained following steps 4, 5, 6, and 7 of Example **29.**

LC-MS (ESI): m/z=456.2 $[M+H]^+$.

$^1H$ NMR (400 MHz, $CDCl_3$) δ = 7.91 (d, 1H), 7.09 (d, 1H), 5.74 (s, 2H), 5.56 (s, 2H), 4.82 (s, 2H), 4.12 (s, 3H), 3.92 (s, 2H), 2.83 (s, 6H), 2.53 (s, 2H), 2.48 (s, 3H), 0.69 (d, 4H).

## Example 37

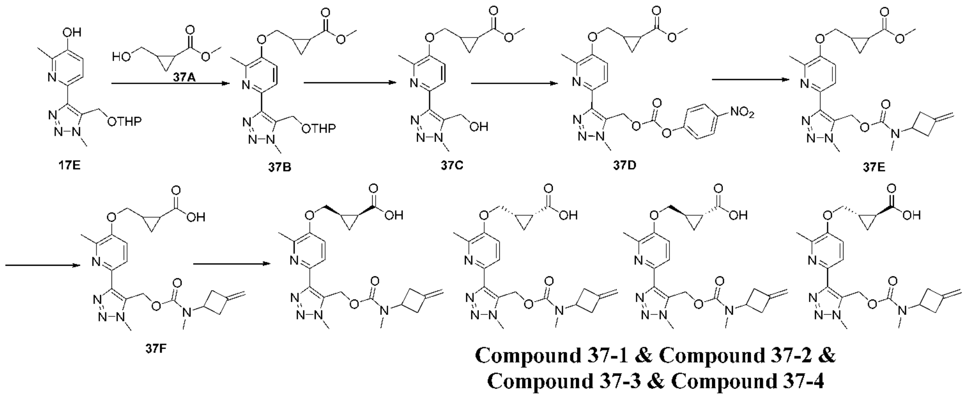

17E 37B 37C 37D 37E

37F

**Compound 37-1 & Compound 37-2 & Compound 37-3 & Compound 37-4**

Starting from compound **17E** and following step 1 to step 5 of Example 25, compound **37F** (574 mg, 91.6%) was obtained.

Compound **37F** was subjected to SFC resolution to afford compound **37-1** (SFC retention time: 2.311 min, 98 mg), compound **37-2** (SFC retention time: 2.173 min, 112 mg), compound **37-3** (SFC retention time: 1.763 min, 42.3 mg) and compound **37-4** (SFC retention time: 1.374 min, 39.1 mg).

First SFC analytical method: instrument: Waters 150 Prep-SFC, column: Chiral WHEIK column; mobile phase: A: $CO_2$, B: 0.1%$NH_3$•$H_2O$ in MeOH; gradient: 35% B gradient elution; flow rate: 120 mL/min; column temperature: room temperature; wavelength: 220 nm; cycle time: 4.3 min; sample preparation: sample concentration 10 mg/mL, methanol solution injection: 3 mL per injection. After separation, the fractions were dried to afford compound **37-1** and compound **37-2.**

Second SFC analytical method: instrument: Waters 150 Prep-SFC, column: Chiral AD column; mobile phase: A: $CO_2$, B: 0.1%$NH_3$•$H_2O$ in MeOH; gradient: 30% B gradient elution; flow rate: 120 mL/min; column temperature: room temperature; wavelength: 220 nm; cycle time: 8 min; sample preparation: sample concentration 10 mg/mL, methanol solution injection: 3 mL per injection. After separation, the fractions were dried to afford compound **37-3** and compound **37-4.**

Compound **37-1** (SFC retention time: 2.311 min): $^1$H NMR (400 MHz, DMSO-$d_6$) δ 7.86 - 7.79 (m, 1H), 7.44 - 7.36 (m, 1H), 5.65 (s, 2H), 4.79 (s, 2H), 4.45 - 4.37 (m, 1H), 4.15 - 4.00 (m, 4H), 2.92 - 2.56 (m, 8H), 2.37 (s, 3H), 1.83 - 1.68 (m, 2H), 1.16 - 1.04 (m, 1H), 1.02 - 0.92 (m, 1H).

LC-MS (ESI): m/z = 442.3 $[M+H]^+$.

Compound **37-2** (SFC retention time: 2.173 min): $^1$H NMR (400 MHz, DMSO-$d_6$) δ 7.86 - 7.79 (m, 1H), 7.44 - 7.36 (m, 1H), 5.65 (s, 2H), 4.79 (s, 2H), 4.45 - 4.37 (m, 1H), 4.15 - 4.00 (m, 4H), 2.92 - 2.56 (m, 8H), 2.37 (s, 3H), 1.83 - 1.68 (m, 2H), 1.16 - 1.04 (m, 1H), 1.02 - 0.92 (m, 1H).

LC-MS (ESI): m/z = 442.3 $[M+H]^+$.

Compound **37-3** (SFC retention time: 1.763 min): $^1$H NMR (400 MHz, DMSO-$d_6$) δ 7.86 - 7.79 (m, 1H), 7.44 - 7.36 (m, 1H), 5.65 (s, 2H), 4.79 (s, 2H), 4.45 - 4.37 (m, 1H), 4.15 - 4.00 (m, 4H), 2.92 - 2.56 (m, 8H), 2.37 (s, 3H), 1.83 - 1.68 (m, 2H), 1.16 - 1.04 (m, 1H), 1.02 - 0.92 (m, 1H).

LC-MS (ESI): m/z = 442.3 $[M+H]^+$.

Compound **37-4** (SFC retention time: 1.374 min): $^1$H NMR (400 MHz, DMSO-$d_6$) δ 7.86 - 7.79 (m, 1H), 7.44 - 7.36 (m, 1H), 5.65 (s, 2H), 4.79 (s, 2H), 4.45 - 4.37 (m, 1H), 4.15 - 4.00 (m, 4H), 2.92 - 2.56 (m, 8H), 2.37 (s, 3H), 1.83 - 1.68 (m, 2H), 1.16 - 1.04 (m, 1H), 1.02 - 0.92 (m, 1H).

LC-MS (ESI): m/z = 442.3 $[M+H]^+$.

## Example 38

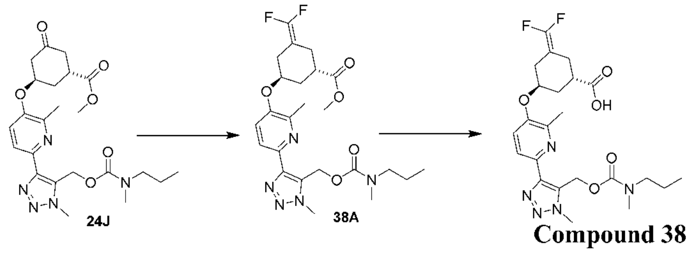

Compound 38

Step 1: Compound **24J** (360.0 mg, 0.76 mmol) was added to a mixed solvent of dry toluene (10 mL) and N,N-dimethylformamide (3 mL), followed by lithium iodide (407.0 mg, 3.04 mmol), and the mixture was purged three times with nitrogen. Subsequently, (trifluoromethyl)trimethylsilane (540.0 mg, 3.80 mmol) and tri-n-butylphosphine (921.2 mg, 4.56 mmol) were successively added. After the addition, the resulting mixture was warmed to 45°C and reacted for 16 hours. Upon completion of the reaction as monitored by TLC, the reaction solution was slowly added to 50 mL of ice water and extracted three times with ethyl acetate (50 mL). The organic phases were combined, washed with a saturated sodium chloride aqueous solution, dried over anhydrous sodium sulphate, and filtered. The filtrate was concentrated, and the crude product was purified by column chromatography (eluents: petroleum ether/ethyl acetate = 1/3) to afford compound **38A** (90.0 mg, yield: 23%).

LC-MS (ESI): m/z=508.2 $[M+H]^+$.

Step 2: **38A** (90.0 mg, 0.18 mmol) was dissolved in a mixed solvent of methanol (3 mL), tetrahydrofuran (3 mL) and water (3 mL), and then lithium hydroxide (37 mg, 0.90 mmol) was added, and the mixture was reacted at room temperature for 6 hours. After the reaction was completed, 1 M dilute hydrochloric acid was directly added to adjust the pH to weakly acidic. A small amount of water was then added, and the mixture was extracted three times with ethyl acetate (20 mL). The organic phases were combined and concentrated, and the residue was subsequently purified by preparative HPLC to afford compound **38** (30.0 mg, 32%).

LC-MS (ESI): m/z=494.2 $[M+H]^+$.

$^1$H NMR (400 MHz, $CD_3Cl$) δ 8.01 (d, 1H), 7.84 (d, 1H), 5.60 (s, 2H), 4.97 (s, 1H), 4.21 (s, 3H), 3.23 - 3.19 (m, 2H), 2.88 - 2.84 (m, 4H), 2.69 - 2.59 (m, 5H), 2.40 - 2.36 (m, 1H), 2.28 - 2.24 (m, 2H), 2.09 - 2.03 (m, 1H), 1.55 - 1.45 (m, 2H), 0.88 - 0.77 (m, 3H).

## Example 39

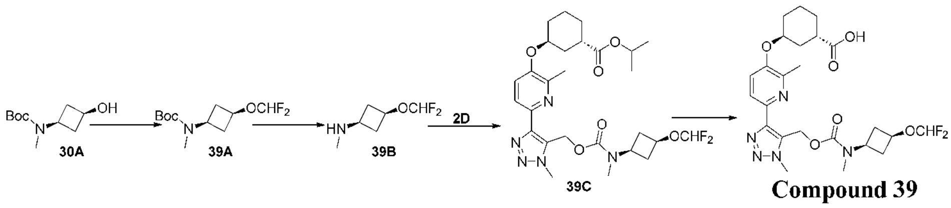

Compound 39

Starting from compound **30A** and following step 1 to step 4 of Example 30, compound 39 (54 mg, 46%) was obtained.

LC-MS (ESI): m/z =524.4 $[M+H]^+$.

$^1$H NMR (400 MHz, $CDCl_3$) δ 8.06 - 8.03 (m, 1H), 7.77 - 7.74 (m, 1H), 6.33 - 5.95 (m, 1H), 5.62 - 5.45 (m, 2H), 4.81 (s, 1H), 4.35 - 4.27 (m, 1H), 4.22 - 4.03 (m, 4H), 2.91 - 2.79 (m, 4H), 2.68 (s, 3H), 2.64 - 2.56 (m, 2H), 2.32 - 2.21 (m, 2H), 2.12 - 1.95 (m, 2H), 1.94 - 1.71 (m, 5H), 1.70 - 1.57 (m, 1H).

## Example 40

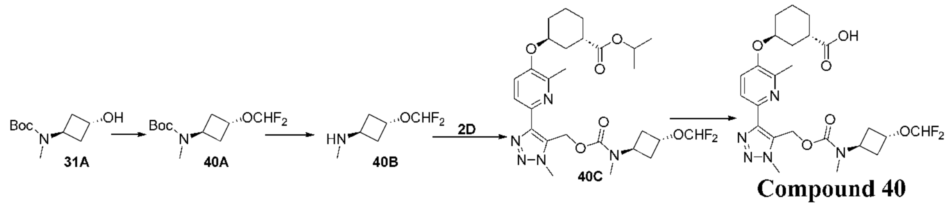


31A 40A 40B 40C Compound 40

Starting from **31A** (400 mg, 1.99 mmol) and following step 1 to step 4 of Example 30, compound **40** (41 mg) was synthesised.

LC-MS (ESI): m/z =524.3 $[M+H]^+$.

$^1H$ NMR (400 MHz, $CDCl_3$) δ 8.08 - 8.05 (m, 1H), 7.87 - 7.84 (m, 1H), 6.37 - 5.96 (m, 1H), 5.64 - 5.48 (m, 2H), 4.89 - 4.61 (m, 3H), 4.19 (s, 3H), 2.90 - 2.81 (m, 4H), 2.75 (s, 3H), 2.54 - 2.46 (m, 2H), 2.46 - 2.37 (m, 2H), 2.11 - 1.96 (m, 2H), 1.95 - 1.70 (m, 5H), 1.69 - 1.59 (m, 1H).

## Example 41

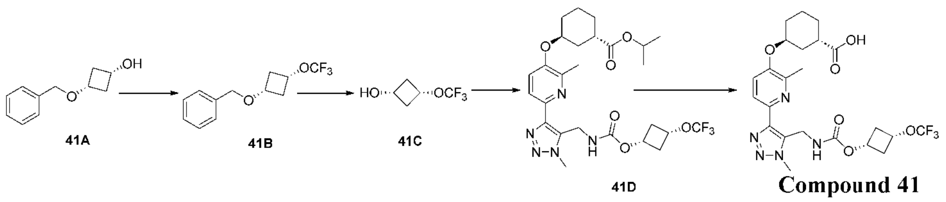

41A 41B 41C 41D Compound 41

Step 1: Starting from **41A** (890 mg, 5.0 mmol) and following the synthetic method described in step 1 of Example **30,** compound **41B** (300 mg, 21.40%) was synthesised.

Step 2: At room temperature, **41B** (300 mg, 1.22 mmol) was dissolved in dry tetrahydrofuran (10 mL), palladium hydroxide on carbon (856 mg, 1.22 mmol) and hydrochloric acid (5 mg, 0.12 mmol) were added, and the mixture was reacted at room temperature under hydrogen atmosphere for 16 hours. Upon complete depletion of starting materials as monitored by TLC, the reaction was stopped. The reaction system was filtered to afford compound **41C** (180 mg, 94.64%).

Step 3: At room temperature, **41C** (180 mg, 1.15 mmol) was dissolved in dry tetrahydrofuran (5 mL). Under nitrogen atmosphere, triethylamine (350 mg, 3.45 mmol) and N,N'-carbonyldiimidazole (186 mg, 1.15 mmol) were added, and the mixture was reacted for another 1 hour. Subsequently, **11D** (534 mg, 1.38 mmol) was added, and the mixture was reacted at 80°C under nitrogen atmosphere for 16 hours. Upon complete depletion of starting materials as monitored by TLC, the reaction was stopped. Ethyl acetate (20 mL) was added to the reaction solution. The organic phase was washed with water (30 mL × 3) and a saturated sodium chloride aqueous solution (30 mL × 2), dried over anhydrous sodium sulphate, and filtered. The filtrate was concentrated, and the residue was separated and purified by column chromatography (PE : EA = 2 : 1) to afford compound **41D** (70 mg, 10.66%).

LC-MS (ESI): m/z = 570.3 $[M+H]^+$.

Step 4: Starting from **41D** (70 mg, 0.12 mmol) and following the synthetic method described in step 4 of Example 1, compound **41** (25 mg, 38.56%) was synthesised.

LC-MS (ESI): m/z = 528.3 $[M+H]^+$.

$^1H$ NMR (400 MHz, DMSO-$d_6$) δ 7.85 - 7.83 (d, 1H), 7.69 (s, 1H), 7.51 - 7.49 (d, 1H), 4.78 - 4.74 (t, 3H), 4.55 (s, 2H), 4.04 (s, 3H), 2.83 - 2.80 (m, 2H), 2.67 - 2.60 (m, 1H), 2.45 (s, 3H), 2.17 - 2.14 (m, 2H), 2.05 - 1.99 (m, 1H), 1.88 - 1.75 (m, 3H), 1.67 - 1.46 (m, 4H).

## Example 42:

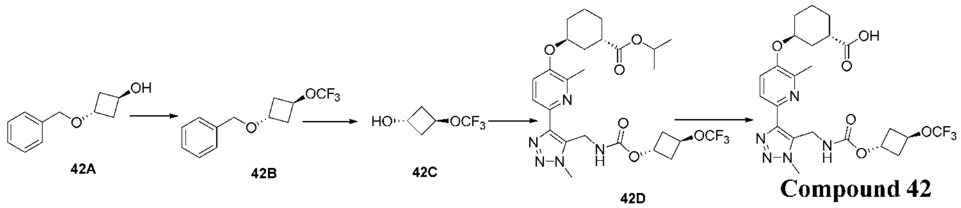
42A 42B 42C 42D Compound 42

Starting from **42A** (890 mg, 5.0 mmol) and following the synthetic route described in Example **41,** compound **42** (27 mg, 29.15%) was synthesised.

LC-MS (ESI): m/z = 528.3 $[M+H]^+$.

$^1$H NMR (400 MHz, DMSO-$d_6$) δ 7.85 - 7.83 (d, 1H), 7.68 (s, 1H), 7.52 - 7.50 (d, 1H), 5.00 - 4.93 (d, 3H), 4.78 - 4.76 (d, 4H), 4.05 (s, 3H), 2.66 - 2.64 (m, 1H), 2.46 (s, 3H), 2.05 - 1.97 (m, 2H), 1.91 - 1.73 (m, 4H), 1.69 - 1.43 (d, 4H).

**Example 43**

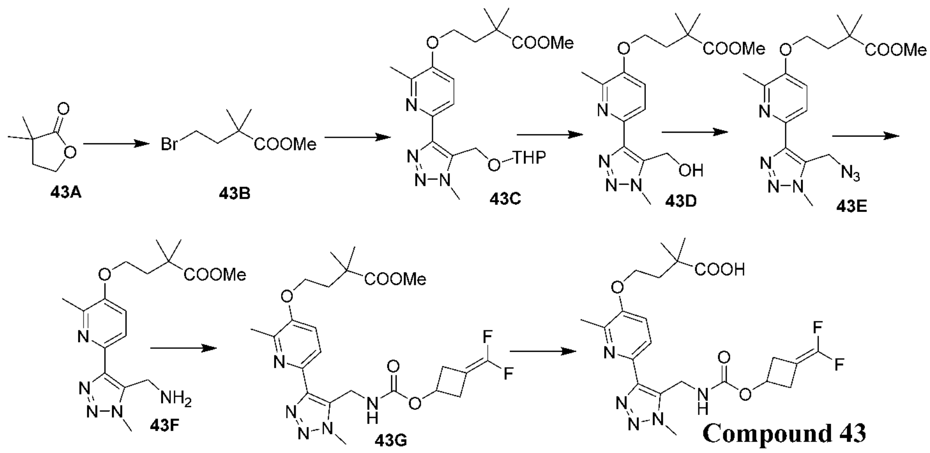
43A 43B 43C 43D 43E 43F 43G Compound 43

Step 1: Compound **43B** (1.5 g) was synthesised from compound **43A** (1.5 g, 13.05 mmol) according to the method reported in patent WO 2011046771.

Compound **43D** (0.43 g) was obtained following step 1 and step 2 of Example **36.**

Step 4: Compound **43D** (0.1 g, 0.29 mmol) was dissolved in dry tetrahydrofuran (5 ml), diphenyl phosphorazidate (44 mg, 1.16 mmol) and 1,8-diazabicyclo[5,4,0]undec-7-ene (178 mg, 1.16 mmol) were successively added, and the mixture was stirred at room temperature for 1 hour. Upon completion of the reaction as monitored by TLC, the reaction system was directly concentrated, and the resulting residue was purified by normal-phase column chromatography to afford compound **43E** (90 mg, yield: 83%).

LC-MS (ESI): m/z=374.2 $[M+H]^+$.

Step 5: Compound **43E** (90 mg, 0.24 mmol) was dissolved in tetrahydrofuran/water (3 ml/1 ml), triphenylphosphine (94 mg, 0.36 mmol) was added, and the mixture was stirred at room temperature for 1 hour. Upon completion of the reaction as monitored by TLC, the reaction system was directly concentrated, and the resulting residue was purified by normal-phase column chromatography to afford compound **43F** (53 mg, yield: 64%).

LC-MS (ESI): m/z=348.2 $[M+H]^+$.

Starting from compound **43F** and compound **22D** and following step 4 and step 5 of Example **22,** compound **43** (20 mg) was obtained.

LC-MS (ESI): m/z=480.2 $[M+H]^+$.

$^1$H NMR (400 MHz, $CDCl_3$) δ 8.03 (d, 1H), 7.21 (d, 1H), 7.16 (s, 1H), 5.03 - 4.95 (m, 1H), 4.57 (d, 2H), 4.18 (s, 3H), 4.11 (t, 2H), 3.06 - 3.00 (m, 2H), 2.77 - 2.66 (m, 2H), 2.48 (s, 3H), 2.17 (t, 2H), 1.35 (s, 6H).

**Example 44**

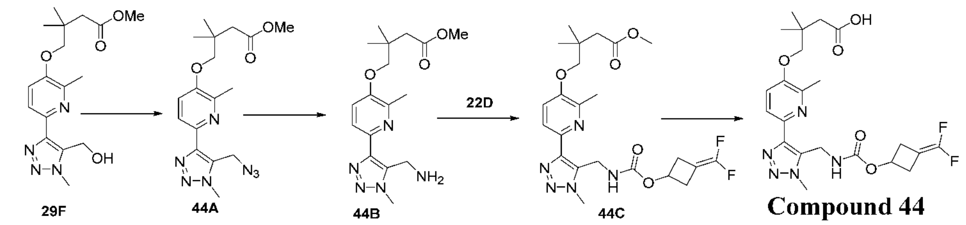


29F 44A 44B 44C Compound 44

Step 1: Compound **29F** (85 mg, 0.24 mmol) was added to tetrahydrofuran (5 ml), followed by DPPA (180 mg, 0.48 mmol) and DBU (73 mg, 0.48 mmol), and the mixture was stirred at room temperature for 2 hours. Upon completion of the reaction as monitored by TLC, the reaction system was diluted with water, extracted with ethyl acetate, dried over anhydrous sodium sulphate, and filtered. The filtrate was concentrated under reduced pressure, and the resulting residue was purified by silica gel column chromatography to afford compound **44A** (75 mg, yield: 82%).

LC-MS (ESI): m/z=374.2[M+H]$^+$.

Step 2: Compound **44A** (75 mg, 0.20 mmol) was added to a mixed solvent of tetrahydrofuran (3 mL) and water (1 mL), followed by triphenylphosphine (100 mg, 0.40 mmol), and the mixture was stirred at room temperature under nitrogen atmosphere for 12 hours. Upon completion of the reaction as monitored by TLC, the reaction system was concentrated under reduced pressure, and the resulting residue was purified by silica gel column chromatography to afford compound **44B** (55 mg, yield: 79%).

LC-MS (ESI): m/z=348.2[M+H]$^+$.

Step 3: Compound **44B** (55 mg, 0.16 mmol) and compound **22D** (68 mg, 0.24 mmol) were added to a reaction flask and dissolved in tetrahydrofuran (5 ml), and then DIPEA (41 mg, 0.32 mmol) was added, and the mixture was reacted at room temperature for 3 hours. Upon completion of the reaction as monitored by TLC, the reaction system was diluted with water, extracted with ethyl acetate, dried over anhydrous sodium sulphate, and filtered. The filtrate was concentrated under reduced pressure, and the resulting residue was purified by silica gel column chromatography to afford compound **44C** (60 mg, yield: 77%).

LC-MS (ESI): m/z=494.2 [M+H]$^+$.

Step 4: Compound **44C** (60 mg, 0.12 mmol) was added to a reaction flask and dissolved in THF : MeOH : $H_2O$ = 5 : 1 : 1 (5 ml), and then lithium hydroxide monohydrate (25 mg, 0.60 mmol) was added, and the mixture was reacted at 35°C for 16 hours. Upon completion of the reaction as monitored by TLC, the reaction system was adjusted to approximately pH 7 with 1M HCl, and concentrated under reduced pressure, and the resulting residue was purified and separated by reversed-phase column chromatography to afford compound **44** (16 mg, yield: 27%).

LC-MS (ESI): m/z=480.3[M+H]$^+$.

$^1$H NMR (400 MHz, $CD_3OD$) δ 7.90-7.75 (m, 1H), 7.41-7.34 (m, 1H), 5.02-4.94 (m, 1H), 4.75 (s, 2H), 4.15 (s, 3H), 3.91(s, 2H), 3.08-2.97 (m, 2H), 2.78-2.62 (m, 2H), 2.53 (s, 3H), 2.44 (s, 2H), 1.21 (s, 6H).

**Example 45**

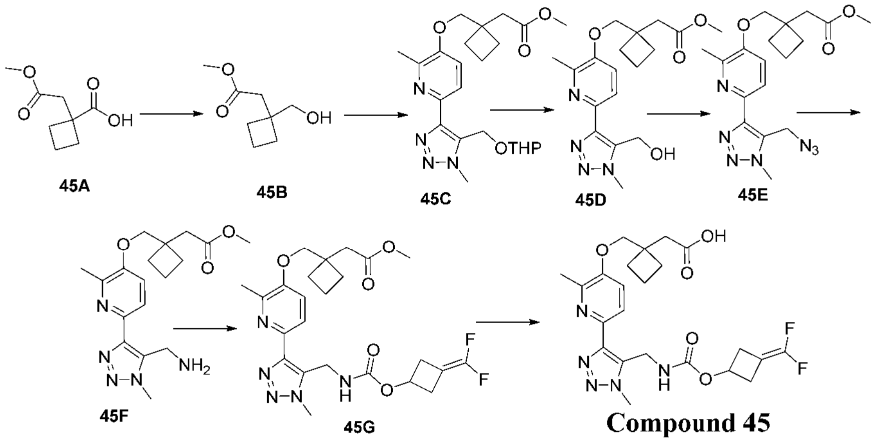

45A 45B 45C 45D 45E 45F 45G Compound 45

Step 1: Compound **45A** (1.0 g, 5.81 mmol) was dissolved in THF (20 ml), a borane dimethyl sulphide solution (0.8 ml, 10 M in DMS) was added at 0°C, and the mixture was warmed to room temperature and reacted for 16 hours. Upon completion of the reaction as monitored by TLC, the reaction system was quenched with methanol and concentrated under

reduced pressure, and the resulting residue was purified by silica gel column chromatography to afford compound **45B** (0.9 g, yield: 97.82%).

$^1H$ NMR (400 MHz, DMSO-$d_6$) δ 4.65 - 4.61 (m, 1H), 3.56 (s, 3H), 3.39 - 3.36 (m, 2H), 2.43 (s, 2H), 1.84 - 1.77 (m, 6H).

Step 2: Compound **17E** (0.95 g, 3.12 mmol), compound **45B** (0.89 g, 5.62 mmol) and tributylphosphine (1.9 g, 9.36 mmol) were added to a reaction flask and dissolved in toluene (50 ml), and then ADDP (2.35 g, 9.36 mmol) was added, and the mixture was reacted at 55°C for 16 hours. Upon completion of the reaction as monitored by TLC, the reaction system was cooled to room temperature and concentrated under reduced pressure, and the resulting residue was purified by silica gel column chromatography to afford compound **45C** (1.2 g, yield: 86.48%).

LC-MS (ESI): m/z=445.3[M+H]+.

Step 3: Compound **45C** (1.2 g, 2.70 mmol) was added to a reaction flask and dissolved in methanol (15 ml), and then PPTS (0.68 g, 2.70 mmol) was added, and the mixture was reacted at 60°C for 4 hours. Upon completion of the reaction as monitored by TLC, the reaction system was concentrated under reduced pressure, and the resulting residue was purified by silica gel column chromatography to afford compound **45D** (0.56 g, yield: 57.56%).

LC-MS (ESI): m/z=361.2[M+H]+.

Step 4: Compound **45D** (0.56 g, 1.55 mmol) was added to a reaction flask and dissolved in tetrahydrofuran (10 ml), and then DPPA (850 mg, 3.10 mmol) and DBU (470 mg, 3.10 mmol) were added, and the mixture was reacted at room temperature for 2 hours. Upon completion of the reaction as monitored by TLC, the reaction system was concentrated under reduced pressure, and the resulting residue was purified by silica gel column chromatography to afford compound **45E** (0.55 g, yield: 91.84%).

LC-MS (ESI): m/z=386.3[M+H]+.

Step 5: Compound **45E** (0.55 g, 1.43 mmol) was added to a reaction flask and dissolved in THF : $H_2O$ = 5 : 1 (12 ml), and then triphenylphosphine (750 mg, 2.86 mmol) was added, and the mixture was reacted at room temperature for 1 hour. Upon completion of the reaction as monitored by TLC, the reaction system was concentrated under reduced pressure, and the resulting residue was purified by silica gel column chromatography to afford compound **45F** (0.25 g, yield: 48.74%).

LC-MS (ESI): m/z=360.3[M+H]+.

Step 6: Compound **45F** (0.25 g, 0.70 mmol) was added to a reaction flask and dissolved in dichloromethane (10 ml), and then DIPEA (0.36 ml, 2.1 mmol) and compound **22D** (0.24 g, 0.84 mmol) were added, and the mixture was reacted at room temperature for 16 hours. Upon completion of the reaction as monitored by TLC, the reaction system was concentrated under reduced pressure, and the resulting residue was purified by silica gel column chromatography to afford compound **45G** (0.3 g, yield: 85.32%).

LC-MS (ESI): m/z=506.2[M+H]+.

Step 7: Compound **45G** (0.3 g, 0.59 mmol) was added to a reaction flask and dissolved in THF : $H_2O$ : MeOH = 3 : 1 : 1 (10 ml), and then lithium hydroxide (28 mg, 1.18 mmol) was added, and the mixture was reacted at room temperature for 3 hours. Upon completion of the reaction as monitored by TLC, the reaction system was concentrated under reduced pressure, and the resulting residue was purified by reversed-phase column chromatography to afford compound **45** (0.1 g, yield: 34.28%).

$^1H$ NMR (400 MHz, DMSO-$d_6$) δ 7.86 - 7.81 (m, 1H), 7.74 - 7.66 (m, 1H), 7.48 - 7.43 (m, 1H), 4.98 - 4.88 (m, 1H), 4.80 - 4.72 (m, 2H), 4.08 (s, 2H), 4.04 (s, 3H), 3.07 - 2.96 (m, 2H), 2.72 - 2.61 (m, 2H), 2.58 (s, 2H), 2.43 (s, 3H), 2.06 - 1.89 (m, 6H).

LC-MS (ESI): m/z=492.2[M+H]+.

**Example 46**

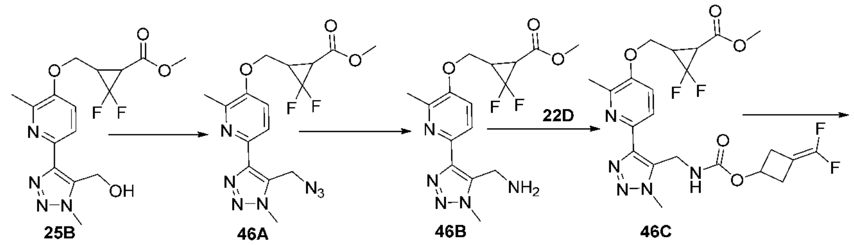


25B 46A 46B 46C

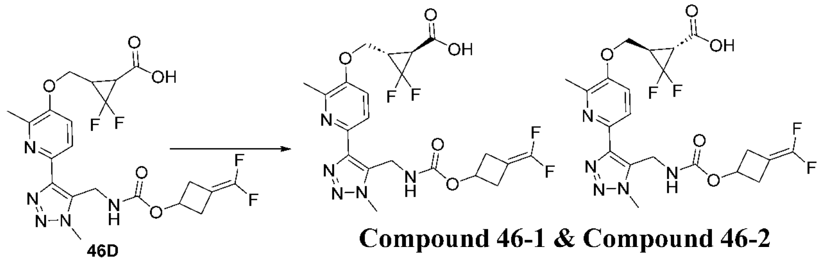

46D

**Compound 46-1 & Compound 46-2**

Step 1: Compound **25B** (600 mg, 1.63 mmol) and 1,8-diazabicyclo[5.4.0]undec-7-ene (496 mg, 3.26 mmol) were dissolved in tetrahydrofuran (15 ml), and then diphenyl phosphorazidate (897 mg, 3.26 mmol) was added, and the mixture was stirred and reacted at room temperature for 4 hours. Upon completion of the reaction as monitored by TLC, the reaction system was concentrated under reduced pressure, and the resulting residue was purified by silica gel column chromatography (PE : EA = 2 : 1) to afford compound **46A** (430 mg, yield: 67.12%).

LC-MS (ESI): m/z=394.1[M+H]$^+$.

Step 2: Compound **46A** (430 mg, 1.09 mmol) was dissolved in a mixed solvent of tetrahydrofuran (10 ml) and water (2 ml), and then triphenylphosphine (573 mg, 2.19 mmol) was added, and the mixture was stirred and reacted at room temperature for 4 hours. Upon completion of the reaction as monitored by TLC, the reaction system was concentrated under reduced pressure, and the resulting residue was purified by silica gel column chromatography (DCM : MeOH = 10 : 1) to afford compound **46B** (200 mg, yield: 50.0%).

LC-MS (ESI): m/z=368.2[M+H]$^+$.

Step 3: Compound **46B** (200 mg, 0.54 mmol) was added to a reaction flask and dissolved in dichloromethane (20 ml), and then DIPEA (210 mg, 1.63 mmol) and **22D** (230 mg, 0.81 mmol) were added, and the mixture was reacted at room temperature for 16 hours. Upon completion of the reaction as monitored by TLC, the reaction system was concentrated under reduced pressure, and the resulting residue was purified by silica gel column chromatography (PE : EA = 1 : 1) to afford compound **46C** (230 mg, yield: 83.03%).

LC-MS (ESI): m/z=514.2[M+H]$^+$.

Step 4: Compound **46C** (230 mg, 0.45 mmol) was added to a reaction flask and dissolved in THF : MeOH : $H_2O$ = 1 : 1 : 1 (12 ml), and then anhydrous lithium hydroxide (43 mg, 1.79 mmol) was added, and the mixture was stirred and reacted at room temperature for 18 hours. Upon completion of the reaction as monitored by TLC, dilute hydrochloric acid was added to adjust the pH to weakly acidic, and the mixture was extracted with ethyl acetate (20 mL×4). The organic phases were combined, dried over anhydrous sodium sulphate, and filtered. The filtrate was concentrated under reduced pressure, and the resulting residue was separated by preparative HPLC to afford compound **46D** (190 mg, yield: 85.20%).

LC-MS (ESI): m/z=500.3[M+H]$^+$.

Step 5: Compound **46D** was subjected to SFC resolution to afford compound **46-1** (SFC retention time: 2.004 min, 70 mg) and compound **46-2** (SFC retention time: 2.219 min, 80 mg).

SFC analytical method: instrument: SHIMADZU LC-30AD SFC, column: Chiral IK column; mobile phase: A: $CO_2$, B: 0.05%DEA in i-PrOH; gradient: 5%-40% B in A; flow rate: 3 mL/min, column temperature: 35°C; wavelength: 220 nm. SFC preparative method: instrument: Waters 150 Prep-SFC, column: Chiral IK column; mobile phase: A: $CO_2$, B: 0.1% NH3•H2O in i-PrOH; gradient: 30% B gradient elution; flow rate: 100 mL/min; column temperature: 25°C; wavelength: 220 nm; cycle time: 7.0 min; sample preparation: sample concentration 8 mg/mL, acetonitrile methanol mixed solution injection: 5.0 mL per injection. After separation, the fractions were dried to afford compound **46-1** and compound **46-2.**

Compound **46-1** (SFC retention time: 2.004 min): $^1$H NMR (400 MHz, DMSO-$d_6$) δ 13.18 (s, 1H), 7.86 - 7.84 (m, 1H), 7.68 (s, 1H), 7.50 - 7.48 (m, 1H), 4.94 - 4.91 (m, 1H), 4.77 - 4.76 (m, 2H), 4.31 - 4.29 (m, 1H), 4.24 - 4.19 (m, 1H), 4.04 (s, 3H), 3.02- 3.00 (m, 2H), 2.81- 2.64 (m, 4H), 2.42 (s, 3H).

LC-MS (ESI): m/z = 500.4 [M+H]$^+$.

Compound **46-2** (SFC retention time: 2.219 min): $^1$H NMR (400 MHz, DMSO-$d_6$) δ 13.18 (s, 1H), 7.86 - 7.84 (m, 1H), 7.69 (s, 1H), 7.50 - 7.48 (m, 1H), 4.95 - 4.91 (m, 1H), 4.77 - 4.76 (m, 2H), 4.34 - 4.29 (m, 1H), 4.24 - 4.20 (m, 1H), 4.04 (s, 3H), 3.02- 3.00 (m, 2H), 2.81- 2.65 (m, 4H), 2.43 (s, 3H).

LC-MS (ESI): m/z = 500.4 $[M+H]^+$.

**Example 47**

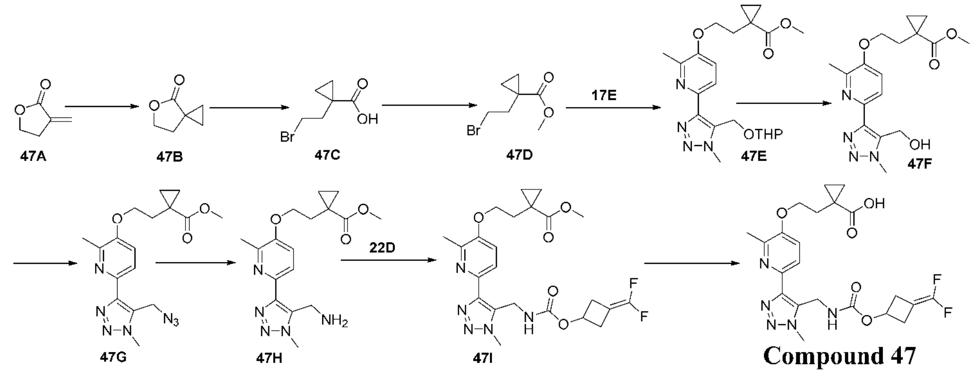


Compound 47

Step 1: A solution of diazomethane (1 mol/L) in diethyl ether (200 ml) was cooled to 0°C, **47A** (5 g, 51mmol) and palladium acetate (0.57 g, 2.5 mmol) were added, and the mixture was purged with $N_2$ and then reacted at room temperature for 3h. The reaction system was filtered, and the filtrate was directly concentrated under reduced pressure to afford **47B** (5 g, 87.5%).

$^1$HNMR (400MHz, MeOD) δ 4.45 - 4.40 (m, 2H), 2.34 (t, 2H), 1.16 - 1.12 (m, 2H), 1.07 - 1.03 (m, 2H).

Step 2: **47B** (5 g, 44.59 mmol) was added portionwise to a solution of 33% HBr in acetic acid (40 ml), and the mixture was reacted at room temperature for 2h. Water (200 ml) was added, the mixture was filtered, and the solid was washed with water to afford **47C** (4 g, 46.47%).

$^1$H NMR (400 MHz, DMSO-d6) δ 12.23 (s, 1H), 3.62 - 3.54 (m, 2H), 2.05 - 1.95 (m, 2H), 1.08 - 1.06 (m, 2H), 0.86 - 0.84 (m, 2H).

Step 3: **47C** (2 g, 10.36 mmol) was added to methanol (20 mL), followed by thionyl chloride (3.70 g, 31.08 mmol), and the mixture was reacted at 70°C for 2 h. Upon completion of the reaction as monitored, the reaction system was cooled to room temperature and directly concentrated under reduced pressure to remove methanol. Ethyl acetate (30 ml) was added, and the mixture was successively washed with water and a saturated sodium chloride solution. The organic phase was concentrated under reduced pressure to afford **47D** (2 g, 93.22%).

Compound **47** (0.03 g) was obtained following steps 2, 3, 4, 5, 6 and 7 of Example 43.

$^1$HNMR (400 MHz, $CDCl_3$) δ 8.03 (d, 1H), 7.35 (s, 1H), 5.04 - 4.94 (m, 1H), 4.60 (s, 2H), 4.30 - 4.26 (m, 2H), 4.18 (s, 3H), 3.06 - 3.00 (m, 2H), 2.75 - 2.69 (m, 2H), 2.58 (s, 3H), 2.12 - 2.10 (m, 2H), 1.45 - 1.40 (m, 2H), 1.00 - 0.95 (m, 2H).

LC-MS (ESI): m/z = 478.2$[M+H]^+$.

**Example 48**

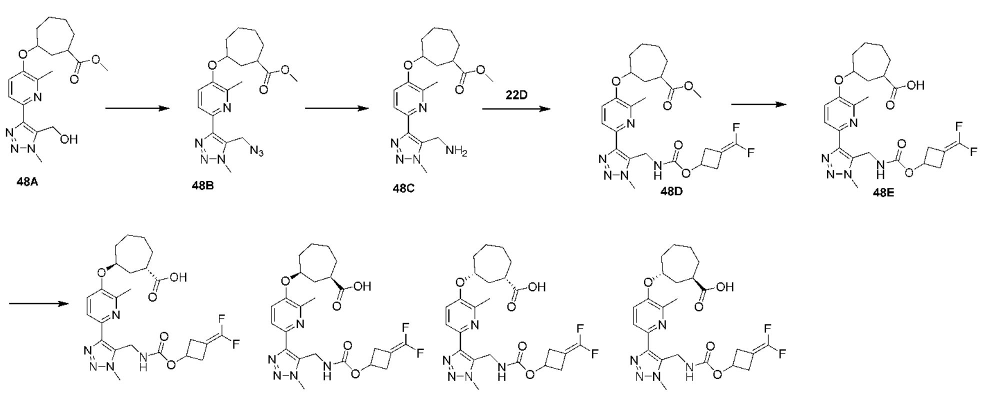


Compounds 48-1, 48-2, 48-3, 48-4

Starting from compound **48A** (580 mg, 1.55 mmol, reference for the synthetic method: WO 2020060916) and following step 4 to step 7 of Example 43, compound **48E** (160 mg, yield: 82%) was obtained.

LC-MS (ESI):m/z=506.2$[M+H]^+$.

Step 5: Compound **48E** was subjected to chiral resolution to afford compounds **48-1** (SFC retention time: 1.671 min, 8 mg), **48-2** (SFC retention time: 1.791 min, 10 mg), **48-3** (SFC retention time: 1.634 min, 6 mg) and **48-4** (SFC retention time: 1.708 min, 3 mg). SFC analytical method: instrument: SHIMADZU LC-30AD, column: Chiral WHELK column; mobile phase: A: $CO_2$, B: 0.1% $NH_3.H_2O$ in MeOH; gradient: 35% B in A; flow rate: 110 mL/min; column temperature: 35°C; wavelength: 220 nm. SFC preparative method: instrument: Waters 150 Prep-SFC, column: Chiral WHELK column; mobile phase: A: $CO_2$, B: isopropanol; gradient: 33% B gradient elution; flow rate: 120 mL/min; column temperature: 25°C; wavelength: 254 nm; cycle time: 8.1 min; sample preparation: sample concentration 2 mg/mL, ethanol solution injection: 2 mL per injection. After separation, the fractions were lyophilised at -80°C to afford compounds **48-1, 48-2, 48-3 and 48-4.**

Compound **48-1** (SFC retention time: 1.671 min): $^1$H NMR (400 MHz, DMSO-$d_6$) δ 12.07 (s, 1H), 7.83 (d, 1H), 7.69(s, 1H), 7.45 (d, 1H), 4.98 - 4.88 (m, 1H), 4.78 - 4.75 (s, 2H), 4.63 - 4.54 (m, 1H), 4.04(s, 3H), 3.08 - 2.96 (m, 2H), 2.72 - 2.62 (m, 2H), 2.57 - 2.52 (m, 1H), 2.40(s, 3H), 2.32 - 2.22 (m, 1H), 2.04 - 1.90 (m, 2H), 1.85 - 1.48 (m, 7H); LC-MS (ESI): m/z =506.2 $[M+H]^+$.

Compound **48-2** (SFC retention time: 1.791 min): $^1$H NMR (400 MHz, DMSO-$d_6$) δ 12.03 (s, 1H), 7.82 (d, 1H), 7.70(s, 1H), 7.48 (d, 1H), 4.98 - 4.87 (m, 1H), 4.81 - 4.72 (m, 3H), 4.04(s, 3H), 3.07 - 2.97 (m, 2H), 2.72 - 2.60 (m, 3H), 2.42 (s, 3H), 2.19 - 2.10 (m, 1H), 2.05 - 1.91 (m, 3H), 1.83 - 1.41 (m, 6H); LC-MS (ESI): m/z =506.2 $[M+H]^+$.

Compound **48-3** (SFC retention time: 1.634 min): $^1$H NMR (400 MHz, DMSO-$d_6$) δ 12.07 (s, 1H), 7.83 (d, 1H), 7.69(s, 1H), 7.45 (d, 1H), 4.98 - 4.87 (m, 1H), 4.79 - 4.75 (m, 2H), 4.63 - 4.53 (m, 1H), 4.04(s, 3H), 3.08 - 2.96 (m, 2H), 2.71 - 2.60 (m, 2H), 2.57 - 2.51 (m, 1H), 2.40(s, 3H), 2.33 - 2.22 (m, 1H), 2.04 - 1.91 (m, 2H), 1.85 - 1.47 (m, 7H); LC-MS (ESI): m/z =506.2 $[M+H]^+$.

Compound **48-4** (SFC retention time: 1.708 min): $^1$H NMR (400 MHz, DMSO-$d_6$) δ 11.82 (s, 1H), 7.82 (d, 1H), 7.70(s, 1H), 7.48 (d, 1H), 4.97 - 4.88 (m, 1H), 4.78 - 4.72 (m, 3H), 4.04(s, 3H), 3.07 - 2.97 (m, 2H), 2.72 - 2.61 (m, 3H), 2.42 (s, 3H), 2.17 - 2.09 (m, 1H), 2.05 - 1.91 (m, 3H), 1.83 - 1.41 (m, 6H); LC-MS (ESI): m/z =506.2 $[M+H]^+$.

**Biological assay and evaluation**

**1. Cell calcium flux assay**

1) The test compound was diluted to a 400X stock solution with DMSO in a 384-well plate.
2) 1 μl of compound solution from step 1 was transferred into 39 ul of assay buffer to prepare a 10X working solution in a 384-well plate using a Bravo automated liquid handling platform.
3) CHO-LPA1 cells were cultured in F12 medium (10% FBS).
4) When cells reached 80% confluence, the cells were dissociated using 0.25% trypsin-EDTA.
5) Cell density was determined and the cells were diluted to 4x10e5/ml using F12 (10% FBS).
6) 30 μl of the cell suspension was dispensed into a 384-well plate (corning 3764#) using a multidrop automated dispenser, resulting in 12,000 cells per well. The plate was incubated at 37°C with 5% $CO_2$ for 18-20 hours.
7) The medium was replaced with 25 uL of serum-free medium and incubated overnight.
8) 10 ul of 3.5X loading dye was added to each well of the cell plate. The plate was incubated in the dark at 37°C with 5% $CO_2$ for 0.5-1 hours.
9) After incubation, 5 μl of the 10X working solution from step 2 was transferred to the cell plate.
10) The cell plate was incubated in the dark at 25°C for 15 minutes, and then the calcium signals were read.
11) A 5X agonist (LPA) working solution in 1X HBSS+20 mM HEPES+0.1% BSA was prepared in a 384-well assay plate (greiner 784075#), with at least 20 μl per well. The agonist concentration used in this assay was determined based on the dose-response curve from a prior agonist mode experiment, with the EC80 value serving as the final agonist concentration.
12) Data were read and saved using FLIPR at room temperature with the specified settings.
13) The signal values were plotted against compound concentrations, and the curve fitting was performed and the $IC_{50}$ values were calculated using nonlinear regression analysis in GraphPad Prism software.

Experimental results: The compounds of the present invention had a significant antagonistic effect on LPAR1 enzyme activity in vitro, and the $IC_{50}$ value of the example compounds for LPAR1 enzyme activity was less than 100 μM. $IC_{50}$ values are represented by grades A, B, C, and D, where A represents $0 < IC_{50} \leq 10$ nM, B represents 10 nM $< IC_{50} \leq 50$ nM, C represents 50 nM $< IC_{50} \leq 100$ nM, and D represents $IC_{50} > 100$ nM. The test results of some examples were shown

in Table 1.

Table 1 Antagonistic activity of the compounds of the present invention against LPAR1

| Compound | LPAR1 $IC_{50}$ (nM) | Compound | LPAR1 $IC_{50}$ (nM) |
|---|---|---|---|
| Compound 4 | A | Compound 25 | A |
| Compound 5 | A | Compound 26 | A |
| Compound 6 | B | Compound 27 | A |
| Compound 7 | A | Compound 28 | A |
| Compound 8-1 | A | Compound 29 | A |
| Compound 9-1 | A | Compound 30 | A |
| Compound 11 | A | Compound 31 | A |
| Compound 12 | A | Compound 32-1 | A |
| Compound 13 | B | Compound 32-4 | A |
| Compound 14 | A | Compound 34-2 | A |
| Compound 15 | B | Compound 35-2 | A |
| Compound 17-2 | A | Compound 36 | A |
| Compound 18 | A | Compound 38 | A |
| Compound 19 | A | Compound 39 | A |
| Compound 20-1 | B | Compound 40 | A |
| Compound 21 | A | Compound 41 | A |
| Compound 22 | A | Compound 42 | A |
| Compound 23 | A | Compound 48-2 | B |
| Compound 24 | A | | |

Experimental conclusion: The compounds of the present invention, such as the example compounds, exhibit high antagonistic activity against the LPAR1 receptor, with $IC_{50}$ values of 2 nM for compounds 4, 5, 7, 12, and 19.

**2. Pharmacokinetic test in mice**

2.1 Experimental animals: Male C57 mice, 20-25 g, 6 mice/compound, purchased from Chengdu Ddossy Experimental Animals Co., Ltd.

2.2 Experimental design: On the day of the experiment, the C57 mice were randomly grouped according to their body weights. The animals were fasted with water available for 12-14 h one day before the administration, and were fed 4 h after the administration.

Table 2. Administration information

| Group | Number | Administration information | | | | | |
|---|---|---|---|---|---|---|---|
| | Male | | Administration dosage (mg/kg) | Administration concentration (mg/mL) | Administration volume (mL/kg) | Collected sample | Mode of administration |
| G1 | 3 | Test compound | 1 | 0.2 | 5 | Plasma | Intravenous administration |
| G2 | 3 | | 3 | 0.6 | 5 | Plasma | Oral gavage administration |
| Note: vehicle for intravenous administration: 5% DMA+5% Solutol+90% Saline; vehicle for oral gavage administration: 10% Cremophor-EL+40% PEG400+50% 1XPBS (pH = 7.4). | | | | | | | |

Before and after administration, 0.06 mL of blood was taken from the orbit under isoflurane anaesthesia and placed in an EDTAK2 centrifuge tube. The tube was centrifuged at 5000 rpm at 4°C for 10 min, and plasma was collected. The blood collection time points for the intravenous administration group and oral gavage administration group were: 0 min, 5 min, 15 min, 30 min, 1 h, 2 h, 4 h, 6 h, 8 h, and 24 h. Before analysis and detection, all samples were stored at -80°C, and the samples were quantitatively analysed by LC-MS/MS.

Table 3. Pharmacokinetic parameters of test compounds in plasma of mice

| Test compound | Mode of administration | $C_0$ or $C_{max}$ (ng/mL) | CL (mL/min/kg) | $Vd_{ss}$ (L/kg) | $AUC_{0-t}$ (hr*ng/mL) | F (%) |
|---|---|---|---|---|---|---|
| Compound 4 | i.v. (1 mg/kg) | 1066±134 | 33.2±5.6 | 5.11±1.0 | 510±95 | - |
| | i.g. (3 mg/kg) | 687±331 | - | - | 2179±672 | ≥98 |
| Compound 12 | i.v. (1 mg/kg) | 1938±258 | 17.5±2.4 | 2.57±0.23 | 962±131 | - |
| | i.g. (3 mg/kg) | 581±18 | - | - | 1452±81 | - |
| Compound 22 | i.v. (1 mg/kg) | 1537±290 | 10.4±0.55 | 2.77±0.16 | 1570±74 | - |
| | i.g. (3 mg/kg) | 1788±180 | - | - | 5606±1406 | ≥98 |
| Compound 26 | i.v. (1 mg/kg) | 2268±193 | 10.3±0.52 | 2.83±0.53 | 1580±50 | - |
| | i.g. (3 mg/kg) | 659±55 | - | - | 2289±102 | - |
| Compound 47 | i.v. (1 mg/kg) | 1361±179 | 16.8±5.7 | 5.40±1.4 | 1001±333 | - |
| | i.g. (3 mg/kg) | 566±122 | - | - | 2739±325 | 91.3 |
| -: not applicable. | | | | | | |

Conclusion: The compounds of the present invention, such as the example compounds, have good pharmacokinetic characteristics in mice, for example, compounds 4, 12, 22, 26 and 47 have excellent pharmacokinetic characteristics in mice.

### 3. Pharmacokinetic test in rats

3.1. Experimental animals: Male SD rats, about 220 g, 6-8 weeks old, 6 rats/compound, purchased from Chengdu Ddossy Experimental Animals Co., Ltd.

3.2. Experimental design: On the day of the experiment, the SD rats were randomly grouped according to their body weights. The animals were fasted with water available for 12-14 h one day before the administration, and were fed 4 h after the administration.

Table 4. Administration information

| Group | Number | Administration information | | | | | |
|---|---|---|---|---|---|---|---|
| | Male | | Administration dosage (mg/kg) | Administration concentration (mg/mL) | Administration volume (mL/kg) | Collected sample | Mode of administration |
| G1 | 3 | Test compound | 1 | 0.2 | 5 | Plasma | Intravenous administration |
| G2 | 3 | | 3 | 0.6 | 5 | Plasma | Oral gavage administration |
| Note: vehicle for intravenous administration: 5% DMA+5% Solutol+90% Saline; vehicle for oral gavage administration: 10% Cremophor-EL+40% PEG400+50% 1XPBS (pH = 7.4). | | | | | | | |

Before and after administration, 0.15 mL of blood was taken from the orbit under isoflurane anaesthesia and placed in an EDTAK2 centrifuge tube. The tube was centrifuged at 5000 rpm at 4°C for 10 min, and plasma was collected. Before analysis and detection, all samples were stored at -80°C, and the samples were quantitatively analysed by LC-MS/MS.

Table 5. Pharmacokinetic parameters of test compound in plasma of rats

| Test compound | Mode of administration | $C_0$ or $C_{max}$ (ng/mL) | CL (mL/min/kg) | $Vd_{ss}$ (L/kg) | $AUC_{0-t}$ (hr*ng/mL) | F (%) |
|---|---|---|---|---|---|---|
| Compound 22 | i.v. (1 mg/kg) | 2428±221 | 9.45±1.7 | 1.21±0.17 | 1794±321 | - |
| | i.g. (3 mg/kg) | 1630±162 | - | - | 6713±652 | ≥98 |
| Compound 26 | i.v. (1 mg/kg) | 2745±354 | 8.91±1.2 | 1.21±0.50 | 1867±246 | - |
| | i.g. (3 mg/kg) | 1144±145 | - | - | 2878±913 | - |
| Compound 29 | i.v. (1 mg/kg) | 2324±170 | 15.0±2.7 | 3.53±1.6 | 1094±208 | - |
| | i.g. (3 mg/kg) | 739±29 | - | - | 2535±679 | - |
| Compound 47 | i.v. (1 mg/kg) | 3233±495 | 5.95±1.2 | 0.825±0.027 | 2852±532 | - |
| | i.g. (3 mg/kg) | 3060±457 | - | - | 12586±1320 | ≥98 |
| -: not applicable. | | | | | | |

Conclusion: The compounds of the present invention, such as example compounds 22, 26, and 29, have good pharmacokinetic characteristics in rats.

**4. Pharmacokinetic test in beagle dogs**

4.1. Experimental animals: Male beagle dogs, about 8 to 11 kg, 6 beagle dogs/compound, purchased from Beijing Marshall Biotechnology Co., Ltd.

4.2. Experimental method: On the day of the experiment, the beagle dogs were randomly grouped according to their body weights. The animals were fasted with water available for 12-14 h one day before the administration, and were fed 4 h after the administration.

Table 6. Administration information

| Group | Number | Administration information | | | | | |
|---|---|---|---|---|---|---|---|
| | Male | | Administration dosage (mg/kg) | Administration concentration (mg/mL) | Administration volume (mL/kg) | Collected sample | Mode of administration |
| G1 | 3 | Test compound | 1 | 1 | 1 | Plasma | Intravenous administration |

(continued)

| Group | Number | Administration information | | | | | |
|---|---|---|---|---|---|---|---|
| | Male | | Administration dosage (mg/kg) | Administration concentration (mg/mL) | Administration volume (mL/kg) | Collected sample | Mode of administration |
| G2 | 3 | | 3 | 0.6 | 5 | Plasma | **Oral** gavage administration |
| Note: vehicle for intravenous administration: 5% DMA+5% Solutol+90% Saline; vehicle for oral gavage administration: 10% Cremophor-EL+40% PEG400+50% 1XPBS (pH = 7.4). | | | | | | | |

Before and after administration, 1 mL of blood was taken from the jugular veins or limb veins and placed in an EDTAK2 centrifuge tube. The tube was centrifuged at 5000 rpm at 4°C for 10 min, and plasma was collected. Before analysis and detection, all samples were stored at -80°C. The samples were analysed quantitatively by LC-MS/MS.

Conclusion: The compounds of the present invention, such as the example compounds, have good pharmacokinetic characteristics in beagle dogs.

### 5. Pharmacokinetic test in monkeys

5.1. Experimental animals: Male cynomolgus monkeys, 3-5 kg, 3-6 years old, 4 monkeys/compound, purchased from Suzhou Xishan Biotechnology Inc.

5.2. Experimental method: On the day of the experiment, the monkeys were randomly grouped according to their body weights. The animals were fasted with water available for 14 to 18 h one day before the administration, and were fed 4 h after the administration.

Before and after administration, 1.0 mL of blood was taken from the limb veins and placed in an EDTAK2 centrifuge tube. The tube was centrifuged at 5000 rpm at 4°C for 10 min, and plasma was collected. Before analysis and detection, all samples were stored at -80°C, and the samples were quantitatively analysed by LC-MS/MS.

Conclusion: The compounds of the present invention, such as the example compounds, have good pharmacokinetic characteristics in monkeys.

### 6. hERG potassium ion channel test

Experimental platform: electrophysiological manual patch-clamp system
Cell line: Chinese hamster ovary (CHO) cell lines stably expressing hERG potassium ion channel
Experimental method: In Chinese Hamster Ovary (CHO) cells stably expressing hERG potassium channel, whole cell patch-clamp technique was used to record hERG potassium channel current at room temperature. The glass microelectrode was made of a glass electrode blank (BF150-86-10, Sutter) by a puller. The tip resistance after filling the liquid in the electrode was about 2-5 MΩ. The glass microelectrode can be connected to the patch-clamp amplifier by inserting the glass microelectrode into an amplifier probe. The clamping voltage and data recording were controlled and recorded by the pClamp 10 software through a computer. The sampling frequency was 10 kHz, and the filtering frequency was 2 kHz. After the whole cell records were obtained, the cells were clamped at -80 mV, and the step voltage that induced the hERG potassium current (I hERG) was depolarised from -80 mV to +20 mV for 2 s, then repolarised to -50 mV, and returned to -80 mV after 1 s. This voltage stimulation was given every 10 s, and the administration process was started after the hERG potassium current was confirmed to be stable (at least 1 minute). The compound was administered for at least 1 minute at each test concentration, and at least 2 cells ($n \geq 2$) were tested at each concentration.

Data processing: Data analysis processing was carried out by using pClamp 10, GraphPad Prism 5 and Excel software. The inhibition degree of hERG potassium current (peak value of hERG tail current induced at -50 mV) at different compound concentrations was calculated by the following formula:

$$\text{Inhibition \%} = [1 - (I / Io)] \times 100\%$$

where Inhibition% represents the percentage of inhibition of hERG potassium current by the compound, and I and Io

represent the amplitude of hERG potassium current after and before the administration, respectively.

Compound IC50 was calculated using GraphPad Prism 5 software by fitting according to the following equation:

$$Y = Bottom + (Top-Bottom)/(1+10^((LogIC50-X)*HillSlope))$$

where X represents the Log value of the tested concentration of the test compound, Y represents the percentage inhibition at the corresponding concentration, and Bottom and Top represent the minimum and maximum percentage inhibitions, respectively.

Conclusion: The compounds of the present invention, such as the example compounds, do not inhibit hERG.

**7. CYP enzyme inhibition test**

The purpose of this study was to evaluate the effect of the test compound on the activity of five isoenzymes (CYP1A2, CYP2C9, CYP2C19, CYP2D6 and CYP3A4) of human liver microsomal cytochrome P450 (CYP) by using an *in vitro* testing system. The specific probe substrates of CYP450 isoenzymes were co-incubated with human liver microsomes and test compounds of different concentrations, respectively, and reduced nicotinamide adenine dinucleotide phosphate (NADPH) was added to initiate the reaction. After the completion of the reaction, the sample was treated, and liquid chromatography-tandem mass spectrometry (LC-MS/MS) was used to quantitatively detect metabolites produced by the specific substrates, changes in CYP enzyme activity were determined, and IC50 value was calculated to evaluate the inhibitory potential of the test compound on each CYP enzyme subtype. Under the test conditions, the incubation concentration was 0-30 μM.

Conclusion: The compounds of the present invention, such as the example compounds, do not inhibit the CYP enzyme.

**8. Liver microsomal stability test**

In this experiment, liver microsomes of five species, including human, dog, rat, and mouse, were used as *in vitro* models to evaluate the metabolic stability of the test compound.

At 37°C, 1 μM test compound was co-incubated with microsomal protein and coenzyme NADPH. At given time points of the reaction (5 min, 10 min, 20 min, 30 min and 60 min), the reaction was terminated by adding ice-cold acetonitrile containing an internal standard. The LC-MS/MS method was used to measure the concentration of the test compound in the sample. $T_{1/2}$ was calculated using the natural logarithm (ln) of the residual rate of the drug in the incubation system and the incubation time. In addition, the intrinsic clearance in liver microsomes $CL_{int(mic)}$ and the intrinsic clearance in liver $CL_{int(Liver)}$ were further calculated.

Conclusion: The compounds of the present invention, such as the example compounds, have good stability in liver microsomes.

**9. Caco2 permeability test**

In the experiment, a monolayer of Caco-2 cells was used, and incubated in triplicate in a 96-well Transwell plate. A transport buffer solution (HBSS, 10 mM HEPES, pH 7.4 ± 0.05) containing the compound of the present invention (2 μM) **or the control** compound digoxin (10 μM), nadolol (2 μM) or metoprolol (2 μM) was added to the administration end hole on the apical side or basal side. A DMSO-containing transport buffer solution was added to the corresponding receiving end hole. After incubation for 2 hours at 37°C ± 1°C, the cell plate was removed, and an appropriate amount of samples was taken from both the apical side and basal side and transferred to a new 96-well plate. Subsequently, acetonitrile containing an internal standard was added to precipitate the protein. The samples were analysed using LC MS/MS, and the concentrations of the compound of the present invention and the control compound were determined. Concentration data were used to calculate apparent permeability coefficients for transport from the apical side to the basal side, and from the basal side to the apical side of the cell monolayer, and thus to calculate the efflux ratio. The integrity of the cell monolayer after 2 hours of incubation was evaluated by leakage of Lucifer Yellow.

Table 7

| Name of compound | Average apparent permeability coefficient ($10^{-6}$ cm/s) | | Efflux ratio |
|---|---|---|---|
| | A-B | B-A | |
| Compound 26 | 4.31 | 29.2 | 6.79 |

Conclusion: The compounds of the present invention, such as the example compounds, exhibit good permeability.

**10. Mouse model of bleomycin (BLM)-induced idiopathic pulmonary fibrosis (IPF)**

1) Screening and grouping: A total of 48 nine-week-old male C57BL/6j mice (experimental animals) were used in this study. Prior to the start of the experiment, the animals were divided into a sham-operated group (Group 1, 8 mice) and a model group (40 mice) based on their body weights. One week after model establishment, the model group animals were further divided into six subgroups (Groups 2-6, n = 8 per group) based on their body weights.
2) Animal model establishment: On day 1 of the experiment, the animals were anesthetised with Zoletil (50 mg/kg) and Xylazine (10 mg/kg). Mice in the model group received an intratracheal (i.t.) injection of bleomycin at a dose of 0.66 mg/kg (1 U/kg) in a volume of 50 μL on day 1. Mice in the sham-operated group (Group 1, n = 10) received an intratracheal injection of normal saline in a volume of 50 μL.
3) Mode of administration of test compounds: Starting from day 7 of the experiment, the animals were treated as follows. Group 3: compound 22 was administered by oral gavage at a dose of 1 mg/kg at a volume of 10 mL/kg body weight, twice daily. Group 4: compound 22 was administered by oral gavage at a dose of 3 mg/kg at a volume of 10 mL/kg body weight, twice daily. Group 5: BMS-986278 was administered by oral gavage at a dose of 10 mg/kg at a volume of 10 mL/kg body weight, twice daily. Group 6: nintedanib was administered by oral gavage at a dose of 60 mg/kg at a volume of 10 mL/kg body weight, once daily. Sham-operated group (Group 1) and model group (Group 2): the vehicle was administered by oral gavage at a volume of 10 mL/kg body weight, twice daily.
4) Test indicators: Lung tissues were collected at the study endpoint for pathological evaluation.

Results: Administration of bleomycin (0.66 mg/kg, i.t.) significantly increased the Modified Ashcroft score and the fibrotic area in lung tissues of model mice 21 days post-injection. Compared with the vehicle-treated model group, oral gavage administration (twice daily for 14 consecutive days) of test compound 22 at doses of 1 mg/kg and 3 mg/kg significantly reduced both the Modified Ashcroft score and the fibrotic area in lung tissues of model mice at the study endpoint. The group treated with 3 mg/kg compound 22 achieved a Modified Ashcroft score of less than 4 and a fibrotic area of less than 20%. At a dose of 1 mg/kg, the efficacy of test compound 22 was comparable to that of BMS-986278 (10 mg/kg) and nintedanib (60 mg/kg). At a dose of 3 mg/kg, compound 22 showed superior efficacy to both BMS-986278 and nintedanib.

## Claims

1. A compound as shown in formula (I), or a stereoisomer, a deuterated substance, or a pharmaceutically acceptable salt thereof, **characterised in that**

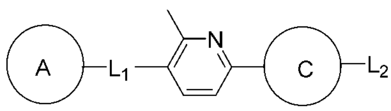

(I)

ring C is

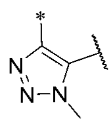

,

wherein the "*" end is connected to a pyridine ring on the left side, and the

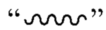

end is connected to $L_2$;

ring A is selected from 3- to 12-membered carbocyclyl and 4- to 12-membered heterocyclyl, or ring A is absent, wherein the heterocyclyl contains 1-3 heteroatoms selected from N, O and S; the carbocyclyl and heterocyclyl are optionally further substituted with 1-4 $R^A$;

each $R^A$ is independently selected from H, halogen, =O, CN, OH, $C_{1-6}$ alkyl, $C_{2-6}$ alkenyl, $C_{2-6}$ alkynyl, $-OC_{1-4}$ alkyl, $-(CH_2)_p$-(5- to 10-membered heterocyclyl), -(a 3- to 6-membered carbocyclic ring)-$COOR^{a1}$, $-(CH_2)_p-COOR^{a1}$, $-(CH_2)_p-C(O)NR^{a1}R^{a2}$, $-(CH_2)_p-C(O)NHC(O)R^{a1}$, $-(CH_2)_p-C(O)NHS(O)_2R^{a1}$, $-(CH_2)_p-C(O)NHS(O)R^{a1}$, $-(CH_2)_p-S(O)_2OH$, $-(CH_2)_p-S(O)_2NHC(O)R^{a1}$, $-(CH_2)_P-P(O)(OH)_2$, $=CH_2$, $=CF_2$, $=CH-CH_3$ or $=C-(CH_3)_2$, wherein the heterocyclyl contains 1-3 heteroatoms selected from N, O and S; the alkyl, alkenyl, alkynyl and heterocyclyl are optionally further substituted with 1-4 groups selected from halogen, OH, $NH_2$, CN, and -O-halogenated $C_{14}$ alkyl;

$L_1$ is selected from a bond, $C_{16}$ alkyl, $C_{2-6}$ alkenyl, $C_{2-6}$ alkynyl, -O-, $-O-C_{1-6}$ alkyl-, -S-, $-S-C_{1-6}$ alkyl-, $-C(O)NR^{L1}-$, $-NR^{L1}C(O)-$,

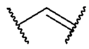,

$-NR^{L1}-$, and $-NR^{L1}-C_{1-6}$ alkyl-, wherein the alkyl, alkenyl, and alkynyl are optionally further substituted with 1-4 $R^{L1}$;

each $R^{L1}$ is independently selected from H, halogen, OH, $C_{1-4}$ alkyl, halogenated $C_{1-4}$ alkyl, 3- to 6-membered cycloalkyl, and -COOH, wherein the alkyl and cycloalkyl are optionally further substituted with 1-4 groups selected from halogen, OH, and $NH_2$;

when ring A is absent, $L_1$ is selected from $-OC_{1-6}$ alkyl substituted with one COOH, $C_{2-6}$ alkenyl, and $C_{2-6}$ alkynyl;

$L_2$ is selected from $-(CR^{L21}R^{L22})p-OC(O)-N(R^{L23})2$, - $(CR^{L21}R^{L22})pN(R^{L24})S(O_2N(R^{L25})_2$, $-(CR^{L21}R^{L22})_pN^{L26})C(O)N(R^{L27})(R^{L28})$, $-(CR^{L21}R^{L22})_pN(R^{L29})(R^{L30})$, $-C(=O)N(R^{L31})_2$, $-N(R^{L31})-C(=O)OR^{L32}$, $-(CH2)_pR^{L33}$, $-(CR^{L21}R^{L22})_p-C(O)-N(R^{L29})-C(O)R^{L21}$, $-(CR^{L21}R^{L22})_p-C(O)-N(R^{L29})-S(O)_2R^{L21}$, -(3-to 6-membered cycloalkyl)-$C(=O)N(R^{L25})_2$, -(4- to 7-membered heterocyclyl)-$C(=O)N(R^{L25})_2$, -($C_{2-4}$ alkenyl)-(4- to 7-membered heterocyclyl)-$N(R^{L25})_2$, - $(CR^{L21}R^{L22})_p-SC(O)-N(R^{L25})_2$, $-(CR^{L21}R^{L22})_pN(R^{L29})C(S)N(R^{L27})(R^{L28})$,

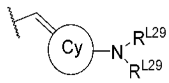,

$-(CR^{L21}R^{L22})_p-OC(O)-R^{L23}$, $-(CR^{L21}R^{L22})_p-O-R^{L23}$, and $-(CR^{L21}R^{L22})_p-N(R^{L26})C(O)O-R^{L23}$, wherein Cy is 4- to 7-membered heterocyclyl; the cycloalkyl and heterocyclyl are optionally further substituted with 1-4 groups selected from halogen, =O, OH, $NH_2$, CN, $C_{1-4}$ alkyl, $C_{1-4}$ alkoxy, halogenated $C_{1-4}$ alkyl, $C_{3-6}$ cycloalkyl, and 5- to 7-membered heterocyclyl;

$R^{a1}$, $R^{a2}$, $R^{L1}$, $R^{L2}$, $R^{L21}$, $R^{L22}$, $R^{L23}$, $R^{L24}$, $R^{L25}$, $R^{L26}$, $R^{L27}$, $R^{L28}$, $R^{L29}$, $R^{L30}$, and $R^{L31}$ are each independently selected from H, halogen, $C_{1-4}$ alkyl, $C_{2-6}$ alkenyl, $C_{2-6}$ alkynyl, $C_{1-4}$ alkoxy, 5- to 14-membered heterocyclyl, $-(CH_2)_p$-(4- to 7-membered heterocyclyl), $-(CH_2)_p-C_{3-10}$ cycloalkyl, $-(CH_2)_p-O-C_{3-10}$ cycloalkyl, and $-O-C_{3-10}$ cycloalkyl, wherein the heterocyclyl contains 1-3 heteroatoms selected from N, O and S; the alkyl, alkenyl, alkynyl, cycloalkyl, and heterocyclyl are optionally further substituted with 1-4 groups selected from halogen, =O, $=CH_2$, $=CF_2$, $=CH-CH_3$, $=C-(CH_3)_2$, OH, $NH_2$, CN, acetyl, $-SCF_3$, $-S(O)_2CH_3$, $C_{1-4}$ alkyl, $C_{1-4}$ alkoxy, halogenated $C_{1-4}$ alkoxy, halogenated $C_{1-4}$ alkyl, $C_{3-10}$ cycloalkyl, 5- to 12-membered heterocyclyl, and $-C\equiv C-C_{3-10}$ cycloalkyl;

$R^{L32}$ is selected from $-C_{1-4}$ alkyl-(5- to 10-membered heteroaryl) and $-C_{1-4}$ alkyl-(6- to 10-membered aryl), wherein the heteroaryl and aryl are optionally further substituted with 1-3 substituents selected from halogen, CN, OH, $NO_2$, $NH_2$, halogenated $C_{1-4}$ alkyl, $-OC_{1-4}$ alkyl, $C_{2-6}$ alkenyl or $C_{2-6}$ alkynyl;

$R^{L33}$ is selected from $C_{1-4}$ alkyl, halogen, OH, $NH_2$, CN, $C_{2-6}$ alkenyl, $C_{2-6}$ alkynyl, -C(=O)H, -C(=O)OH, $C_{3-10}$ cycloalkyl, $C_{1-4}$ alkoxy, and 5- to 14-membered heterocyclyl, wherein the alkyl, alkoxy, alkenyl, alkynyl, cycloalkyl, alkoxy and heterocyclyl are optionally further substituted with 1-4 groups selected from halogen, OH, $NH_2$, CN, $N_3$, $C_{1-4}$ alkoxy, halogenated $C_{1-4}$ alkoxy, $C_{3-5}$ cycloalkyl, 4- to 6-membered heterocycloalkyl, 6- to 10-membered aryl, and 5- to 10-membered heteroaryl;

alternatively, $R^{L24}$ and $R^{L25}$ or $R^{L26}$ and $R^{L27}$ or two $R^{L23}$, together with the atoms to which they are attached, form a 5- to 7-membered heterocyclic ring containing 1-3 heteroatoms selected from N, O and S, wherein the heterocyclic ring is optionally further substituted with 1-3 groups selected from halogen, =O, OH, $NH_2$, CN, $C_{1-4}$ alkyl, and acetyl;

each p is independently selected from 0, 1, 2, 3 and 4.

**2.** The compound as shown in formula (I), or the stereoisomer, the deuterated substance, or the pharmaceutically acceptable salt thereof according to claim 1, having a structure of formula (I-1), **characterised in that**

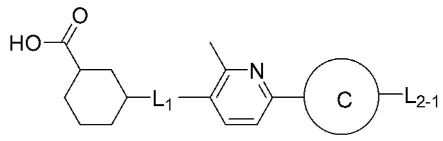

(I-1)

$L_2$-$_1$ is selected from -($CR^{L21}R^{L22}$)$_p$-OC(O)-N($R^{L23}$)$_2$, - ($CR^{L21}R^{L22}$)$_p$$N^{L24}$)S(O)$_2$N($R^{L25}$)$_2$, -($CR^{L21}R^{L22}$)$_p$$N^{L26}$) C(O)N($R^{L27}$)($R^{L28}$),-($CR^{L21}R^{L22}$)$_p$N($R^{L21}$)($R^{L30}$), -($CR^{L21}R^{L22}$)$_p$-C(O)-N($R^{L29}$)-C(O)$R^{L21}$, -($CR^{L21}R^{L22}$)$_p$-C(O)-N($R^{L29}$)-S(O)$_2$$R^{L21}$, -(3- to 6-membered cycloalkyl)-C(=O)N($R^{L25}$)$_2$, -(4- to 7-membered heterocyclyl)-C(=O)N($R^{L25}$)2, -(C2-4 alkenyl)-(4- to 7-membered heterocyclyl)-N($R^{L25}$)2, – ($CR^{L21}R^{L22}$)$_p$-SC(O)-N($R^{L25}$)$_2$, - ($CR^{L21}R^{L22}$)$_p$N($R^{L26}$)C(S)N($R^{L27}$)($R^{L28}$),

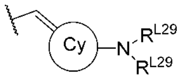 ,

-($CR^{L21}R^{L22}$)$_p$-OC(O)-$R^{L23}$, -($CR^{L21}R^{L22}$)p-O-$R^{L23}$, and -($CR^{L21}R^{L22}$)p-N($R^{L26}$)C(O)O-$R^{L23}$, wherein Cy is 4- to 7-membered heterocyclyl; the cycloalkyl and heterocyclyl are optionally further substituted with 1-4 groups selected from halogen, =O, OH, $NH_2$, CN, $C_1$-$_4$ alkyl, $C_1$-$_4$ alkoxy, halogenated $C_1$-$_4$ alkyl, $C_3$-$_6$ cycloalkyl, and 5- to 7-membered heterocyclyl;

$R^{L21}$ and $R^{L22}$ are each independently selected from H, $C_1$-$_4$ alkyl, $C_2$-$_6$ alkenyl, $C_2$-$_6$ alkynyl, $C_3$-$_{10}$ cycloalkyl, and 5- to 12-membered heterocyclyl, wherein the heterocyclyl contains 1-3 heteroatoms selected from N, O and S; the alkyl, alkenyl, alkynyl, cycloalkyl, and heterocyclyl are optionally further substituted with 1-4 groups selected from halogen, =O, OH, $NH_2$, CN, and $C_1$-$_4$ alkyl;

each $R^{L23}$ is independently selected from $C_1$-$_4$ alkyl, -($CH_2$)$_p$-O-$C_3$-$_6$ cycloalkyl, $C_3$-$_6$ cycloalkyl, -O-$C_3$-$_6$ cycloalkyl, -($CH_2$)$_p$-(4- to 7-membered heterocycloalkyl), 6- to 12-membered bicyclic heterocycloalkyl,

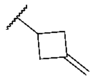, 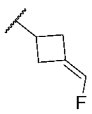, 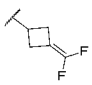, 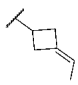,

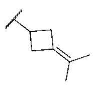, 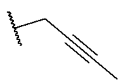, 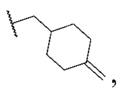, 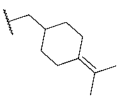, 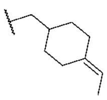, 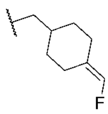, 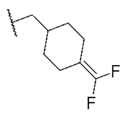, 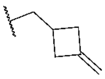,

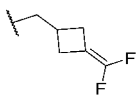, 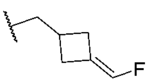, 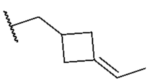, 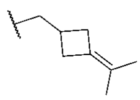, 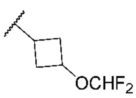, 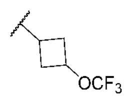,

and 5-to 6-membered heteroaryl, wherein the heterocycloalkyl and heteroaryl contain 1-3 heteroatoms selected from N, O, S, S(O) and S(O)$_2$; the alkyl, heterocycloalkyl and heteroaryl are optionally further substituted with 1-4 groups selected from =O, halogen, OH, -$OCF_3$, -$OCHF_2$, -$SCF_3$, $NH_2$, CN, acetyl, -S(O)$_2$$CH_3$, and

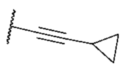;

and when $R^{L21}$ and $R^{L22}$ are both H, and $L_1$ is O, at least one $R^{L23}$ is selected from -($CH_2$)$_p$-(4- to 7-membered heterocycloalkyl), 6- to 12-membered bicyclic heterocycloalkyl, 5- to 6-membered heteroaryl,

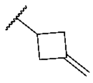, 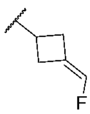, 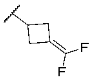, 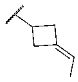,

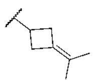, 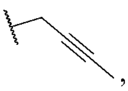, 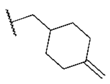, 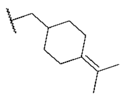, 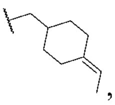, 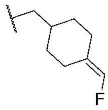, 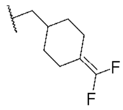, 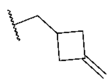,

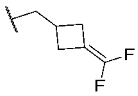, 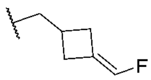, 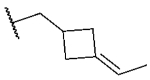, 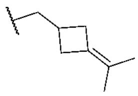, 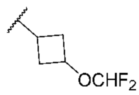, 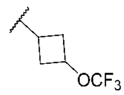, $C_{1-4}$ alkyl substituted with 1-4 groups selected from $-OCF_3$, $-OCHF_2$, $-SCF_3$, $NH_2$, CN, acetyl, $-S(O)_2CH_3$, and

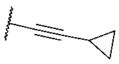,

$-O-C_{3-6}$ cycloalkyl, $-(CH_2)_p-O-C_{3-6}$ cycloalkyl, and $C_{3-6}$ cycloalkyl substituted with 1-4 groups selected from $-OCF_3$, $-OCHF_2$, - $SCF_3$, $NH_2$, CN, acetyl, $-S(O)_2CH_3$, and

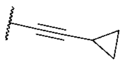,

wherein the heterocycloalkyl and heteroaryl are optionally further substituted with 1-4 groups selected from =O, halogen, OH, $-OCF_3$, $-OCHF_2$, $-SCF_3$, $NH_2$, CN, acetyl, $-S(O)_2CH_3$, and

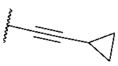;

each $R^{L24}$ is independently selected from H, $C_{2-6}$ alkenyl, and $C_{2-6}$ alkynyl, wherein the alkenyl and alkynyl are optionally further substituted with 1-4 groups selected from halogen, OH, $NH_2$, CN, $C_{3-10}$ cycloalkyl, and 5- to 12-membered heterocyclyl;

$R^{L25}$, $R^{L27}$, and $R^{L28}$ are each independently selected from $C_{1-3}$ alkyl, $C_{2-6}$ alkenyl, and $C_{2-6}$ alkynyl, wherein the alkyl, alkenyl, and alkynyl are optionally further substituted with 1-4 groups selected from halogen, OH, $NH_2$, and CN;

each $R^{L26}$ is independently selected from $C_{2-6}$ alkenyl, $C_{2-6}$ alkynyl, and $C_{3-6}$ cycloalkyl, wherein the alkenyl, alkynyl, and cycloalkyl are optionally further substituted with 1-4 groups selected from halogen, =O, OH, $NH_2$, CN, and $C_{14}$ alkyl;

$R^{L29}$ is selected from H, $C_{1-4}$ alkyl, $C_{2-6}$ alkenyl, and $C_{2-6}$ alkynyl, wherein the alkyl, alkenyl, and alkynyl are optionally further substituted with 1-4 groups selected from halogen, OH, $NH_2$, and CN;

$R^{L30}$ is selected from 6- to 14-membered bicyclic or tricyclic heterocyclyl, wherein the heterocyclyl contains 1-3 heteroatoms selected from N, O and S; the heterocyclyl is optionally further substituted with 1-4 groups selected from halogen, CN, OH, $NH_2$, $C_{1-4}$ alkyl, and halogenated $C_{1-4}$ alkyl;

p is selected from 0, 1, 2, 3 and 4;

alternatively, $R^{L24}$ and $R^{L25}$ or $R^{L26}$ and $R^{L27}$, together with the atoms to which they are attached, form a 5- to 7-membered heterocyclic ring containing 1-3 heteroatoms selected from N, O and S, wherein the heterocyclic ring is optionally further substituted with 1-3 groups selected from halogen, =O, OH, $NH_2$, CN, $C_{1-4}$ alkyl, and acetyl;

alternatively, two $R^{L23}$, together with the atoms to which they are attached, form a 5- to 7-membered heterocyclic ring containing 1-3 heteroatoms selected from N, O and S, wherein the heterocyclic ring is optionally further substituted with 1-3 groups selected from =O, OH, $NH_2$, CN, and acetyl;

provided that (1). $R^{L30}$ is not

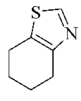, 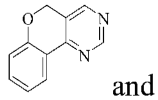 and 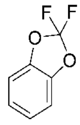;

(2). the compound is not

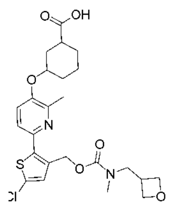, 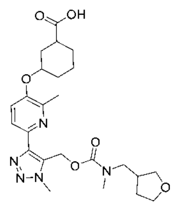, 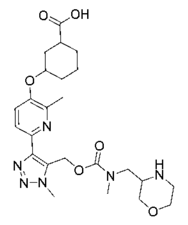

and

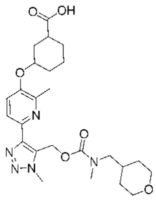.

**3.** The compound as shown in formula (I-1), or the stereoisomer, the deuterated substance, or the pharmaceutically acceptable salt thereof according to claim 2, **characterised in that**

$L_2$-$_1$ is selected from

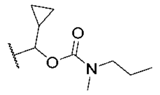, 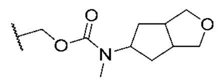, 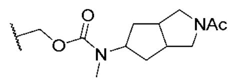,

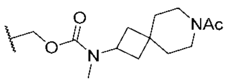, 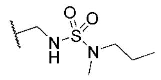, 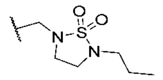, 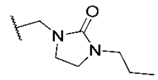, 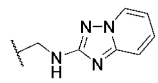,

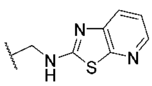, 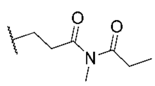, 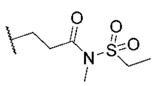, 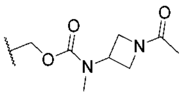, 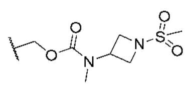,

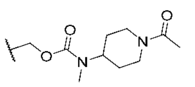, 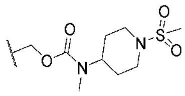, 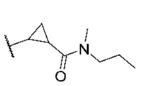, 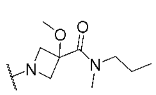, 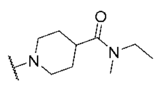,

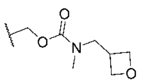, 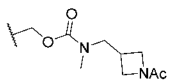, 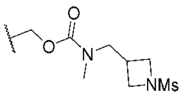, 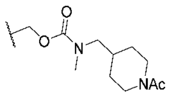, 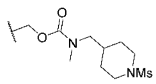,

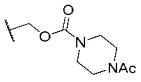, 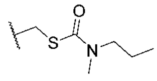, 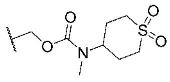, 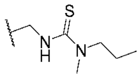, 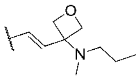, 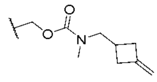,

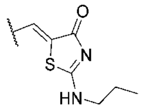, 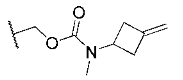, 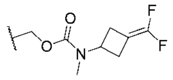, 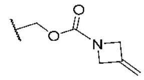, 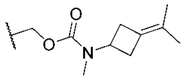,

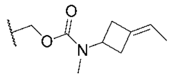, 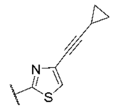, 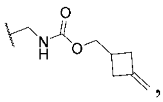, 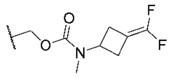, 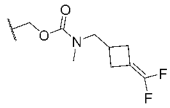, 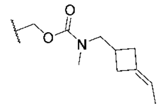,

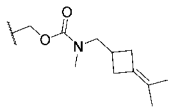, 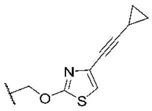, 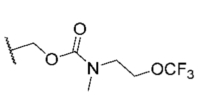, 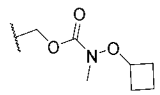, 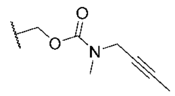,

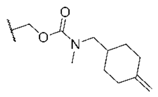, 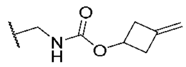, 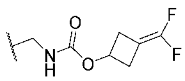, 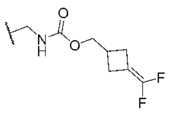, 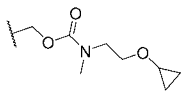,

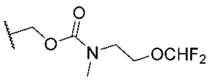, 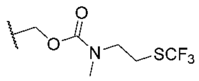, 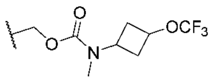, 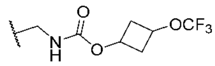,

and

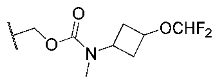;

$L_1$ is selected from a bond, $-OCH_2-$, -S-, $-OCH(CH_3)-$, $-OCH(CF_3)-$,

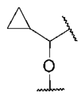,

O-, -C(=O)-, -NHC(O)-, $-CH_2-$, -NH-, $-N(CH_3)-$, and $-NHCH(CH_3)-$.

**4.** The compound as shown in formula (I), or the stereoisomer, the deuterated substance, or the pharmaceutically acceptable salt thereof according to claim 1, **characterised by** having a structure of formula (I-2), (I-2a), (I-2b), or (I-2c), wherein

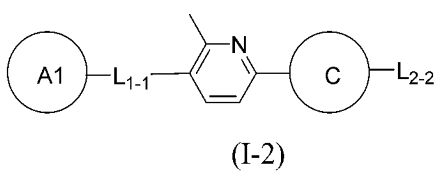

(I-2)

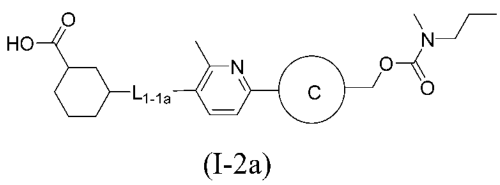

(I-2a)

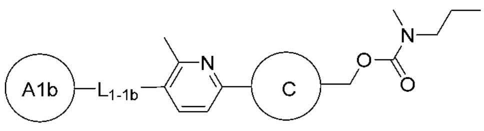

(I-2b)

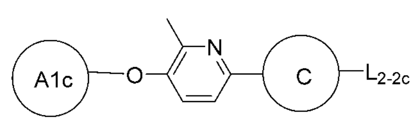

(I-2c)

rings A1, A1b, and A1c are each independently selected from 3- to 12-membered carbocyclyl and 4- to 12-membered heterocyclyl, or are absent, wherein the heterocyclyl contains 1-3 heteroatoms selected from N, O and S; the carbocyclyl and heterocyclyl are optionally further substituted with 1-4 $R^A$;

each $R^A$ is independently selected from H, =O, halogen, CN, OH, $C_1$-$_6$ alkyl, $C_2$-$_6$ alkenyl, $C_2$-$_6$ alkynyl, -$OC_1$-$_4$ alkyl, -$(CH_2)_p$-5- to 10-membered heterocyclyl, - (a 3- to 6-membered carbocyclic ring)-$COOR^{a1}$, -$(CH_2)_p$-$COOR^{a1}$, -$(CH_2)_p$-C(O)$NR^{a1}R^{a2}$, -$(CH_2)_p$-C(O)NHC(O)$R^{a1}$, -$(CH_2)_p$-C(O)NHS$(O)_2R^{a1}$, -$(CH_2)_p$-C(O) NHS(O)$R^{a1}$, -$(CH_2)_p$-S$(O)_2$OH, -$(CH_2)_p$-S$(O)_2$NHC(O)$R^{a1}$, -$(CH_2)_p$-P(O)$(OH)_2$, =$CH_2$, =$CF_2$, =CH-$CH_3$ or =C-$(CH_3)_2$, wherein the heterocyclyl contains 1-3 heteroatoms selected from N, O and S; the alkyl, alkenyl, alkynyl and heterocyclyl are optionally further substituted with 1-4 groups selected from halogen, OH, $NH_2$, CN, and -O-halogenated $C_1$-$_4$ alkyl; and $L_1$-$_1$-A1, $L_1$-$_{1b}$-Alb, and O-A 1 c are not

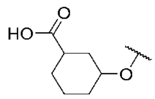;

$L_1$-$_1$ and $L_1$-$_{1b}$ are each independently selected from a bond, $C_1$-$_6$ alkyl, $C_2$-$_6$ alkenyl, $C_2$-$_6$ alkynyl, -O-, -O-$C_1$-$_6$ alkyl-, -S-, -S-$C_1$-$_6$ alkyl-, -C(O)$NR^{L1}$-, - $NR^{L1}$C(O)-,

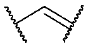,

-$NR^{L1}$-, and -$NR^{L1}$-$C_{1-6}$ alkyl-, wherein the alkyl, alkenyl, and alkynyl are optionally further substituted with 1-4 $R^{L1}$;

$L_1$-$_{1a}$ is selected from a bond, $C_1$-$_4$ alkyl, $C_2$-$_4$ alkenyl, $C_2$-$_4$ alkynyl, -O-$C_1$-$_4$ alkyl-, -S-, -S-$C_1$-$_4$ alkyl-, -C(O)$NR^{L1}$-, -$NR^{L1}$C(O)-,

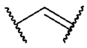,

-$NR^{L1}$-, and -$NR^{L1}$-$C_1$-$_6$ alkyl-, wherein the alkyl, alkenyl, and alkynyl are optionally further substituted with 1-4 $R^{L1}$;

each $R^{L1}$ is independently selected from H, halogen, OH, $C_1$-$_4$ alkyl, halogenated $C_1$-$_4$ alkyl, 3- to 6-membered cycloalkyl, and -COOH, wherein the alkyl and cycloalkyl are optionally further substituted with 1-4 groups selected from halogen, OH, and $NH_2$;

when rings A1 and A1b are each independently absent, $L_1$-$_1$ and $L_1$-$_{1b}$ are each independently selected from -$OC_1$-$_6$ alkyl substituted with one COOH, $C_2$-$_6$ alkenyl, and $C_2$-$_6$ alkynyl;

$L_2$-$_2$ and $L_2$-$_{2c}$ are each independently selected from -$(CR^{L21}R^{L22})$p-OC(O)-N$(R^{L23})_2$, -$(CR^{L21}R^{L22})_p$N$(R^{L24})$ S$(O)_2$N$(R^{L25})_2$,-$(CR^{L21}R^{L22})_p$N$(R^{L26})$C(O)N$(R^{L27})(R^{L28})$, -$(CR^{L21}R^{L22})_p$N$(R^{L29})(R^{L30})$,-$(CRL^{21}R^{L22})_p$-O-C(O)-$R^{L23}$, -$(CR^{L21}R^{L22})_p$ and -$(CR^{L21}R^{L22})_p$-N$(R^{L26})$C(O)O-$R^{L23}$;

$R^{a1}$, $R^{a2}$, $R^{L1}$, $R^{L2}$, $R^{L21}$, $R^{L22}$, $R^{L23}$, $R^{L24}$, $R^{L25}$, $R^{L26}$, $R^{L27}$, and $R^{L28}$ are each independently selected from H, halogen, $C_1$-$_4$ alkyl, $C_2$-$_6$ alkenyl, $C_2$-$_6$ alkynyl, $C_1$-$_4$ alkoxy, 5- to 10-membered heterocycloalkyl, -$(CH_2)_p$-(4- to 7-membered heterocyclyl), -$(CH_2)_p$-$C_3$-$_{10}$ cycloalkyl, and -$(CH_2)_p$-$OC_3$-$_{10}$ cycloalkyl, wherein the heterocyclyl contains 1-3 heteroatoms selected from N, O and S; the alkyl, alkenyl, alkynyl, cycloalkyl, and heterocycloalkyl are optionally further substituted with 1-4 groups selected from halogen, =O, =$CH_2$, =$CF_2$, =CH-$CH_3$, =C-$(CH_3)_2$, OH, $NH_2$, CN, acetyl, -S$(O)_2CH_3$, $C_1$-$_4$ alkyl, $C_1$-$_4$ alkoxy, halogenated $C_1$-$_4$ alkyl, halogenated $C_{14}$ alkoxy, -$SCF_3$, $C_3$-$_{10}$ cycloalkyl, 5- to 12-membered heterocyclyl, and -C=C-$C_3$-$_{10}$ cycloalkyl;

$R^{L29}$ is selected from H, $C_1$-$_4$ alkyl, $C_2$-$_6$ alkenyl, and $C_2$-$_6$ alkynyl, wherein the alkyl, alkenyl, and alkynyl are optionally further substituted with 1-4 groups selected from halogen, OH, $NH_2$, and CN;

$R^{L30}$ is selected from 6- to 14-membered bicyclic or tricyclic heterocyclyl, wherein the heterocyclyl contains 1-3 heteroatoms selected from N, O and S; the heterocyclyl is optionally further substituted with 1-4 groups selected from halogen, CN, OH, $NH_2$, $C_1$-$_4$ alkyl, and halogenated $C_1$-$_4$ alkyl;

alternatively, $R^{L24}$ and $R^{L25}$ or $R^{L26}$ and $R^{L27}$, together with the atoms to which they are attached, form a 5- to 7-membered heterocyclic ring containing 1-3 heteroatoms selected from N, O and S, wherein the heterocyclic ring is optionally further substituted with 1-3 groups selected from halogen, =O, OH, $NH_2$, CN, and $C_1$-$_4$ alkyl;

each p is independently selected from 0, 1, 2, 3 and 4;

provided that

(1). for formula (I-2), when $L_2$-$_2$ is

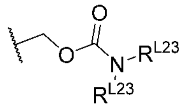,

$L_1$-$_1$-A1 is not selected from

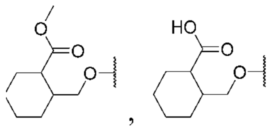, , 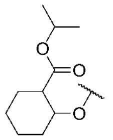, 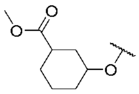, 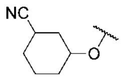, 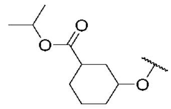,

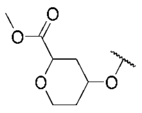, 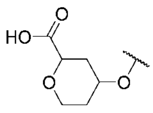, 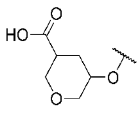, 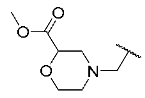, 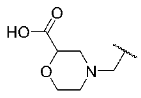, 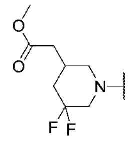,

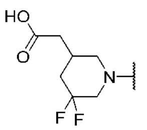, 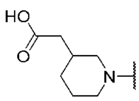, 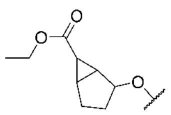, 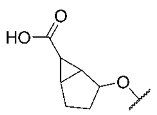, 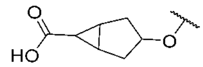,

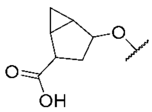, 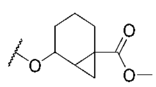, 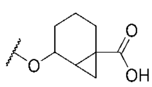, 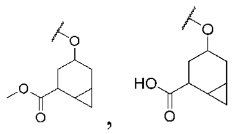, , 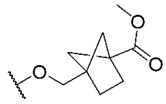,

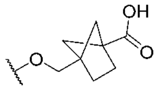, 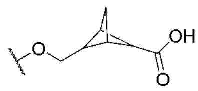, 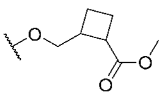, 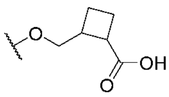, 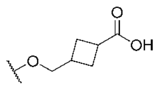,

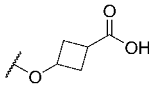, 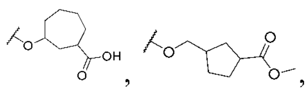, , 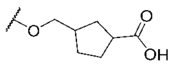, 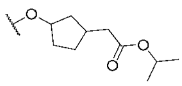, 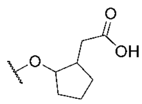,

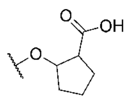, 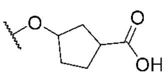, 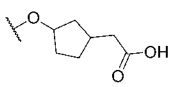, 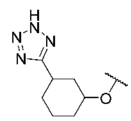, 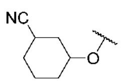, 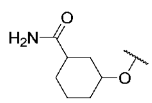

and

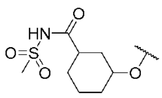;

(2). for formula (I-2), when $L_2$-$_2$ is

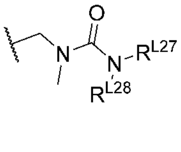,

$L_{1-1}$-A1 is not

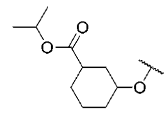;

(3). for formula (I-2b), $L_{1}$-$_{1b}$-Alb is not selected from

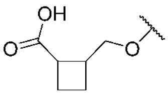,

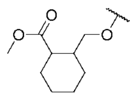, 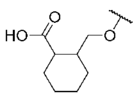, 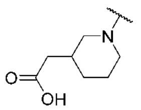, 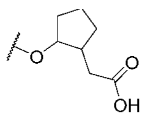, 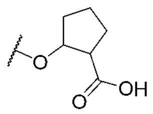, 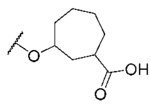,

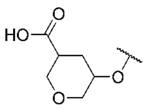, 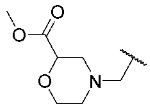, 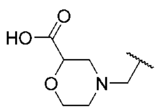, 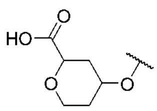, 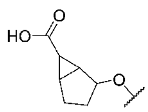, 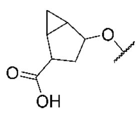,

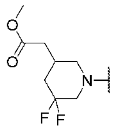, 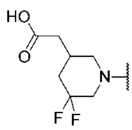, 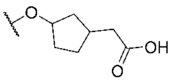, 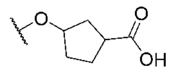, and 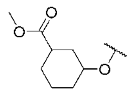;

(4). for formula (I-2c), O-A1c is not selected from

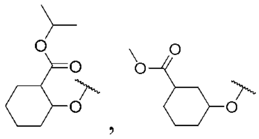,

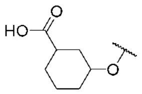, 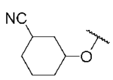, 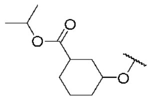, 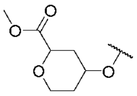, 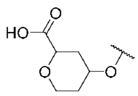, 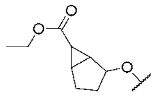,

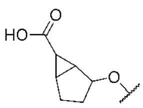, 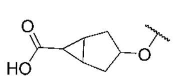, 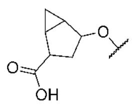, 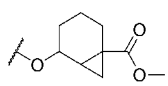, 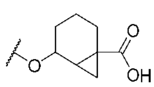, 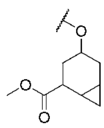,

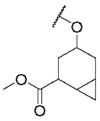, 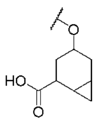, 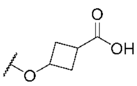, 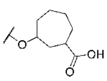, 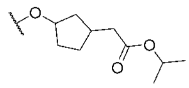, 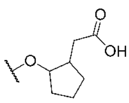, 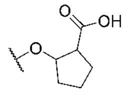,

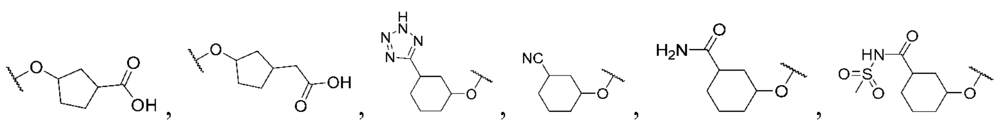,

and

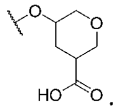.

**5.** The compound as shown in formula (I-2), (I-2b) or (I-2c), or the stereoisomer, the deuterated substance, or the pharmaceutically acceptable salt thereof according to claim 4, **characterised in that**

rings A1, A1b, and A1c are selected from 3- to 8-membered monocyclic cycloalkyl, 6- to 12-membered fused cycloalkyl, 4- to 8-membered monocyclic heterocycloalkyl, 6- to 12-membered fused heterocycloalkyl, 6- to 10-membered spiroheterocyclyl, and 6- to 10-membered bridged heterocyclyl, or are absent, wherein the heterocycloalkyl, fused heterocycloalkyl, spiroheterocyclyl, and bridged heterocyclyl contain 1-3 heteroatoms selected from N, O and S; the cycloalkyl, heterocycloalkyl, fused heterocycloalkyl, spiroheterocyclyl, and bridged heterocyclyl are optionally further substituted with 1-4 $R^A$;

each $R^A$ is independently selected from H, halogen, =O, CN, OH, $C_{14}$ alkyl, $C_{2\text{-}4}$ alkenyl, $C_{2\text{-}4}$ alkynyl, $-OC_{1\text{-}2}$ alkyl, $-(CH_2)_p$-(5- to 8-membered heterocyclyl), - (a 3- to 6-membered carbocyclic ring)-$COOR^{a1}$, $-(CH_2)_p-COOR^{a1}$, $=CH_2$, $=CF_2$, $=CH-CH_3$ or $=C-(CH_3)_2$, wherein the heterocyclyl contains 1-3 heteroatoms selected from N, O and S; the alkyl, alkenyl, alkynyl, and heterocyclyl are optionally further substituted with 1-4 groups selected from halogen, OH, $NH_2$, CN, and -O-halogenated $C_{1\text{-}2}$ alkyl;

$L_{1\text{-}1}$ and $L_{1\text{-}1b}$ are each independently selected from a bond, $C_{1\text{-}4}$ alkyl, $C_{2\text{-}4}$ alkenyl, $C_{2\text{-}4}$ alkynyl, -O-, $-O-C_{1\text{-}4}$ alkyl-, -S-, $-S-C_{1\text{-}4}$ alkyl-, $-C(O)NR^{L1}-$, and - $NR^{L1}C(O)-$, wherein the alkyl, alkenyl, and alkynyl are optionally further substituted with 1-4 $R^{L1}$;

each $R^{L1}$ is independently selected from H, halogen, OH, $C_{1\text{-}2}$ alkyl, halogenated $C_{1\text{-}2}$ alkyl, 3- to 6-membered cycloalkyl, and -COOH, wherein the alkyl and cycloalkyl are optionally further substituted with 1-4 groups selected from halogen, OH, and $NH_2$.

**6.** The compound as shown in formula (I-2), (I-2b) or (I-2c), or the stereoisomer, the deuterated substance, or the pharmaceutically acceptable salt thereof according to claim 5, **characterised in that**

rings A1, A1b, and A1c are each independently selected from one of the groups formed by the following structures optionally substituted with 1-4 $R^A$:

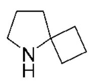, 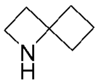, 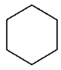, 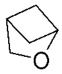, 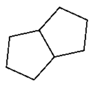, 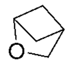, 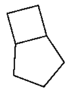, 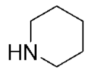, 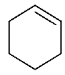, 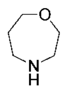, 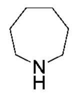,

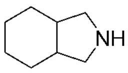, 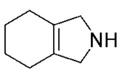, 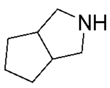, 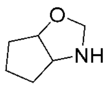, 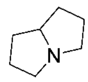, 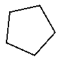, 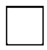, 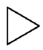, 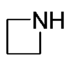, 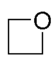,

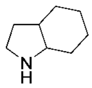, 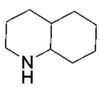, 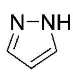, 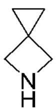, 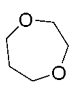, 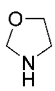, 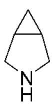, 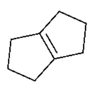, and 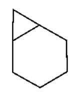;

each $R^A$ is independently selected from H, $C_{1\text{-}2}$ alkyl, halogen, CN, OH, - COOH, $-CH_2-COOH$, $-CH_2CH_2-COOH$, $=CH_2$, $=CF_2$, $=CH-CH_3$ or $=C-(CH_3)_2$;

$L_{1\text{-}1}$ and $L_{1\text{-}1b}$ are each independently selected from a bond, $C_{1\text{-}2}$ alkyl, $-O-C_{1\text{-}2}$ alkyl-, -S-, -O-,

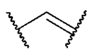,

vinyl, propenyl, ethynyl, -C(O)NH-, and -NHC(O)-, wherein the alkyl is optionally further substituted with 1-4 $R^{L1}$;

each $R^{L1}$ is independently selected from H, halogen, OH, $C_{1\text{-}2}$ alkyl, and 3- to 6-membered cycloalkyl;

$L_{2\text{-}2}$ and $L_{2\text{-}2c}$ are each independently selected from

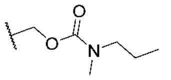 ,

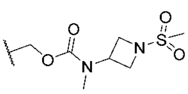 , 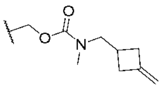 , 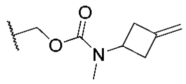 , 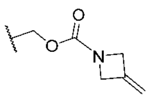 , 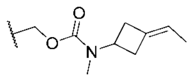 ,

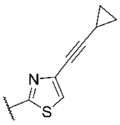 , 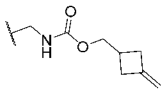 , 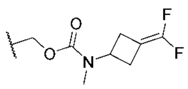 , 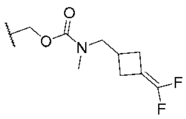 , 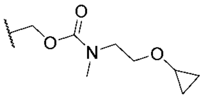 ,

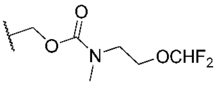 , 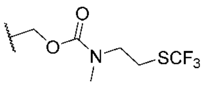 , 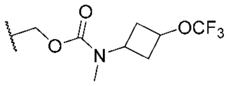 , 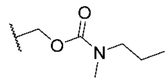 ,

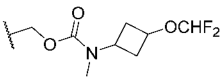 , 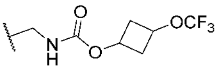 , and 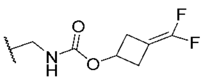 .

7. The compound as shown in formula (I-2), (I-2a), (I-2b) or (I-2c), or the stereoisomer, the deuterated substance, or the pharmaceutically acceptable salt thereof according to claim 6, **characterised in that**

rings A1, A1b, and A1c are each independently selected from the following structures:

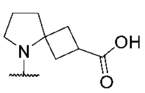 , 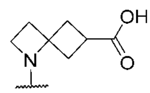 , 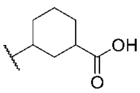 , 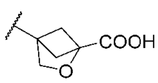 , 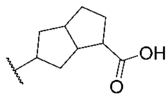 ,

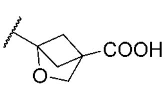 , 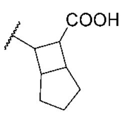 , 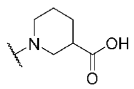 , 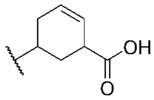 , 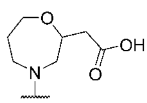 , 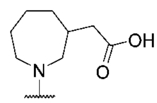 ,

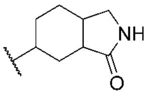 , 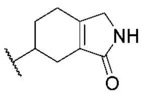 , 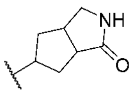 , 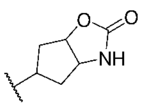 , 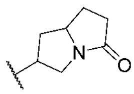 , 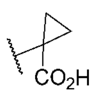 , 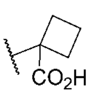 ,

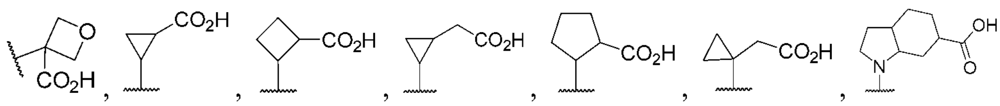 , , , , , , ,

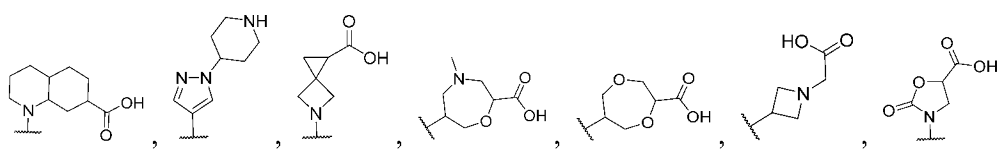 , , , , , , ,

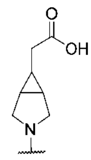 , 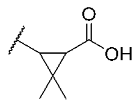 , 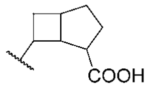 , 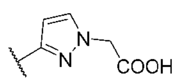 , 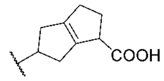 , 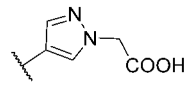 ,

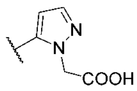 , 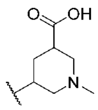 , 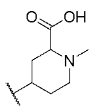 , 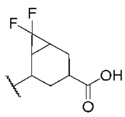 , 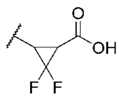 , 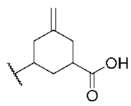 , 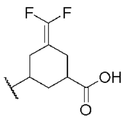 ,

and

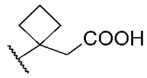 ;

$L_{1}$-$_{1}$ and $L_{1}$-$_{1b}$ are each independently selected from a bond, $-OCH_2-$, - $OCH_2CH_2-$, -S-, $-OCH(CH_3)-$, $-OCH(CF_3)-$,

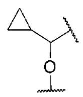 ,

-O-, -C(=O)-, $-CH_2-$, - $CH=CH-CH_2-$, ethynyl, and -NHCO-;
each $L_{1}$-$_{1a}$ is independently selected from a bond, $-OCH_2-$, -S-, $-OCH(CH_3)-$, - $OCH(CF_3)-$,

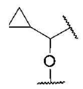 ,

-C(=O)-, $-CH_2-$, $-CH=CH-CH_2-$, ethynyl, -NHCO-, -NH-, - $N(CH_3)-$, and $-NHCH(CH_3)-$.

**8.** The compound as shown in formula (I), or the stereoisomer, the deuterated substance, or the pharmaceutically acceptable salt thereof according to claim 1, **characterised by** having a structure of formula (I-6), wherein

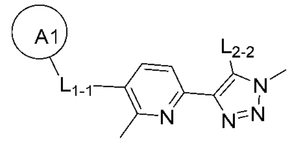

(**I**-6)

$L_{2}$-$_{2}$ is selected from $-(CR^{L21}R^{L22})_p-OC(O)-N(CH_3)R^{L23}$, $-(CR^{L21}R^{L22})_p-OC(O)-NHR^{L23}$, and $-(CR^{L21}R^{L22})_p-N(R^{L26})C(O)O-R^{L23}$;
$R^{L21}$, $R^{L22}$, and $R^{L26}$ are each independently selected from H and $C_{1}$-$_{4}$ alkyl;
$R^{L23}$ is selected from $-(CH_2)_p$-(4- to 7-membered heterocyclyl) and $-(CH_2)_p-C_3$-$_{10}$ cycloalkyl, wherein the heterocyclyl contains 1-3 heteroatoms selected from N, O and S; the cycloalkyl and heterocyclyl are further substituted with 1-4 groups selected from $=CH_2$, $=CF_2$, $=CH-CH_3$, and $=C-(CH_3)_2$;
ring A1 is selected from 3- to 12-membered carbocyclyl and 4- to 12-membered heterocyclyl, or is absent, wherein the heterocyclyl contains 1-3 heteroatoms selected from N, O and S; the carbocyclyl and heterocyclyl are optionally further substituted with 1-4 $R^A$;
when ring A1 is absent, $L_{1}$-$_{1}$ is $-OC_{1}$-$_{6}$ alkyl substituted with one COOH;
$L_{1}$-$_{1}$ is selected from a bond, $C_{1}$-$_{6}$ alkyl, $C_{2}$-$_{6}$ alkenyl, $C_{2}$-$_{6}$ alkynyl, -O-, $-O-C_{1}$-$_{6}$ alkyl-, -S-, $-S-C_{1}$-$_{6}$ alkyl-, -C(O)$NR^{L1}$-,

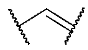,

-$NR^{L1}$-, and -$NR^{L1}$-$C_{1-6}$ alkyl-, wherein the alkyl, alkenyl, and alkynyl are optionally further substituted with 1-4 $R^{L1}$;
$R^A$, $R^{L1}$, and p are as defined in claim 1.

**9.** The compound, or the stereoisomer, the deuterated substance, or the pharmaceutically acceptable salt thereof according to claim 1, **characterised in that** the compound is selected from one of the structures in Table 1 and Table 2.

**10.** A pharmaceutical composition or pharmaceutical preparation, **characterised by** comprising the compound, or the stereoisomer, the deuterated substance, or the pharmaceutically acceptable salt thereof according to any one of claims 1-9, and a pharmaceutically acceptable carrier and/or excipient.

**11.** The pharmaceutical composition or pharmaceutical preparation according to claim 10, **characterised by** comprising 1-1500 mg of the compound, or the stereoisomer, the deuterated substance, or the pharmaceutically acceptable salt thereof according to any one of claims 1-9, and a carrier and/or excipient.

**12.** Use of the compound, or the stereoisomer, the deuterated substance, or the pharmaceutically acceptable salt thereof according to any one of claims 1-9, or the composition according to claim 10 or 11 in the preparation of a medicament for treating/preventing an LPAR1-mediated disease.

**13.** The use according to claim 12, **characterised in that** the LPAR1-mediated disease is selected from idiopathic pulmonary fibrosis, progressive pulmonary fibrosis, systemic sclerosis, benign prostatic hyperplasia, multiple sclerosis, nerve injury, and neuralgia.

**14.** A method for treating a disease in a mammal, **characterised by** comprising administering to a subject a therapeutically effective amount of the compound, or the stereoisomer, the deuterated substance, or the pharmaceutically acceptable salt thereof according to any one of claims 1-11, or the composition according to claim 10 or 11, wherein the therapeutically effective amount is preferably 1-1500 mg, and the disease is selected from idiopathic pulmonary fibrosis, progressive pulmonary fibrosis, systemic sclerosis, benign prostatic hyperplasia, multiple sclerosis, nerve injury, and neuralgia.

# INTERNATIONAL SEARCH REPORT

International application No.
**PCT/CN2024/117612**

## A. CLASSIFICATION OF SUBJECT MATTER

C07D401/14(2006.01)i；C07D401/04(2006.01)i；C07D413/14(2006.01)i；C07D405/14(2006.01)i；A61K31/4192(2006.01)i；A61K31/4439(2006.01)i；A61P11/06(2006.01)i；A61P35/00(2006.01)i

According to International Patent Classification (IPC) or to both national classification and IPC

## B. FIELDS SEARCHED

Minimum documentation searched (classification system followed by classification symbols)

IPC:C07D,A61K,A61P

Documentation searched other than minimum documentation to the extent that such documents are included in the fields searched

Electronic data base consulted during the international search (name of data base and, where practicable, search terms used)

CNTXT, ENTXT, DWPI, CNKI, STN (registry, caplus): 三唑, 吡啶, 环己烷, 氨甲酰基, 甲酸, Triazole, pyridine, cyclohexane, carbamoyl, formic w acid, LPA, 结构式检索, structural formula search

## C. DOCUMENTS CONSIDERED TO BE RELEVANT

| Category* | Citation of document, with indication, where appropriate, of the relevant passages | Relevant to claim No. |
|---|---|---|
| X | CN 109963843 A (BRISTOL MYERS SQUIBB COMPANY) 02 July 2019 (2019-07-02)<br>claims 1-14 and 28-30 | 2-14 |
| X | CN 113473985 A (BRISTOL MYERS SQUIBB COMPANY) 01 October 2021 (2021-10-01)<br>claims 1 and 12-17, and description, embodiments 4 and 5 | 2-14 |
| X | CN 114599648 A (BRISTOL MYERS SQUIBB COMPANY) 07 June 2022 (2022-06-07)<br>claims 1 and 12-16, and description, embodiments 1-2 | 2-14 |
| X | CN 112189010 A (BRISTOL MYERS SQUIBB COMPANY) 05 January 2021 (2021-01-05)<br>claims 1 and 24-29, and description, paragraph 109 | 2-14 |
| X | WO 2022232459 A1 (VIVA STAR BIOSCIENCES (SUZHOU) CO., LTD.) 03 November 2022 (2022-11-03)<br>claim 1, and description, embodiment 30 | 2-14 |
| A | CN 113260310 A (BRISTOL MYERS SQUIBB COMPANY) 13 August 2021 (2021-08-13)<br>claims 1-14 | 2-14 |
| A | US 2021171500 A1 (GILEAD SCIENCES, INC.) 10 June 2021 (2021-06-10)<br>claims 1-81 | 2-14 |

☐ Further documents are listed in the continuation of Box C.　☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **27 November 2024** | **03 December 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | <br>Telephone No. |

Form PCT/ISA/210 (second sheet) (July 2022)

**INTERNATIONAL SEARCH REPORT**

International application No.
**PCT/CN2024/117612**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **14**
because they relate to subject matter not required to be searched by this Authority, namely:

Claim 14 relates to a method for treating a disease. However, the present search is conducted on the basis of the use of the compound in the preparation of a drug.

2. ☑ Claims Nos.: **1**
because they relate to parts of the international application that do not comply with the prescribed requirements to such an extent that no meaningful international search can be carried out, specifically:

Claim 1 defines an overly broad range for substituents, such that a large number of compounds greatly differing in structure are comprised. According to the relevant provisions about support of PCT Article 6, no comprehensive search can be performed with regard to the scope of claim 1. This search is merely limited to claims 2-14.

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/117612**

| Patent document cited in search report | Publication date (day/month/year) | Patent family member(s) | Publication date (day/month/year) |
|---|---|---|---|
| CN 109963843 A | 02 July 2019 | LT 3666771 T | 10 December 2021 |
| | | IL 263767 A | 31 January 2019 |
| | | IL 263767 B | 31 August 2021 |
| | | HRP 20211708 T1 | 04 February 2022 |
| | | HRP 20211708 T8 | 04 March 2022 |
| | | LT 3472148 T | 25 May 2020 |
| | | PT 3666771 T | 27 October 2021 |
| | | UY 37302 A | 02 January 2018 |
| | | MY 195782 A | 13 February 2023 |
| | | CY 1123443 T1 | 31 December 2021 |
| | | RS 60347 B1 | 31 July 2020 |
| | | EA 201892710 A1 | 31 May 2019 |
| | | EA 037585 B1 | 19 April 2021 |
| | | JP 2019518766 A | 04 July 2019 |
| | | JP 7073281 B2 | 23 May 2022 |
| | | US 2017360759 A1 | 21 December 2017 |
| | | US 10071078 B2 | 11 September 2018 |
| | | RS 62524 B1 | 30 November 2021 |
| | | SI 3666771 T1 | 31 December 2021 |
| | | US 2021244711 A1 | 12 August 2021 |
| | | MX 2018015563 A | 06 June 2019 |
| | | HUE 049944 T2 | 30 November 2020 |
| | | PE 20190211 A1 | 07 February 2019 |
| | | NZ 750013 A | 25 March 2022 |
| | | SI 3472148 T1 | 31 July 2020 |
| | | BR 112018076558 A2 | 02 April 2019 |
| | | US 2018333395 A1 | 22 November 2018 |
| | | US 10576062 B2 | 03 March 2020 |
| | | TW 201808919 A | 16 March 2018 |
| | | TWI 725200 B | 21 April 2021 |
| | | ES 2785951 T3 | 08 October 2020 |
| | | CA 3029202 A1 | 28 December 2017 |
| | | CA 3029202 C | 01 March 2022 |
| | | DK 3666771 T3 | 15 November 2021 |
| | | EP 3472148 A1 | 24 April 2019 |
| | | EP 3472148 B1 | 11 March 2020 |
| | | CY 1124737 T1 | 22 July 2022 |
| | | PL 3472148 T3 | 14 December 2020 |
| | | PL 3666771 T3 | 27 December 2021 |
| | | KR 20190020049 A | 27 February 2019 |
| | | KR 102377340 B1 | 21 March 2022 |
| | | AR 108838 A1 | 03 October 2018 |
| | | CL 2018003708 A1 | 08 February 2019 |
| | | US 2022249443 A1 | 11 August 2022 |
| | | DK 3472148 T3 | 27 April 2020 |
| | | EP 3666771 A1 | 17 June 2020 |
| | | EP 3666771 B1 | 29 September 2021 |
| | | AU 2017281439 A1 | 07 February 2019 |
| | | AU 2017281439 B2 | 01 July 2021 |
| | | US 2020138789 A1 | 07 May 2020 |

Form PCT/ISA/210 (patent family annex) (July 2022)

**INTERNATIONAL SEARCH REPORT**
**Information on patent family members**

International application No.
**PCT/CN2024/117612**

| Patent document cited in search report | | | Publication date (day/month/year) | Patent family member(s) | | | Publication date (day/month/year) |
|---|---|---|---|---|---|---|---|
| | | | | US | 11007180 | B2 | 18 May 2021 |
| | | | | ES | 2895385 | T3 | 21 February 2022 |
| | | | | CO | 2019000471 | A2 | 08 February 2019 |
| | | | | JP | 2022106974 | A | 20 July 2022 |
| | | | | JP | 7312295 | B2 | 20 July 2023 |
| | | | | AU | 2021209334 | A1 | 26 August 2021 |
| | | | | AU | 2021209334 | B2 | 01 June 2023 |
| | | | | WO | 2017223016 | A1 | 28 December 2017 |
| | | | | TW | 202126629 | A | 16 July 2021 |
| | | | | TWI | 757128 | B | 01 March 2022 |
| | | | | HUE | 057211 | T2 | 28 April 2022 |
| | | | | ME | 03804 | B | 20 April 2021 |
| | | | | US | 2023390249 | A1 | 07 December 2023 |
| | | | | USRE | 49352 | E | 03 January 2023 |
| | | | | SG | 11201811321 | TA | 30 January 2019 |
| | | | | HRP | 20200586 | T1 | 10 July 2020 |
| | | | | PT | 3472148 | T | 08 May 2020 |
| | | | | ZA | 201808580 | B | 26 August 2020 |
| | | | | KR | 20220038537 | A | 28 March 2022 |
| | | | | KR | 102463621 | B1 | 03 November 2022 |
| CN | 113473985 | A | 01 October 2021 | US | 2022041565 | A1 | 10 February 2022 |
| | | | | JP | 2022500470 | A | 04 January 2022 |
| | | | | JP | 7427658 | B2 | 05 February 2024 |
| | | | | KR | 20210061383 | A | 27 May 2021 |
| | | | | ES | 2946657 | T3 | 24 July 2023 |
| | | | | EP | 3852746 | A1 | 28 July 2021 |
| | | | | EP | 3852746 | B1 | 29 March 2023 |
| | | | | WO | 2020060915 | A1 | 26 March 2020 |
| CN | 114599648 | A | 07 June 2022 | EP | 3986551 | A1 | 27 April 2022 |
| | | | | WO | 2020257135 | A1 | 24 December 2020 |
| | | | | KR | 20220024548 | A | 03 March 2022 |
| | | | | JP | 2022536858 | A | 19 August 2022 |
| | | | | JP | 7465898 | B2 | 11 April 2024 |
| | | | | US | 2022235026 | A1 | 28 July 2022 |
| CN | 112189010 | A | 05 January 2021 | EA | 202091506 | A1 | 22 September 2020 |
| | | | | BR | 112020011776 | A2 | 17 November 2020 |
| | | | | EP | 3710438 | A1 | 23 September 2020 |
| | | | | EP | 3710438 | B1 | 20 October 2021 |
| | | | | HRP | 20211918 | T1 | 18 March 2022 |
| | | | | HRP | 20211918 | T8 | 15 April 2022 |
| | | | | PE | 20210655 | A1 | 31 March 2021 |
| | | | | ES | 2898364 | T3 | 07 March 2022 |
| | | | | MX | 2020006315 | A | 04 August 2022 |
| | | | | AR | 113964 | A1 | 01 July 2020 |
| | | | | SG | 11202005699 | QA | 29 July 2020 |
| | | | | IL | 275348 | A | 30 July 2020 |
| | | | | IL | 275348 | B1 | 01 April 2023 |
| | | | | IL | 275348 | B2 | 01 August 2023 |
| | | | | RS | 62710 | B1 | 31 January 2022 |
| | | | | KR | 20200100721 | A | 26 August 2020 |

Form PCT/ISA/210 (patent family annex) (July 2022)

**INTERNATIONAL SEARCH REPORT**
**Information on patent family members**

International application No.
**PCT/CN2024/117612**

| Patent document cited in search report | Publication date (day/month/year) | Patent family member(s) | Publication date (day/month/year) |
|---|---|---|---|
| | | KR 102702229 B1 | 02 September 2024 |
| | | HUE 057366 T2 | 28 May 2022 |
| | | CL 2020001547 A1 | 25 September 2020 |
| | | SI 3710438 T1 | 31 January 2022 |
| | | US 2020148665 A1 | 14 May 2020 |
| | | US 11008300 B2 | 18 May 2021 |
| | | WO 2019126090 A1 | 27 June 2019 |
| | | US 2022324829 A1 | 13 October 2022 |
| | | US 11697646 B2 | 11 July 2023 |
| | | TW 201927765 A | 16 July 2019 |
| | | TWI 830713 B | 01 February 2024 |
| | | US 2019185446 A1 | 20 June 2019 |
| | | US 10662172 B2 | 26 May 2020 |
| | | US 2023339884 A1 | 26 October 2023 |
| | | PT 3710438 T | 22 November 2021 |
| | | AU 2018392321 A1 | 06 August 2020 |
| | | AU 2018392321 B2 | 10 March 2022 |
| | | JP 2021508686 A | 11 March 2021 |
| | | JP 7274486 B2 | 16 May 2023 |
| | | JP 7274486 B6 | 15 February 2024 |
| | | CY 1124863 T1 | 25 November 2022 |
| | | NZ 766284 A | 28 July 2023 |
| | | PL 3710438 T3 | 31 January 2022 |
| | | DK 3710438 T3 | 06 December 2021 |
| | | LT 3710438 T | 27 December 2021 |
| | | EP 4011875 A1 | 15 June 2022 |
| | | CA 3085586 A1 | 27 June 2019 |
| WO 2022232459 A1 | 03 November 2022 | JP 2024517769 A | 23 April 2024 |
| | | MX 2023012830 A | 19 January 2024 |
| | | AU 2022264579 A1 | 14 December 2023 |
| | | IL 308035 A | 01 December 2023 |
| | | KR 20240044386 A | 04 April 2024 |
| | | CA 3218258 A1 | 03 November 2022 |
| | | EP 4330249 A1 | 06 March 2024 |
| CN 113260310 A | 13 August 2021 | AU 2019362770 A1 | 03 June 2021 |
| | | SG 11202103731 QA | 28 May 2021 |
| | | EP 3866692 A2 | 25 August 2021 |
| | | EP 3866692 A4 | 22 December 2021 |
| | | WO 2020081410 A2 | 23 April 2020 |
| | | WO 2020081410 A3 | 23 July 2020 |
| | | US 2021379210 A1 | 09 December 2021 |
| | | CA 3116129 A1 | 23 April 2020 |
| | | KR 20210076077 A | 23 June 2021 |
| | | JP 2022508699 A | 19 January 2022 |
| | | JP 7367038 B2 | 23 October 2023 |
| US 2021171500 A1 | 10 June 2021 | US 11548871 B2 | 10 January 2023 |
| | | CA 3158743 A1 | 20 May 2021 |
| | | JP 2023501599 A | 18 January 2023 |
| | | JP 7431961 B2 | 15 February 2024 |
| | | KR 20220101137 A | 19 July 2022 |

Form PCT/ISA/210 (patent family annex) (July 2022)

**INTERNATIONAL SEARCH REPORT**
**Information on patent family members**

International application No.
**PCT/CN2024/117612**

| Patent document cited in search report | Publication date (day/month/year) | Patent family member(s) | | | Publication date (day/month/year) |
|---|---|---|---|---|---|
| | | WO | 2021097039 | A1 | 20 May 2021 |
| | | TW | 202128644 | A | 01 August 2021 |
| | | TWI | 767410 | B | 11 June 2022 |
| | | US | 2023212149 | A1 | 06 July 2023 |
| | | US | 11999717 | B2 | 04 June 2024 |
| | | EP | 4058144 | A1 | 21 September 2022 |
| | | AU | 2020384883 | A1 | 12 May 2022 |
| | | AU | 2020384883 | B2 | 16 November 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017223016 A1
- WO 2019126093 A
- EP 585130 A2
- WO 2013090929 A1
- CN 116789556 A
- WO 2008141462 A
- WO 2011046771 A
- WO 2020060916 A


### Non-patent literature cited in the description


- *Journal of Medicinal Chemistry*, 2021, vol. 64 (21), 15549-15581
- *ChemMedChem.*, July 2012, vol. 7 (7), 1230-6
- *Helvetica Chimica Acta*, 1992, vol. 75 (3), 766-772